# EUROPEAN PATENT SPECIFICATION

(11) **EP 0 832 222 B1**
(45) Date of publication and mention of the grant of the patent: **26.07.2006**
(21) Application number: 96918643.6
(22) Date of filing: 31.05.1996
(51) Int. Cl.: C12N 15/12, C07K 14/435, C12N 5/10, A01K 67/027, A01K 67/033, A61K 38/17, A01H 5/00, C07K 16/18, C12N 5/26

(54) **LINC-53 FROM C. ELEGANS AND ITS USES IN LISTING COMPOUNDS INVOLVED IN THE CONTROL OF CELL BEHAVIOUR AND PHARMACEUTICAL COMPOSITIONS**
LINC-53 AUS C. ELEGANS UND SEINE VERWENDUNGEN
PROCEDES POUR IDENTIFIER DES COMPOSES QUI REGULENT LE COMPORTEMENT DES CELLULES, COMPOSES IDENTIFIES, COMPOSITIONS PHARMACEUTIQUES CONTENANT CES COMPOSES ET LEUR UTILISATION POUR REGULER LE COMPORTEMENT DE CELLULES

(30) Priority: 31.05.1995 GB 9510944
(43) Date of publication of application: 01.04.1998
(73) Proprietor: Bogaert, Thierry, 8500 Kortrijk (BE); Stringham, Eve, Surrey, British Columbia V3V 5R5 (CA); Vandekerckhove, Joel, B-Loppem (BE)
(72) Inventor: Bogaert, Thierry, 8500 Kortrijk (BE); Stringham, Eve, Surrey, British Columbia V3V 5R5 (CA); Vandekerckhove, Joel, B-Loppem (BE)
(74) Representative: Baldock, Sharon Claire
(86) International application number: PCT/EP1996/002311
(87) International publication number: WO 1996/038555

(56) References cited:
- EMBL Database Entry CEF45E10 Accession number Z47810; 26 January 1995 XP002019188 & NATURE, vol. 368, no. 6466, 3 April 1994, LONDON GB, pages 32-38, R. WILSON ET AL.: "2.2 Mb of contiguous nucleotide sequence from chromosome III of C. elegans"
- JOURNAL OF NEUROSCIENCE 13 (10). 1993. 4254-4271. ISSN: 0270-6474, XP000612286 HEKIMI S ET AL: "Axonal guidance defects in a Caenorhabditis elegans mutant reveal cell-extrinsic determinants of neuronal morphology."

## Description

The present invention relates to processes for the identification of compounds which inhibit or enhance the rate and direction of cell migration or the control of cell shape, the compounds identified and pharmaceutical formulations containing such compounds together with their use in the regulation of cell behaviour. The invention also relates to an UNC-53 protein encoded by nucleic acid in the cells of the nematode worm C. elegans and cDNA sequences encoding an UNC-53 protein or functional equivalents thereof.

The control of cell motility, cell shape and the outgrowth of axones or other cell outgrowths is an essential feature in the morphogenesis and function of both unicellular and multicellular organisms. The control of this process is disturbed in a variety of disease states in which for example the Receptor Tyrosine Kinase (RTK) signal transduction pathways or the like or their downstream intra-cellular pathways (which are shared with other extra-cellular receptors, including cell adhesion molecules like N-CAMS and integrins) are overstimulated.

Some cell surface proteins and extracellular molecules controlling the directionality and potential of cell migration have been identified. However the processes in which these proteins or molecules are involved to effect cell migration, shape or rate of cell differentiation are not understood.

It is generally considered that a long-range migration of a cell process (which may also be known as a growth cone spike) is a stepwise event, whereby prior to and after each extension there is the formation of a structure at the leading edge of the cell which senses signals in the environment instructing the cell to either stabilize a cell process extending in a preferred direction, or to cause a cell process lamellipodium to extend a process in a given direction. Localized stabilization of the actin cytoskeleton, is a general cell biological process underlying this choice of directional extension.

A gene from the free-living nematode Caenorhabditis elegans, designated"unc-53" has been previously identified and cloned (Abstract, International C. elegans meeting; June 1-5 1991, Madison, Wisconsin, 58, Bogaert and Goh). However, to date no known biological function has been attributed to the UNC-53 gene or its corresponding UNC-53 protein.

Furthermore, EMBL Database Entry CEF45E10 Accession number 247810, dated 26 January 1995 a C. elegans cosmid designated F45E10 containing 17945bp of DNA including 1460bp which shares 100% homology with the UNC-53 gene at C.elegans.

The present inventors have surprisingly identified, through biochemical, genetic, phenotypic and transgenic evidence which is presented herewith, UNC-53 as a signal transducer or signal integrator controlling the rate and directionality of cell migration, and/or cell shape. Key experiments leading to this conclusion were the molecular identification of its domain structure, its biochemical interaction with GRB-2, actin cytoskeleton sequence information and the presence of a potential signal integrating domain in the UNC-53 protein.

An additional key observation is that increased UNC-53 protein activity is proportional to increased cell process extension in the correct direction of cell migration. Reduction of UNC-53 function has previously been shown to lead to a reduction of cell process extension, identifying it as a general component required for cell migration. However, it had not been identified as a component whose level of activity has a determining role in the specification of the quantum and directionality of migration.

The work of the present inventors suggests that UNC-53 plays a central role in quantitatively transducing extracellular signals to the machinery controlling directional cell migration.

The importance of UNC-53 in a variety of cell types in C. elegans has been demonstrated. The gene encodes a signal transduction molecule that transduces a signal from a Receptor Tyrosine Kinase such as for example via the adaptor protein SEM-5/GRB-2, to the machinery controlling directional growth cone extension or stabilization. The UNC-53 protein does this in a highly dosage-dependent fashion whereby reduction of protein activity such as reduction in expression of protein or in the reduction in its activity leads to proportional reduction of cell process extension (cell migration). This is believed to be either by regulated cross-linking of the actin cytoskeleton or by transferring the received signal downstream within the transduction pathway. Higher than wild type UNC-53 expression leads to higher than wild type growth cone extension in the anterior-posterior axis. Both the observed SEM-5/GRB-2 binding to UNC-53 and the predicted ATP/GTP-ase activity of UNC-53 demonstrate a signal transduction role for UNC-53 involved in cell process or growth cone guidance.

UNC-53 is a protein working at the intracellular level. It is so far believed to be the only intracellular protein identified which is involved in the control of directionality and rate of cell migration in response to a specific signal and which integrates different directional signals in defining direction of migration.

Based on the present inventors accumulated knowledge of the unc-53 gene function in C. elegans it is understood that inhibitors or enhancers of the unc-53 gene or the UNC-53 protein will affect the cell motility including (metastasis) via an RTK pathway or the like, or may lead to changes in the shape of the cells (which has been demonstrated in C. elegans body muscle). Applications for such inhibitors and/or enhancers are envisaged in a wide variety of pathologies in which the RTK pathways play a central role, including oncogenesis, psoriasis, cell migration (metastasis), neuronal regeneration/degeneration and immunological disorders among others.

The identification of the biochemical function of the unc-53 gene (and UNC-53 pathway) in the RTK signal transduction pathway is novel and unexpected. No biological function has previously been linked to the unc-53 gene or UNC-53 protein, nor has any homology with any other nucleic acid sequence or gene been recognised.

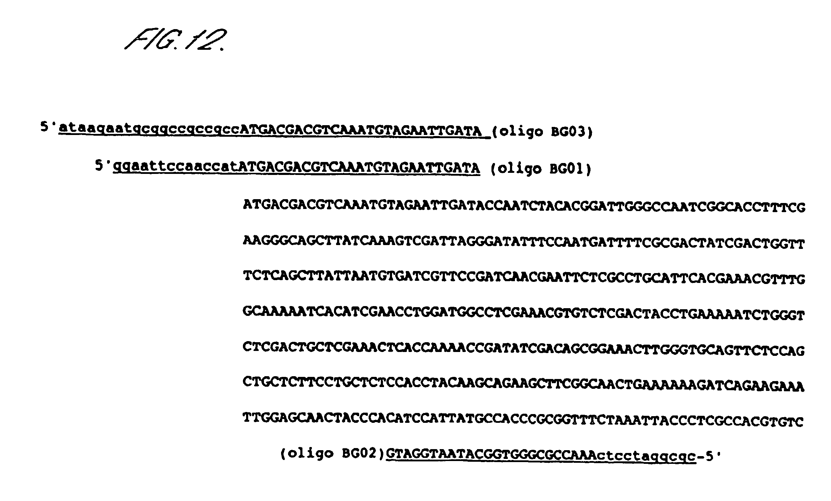

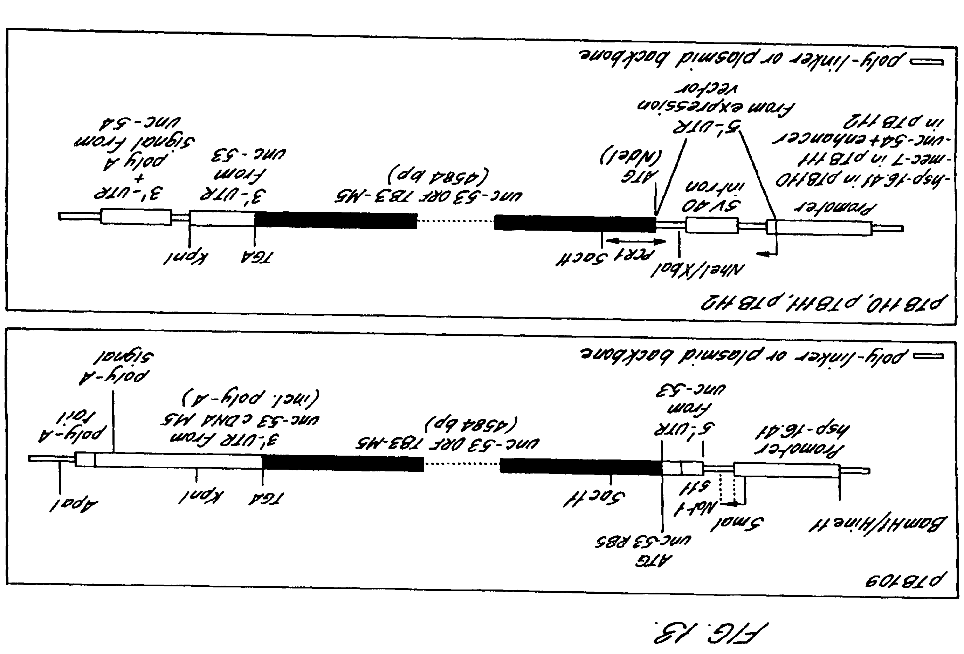

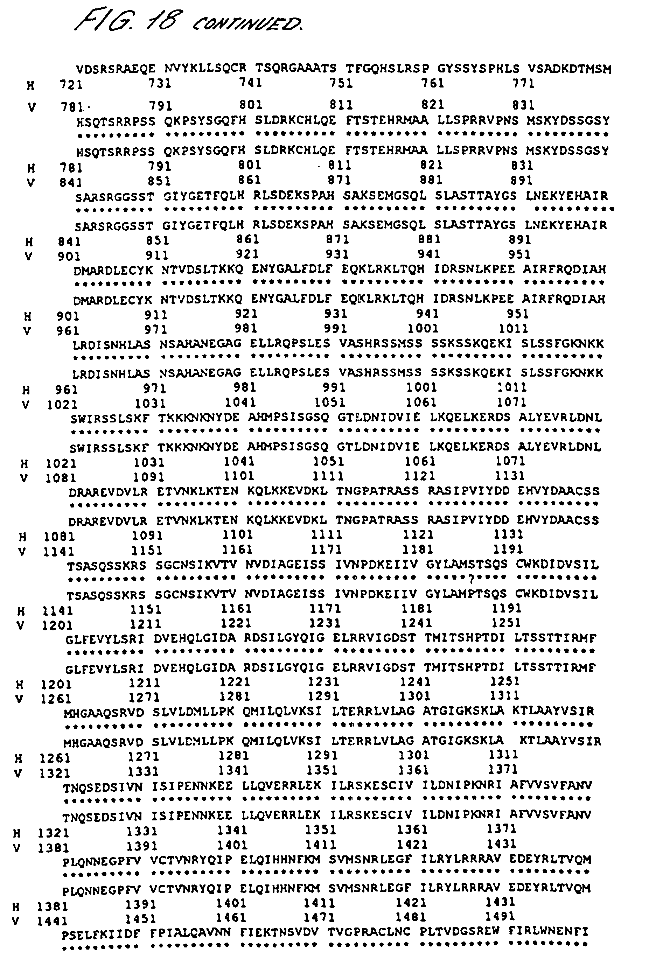

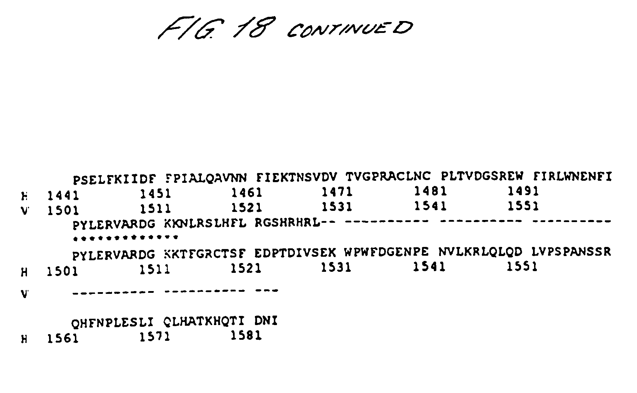

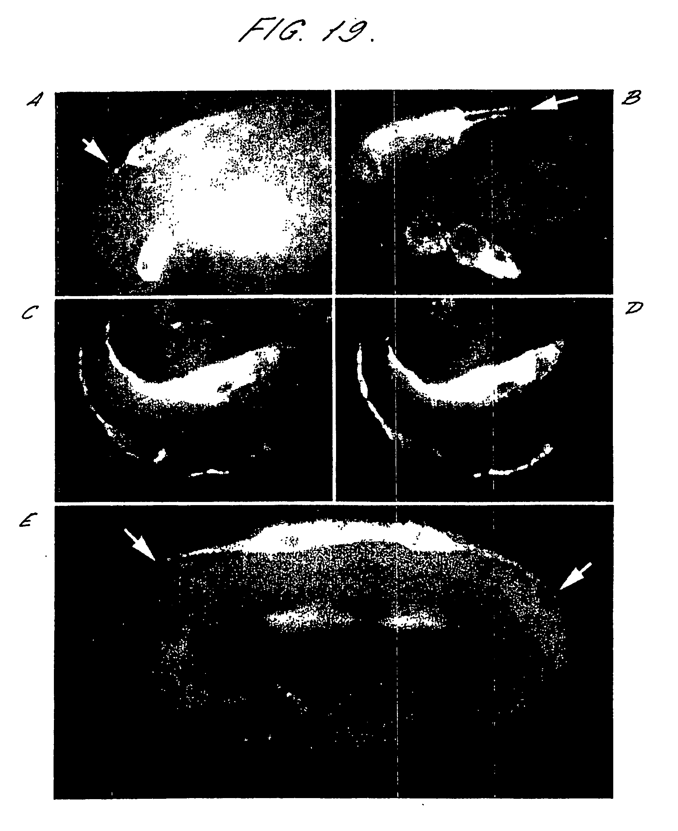

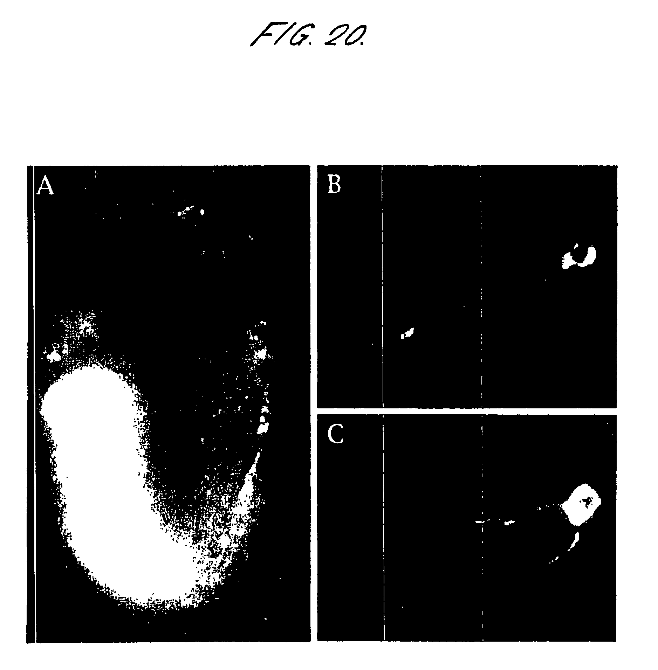

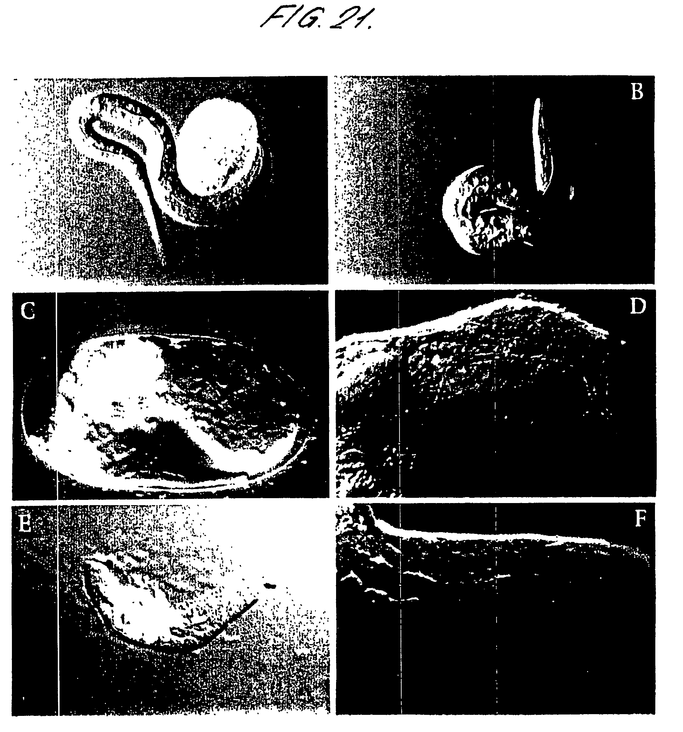

An analysis of the predicted protein sequence of UNC-53 from the gene sequence thereof has revealed the following:
(a) an N-terminal domain with homology to cortical actin binding proteins of the α-actinin and β-spectrin families (designated ABPII in Figure 11). Alignment of UNC-53 with the α-actinin and β-spectrin family of proteins is shown in Fig. 15.).
(b) two putative actin binding sites of the LKK class (ABS1 and ABS2).
(c) two polyproline rich sequences similar to the SH3 binding domains of the SOS family of signal transduction molecules (SH3 binding site) (Fig. 16).
(d) a putative ATP/GTP nucleotide binding site having some of the additional features of the GTP binding domain of RAS-like proteins (Dynamin, NBD).
(e) besides the N-terminal region of the protein, which is similar to actin binding proteins, the predicted protein sequence of UNC-53 identified two putative actin binding sites. The first borders on the 3' end of the region of α-actinin/β-spectrin homology and the second lies in the 3' end of the cDNA sequence.

This suggests that UNC-53 could potentially bind two actin molecules and via actin cross linking, could stabilize a particular cell process to promote directional extension.

In addition, genetic evidence shows that alleles of unc-53 enhance the sex myoblast migration defect of sem-5 mutants. Sem-5 represents the C. elegans homologue of GRB2, the function of these proteins being assigned/attributed to their SH2 and SH3 domains (Clark et al., (1992) Nature 356, 340-344; Stern et al., (1993), Molec. Biol. Cell, 4, 1175-1188). The current model regarding sem-5 function in the migration of sex myoblasts is that sem-5 transduces a signal received at the cell surface by egl-15, a receptor kinase of the fibroblast growth factor family. Together, the genetic and molecular data suggest a role for UNC-53 in both signal transduction and actin binding. We have been able to demonstrate how UNC-53 might act to direct both growth cone rate and directionality. By binding directly to the actin cytoskeleton, UNC-53 may stabilize and cross-link actin molecules (assuming a two actin binding site model) to promote directional growth cone extension. Alternatively, by binding actin, UNC-53 may convey a signal to the cytoskeleton and then via an ATP/GTPase activity transduce the signal to downstream targets. To test these models, biochemical experiments were conducted to determine if any of the sequence similarities observed represented functional domains (see examples 2 to 5). Transgenic analysis as described in examples 6 to 8 support this proposed model.

As described above, the unc-53 gene from C. elegans has been previously identified. However, cDNA sequences substantially corresponding to unc-53 genomic exon sequences of C. elegans or fragments or derivatives thereof have never been previously disclosed. The present inventors have advantageously identified two unc-53 cDNA clones which have been designated as the 7A and 8A clones. The two clones differ in the number of Adenosine(A) residues (7 or 8) in a poly A stretch of the 3' coding region. Therefore, the two clones have different reading frames in the carboxyterminal coding region.

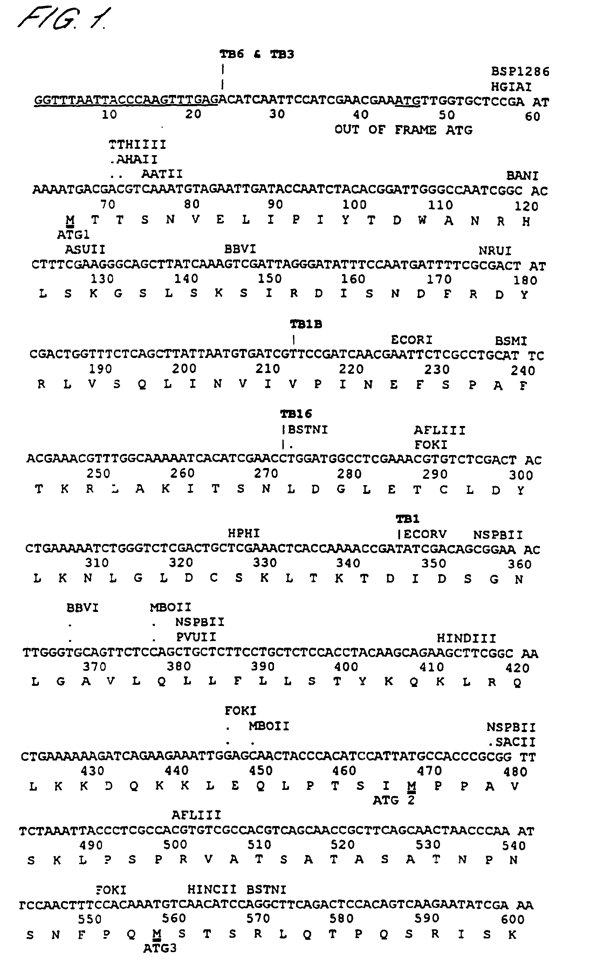

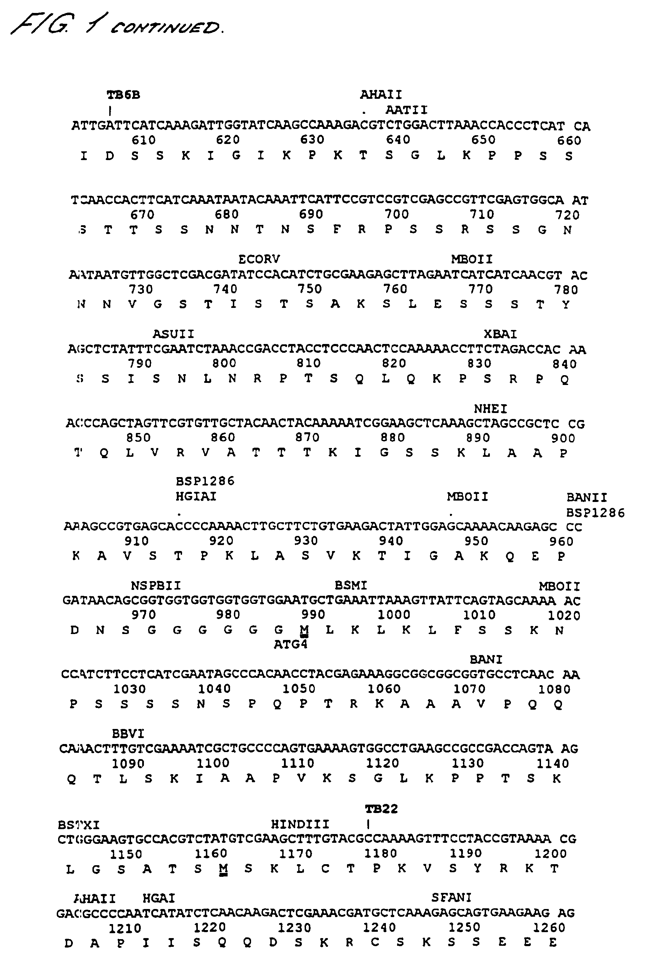

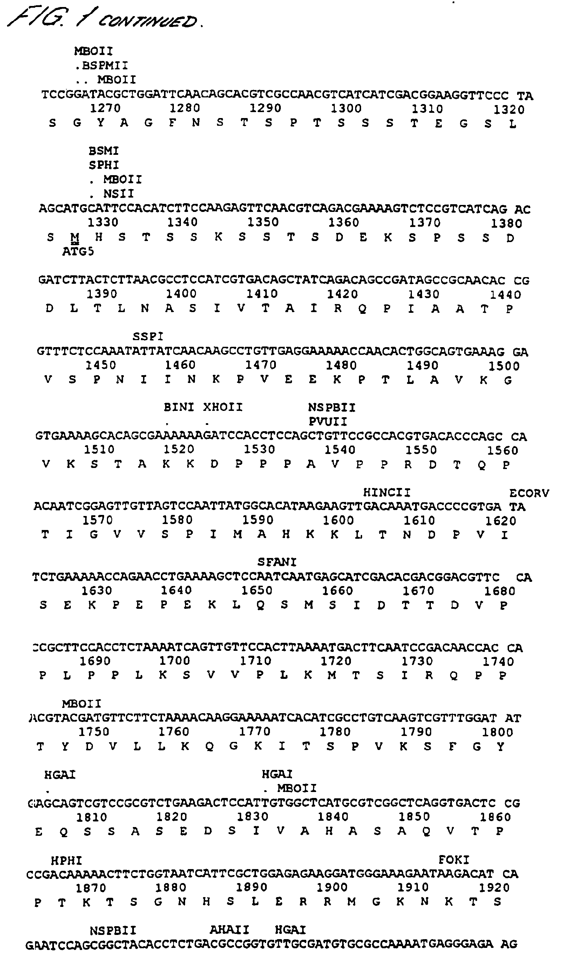

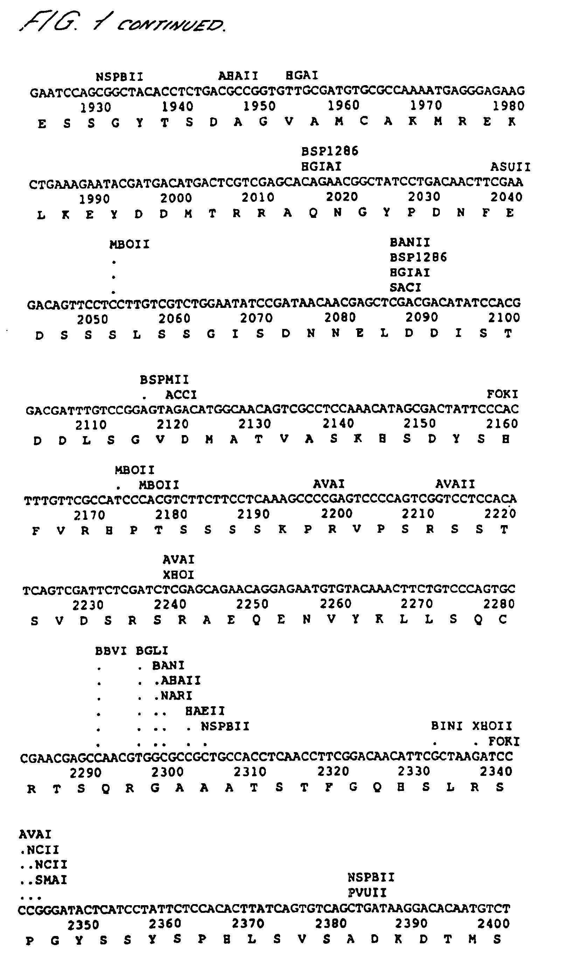

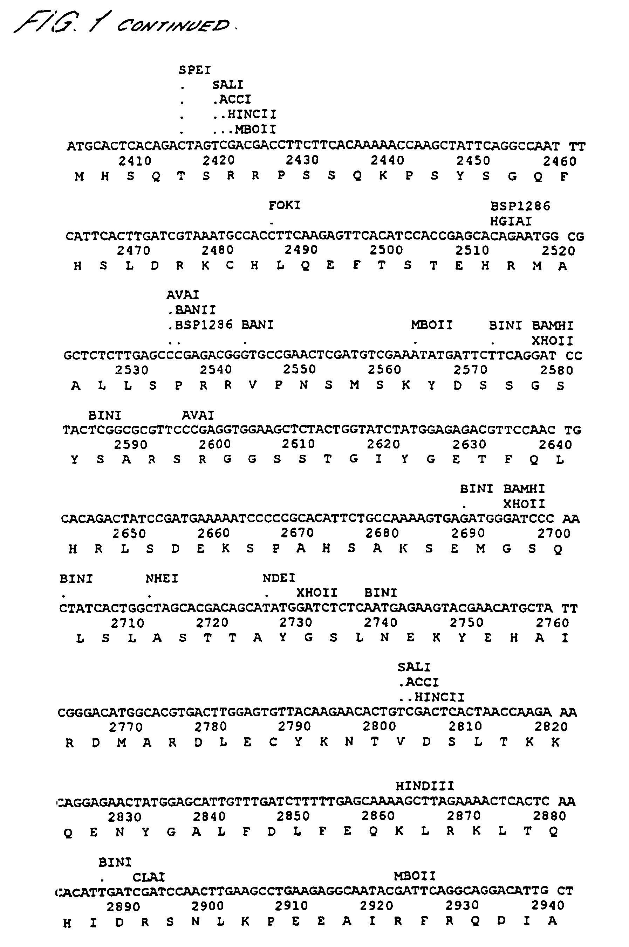

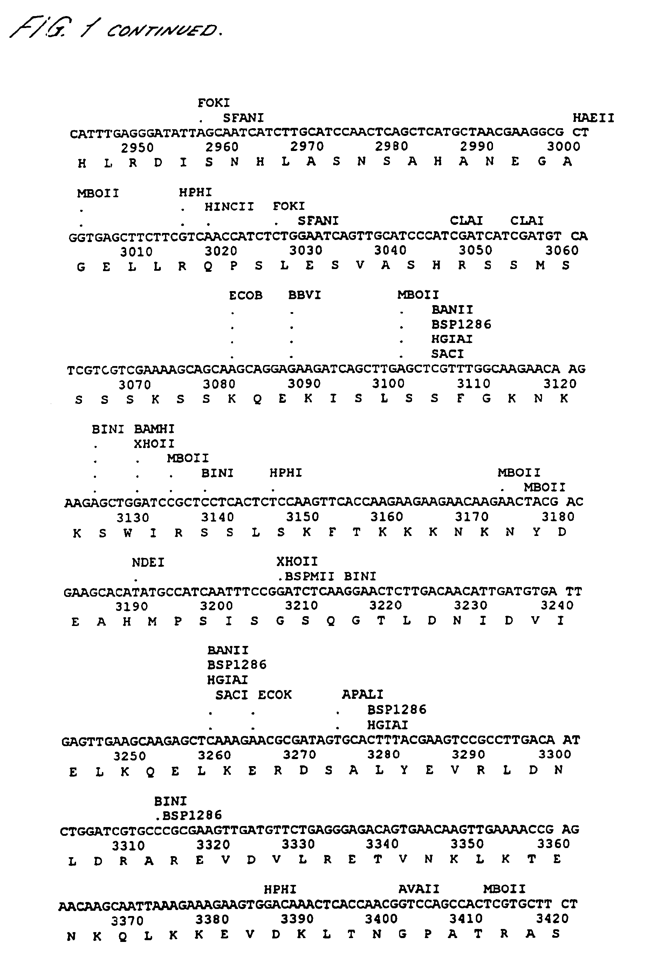

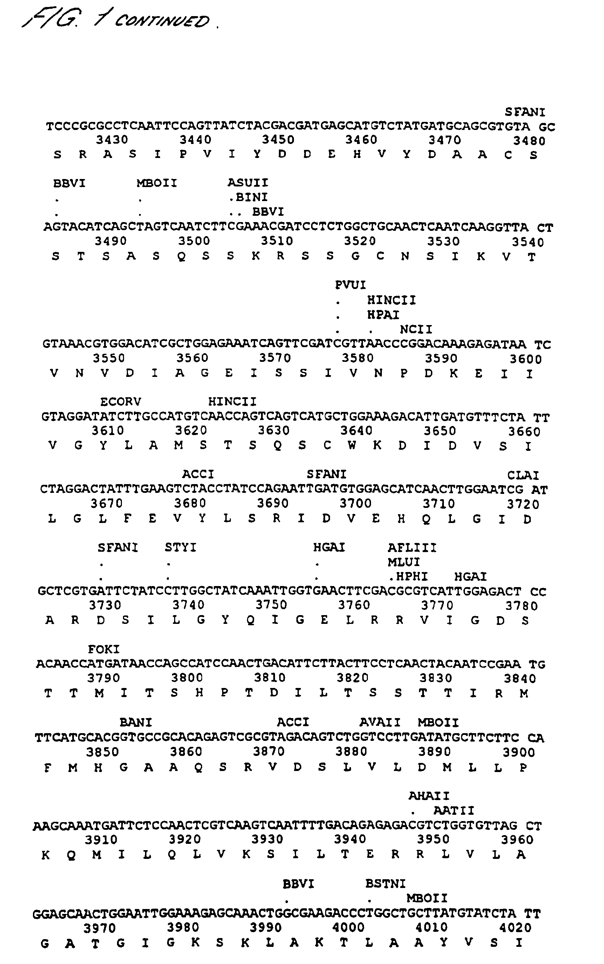

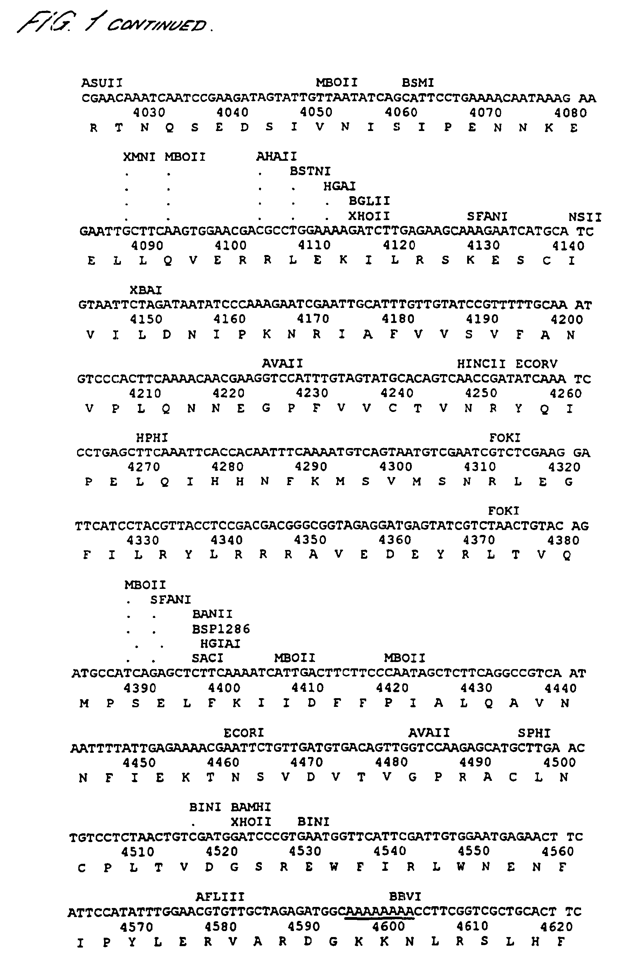

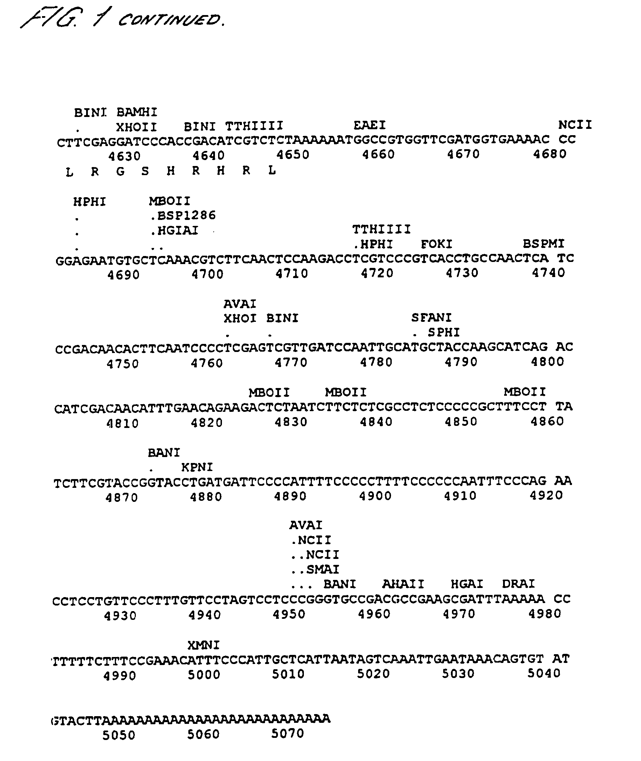

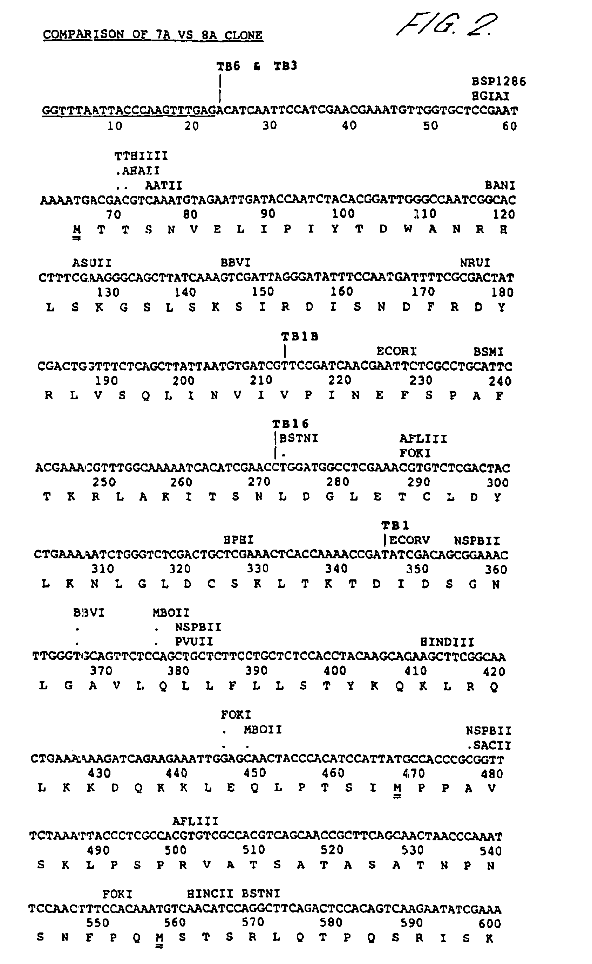

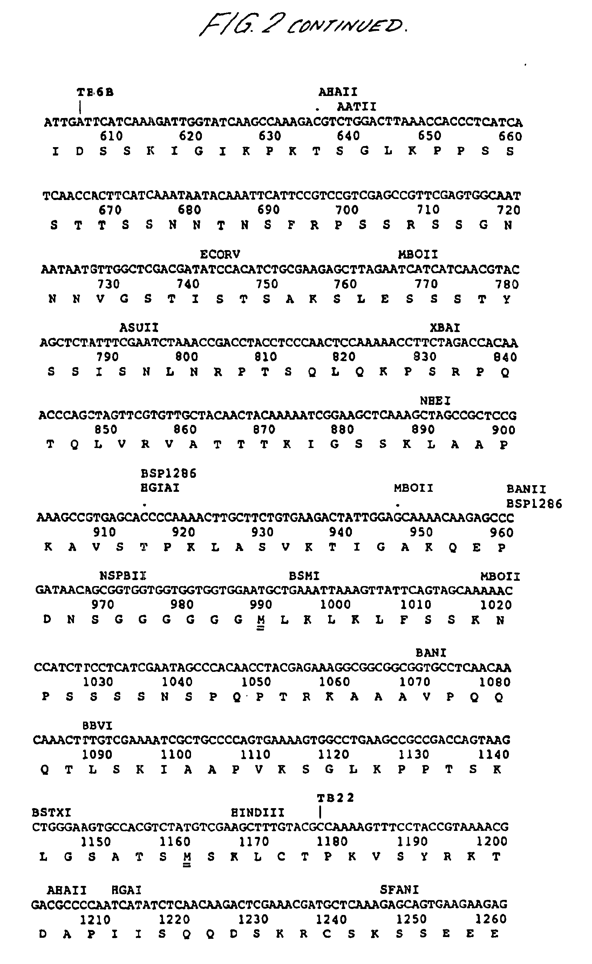

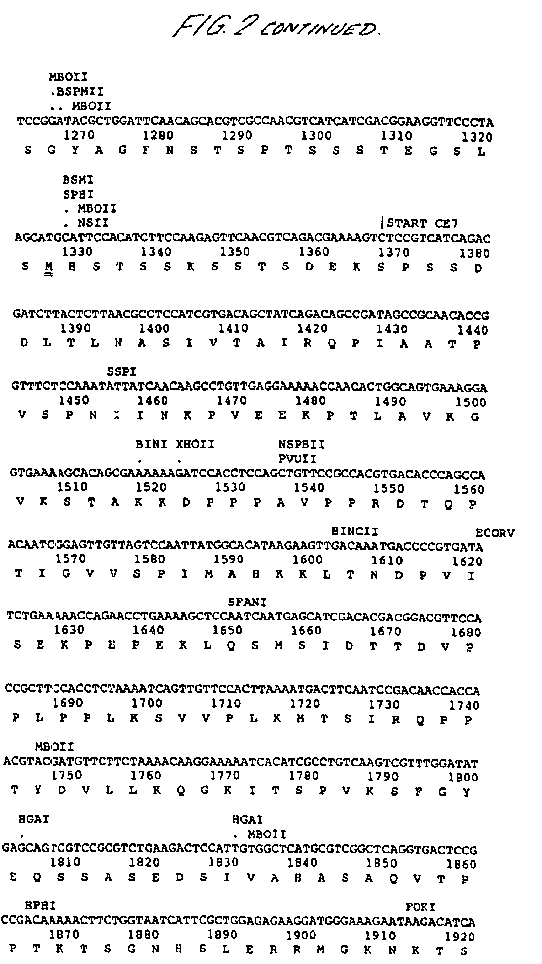

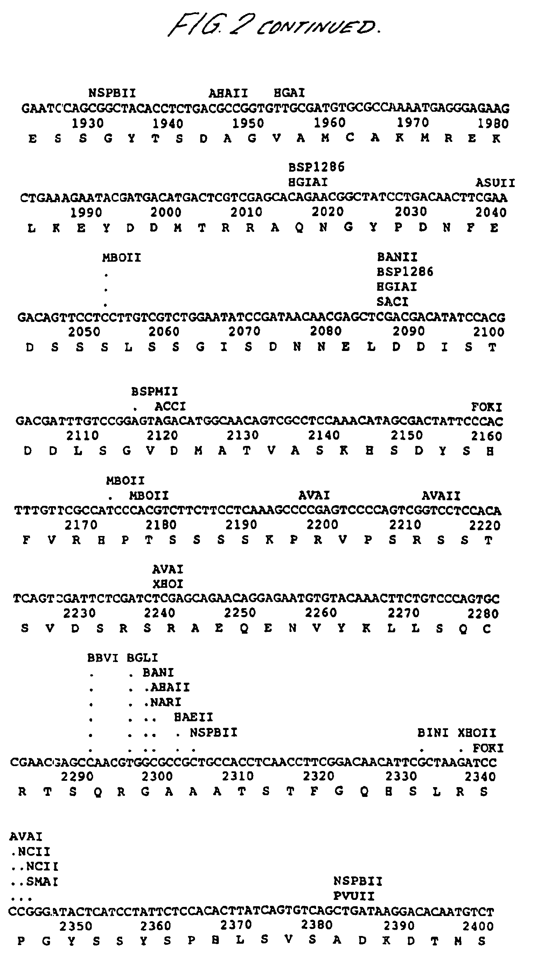

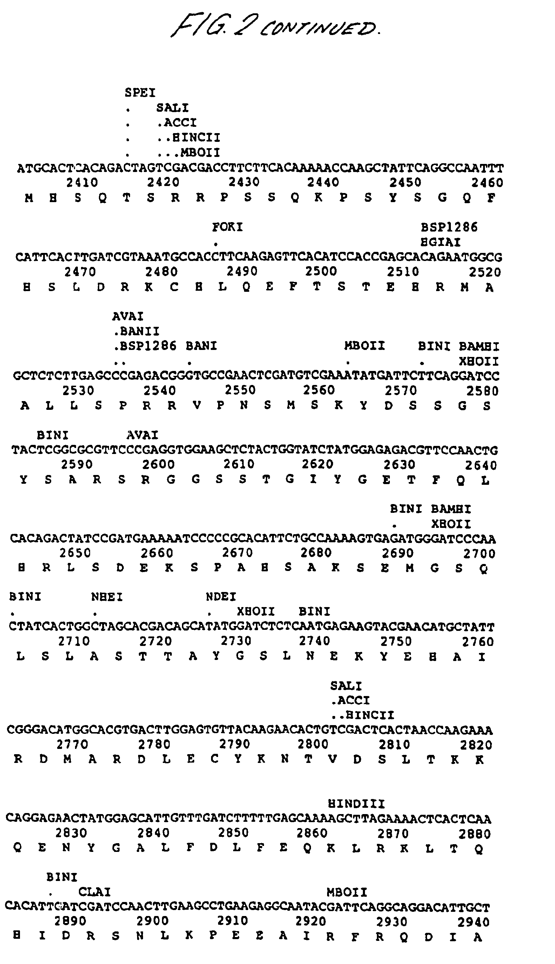

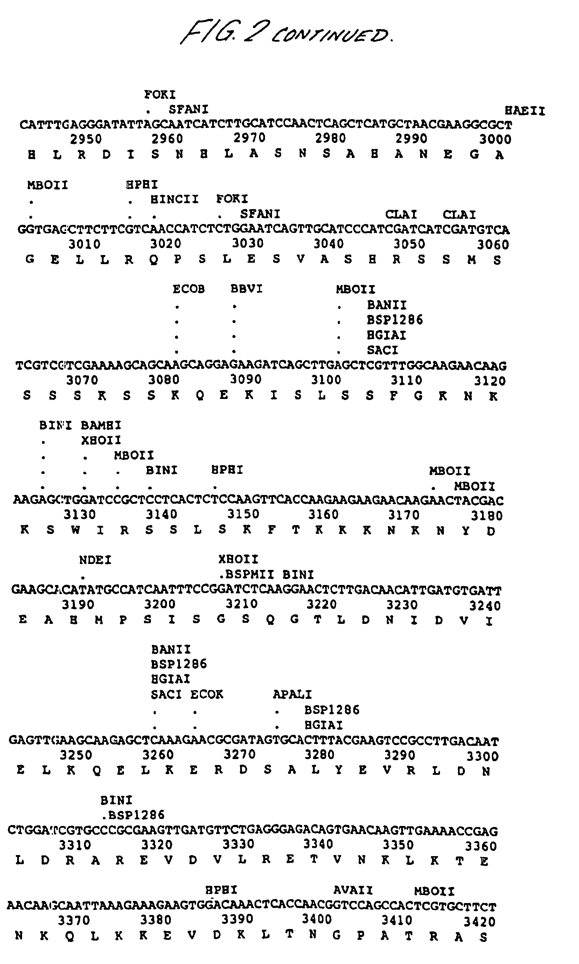

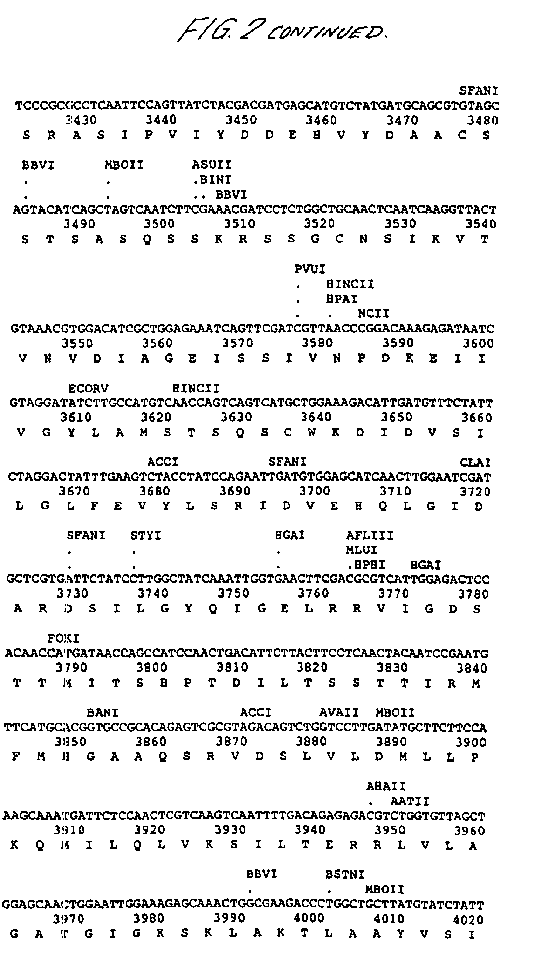

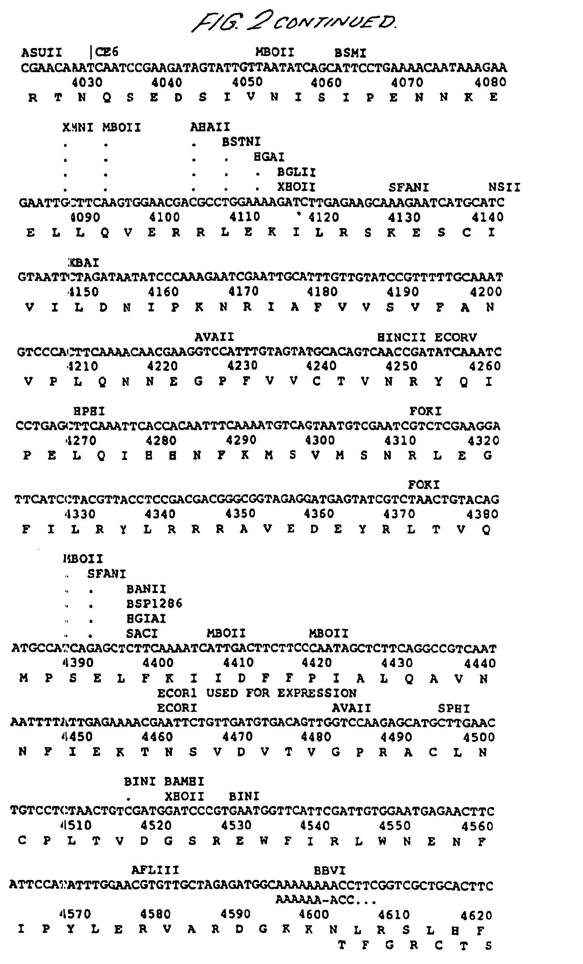

Therefore according to one aspect of the present invention there is provided a cDNA encoding an UNC-53 protein of C. elegans which cDNA comprises at least from nucleotide position 431 to nucleotide position 4647 or alternatively to the 3' poly-A region of the sequence shown in Figure 1. More preferably the cDNA comprises at least from nucleotide position 64 to nucleotide position 4647 or to the 3' poly-A region of the sequence as shown in Figure 1. This cDNA is comprised in the 8A clone having 8A residues in a poly A stretch of the 3' coding region as shown in Figure 1.

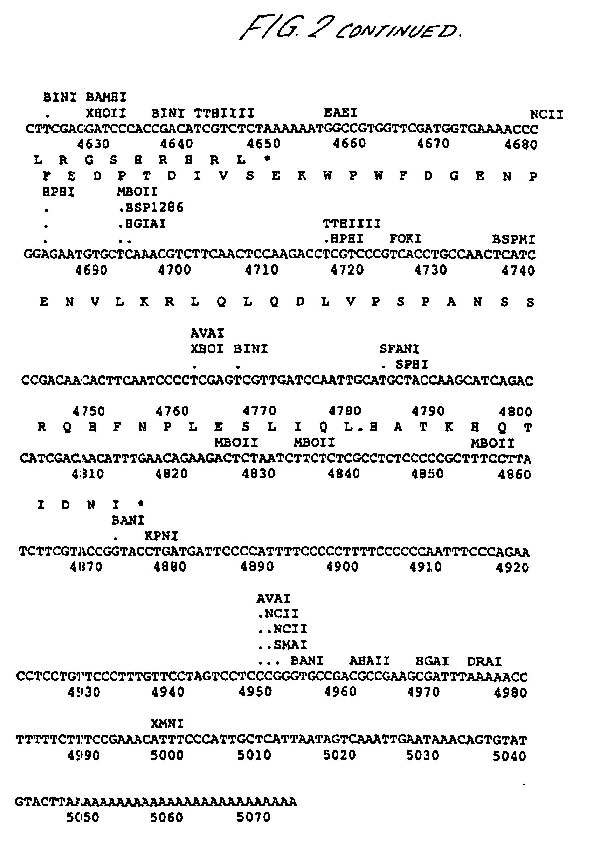

In an alternative embodiment of this aspect of the invention the cDNA comprises at least from nucleotide position 431 to nucleotide position 4812 or alternatively to the 3' poly-A region of the sequence shown in Figure 2 and more preferably at least from position 64 to nucleotide position 4812 or the 3' poly-A region of the sequence shown in Figure 2. This cDNA according to the invention comprises the 7A clone, having only 7 Adenine residues in the poly A stretch of the 3' coding region as shown in the nucleotide sequence of Figure 2 page 8. Each of the cDNA clones according to the invention, may be included in an expression vector which vector may itself be used to transform or transfect a host cell which may be bacterial, animal or plant in origin. Thus, advantageously, once the cDNA corresponding to the unc-53 genome is synthesised using for example reverse transcriptase or the like, a range of cells, tissues or organisms may be transfected following incorporation of the selected cDNA clone into an appropriate expression vector.

The present invention therefore, also further comprises a transgenic cell, tissue or non human organism comprising a transgene capable of expressing UNC-53 protein of C. elegans or a functional equivalent, fragment, derivative or bioprecursor thereof. The term "transgene capable of expressing UNC-53 protein" as used herein means a suitable nucleic acid sequence which leads to the expression of an UNC-53 protein having the same function and/or activity. The transgene may include for example genomic nucleic acid isolated from C. elegans or synthetic nucleic acid or alternatively any of the cDNA clones as described above.

The term "transgenic organism, tissue or cell" as used herein means any suitable organism and/or part of an organism, tissue or cell that contains exogenous nucleic acid either stably integrated in the genome or in an extra chromosomal state wherein said organism is non human.

Preferably, the transgenic cell comprises either a C. elegans cell, an N4 neuroblastoma cell or an MCF-7 breast carcinoma cell. The transgenic organism may be C. elegans itself, or alternatively may be an insect, a non-human animal or a plant. Preferably the unc-53 transgene comprises the unc-53 gene or a functional fragment thereof as disclosed in the claims.

Reference to "tissue or tissue culture" for the purpose of the present invention should be taken to mean such a mutant cell which has been grown in such a culture. Further provided by the present invention is a mutant C. elegans organism which comprises an induced mutation, such as a point mutation in the wild-type unc-53 gene and which mutation affects the regulation of cell motility or shape or the direction of cell migration. Such mutations may be introduced using changes in the cDNA corresponding to qualitative, quantitative direct and indirect changes in the genomic make up.

The term "mutant organism" used herein means any suitable organism that contains genetic information which has been induced to mutate and is thus altered from the wild-type. Therefore naturally occurring mutations in the wild-type organism are not within the scope of this term.

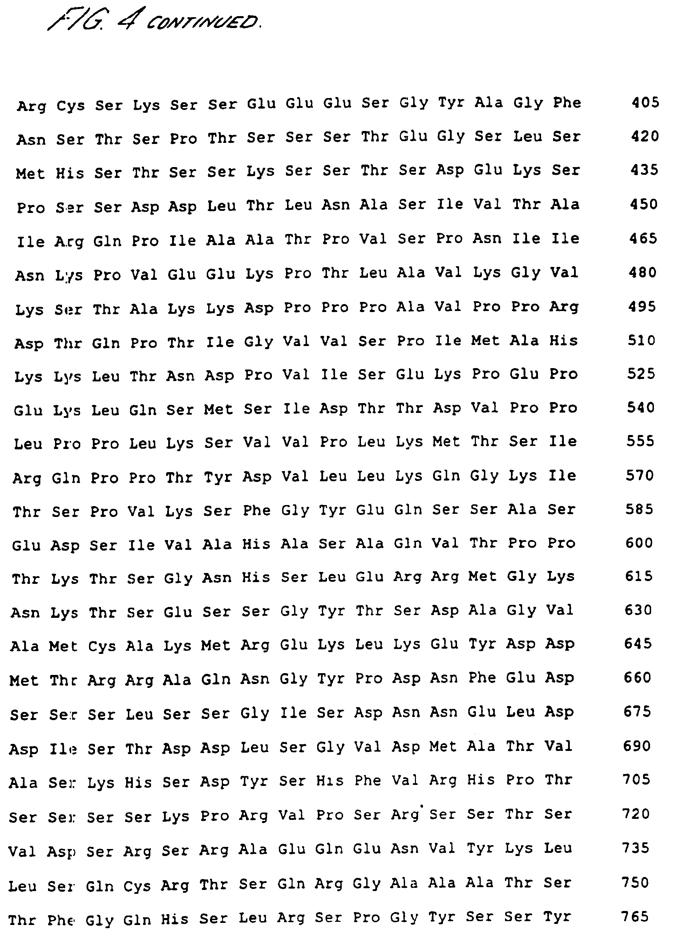

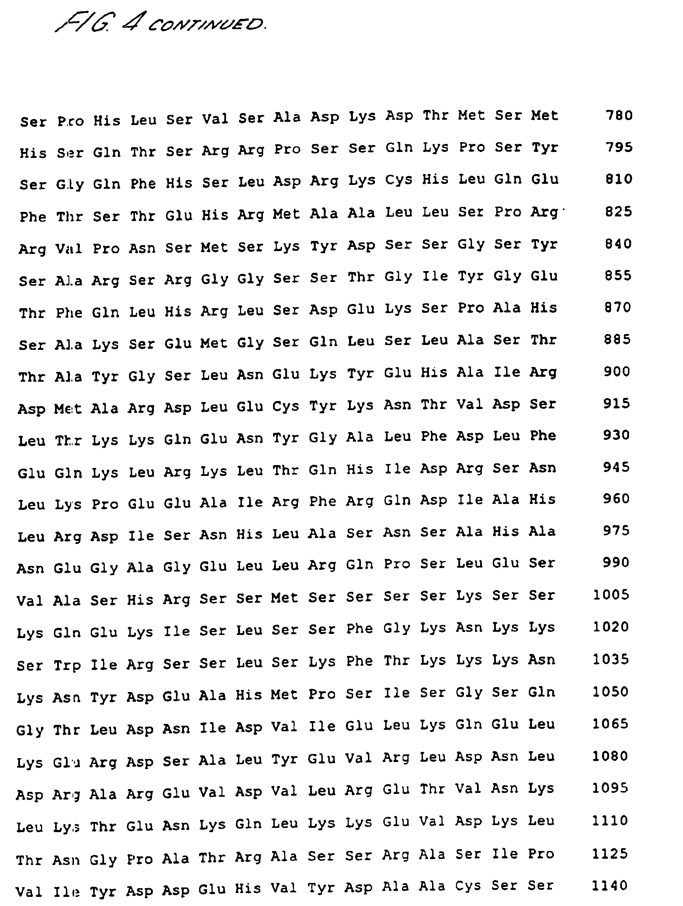

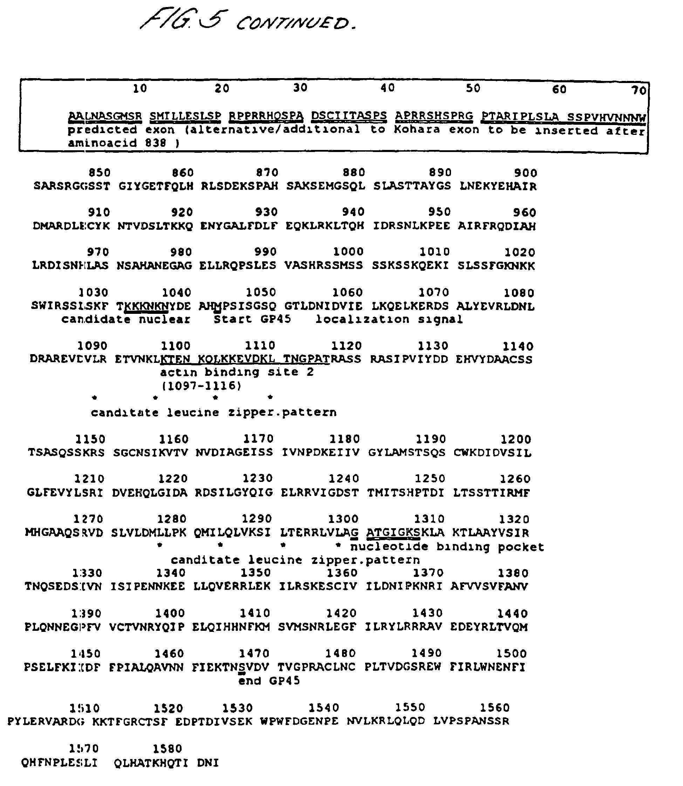

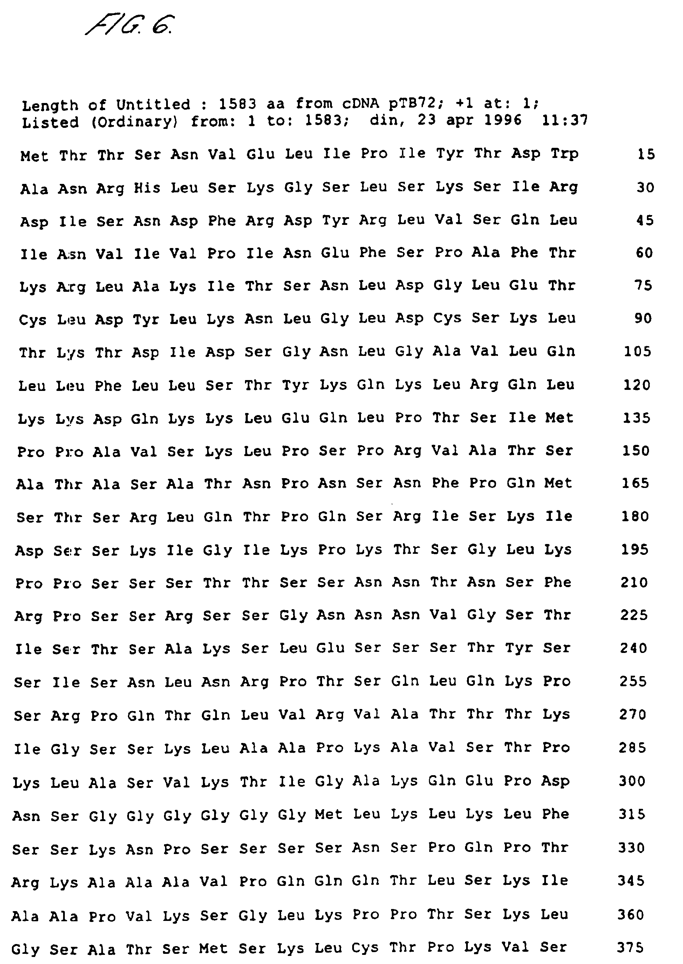

The present invention further comprises an UNC-53 protein which protein may be encoded by a cDNA according to the invention, and which protein has the amino acid sequence shown in Figure 4 from amino acid position 135 to amino acid position 1528; this corresponds to the 8A clone. More preferably the UNC-53 protein, when encoded by a cDNA according to the invention, comprises the amino acid sequence shown in Figure 4. In another aspect of the invention the protein comprises an UNC-53 protein which comprises the sequence of from amino acid position 135 to amino acid position 1583 of the amino acid sequence shown in Figure 6. Preferably, the UNC-53 protein when encoded by a cDNA in accordance with the invention has the amino acid sequence shown in Figure 6.

The UNC-53 protein of C. elegans, may advantageously be used as a medicament to promote neuronal regeneration, revascularisation or wound healing or the treatment of chronic neuro-degenerative disorders or acute traumatic injuries. Similarly, the UNC-53 protein produced by the transgenic cells, tissue or organisms according to the invention may also be used in the preparation of a medicament for treatment of the conditions as described above.

Furthermore, in an alternative embodiment of the invention the nucleic acid sequence itself encoding an UNC-53 protein of C. elegans may also be used as a medicament or, alternatively in the preparation of a medicament, to promote neuronal regeneration, vascularisation or wound healing or for treatment of chronic neuro-degenerative diseases or acute traumatic injuries. Typically neurological ; conditions which may be treated by either an UNC-53 protein, or a nucleic acid according to the invention, comprise peripheral nerve regeneration after trauma; recovery of function of the spinal cord after spinal cord trauma or peripheral neuropathies. Similarly neuro-degeneration diseases which may be treated include Alzheimers disease or Huntingdons disease. Acute traumatic injuries such as stroke, head trauma or haemorrhages may also advantageously be treated.

The nucleic acid sequence according to the invention may comprise a cDNA sequence according to the invention as described above or alternatively may be genomic DNA derived from C. elegans.

The UNC-53 protein of C. elegans, may be incorporated into a pharmaceutically acceptable composition together with a suitable carrier, diluent or an excipient therefor. The pharmaceutical composition may advantageously comprise, additionally or alternatively to the UNC-53 protein according to the invention, the nucleic acid sequence according to the invention as defined above.

The present invention also provides for a method of determining whether a compound is an inhibitor or an enhancer of the regulation of cell shape or motility or the direction of cell migration in a transgenic cell, tissue or organism according to the invention as described herein. The method preferably comprises contacting the compound with a transgenic cell, tissue or organism according to the invention as described above, and screening for a phenotypic change in the cell, tissue or organism. Preferably the compound comprises an inhibitor or enhancer of a protein of the signal transduction pathway of the cell, tissue or organism of which UNC-53 is a component or is an inhibitor or enhancer of a parallel or redundant signal transduction pathway. Such enhancers or inhibitors are defined by particular phenotypic changes in the transgenic cell, tissue or organism, for example changes in cell shape or mobility or the direction of cell migration. Preferably the compound is an inhibitor or an enhancer of the activity of UNC-53 protein of C. elegans or a functional equivalent, derivative or bioprecursor thereof, which protein is expressed in the transgenic cell, tissue or organism as defined herein.

Preferably the phenotypic change to be screened comprises a change in cell shape or a change in cell motility. Where a transgenic cell is used in accordance with one embodiment of the method of the invention, an N4 neuroblastoma cell may be used and in such an embodiment the phenotypic change to be screened may be the length of neurite growth or changes in filipodia outgrowth or alternatively changes in ruffling behaviour or cell adhesion. In an alternative embodiment of the method of the invention, the transgenic cell may comprise an MCF-7 breast carcinoma cell. Typically in such an embodiment the phenotypic change to be screened comprises the extent of phagokinesis. The method according to the invention, may also utilise a mutant cell or mutant organism according to the invention as described above, where the mutant cell is capable of growing in tissue culture and either of which cell or organism has a mutation in the wild-type unc-53 gene.

In accordance with the present invention, a "phenotypic change", may be any phenotype resulting from changes at any suitable point in the life cycle of the cell, tissue or organism defined above, which change can be attributed to the expression of the transgene such as for example, growth, viability, morphology, behaviour, movement, cell migration or cell process or growth cone extension of cells and includes changes in body shape, locomotion, chemotaxis, mating behaviour or the like. The phenotypic change may preferably be monitored directly by visual inspection or alternatively by for example measuring indicators of viability including endogenous or transgenically introduced histochemical markers or other reporter genes, such as for example β-galactosidase.

Preferably the compound is identified by the method according to the invention as an inhibitor or an enhancer, by utilising differences of phenotype of the cell, tissue or organism, which are visible to the eye. Alternatively indicators of viability including endogenous or transgenically introduced histochemical markers or a reporter gene may be used.

The present invention also provides a kit for determining whether a compound is an enhancer or an inhibitor of the regulation of cell motility or shape or the direction of cell migration, which kit comprises at least a plurality of transgenic or mutant cells according to the invention as described above and a plurality of wild-type cells of the same cell type or cell line or tissue culture.

For the purposes of the present invention, the term "unc-53 gene includes the nucleic acid sequence shown in Figure 1, including the differentially spliced isoforms and transcriptional start of the unc-53 gene sequence and which sequence encodes an UNC-53 protein.

The present invention also advantageously provides a process for the preparation of binding antibodies which recognise proteins or fragments thereof involved in the rate and direction of cell migration or the control of cell shape, for the above methods. Preferably the antibody is monoclonal antibody and more preferably monoclonal antibody 16-48-2.

The monoclonal antibody for binding to UNC-53 (or its functional equivalent) may be prepared by known techniques as described by Kohler R. and Milstein C., (1975) Nature 256, 495 to 497. reporter molecule in a GAL-4 transcription deficient yeast cell into which the vectors have been transformed. Preferably, a reporter molecule such as β-galactosidase is activated upon restoration of transcription of the yeast galactose metabolism genes. This method enables any interactions between proteins involved in the signal transduction pathway to be investigated.

Any proteins identified in the signal transduction pathway of the cell, which may be for example a mammalian cell, may also be included in a pharmaceutical composition together with a carrier, diluent or excipient therefor.

The present invention also provides a process for producing an UNC-53 protein of C. elegans which process comprises culturing the cells transformed or transfected with a cDNA expression vector having any of the cDNA sequences according to the invention as described above, and recovering the expressed UNC-53 protein. The cell may advantageously be a bacterial, animal, insect or plant cell.

In accordance with the present invention, a defined nucleic acid sequence includes not only the identical nucleic acid but also any minor base variations from the natural nucleic acid sequence including in particular, substitutions in bases which result in a synonymous codon (a different codon specifying the same amino acid), due to the degenerate code in conservative amino acid substitution. The term "nucleic acid sequence" also includes the complimentary sequence to any single stranded sequence given which includes the definition above regarding base variations.

Furthermore, a defined protein, polypeptide or amino acid sequence according to the invention, includes not only the identical amino acid sequence but also minor amino acid variations from the natural amino acid sequence including conservative amino acid replacements (a replacement by an amino acid that is related in its side chains). Also included are amino acid sequences which vary from the natural amino acid but result in a polypeptide which is immunologically identical or similar to the polypeptide encoded by the naturally occurring sequence. Such polypeptides may be encoded by a corresponding nucleic acid sequence.

The invention may be more clearly understood from the following description with reference to the accompanying drawings and photographs, in which

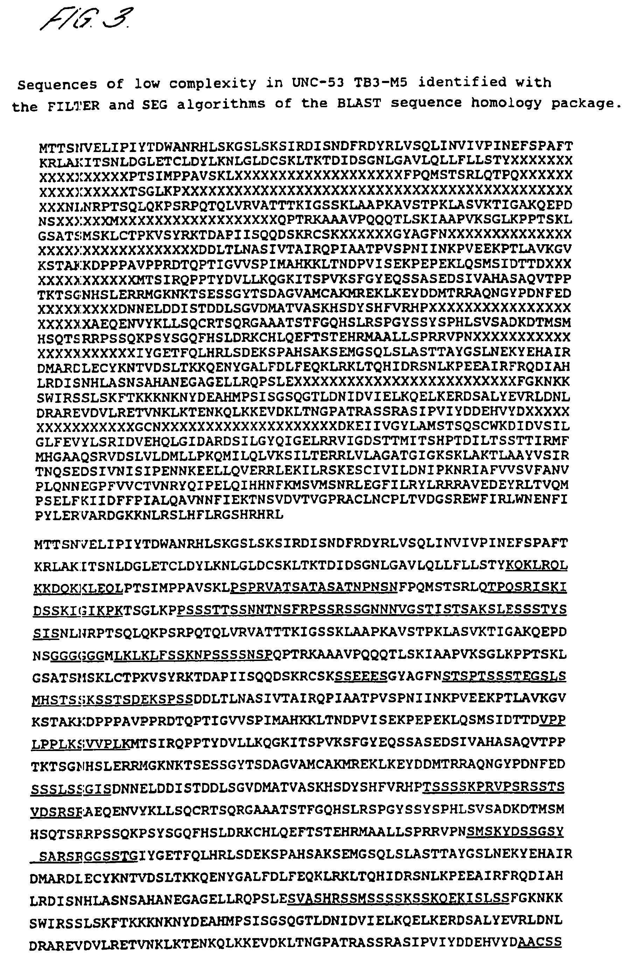

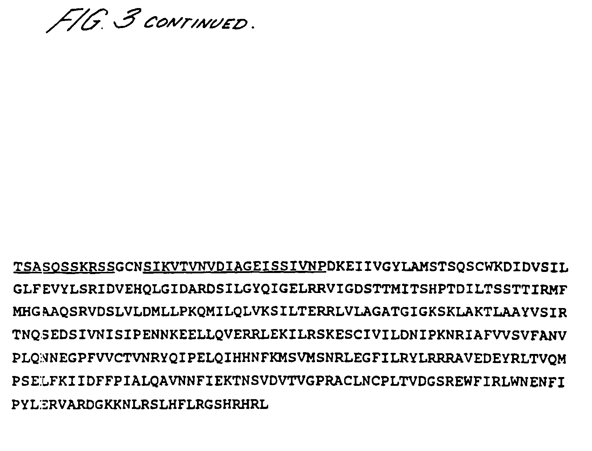

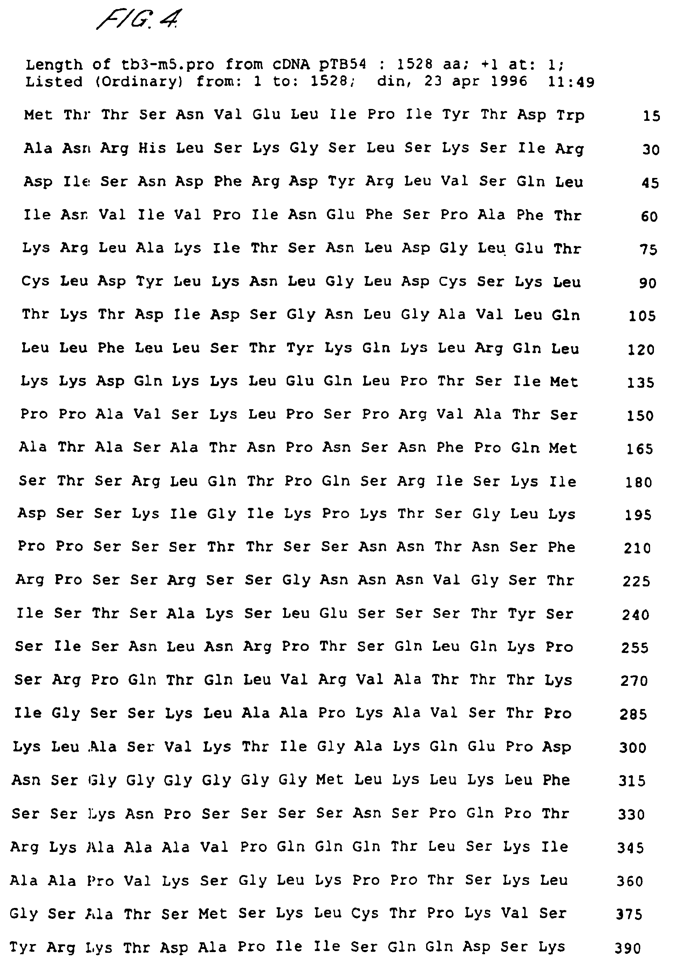

Fig. 1 shows one strand of the C. elegans unc-53 mRNA translated into DNA (U to T) (5073 bases) which corresponds to the 8A clone variant encoding the corresponding 8A protein shown in Figure 3. Designations "TB" are positions onto which SL1 transplices have been identified at the 5' end of the sequence. Different mRNAs which differ in their 5' end therefore exist. Potential start methionines are double underlined (M). Restriction endonuclease sites are indicated. A region of 8 sequential A bases at positions 4594 to 4601 is underlined. This region differs from the corresponding region of the known sequence in the database (F45E10.1) by having 8 rather than 7 A'denine (A) bases resulting in a frame shift (see Fig 15) and corresponds to the 7A form of the protein. The nucleic acid sequence from the database is also included in the nucleic acid sequences of the present application for reference only.

Fig. 2 shows a comparison of the sequences of the 7A and 8A clones of Figure 1.

Fig. 3 shows the predicted C. elegans amino acid UNC-53 sequence corresponding to the nucleic acid sequence of the 8A clone shown numbered from 1 to 1528. Again, potential start methionines are double underlined (M). Designations "tb" are regions for PCR clones to identify PCR products. Other regions of interest are identified. The region indicated as S4 is part of a lambda clone - 16.8 kb of the UNC-53 nucleic acid. This sequence, when translated is part only of the UNC-53 protein. Yet, injection of this part gives transformation rescue in organisms, i.e. providing additional evidence for the existence of shorter forms of the protein.

Fig. 4 shows the predicted C. elegans amino acid sequences of Figure 3 in the three letter code for indicating amino acids.

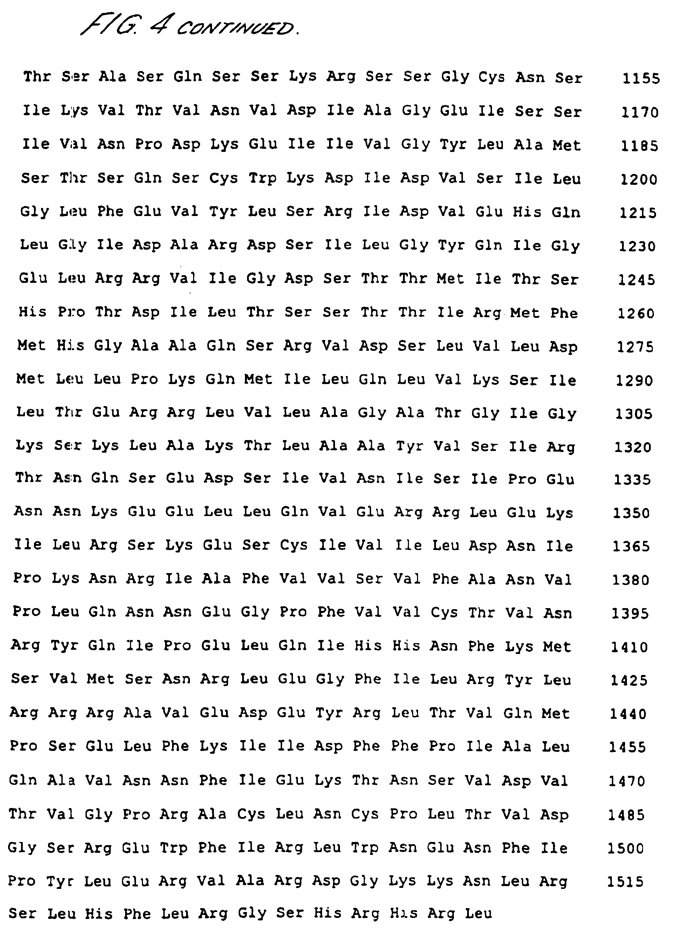

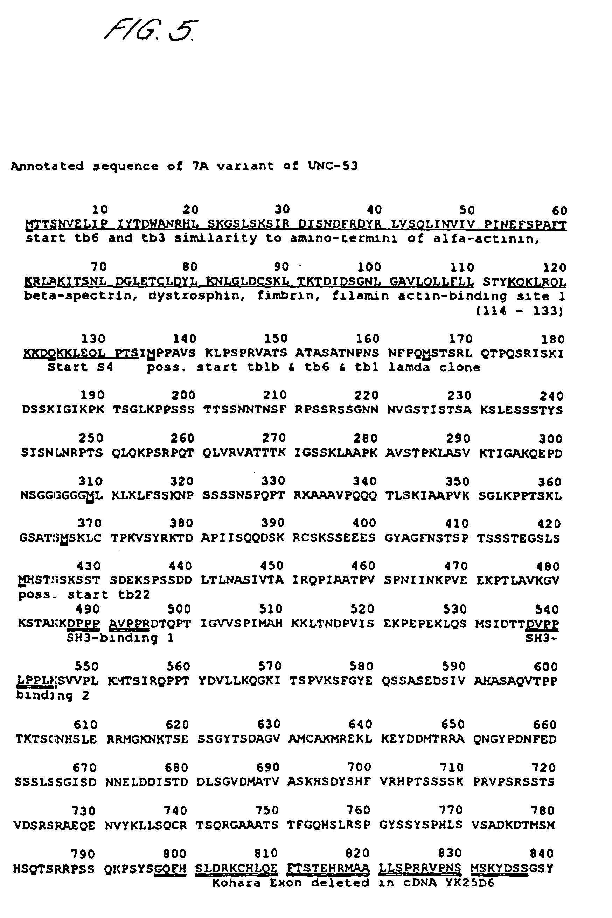

Fig. 5 shows the predicted C. elegans amino acid sequence UNC-53 sequence corresponding to the nucleic acid sequence of the 7A clone of Figure 2 shown numbered from 1 to 1583.

Fig. 6 shows the amino acid sequence of Figure 5 in the corresponding three letter code format for indicating amino acids.

Fig. 7 shows sequences of low complexity of the amino acid sequence of the corresponding nucleic acid sequence of the 8A clone of Fig. 3 identified with the filter and SEG algorithms of the BLAST sequence homology package. Regions of low complexity are indicated by "X" for the first copy of the sequence and by underlined amino acids for the second copy.

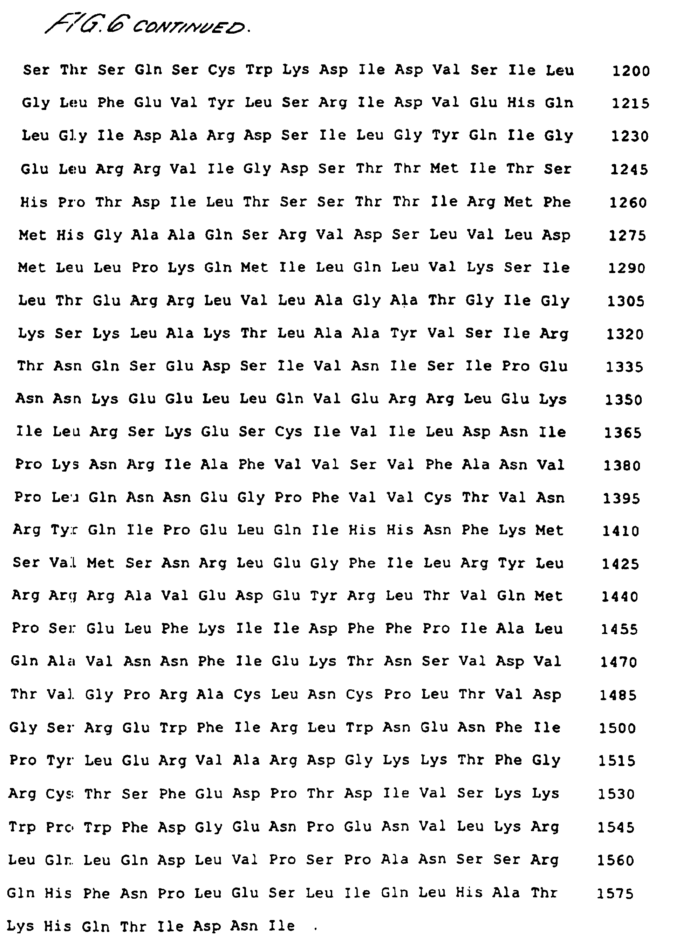

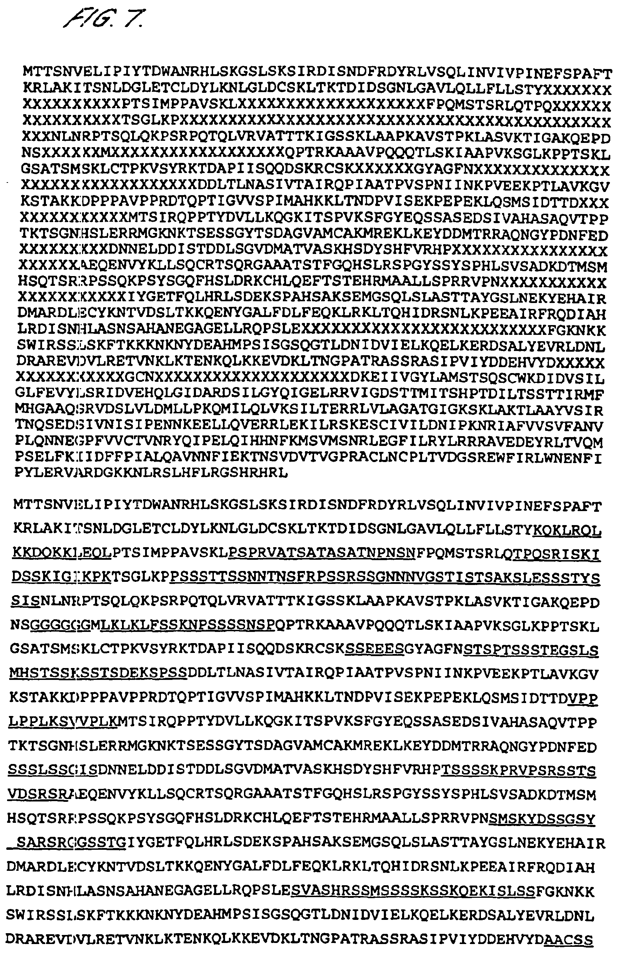

Fig. 8 shows, schematically, the known branches of the highly conserved Receptor Tyrosine Kinase/GRB2 signal transduction pathway including UNC-53.

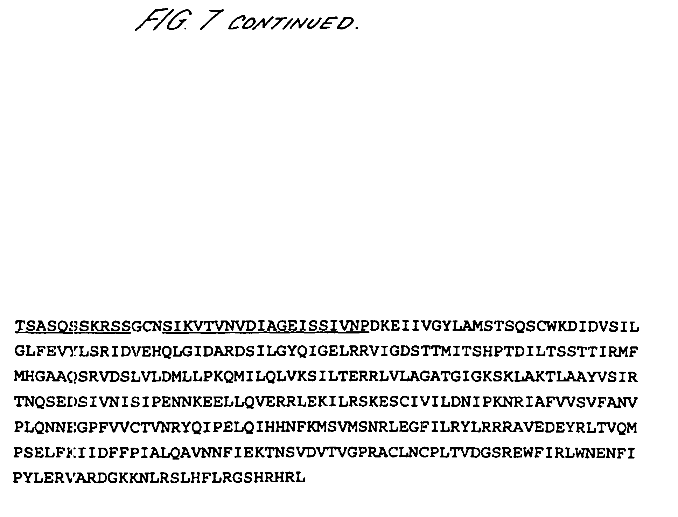

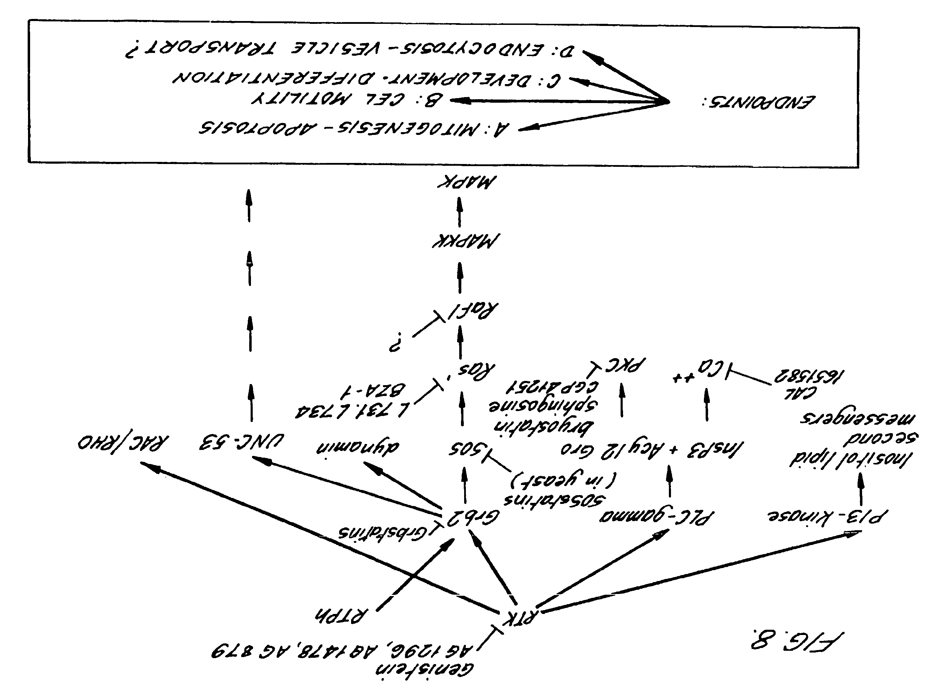

Fig. 9 shows, schematically, the differences in cells with increased and decreased UNC-53 expression from the wild type.

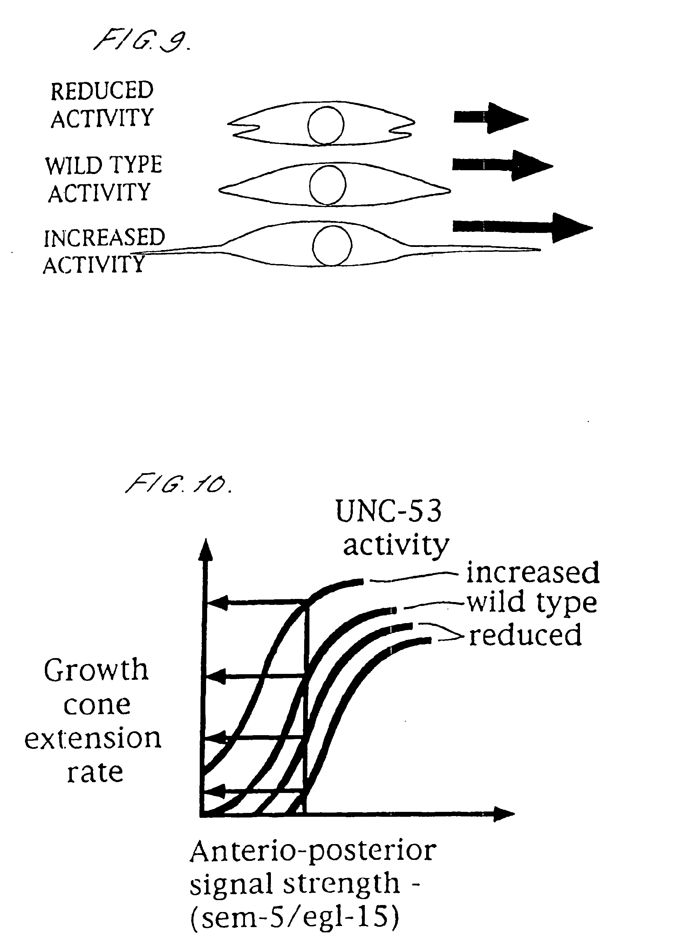

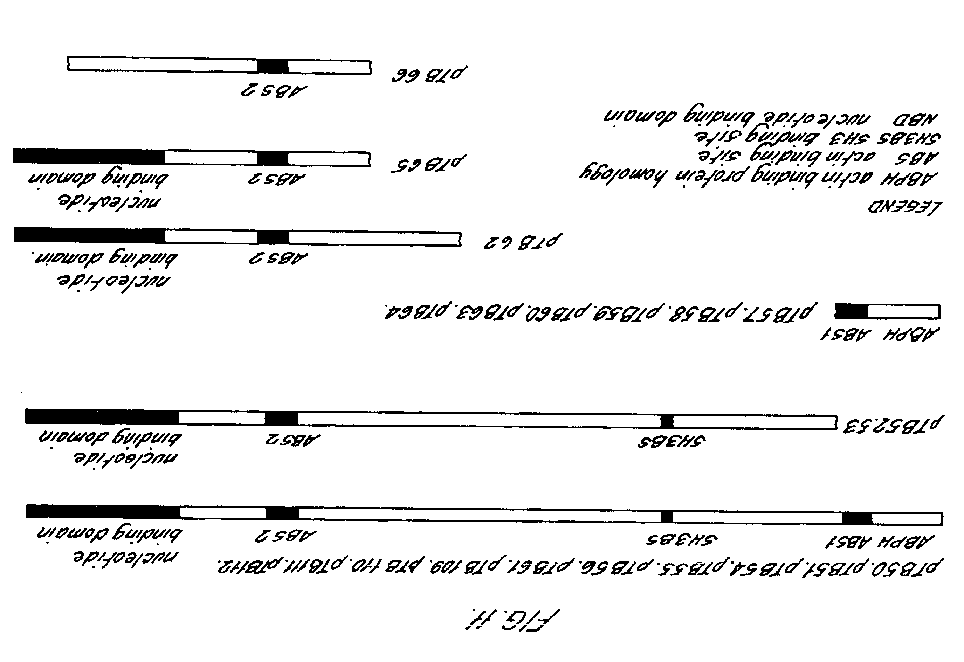

Fig. 10 is a graph of the effect of anterior-posterior signal strength on growth cone extension rate of C. elegans organisms, with increased and decreased UNC-53 expression from the wild type. This graph translates the observation that UNC-53 acts in a dosage-dependent way to direct the rate of extension in the anterior/posterior axis into a model. The signal received e.g. (egl-15) is an RTK mediated signal which is postulated to be received by UNC-53 and which results in extension in the anterior/posterior axis. The graph shows an allelic series of organisms with a graded reduction in extension from increased UNC-53 expression down through wild type to a reduced UNC-53 expression. The prediction is thus: for the same level of RTK mediated signal the increased/decreased growth in the anterior/posterior axis depends on the level of expression of UNC-53 in any organism. The graph also reflects the prediction that for organisms with a particular level of UNC-53 overexpression there is no requirement for a signal before growth cone extension occurs. This extension is likely to be in a random direction or influenced by alternative factors.

Fig. 11 shows constructs of unc-53 nucleic acid including identified functional domains .

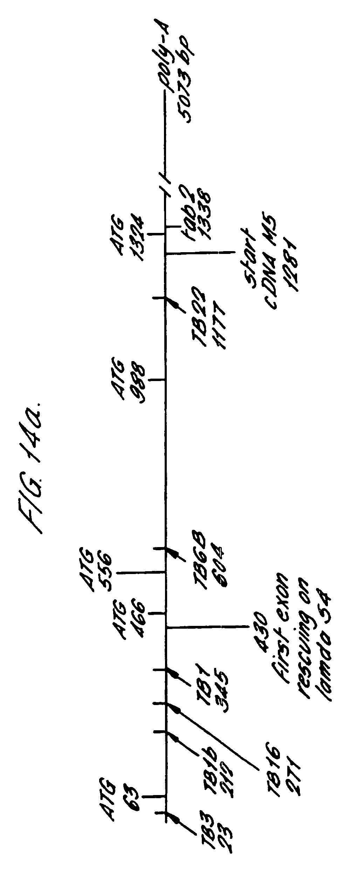

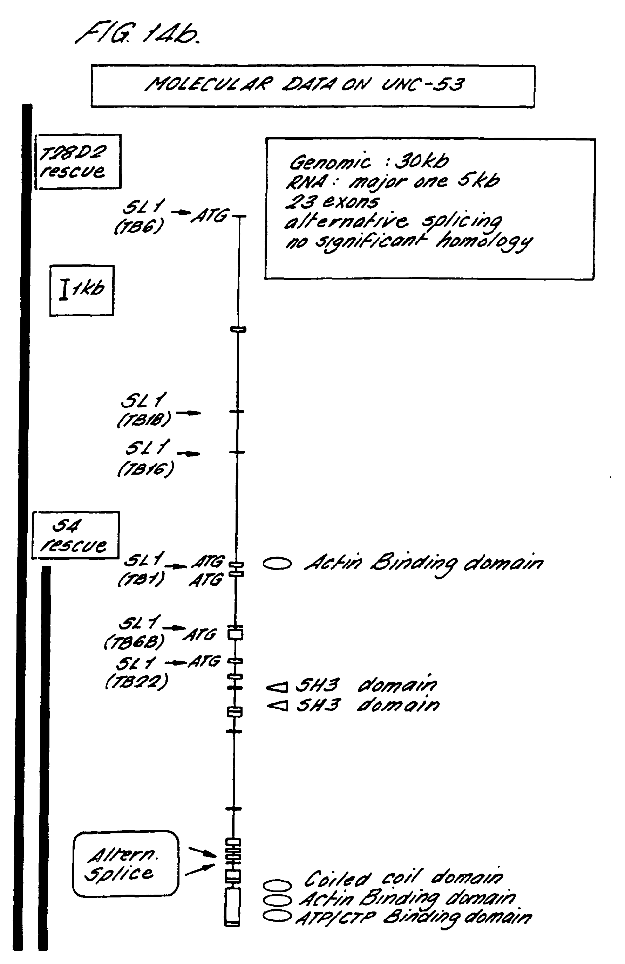

Fig. 12 shows 5' amino terminus of the cDNA encoding from the first methionine amino acid through the actin binding protein homology domain (amino acids 1-133 from Fig. 1) and oligonucleotides designated oligo BG01, BG02 and BG03 (amplification strategies of amino terminus of the unc-53 cDNA). Combinations of oligo BG02 with either oligo BG02 or BG03 were used to amplify the 5' terminus of the cDNA from the first methionine through the actin binding protein homology domain (amino acids 1-133). All of the oligonucleotides are underlined and sequences identical to the cDNA are shown in upper-case. In addition to unc-53 sequence, oligo BG02 contains a stop codon and the recognition sequence for BamHI endonuclease. Oligo BG01 has engineered EcoRI and NdeI recognition sites for inclusion in bacterial expression vectors. Both constructs remove the 5' untranslated region of unc-53 and oligo BG03 contains a NotI cleavage site. Oligo BG03 has an improved ribosome binding site similar to mammalian ribosome binding sites. Use of BG03 in PCR thus results in constructs optimised for mammalian expression.

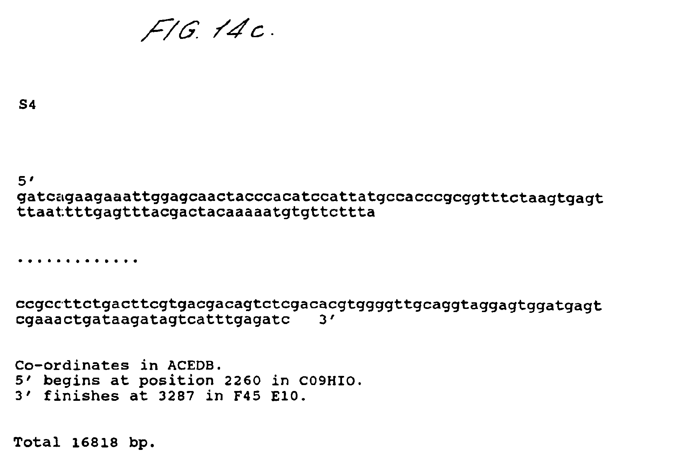

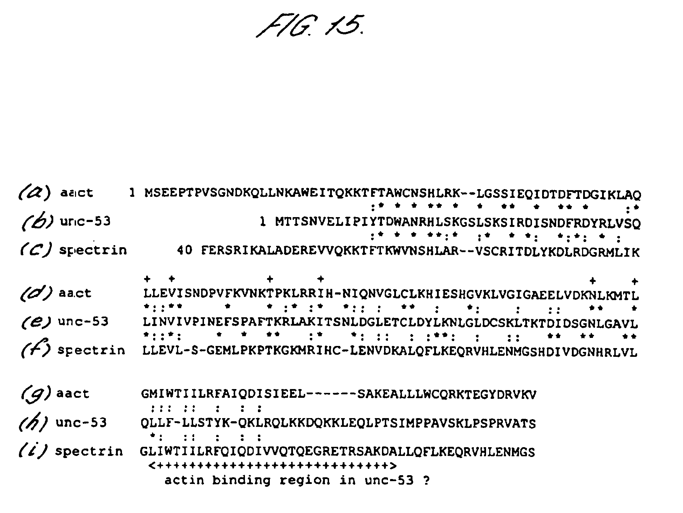

Figure 13 shows, schematically, constructs of the plasmids pTB109, pTB110, pTB111 and pTB112.

Fig. 14(a) shows a summary of transcript starts at the 5' end of the unc-53 gene. Different identified transcript starts and corresponding in-frame ATG-codons are marked. Tab2 is the oligo from within cDNA M5 which was used in RT PCR experiment to identify/isolate the 5' ends of different UNC-53 mRNAs.

Figure 14(b) shows the location of the different transcript starts on the genomic DNA and the position of the S4 Lambda clone with respect to genomic DNA.

Figure 14(c) shows the sequence near the 5' and 3' ends of the lambda S4 clone, identifying its composition corresponding to the 5' and at position 2260 of comid COGHIO and the 3' end of F45R10 at position 3287.

Fig. 15 shows the alignment of UNC-53 protein with the carboxytermini of the α-actinin and β-spectrin family (QY is UNC-53).

Fig 16 shows the predicted actin binding sites of UNC-53. The comparison shows internal LKK repeats.

Fig. 17 shows the alignment of the candidate SH3 binding sites in UNC-53 with known SH3 sites of other named proteins. Proteins at positions 4 and 7 are critical for binding into SH3 pockets.

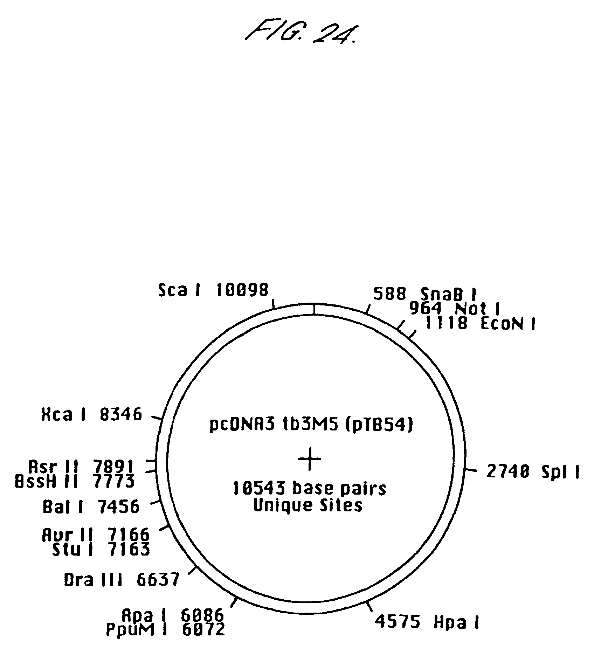

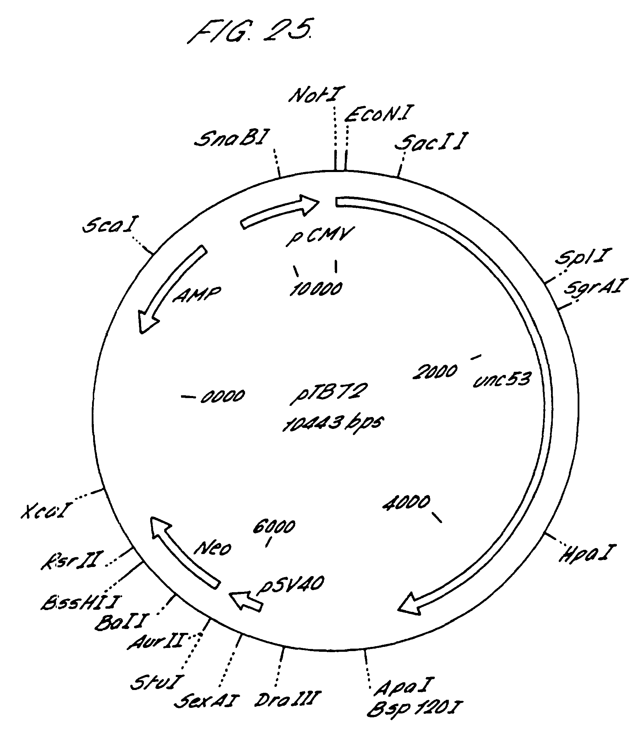

Fig. 18 shows the alignment of the predicted amino acid sequences from F45E10.1 (available in public database) with UNC-53. The different identified amino acid is shown at position 1186. The frameshift which results in the different amino acid sequence from position 1513 is a result of the different number of adenine bases in the nucleic acid sequence (see Fig. 1).

Fig. 19 is a series of photographs of C. elegans embryos (strain TB4Ex25 (Table 1) [UNC-53-UNC-54 construct]). The photographs show increased outgrowth in the anterior-posterior axis of body wall cells in the C. elegans embryos which overexpress UNC-53 (immunofluorescence with UNC-53 mab 16-48-2) Individual photographs are as follows:
A: early embryo comma stage
B: 1.5 fold stage embryo
C: 3 fold stage embryo, first plane of focus
D: 3 fold stage embryo, second plane of focus
E: 3 fold stage, mosaic animal, 3-cells in a quadrant giving expression.

This demonstrates that immunofluorescence provides a measure of the expression in the transgenic lines of UNC-53.

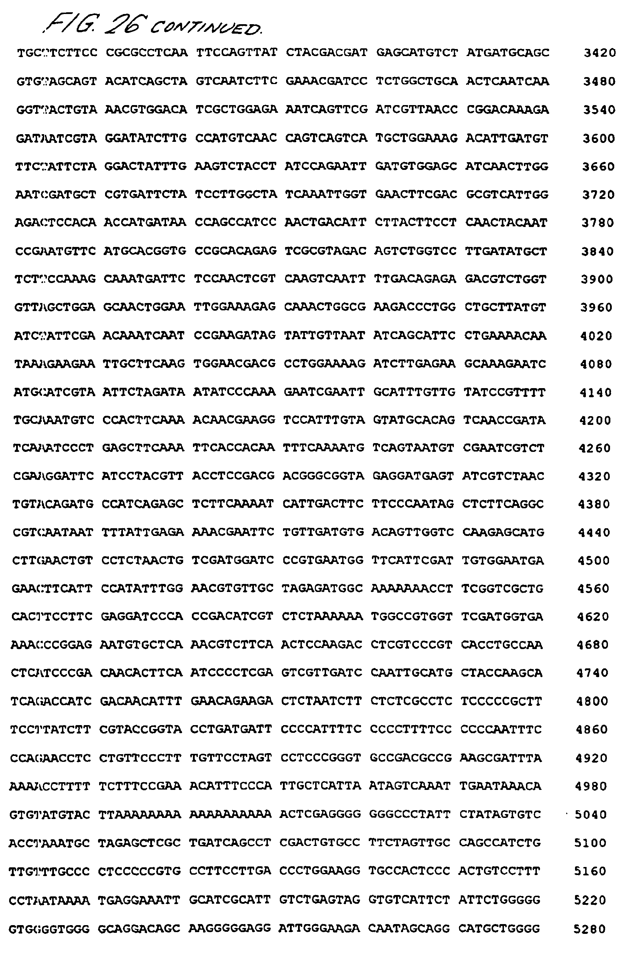

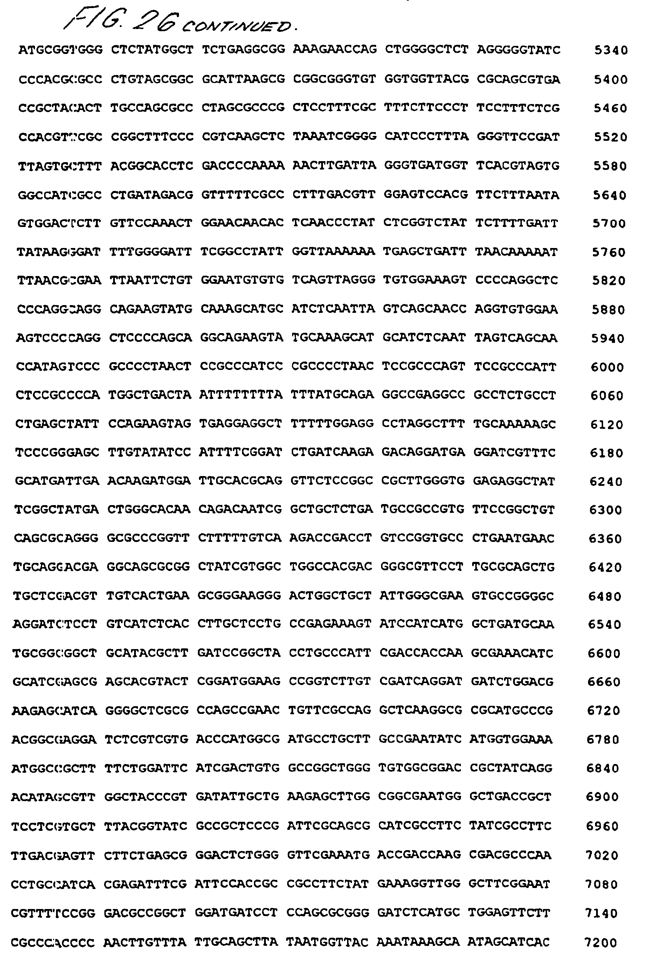

Fig. 20A is a photograph of C. elegans embryo containing DNA construct pTB110 (strain TBAIn76(table 1)). Shown is expression of UNC-53 following heat shock.

Fig. 20B and C are photographs of C. elegans embryos containing DNA construct pTB111 (strain TB1Ex6 (table 1)). Shown is transgenic expression of UNC-53 in mechano-sensory neurons.

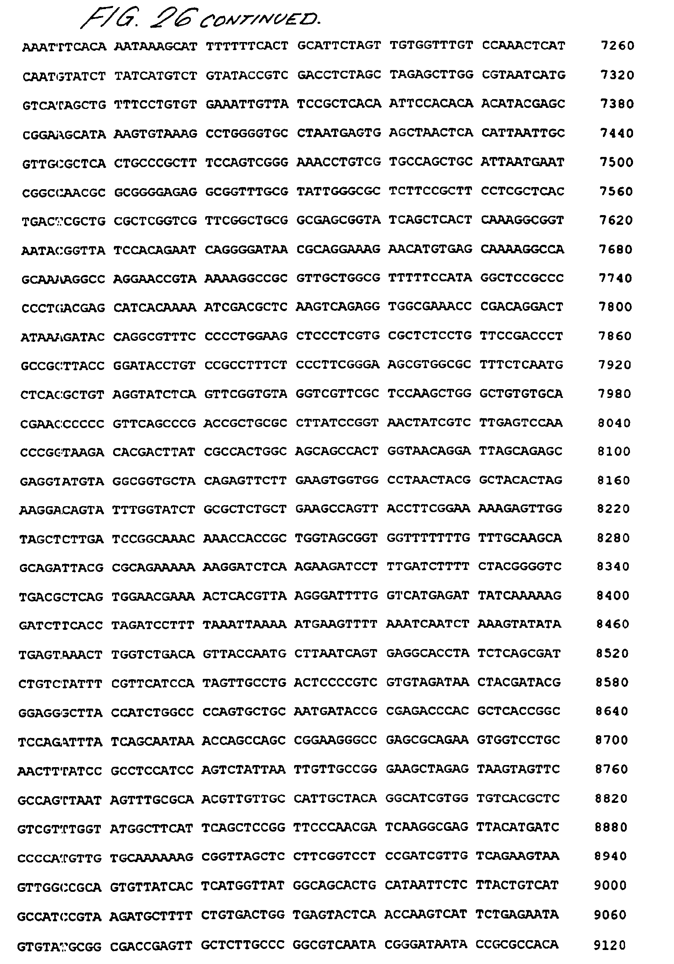

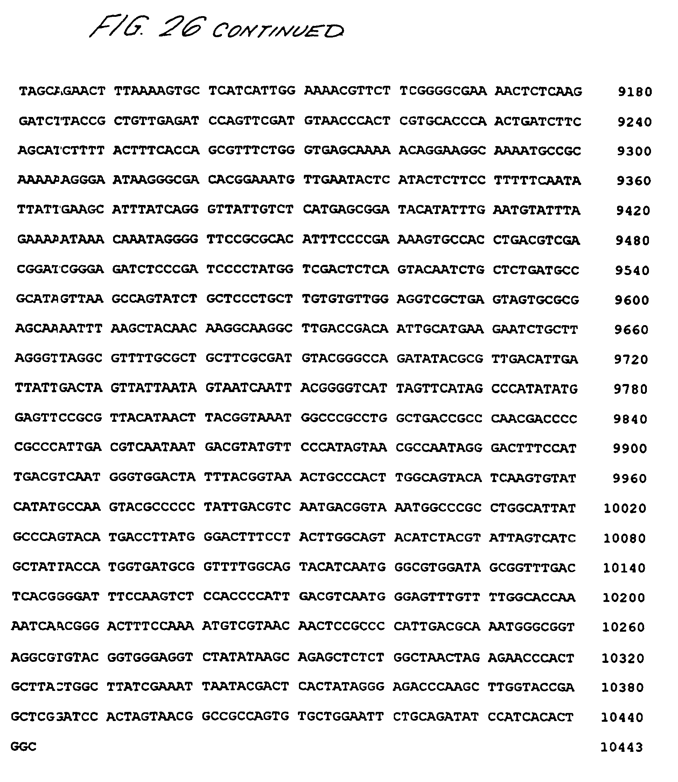

Fig. 21 shows photographs of the following:
A: A wild-type UNC-53 L1 larva of genotype 4-25 (strain TB4Ex25) as in photographs 19B, C and D.
B: L1 larva of 4-25 with morphological defects associated with muscle abnormalities.
C: Lethal phenotype of 4-25.
D: L1 larva of 4-25 showing misshapen animal and muscle cells with increased extensions. Also shows constipation problems associated with abnormal muscle pattern.
E: L1 larva of the heat-shock line TBAIn76 (table 1) exhibiting morphological abnormalities following heat shock and recovery.
F: L1 larva of line TBAIn76 (table 1) showing morphological defects in the pharynx.

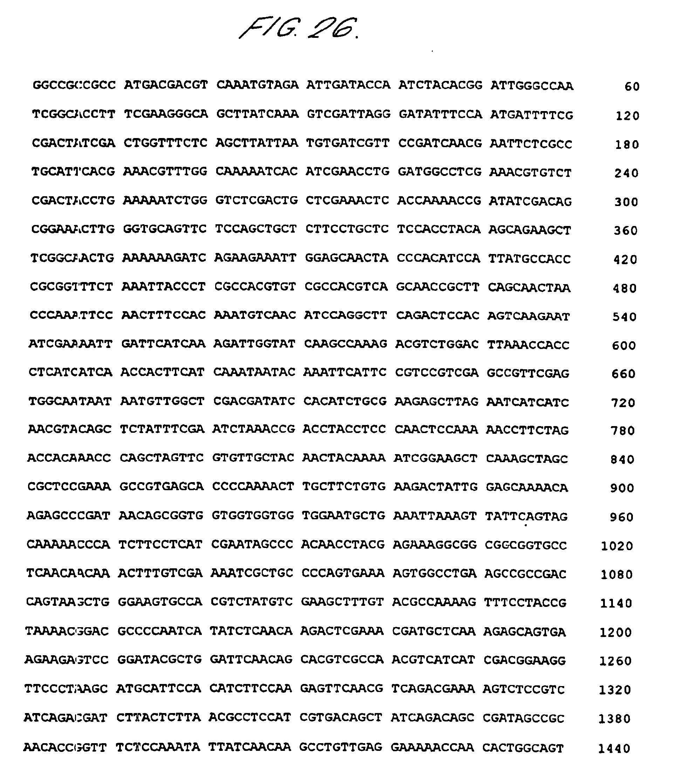

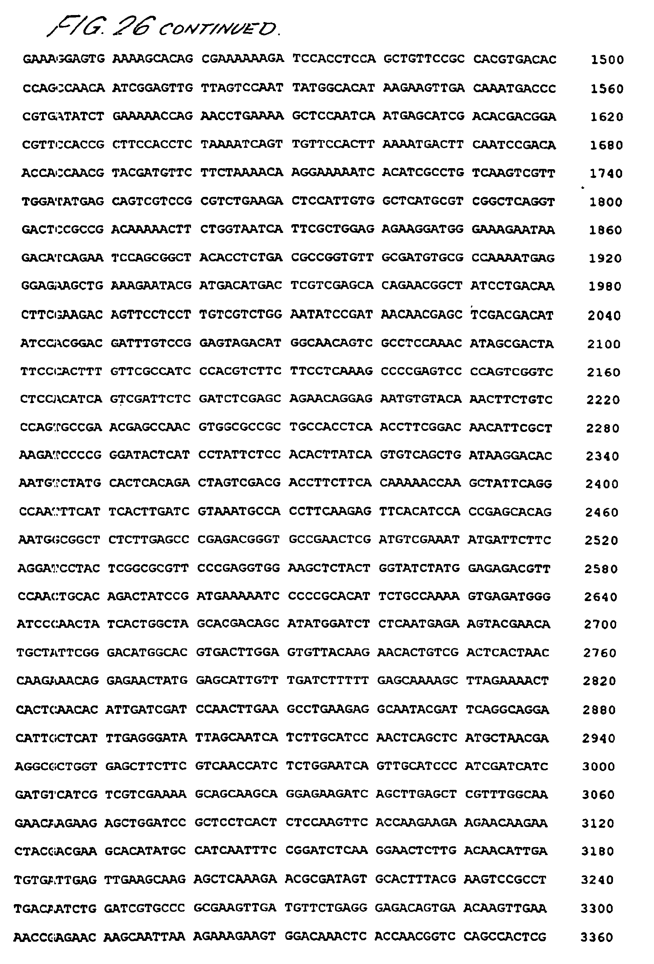

All Figs. 19, 20 and 21 are Normarski optics of live embryos.

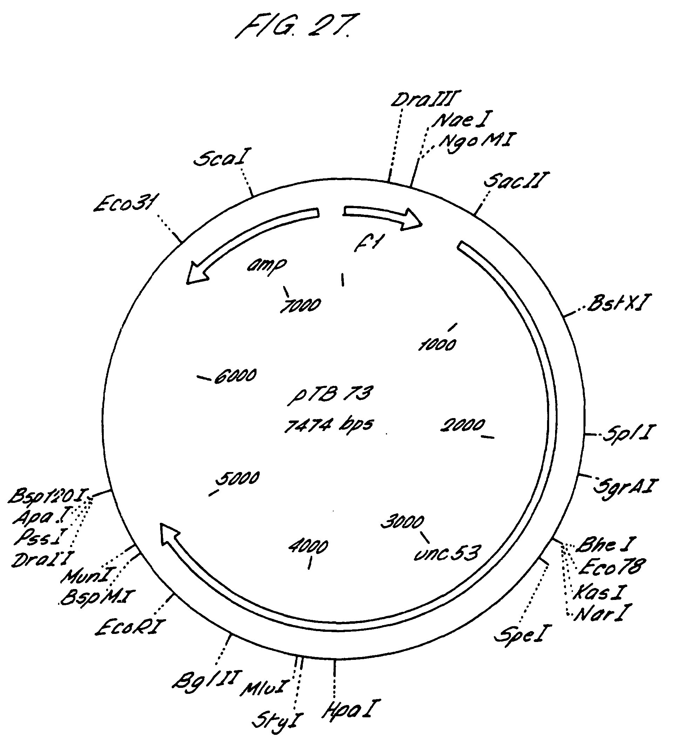

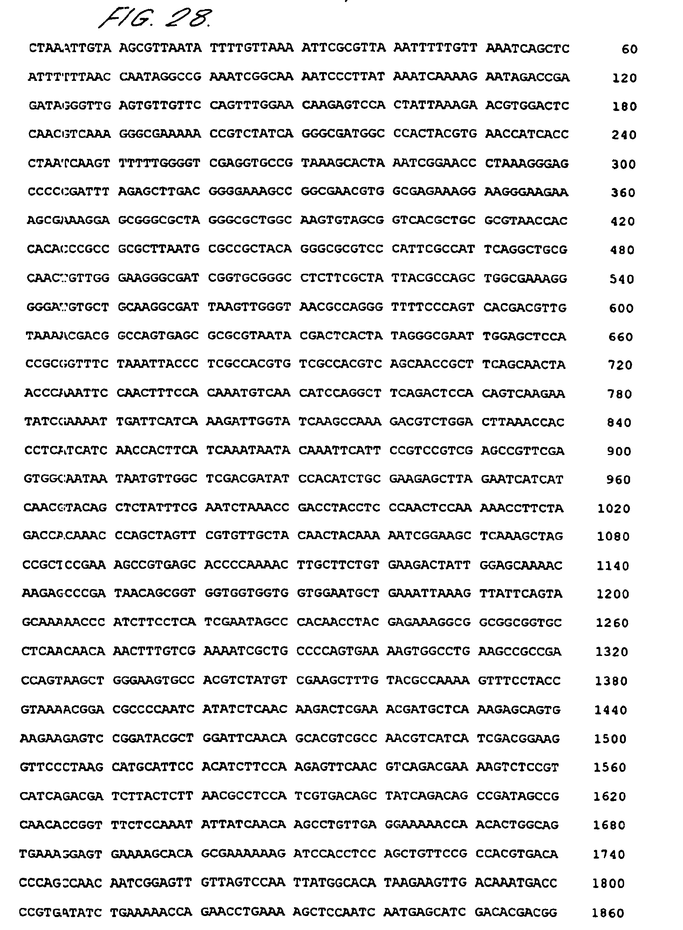

Fig. 22 is a map of plasmid pTB110 (tables 1 and 2) a heat shock promoter fusion, indicating restriction endonuclease sites.

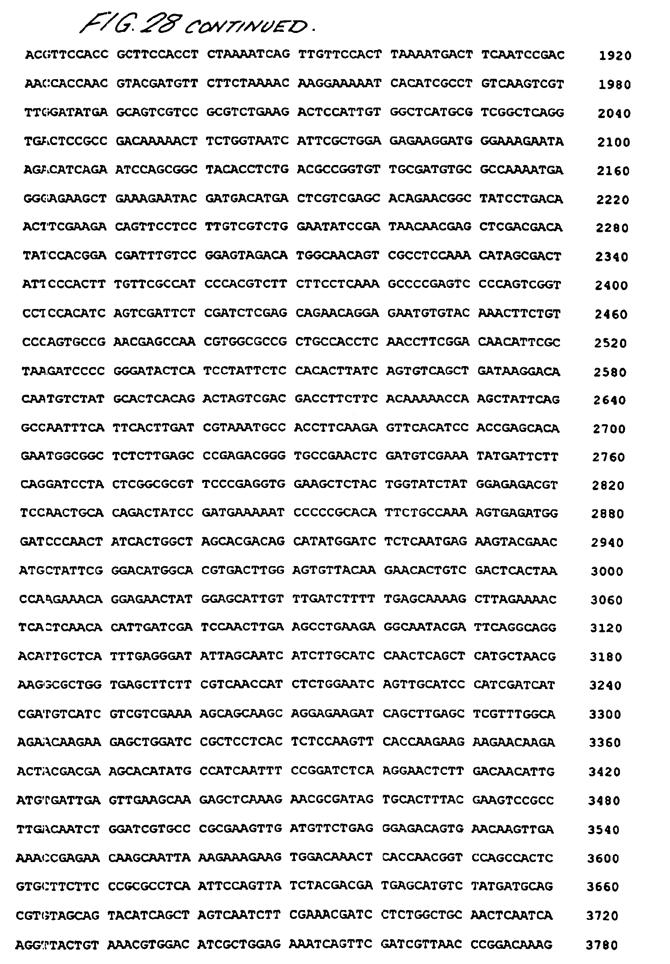

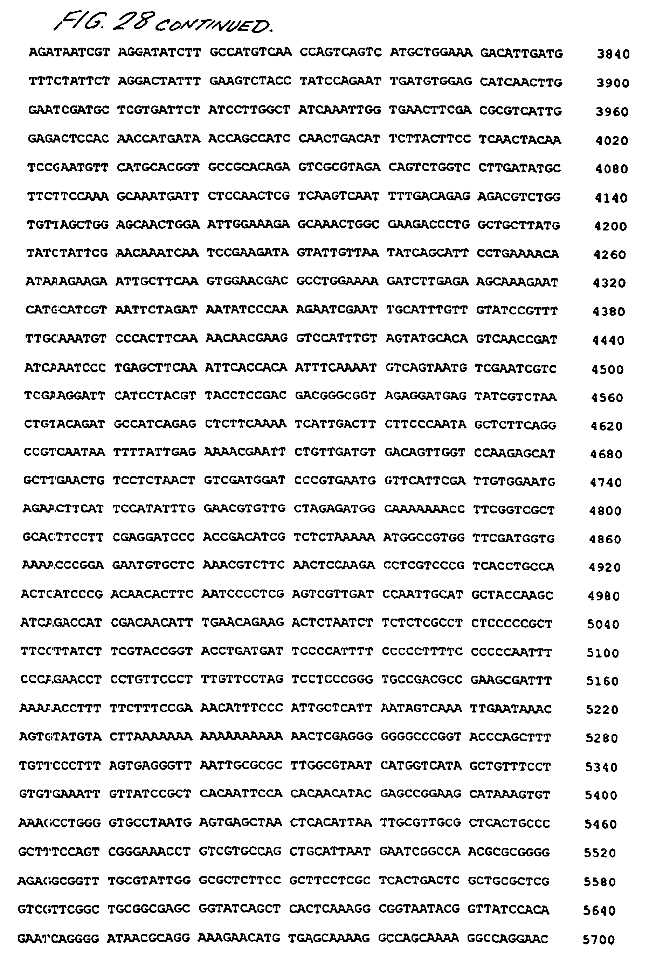

Fig. 23 is a map of plasmid pTB112 (tables 1 and 2) a muscle specific UNC-54 fusion, indicating restriction endonuclease sites.

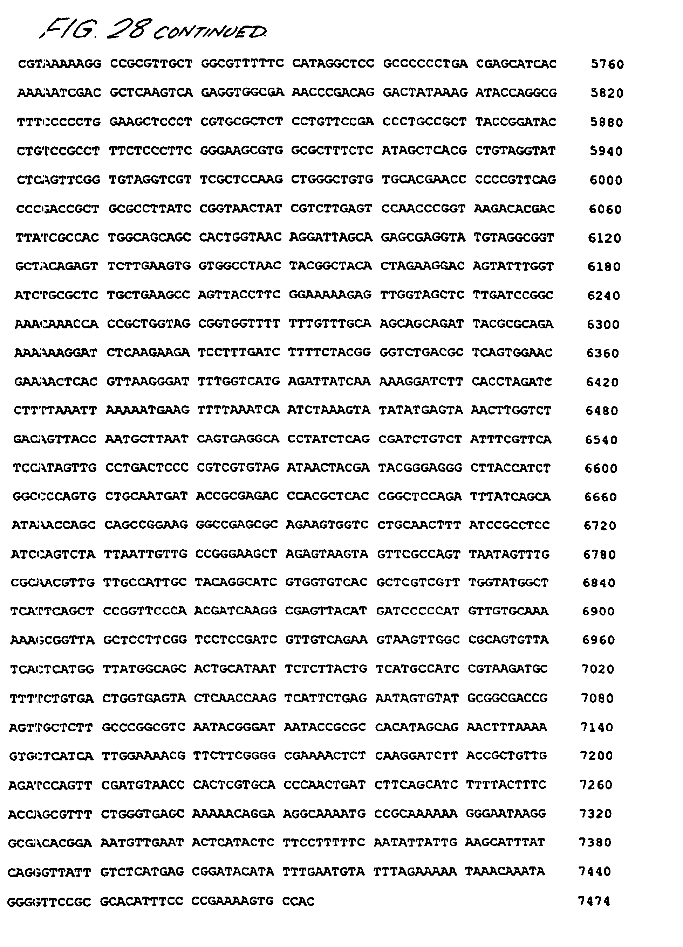

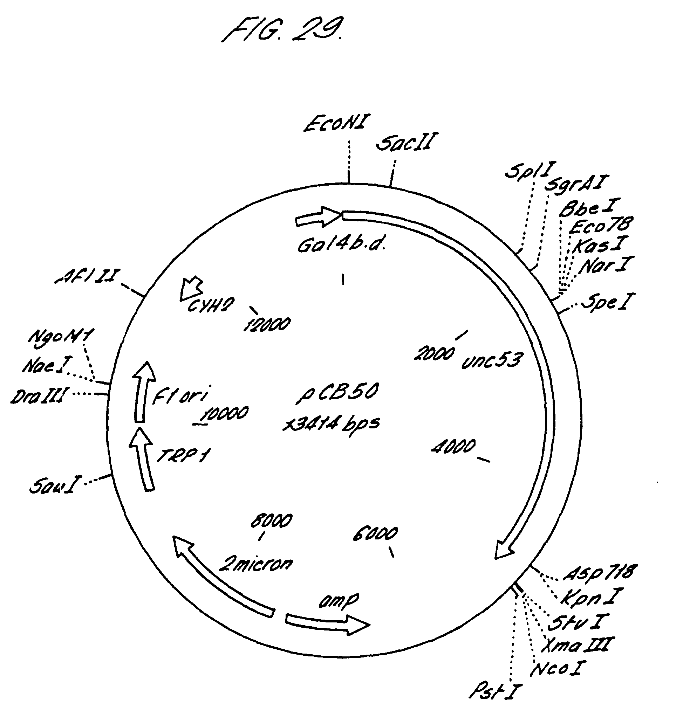

Fig. 24 is a map of plasmid pTB54 (the 8A clone variant) (tables 1 and 2). In the construction of this plasmid the complete unc-53 cDNA (tb3M5) of the 8A variant, including 5' and 3' UTRs was cloned as a NotI-ApaI fragment into the mammalian expression vector pcDNA3 (Invitrogen).

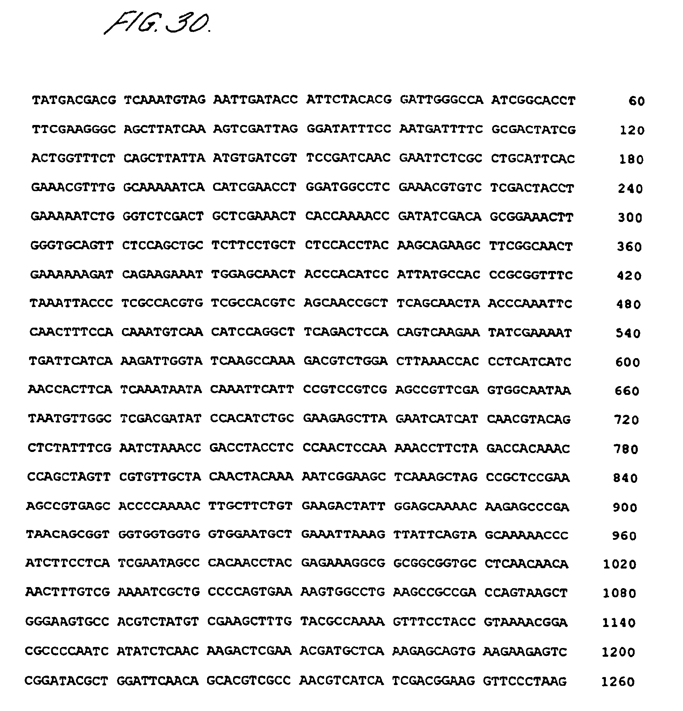

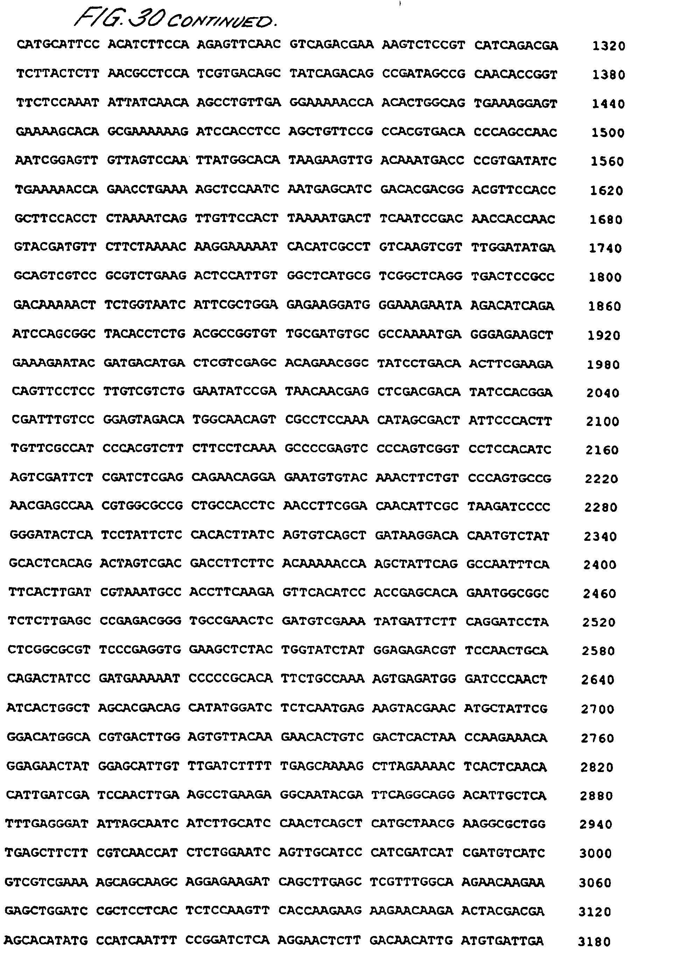

Figure 25 is a map of plasmid pTB72 (the construct encoding the 7A clone variant of UNC-53 cDNA of Figure 2.

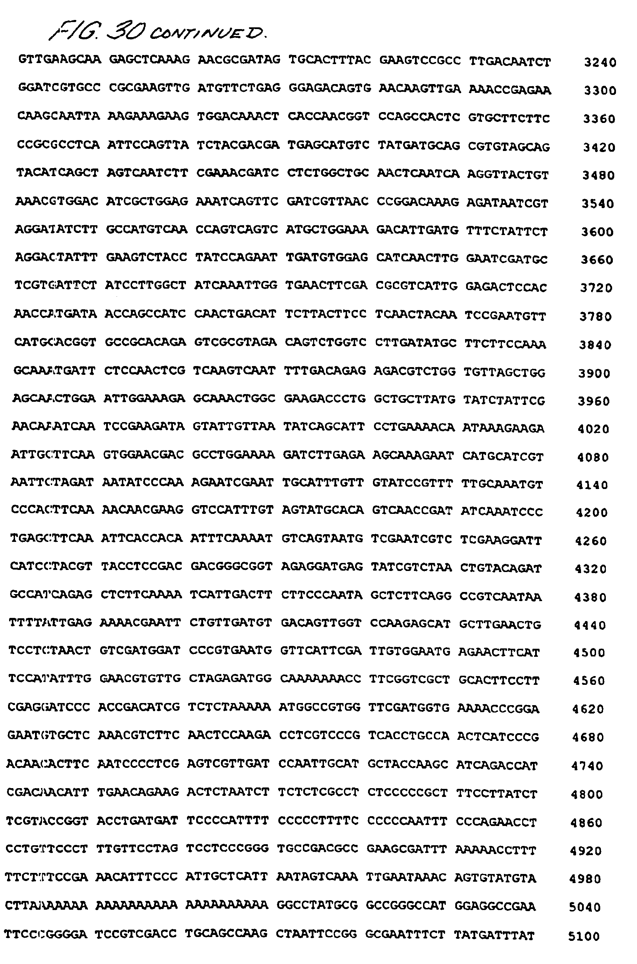

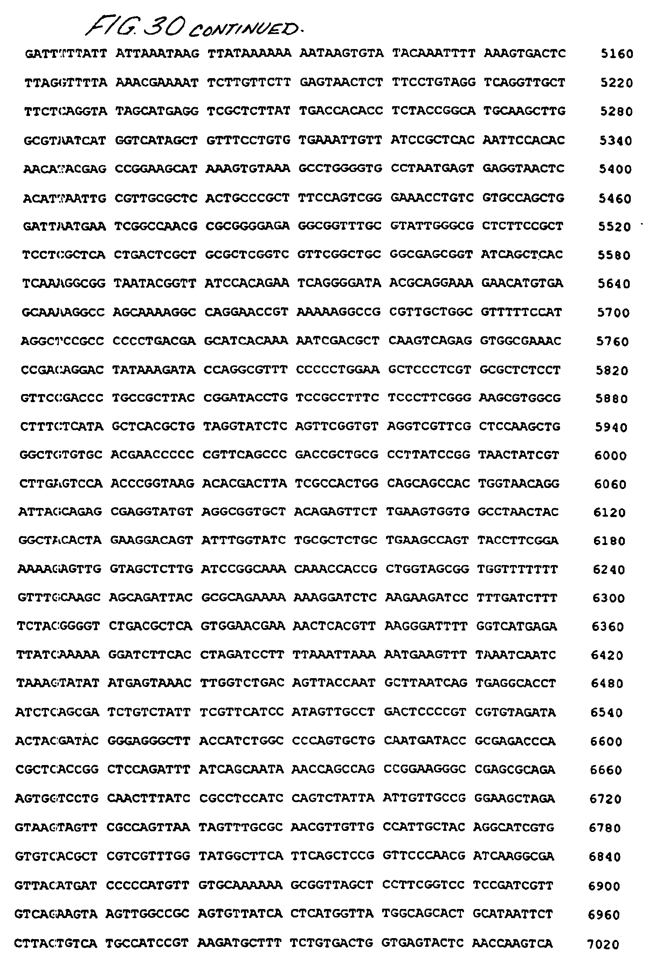

Figure 26 is nucleotide sequence of the plasmid map of Figure 25.

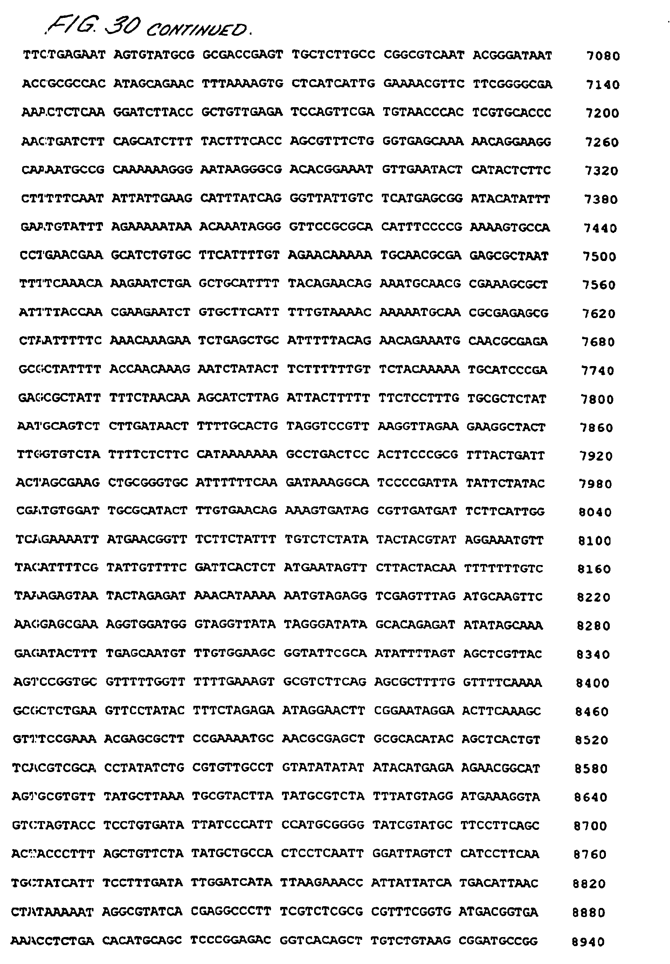

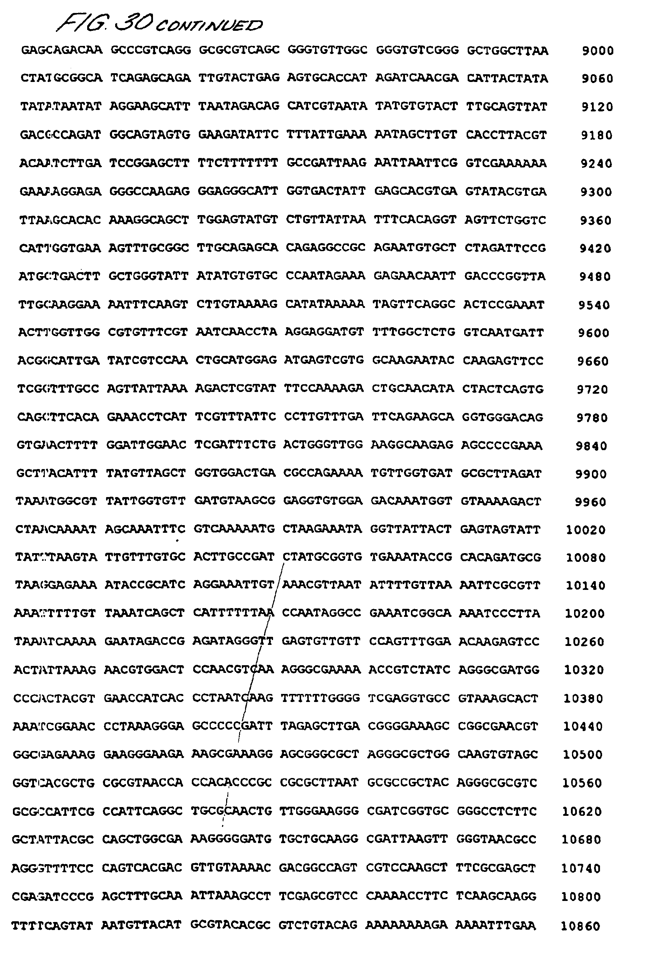

Figure 27 is a map of plasmid pTB73.

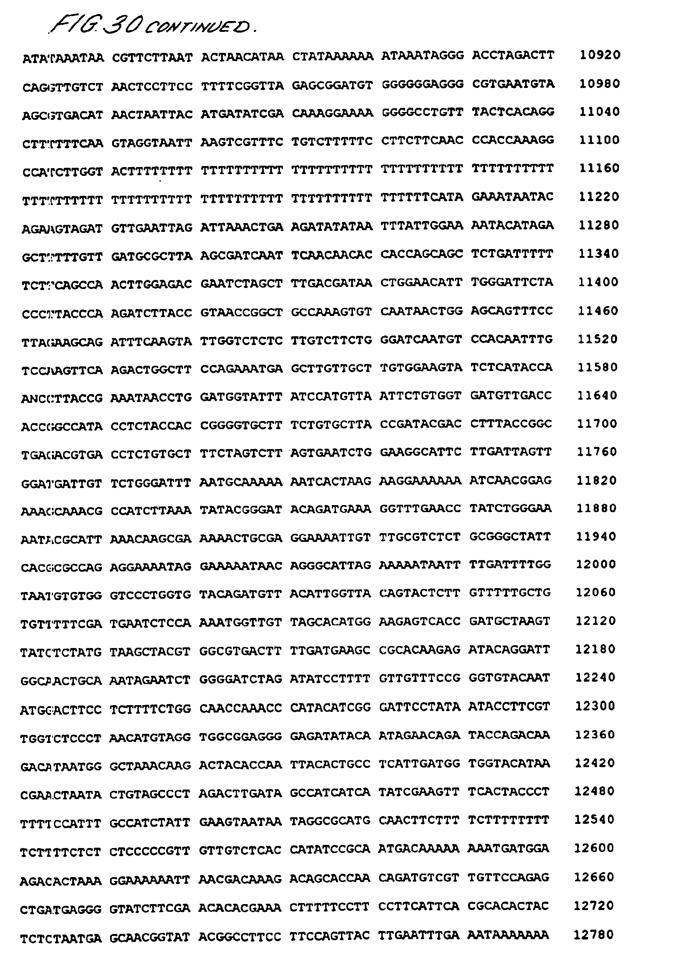

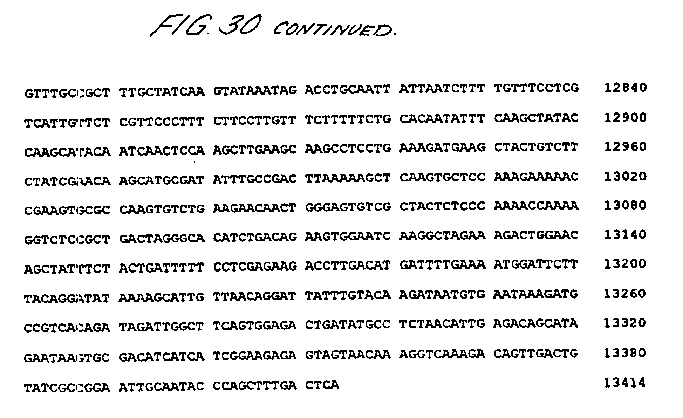

Figure 28 is a nucleotide sequence of plasmid pTB73 of Figure 27.

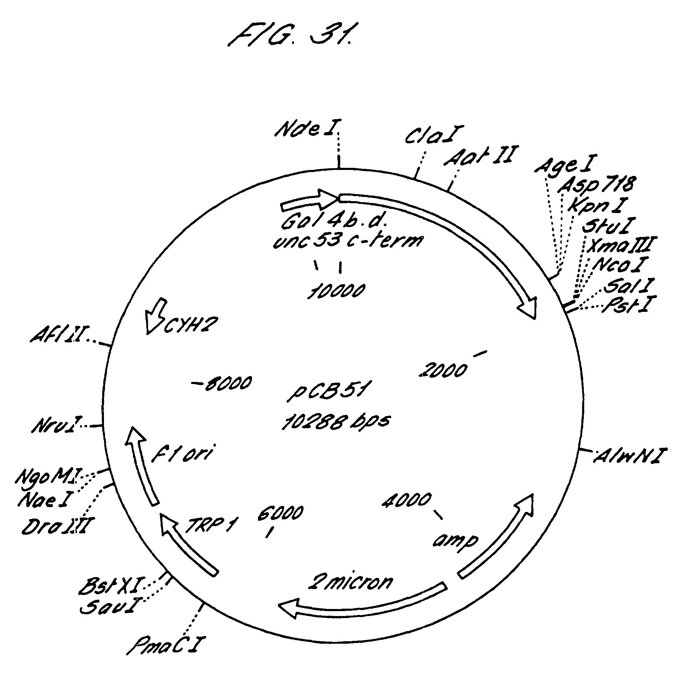

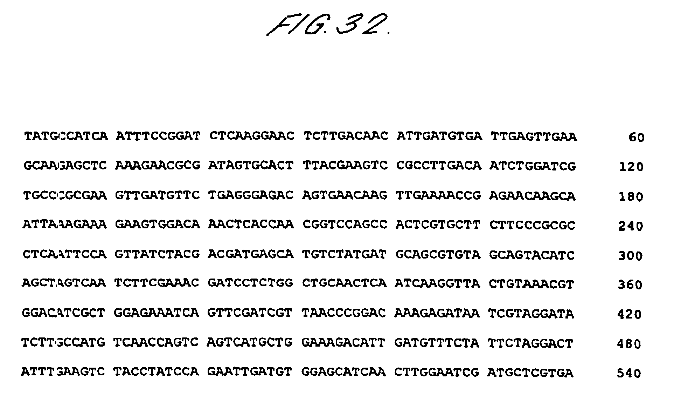

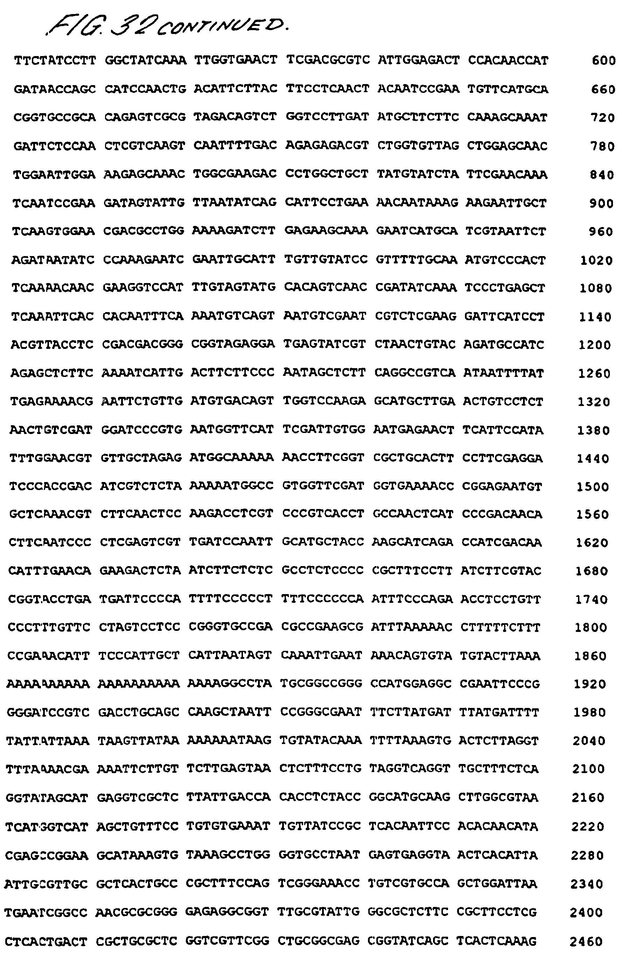

Figure 29 is a map of plasmid pCB50.

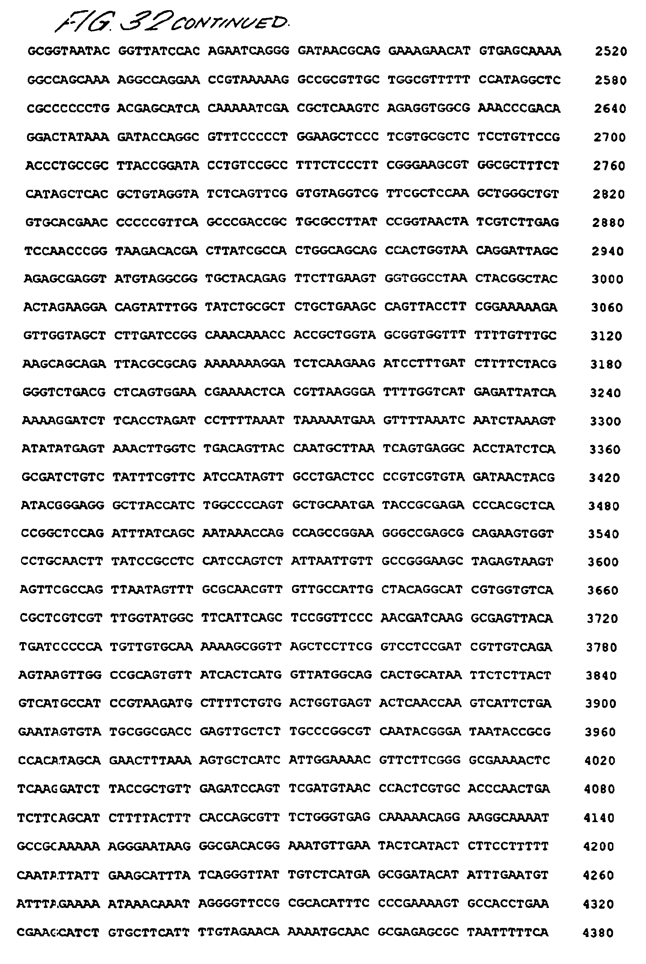

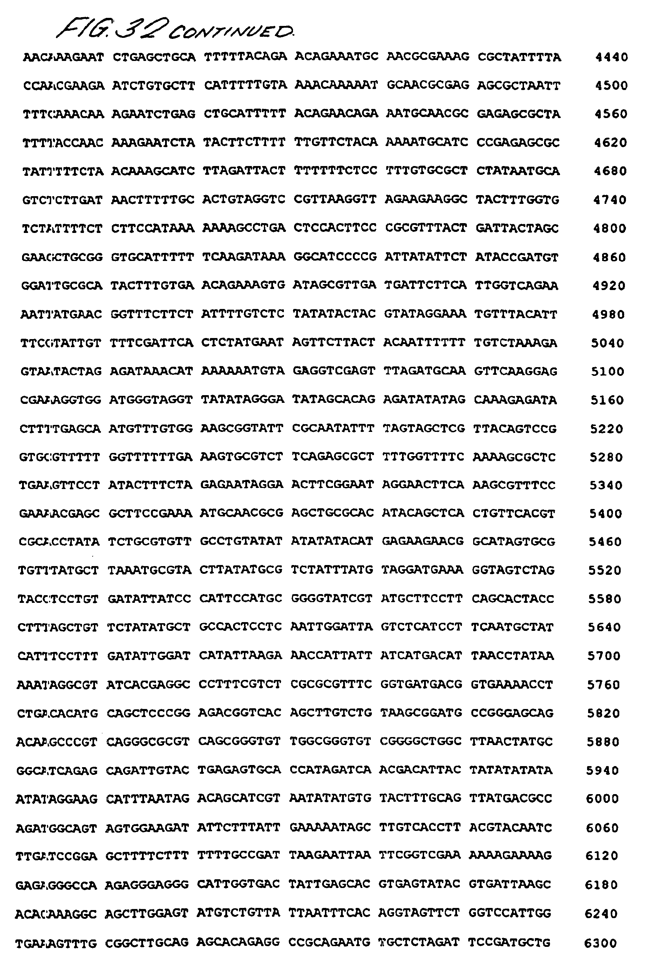

Figure 30 is a nucleotide sequence of plasmid pCB50 of Figure 29.

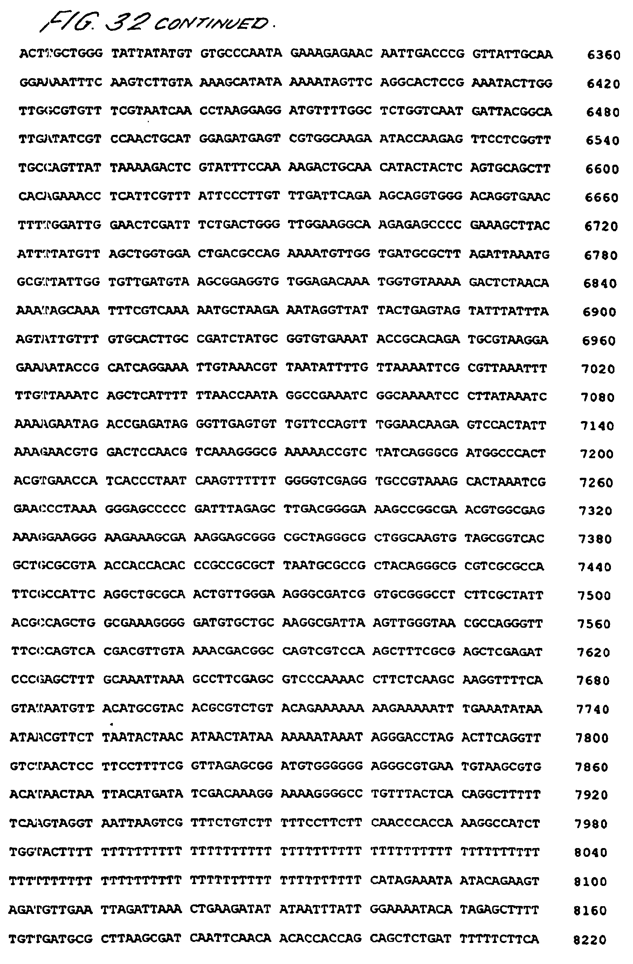

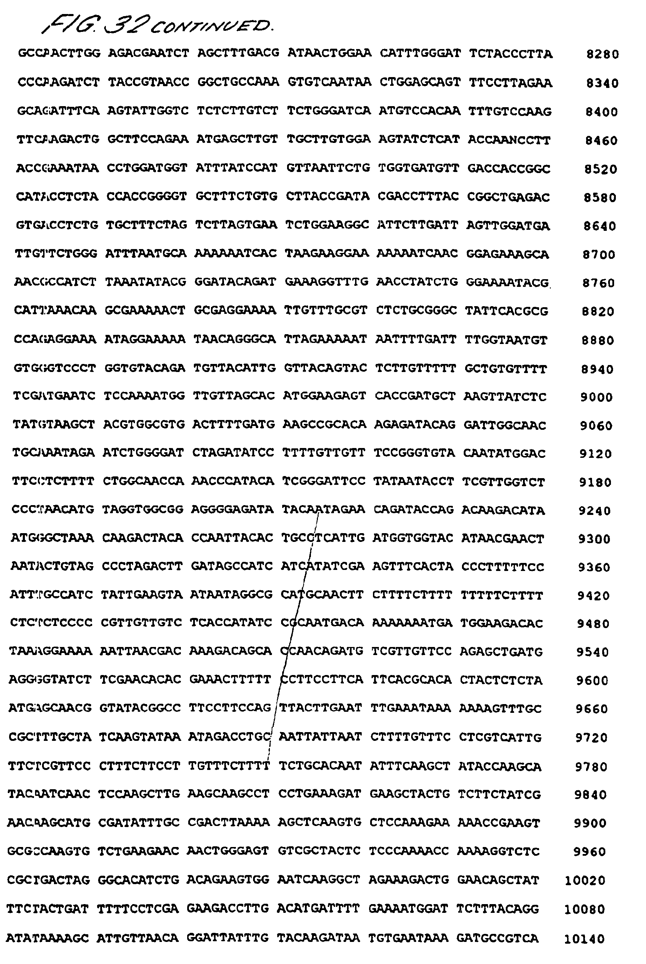

Figure 31 is a map of plasmid pCB51.

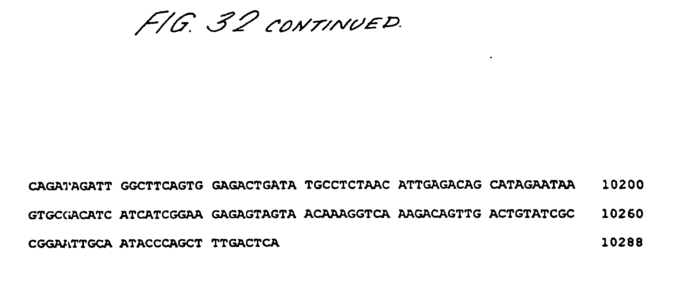

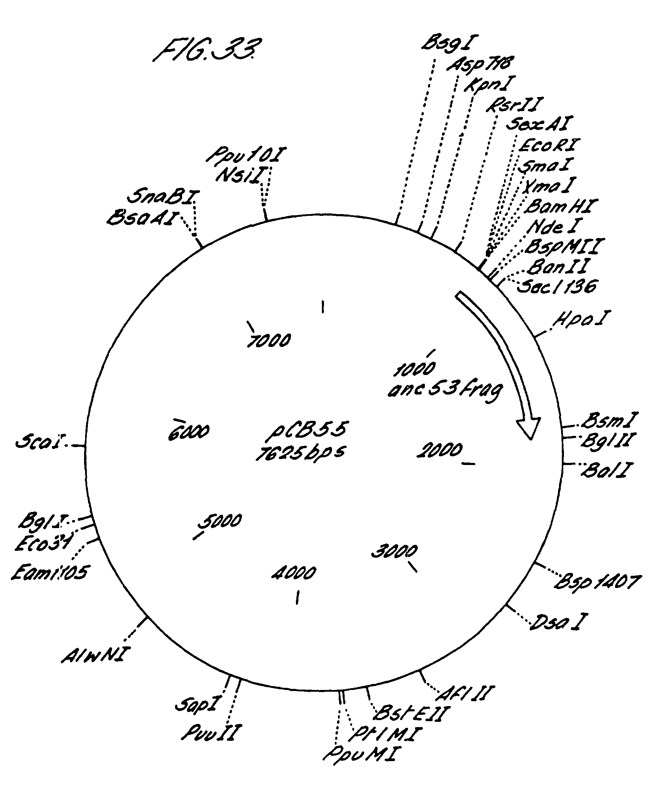

Figure 32 is a nucleotide sequence of the plasmid pCB51 of Figure 31.

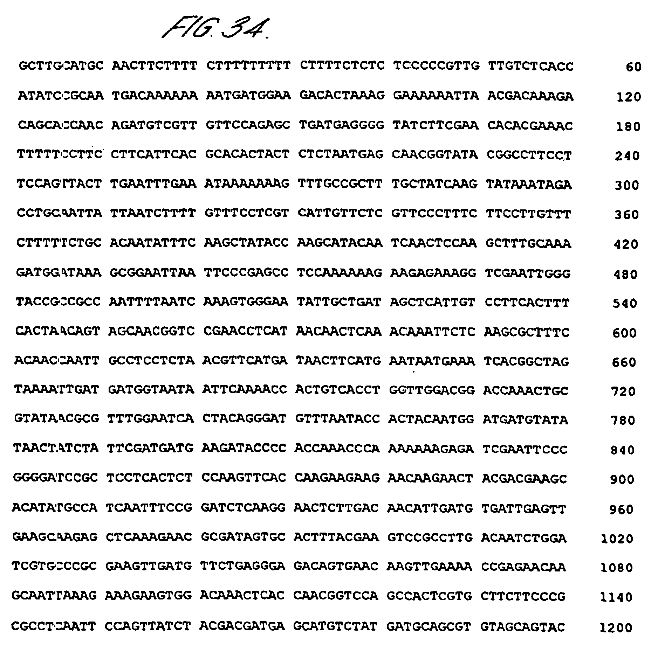

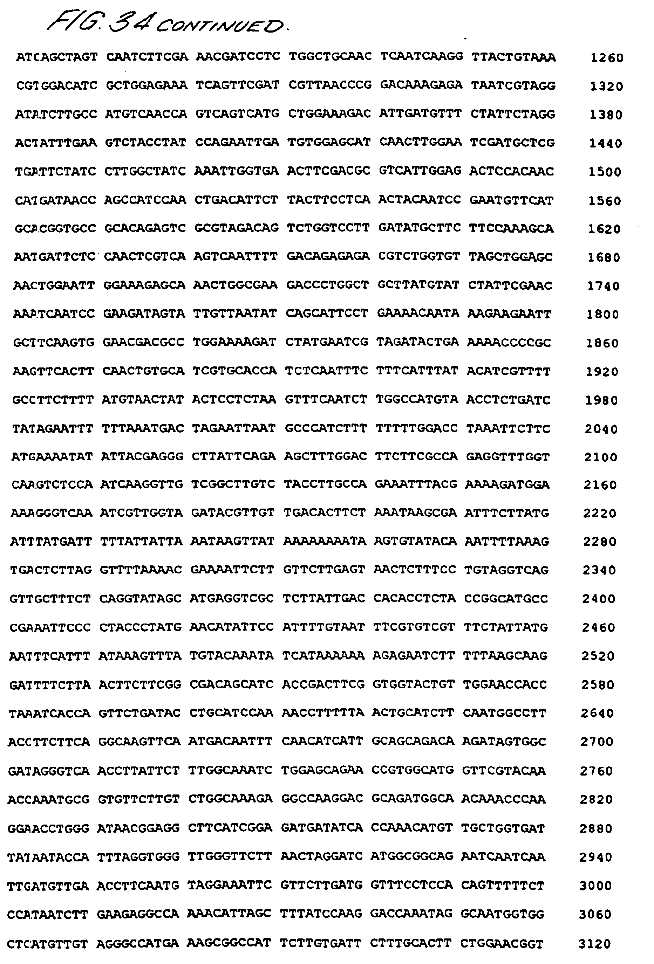

Figure 33 is a map of plasmid ppCB55.

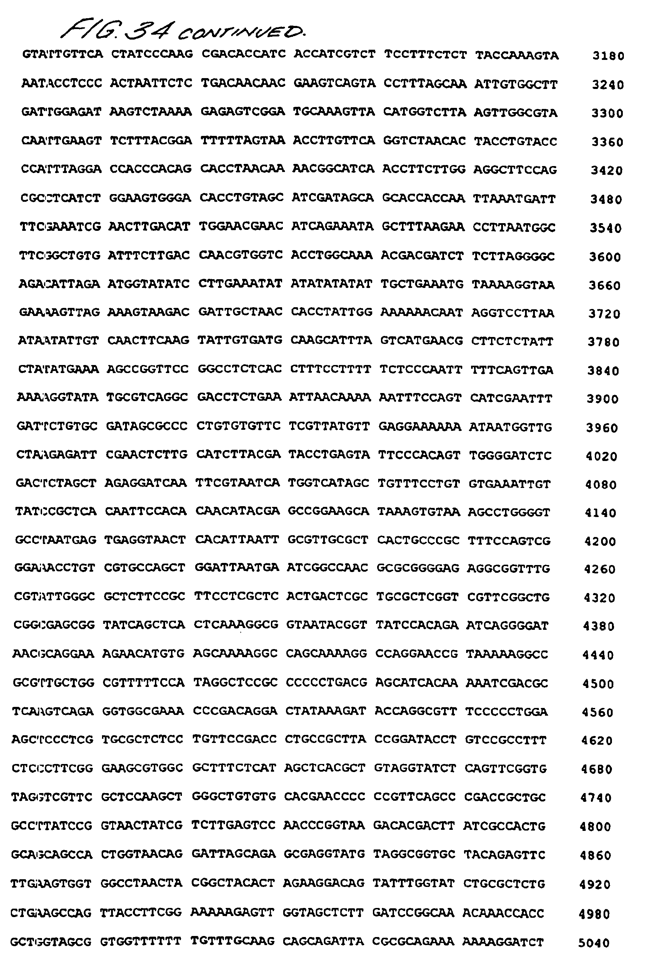

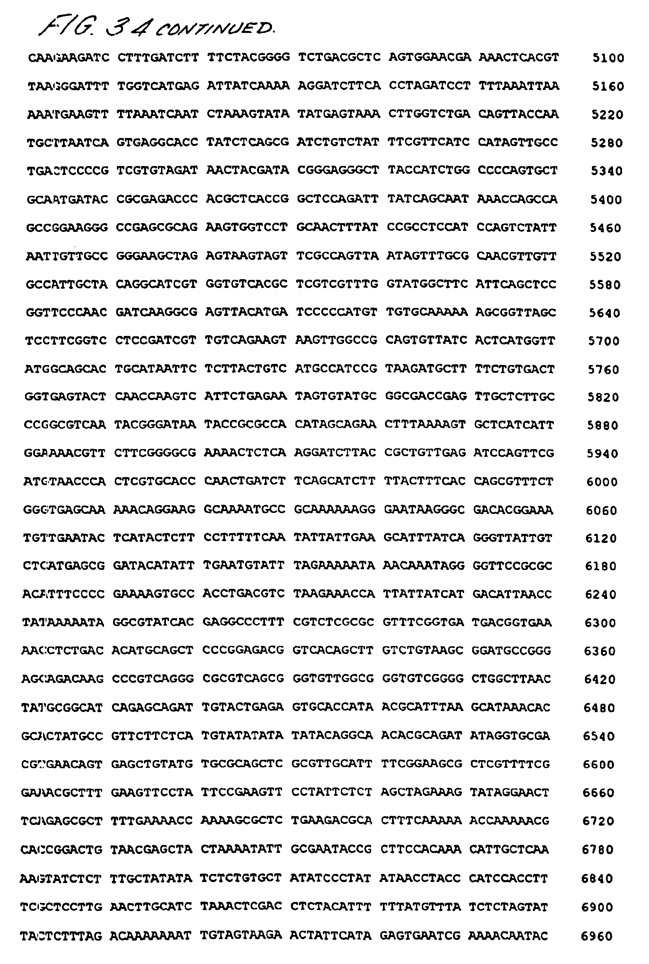

Figure 34 is a nucleotide sequence of plasmid pCB55 of Figure 33.

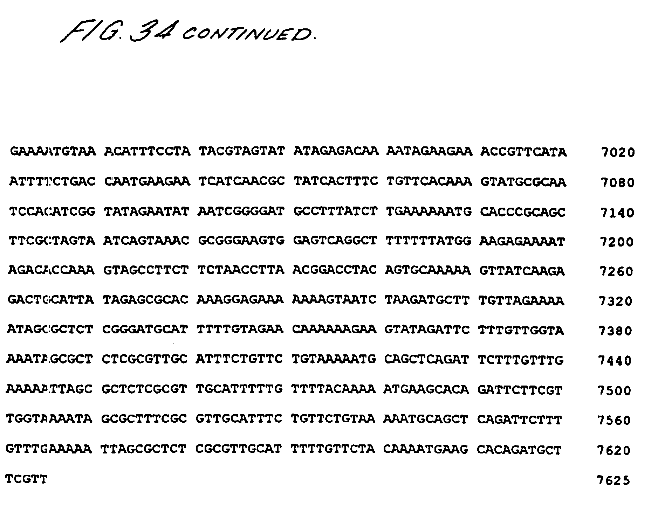

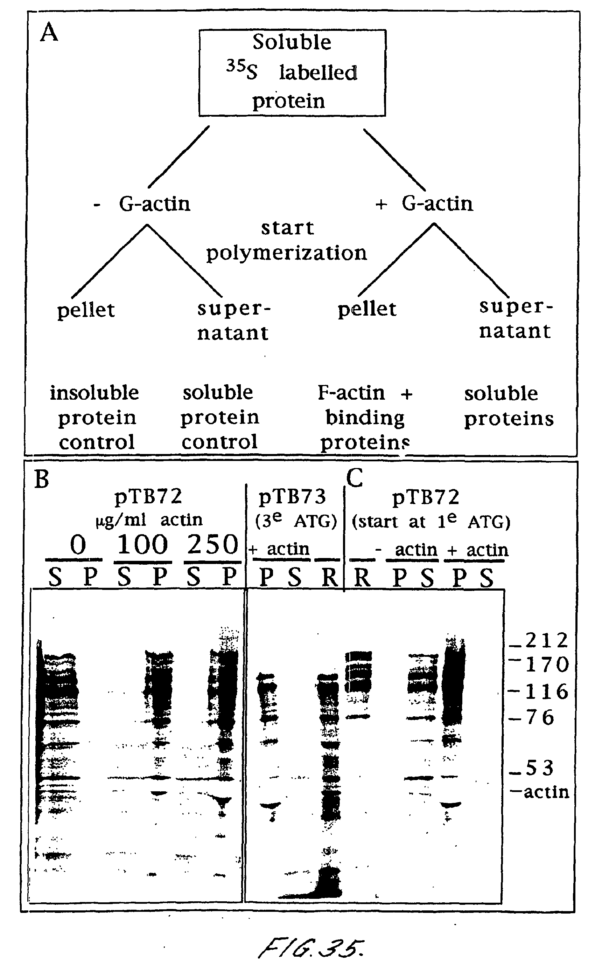

Figure 35A illustrates a flowchart of the actin co-sedimentation assay. Soluble UNC53 protein was incubated with monomeric G-actin in a buffer containing ATP. Polymerization of G-actin to F-actin was induced by increasing the salt concentration to 100 mM, F-actin protein complexes were collected by centrifugation and analyzed by SDS-PAGE and fluorography.

Figure 35(B) illustrates the concentration series of the actin co-sedimentation assay. The full length UNC-53 encoding cDNA (pTB72) was transcribed and translated *in vitro* and co-sedimented with F-actin at a starting G-actin concentrations ranging from 0 to 250 mg/ml. See methods for details. S=supernatant after airfuging. P=pellet after airfuging.

Figure 35(C) illustrates both the full length (pTB72) and amino terminal deleted UNC53 (pTB73) protein co-sediment with F-actin. Starting G-actin concentration was 500 mg/ml. S=supernatant, P=pellet, R= starting *in vitro* reaction.

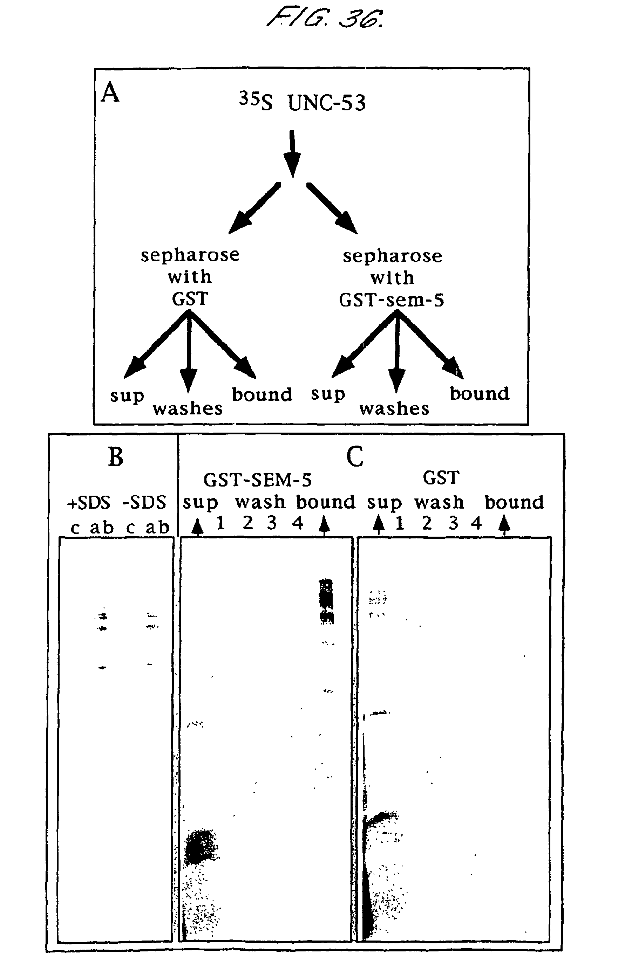

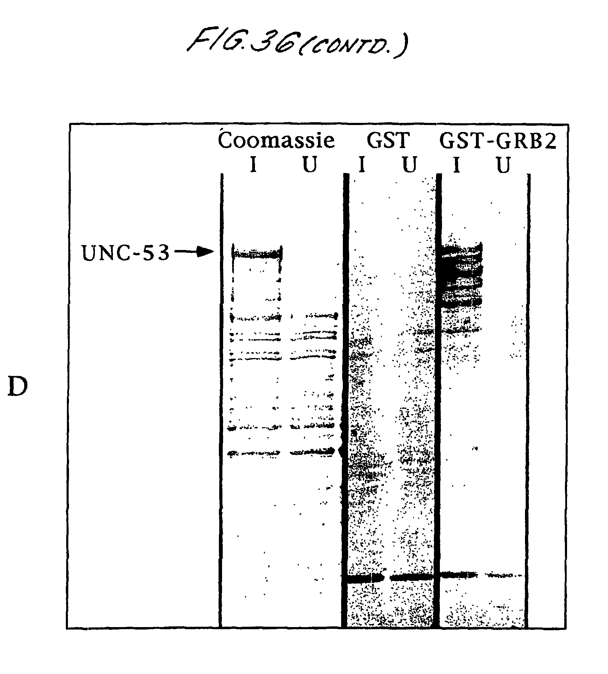

Figure 36(A) is a flowchart of a SEM-5 binding experiment. The truncated UNC53 cDNA (pTB50) was transcribed and translated *in vitro* and incubated with SEM5-GST sepharose or GST sepharose. After four washes, the remaining proteins bound to the matrix were analyzed by SDS-PAGE and fluorography.

Figure 36(B) illustrates an immunoprecipitation experiment of radioactively labelled UNC53 proteins from the TnT pTB50 reaction shows that monoclonal antibody 16-48-2 recognizes both the native (-SDS lanes) and denatured (+SDS) protein products *in vitro.* c=control reaction without anti-UNC53 monoclonal antibody 16-48-2. ab=reaction with monoclonal antibody 16-48-2. See methods for details.

Figure 36(C) illustrates the results of SEM-5-GST binding experiments outlined in (a). *In vitro* translated UNC53 protein were analyzed by SDS-PAGE and fluorography. See methods for details. sup=supernatant

Figure 36(D) illustrates a western blot overlay experiment of UNC-53 (construct pTB61) expressed in bacterial cells. Cell lysates were denatured in Laemmli buffer and the proteins separated by 5-25% gradient SDS-PAGE. The arrowhead indicates the presence of full length UNC-53 in the induced bacterial lysate. Additional gels were blotted to nylon membrane, incubated with biotinylated GST or biotinylated GST-GRB2 protein and bound protein complexes subsequently detected with a streptavidin-alkaline phosphatase conjugated antibody. See methods for details. U=uninduced bacterial cell lysate, I=induced bacterial cell lysate.

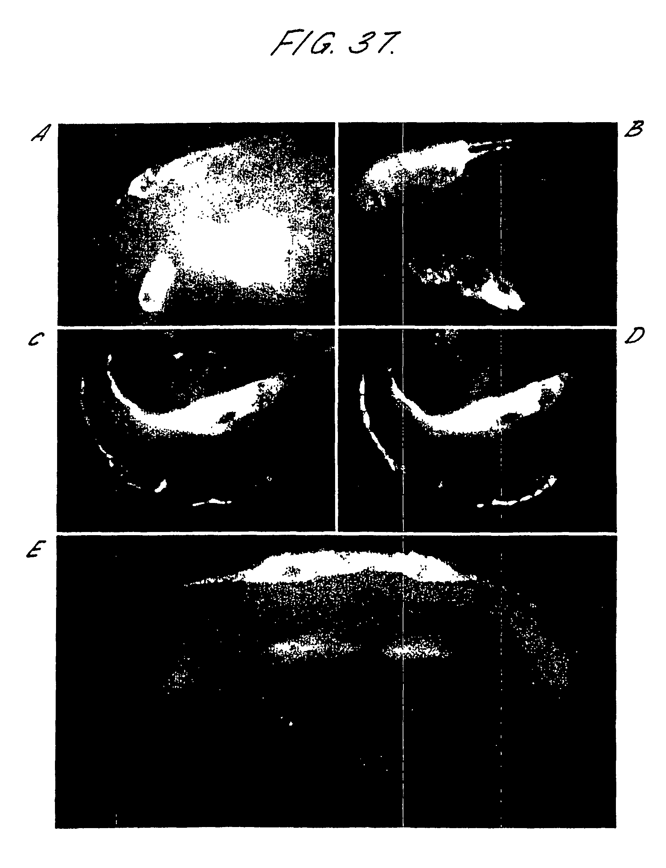

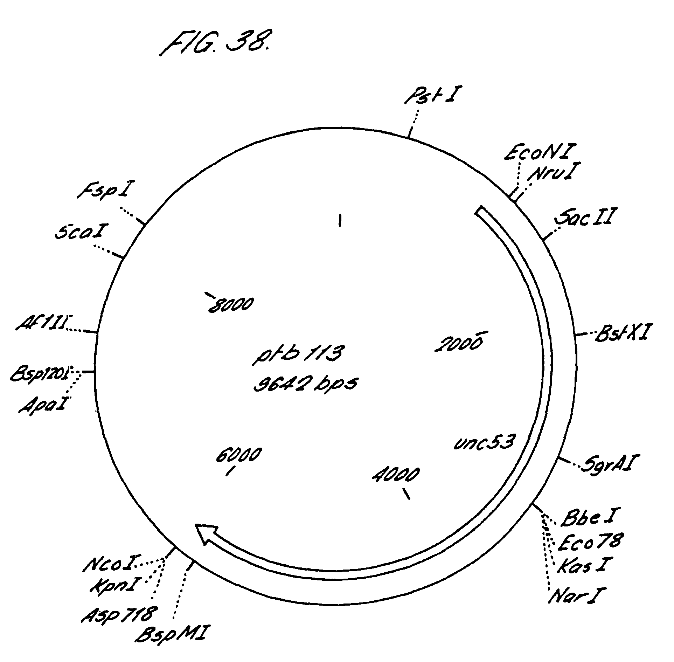

Figure 37 is a series of photographs of C. elegans which illustrates overexpression of UNC-53 in body muscle cells results in over-extension along the longitudinal axis. Transgenic C. elegans embryos carrying the construct pTB113 were analyzed for UNC-53 activity by immunohistochemistry with the 16-48-2 antibody. Starting from the photograph (a) of the top left panel of Figure 37.

(A) and (B) illustrate ectopic growth cone spikes (indicated by the arrowheads) are observed early in myogenesis in the comma stage embryo. (C) and (D) illustrate over-extension of muscle cells in the head region of a three fold embryo during outgrowth. (E) illustrates over-extension is clearly observed along the anterior-posterior axis (indicated by the arrowheads) of a late 3 fold embryo.

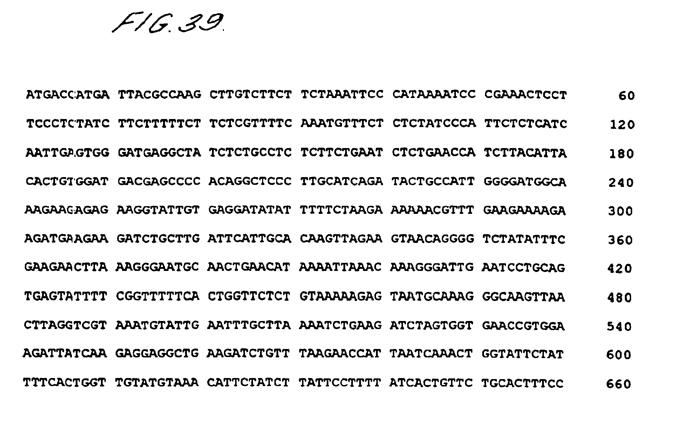

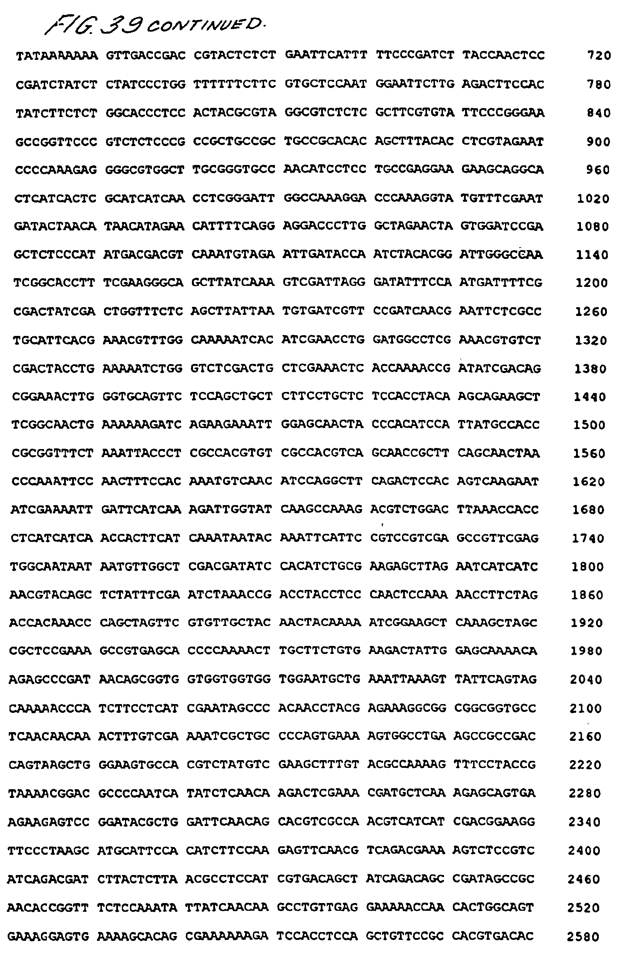

Figure 38 is a map of plasmid ptb113.

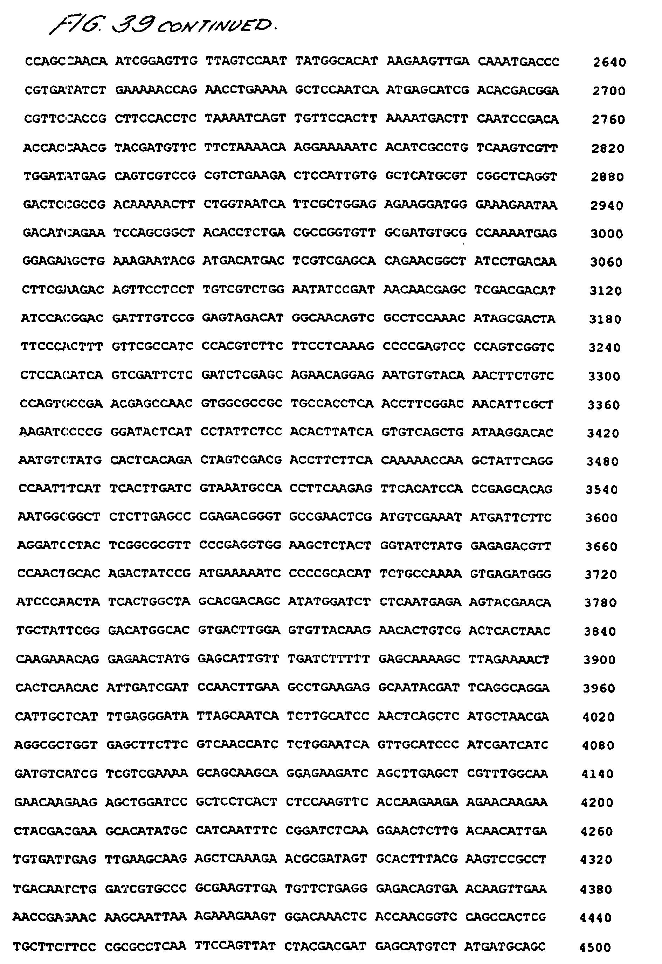

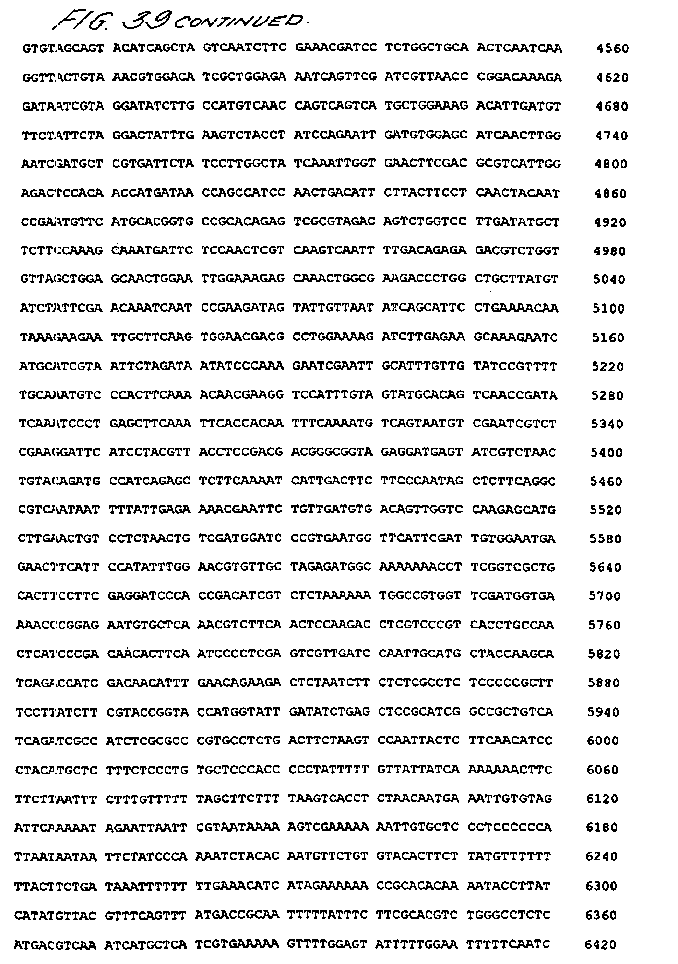

Figure 39 is a nucleotide sequence of the plasmid ptb113 of Figure 38.

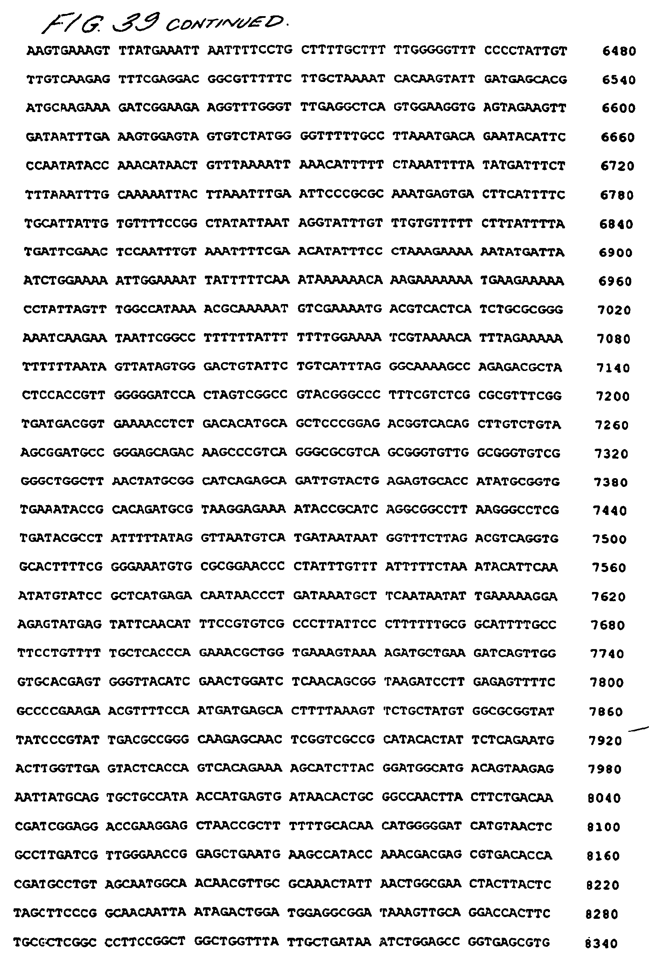

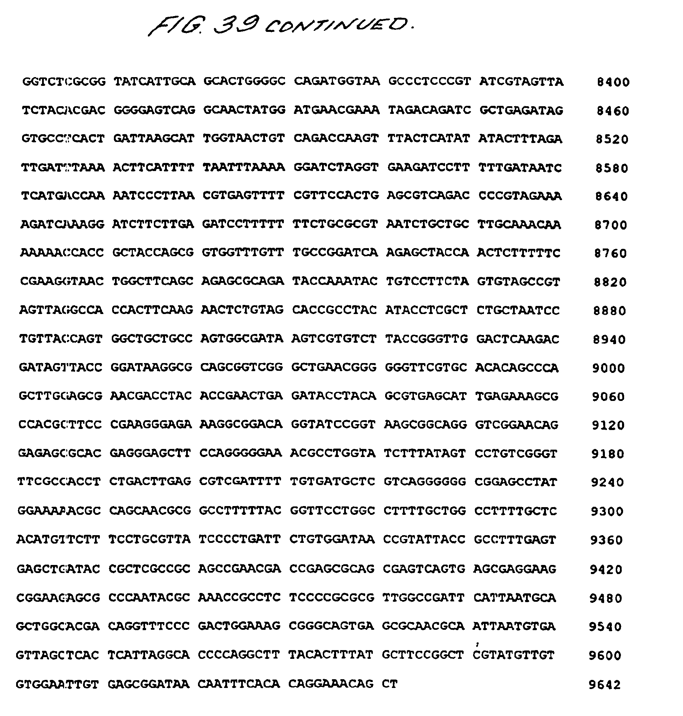

Figure 40 illustrates neurite tree length and fraction positive cells enhancement in a transfected cell C9 compared to wild-type cells C0. Black bars indicate fraction positive cells whereas hatched bars indicate neurite tree length cells, as described in example 8.

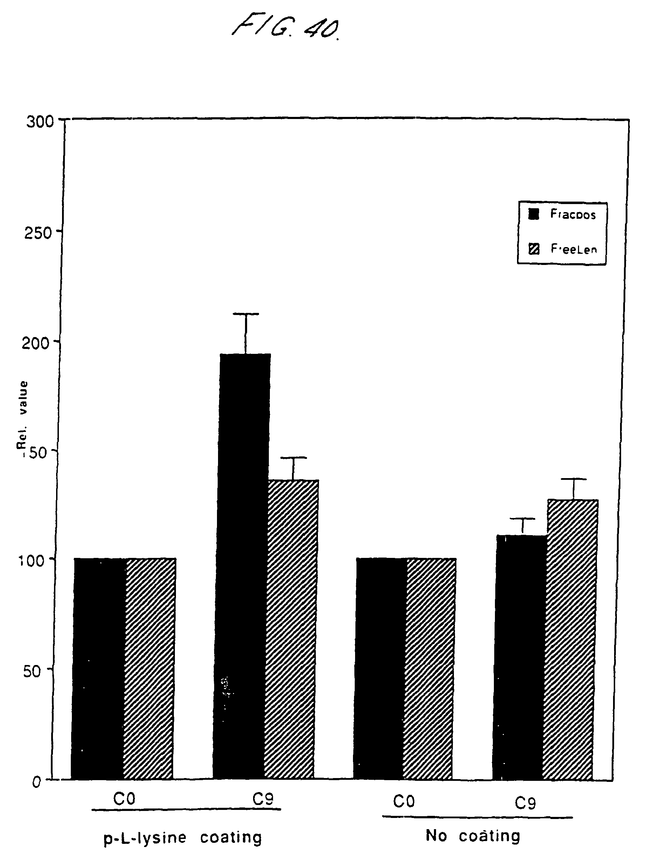

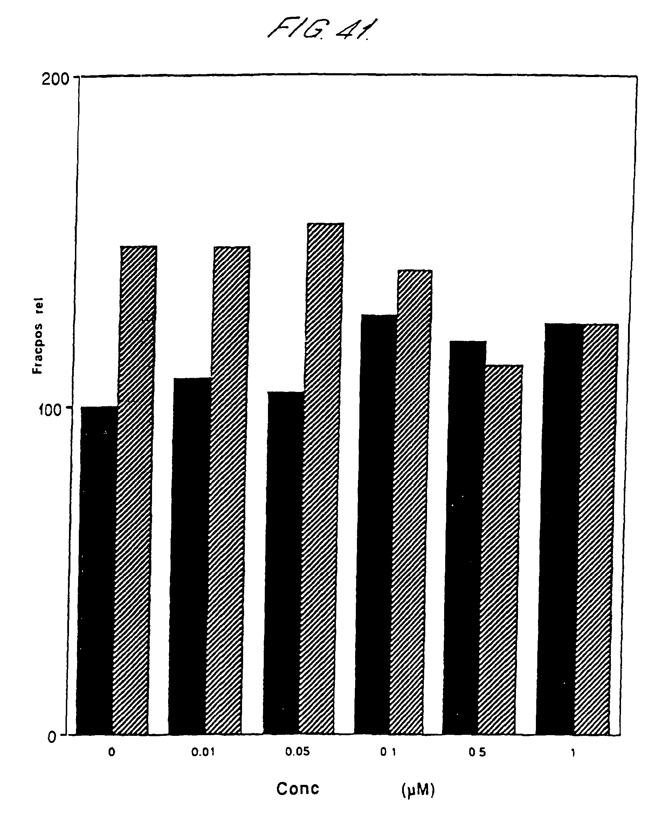

Figure 41 illustrates the results obtained following application of compound (I-(IH-pyrrol-2-ylmethyl)-2-piperidinone) to N4 transfected cells. The dark coloured bars indicate fraction positive CO clones whereas the hatched bars of the chart indicate fraction positive C9 clones.

The following sequence listings are referred to in the specification.

Sequence ID No 1: is a nucleic acid sequence corresponding to the 7A nucleic acid sequence variant of Figure 2.

Sequence 1D No 2: is a nucleic acid sequence corresponding to the 8A nucleic acid sequence variant of figure 1.

Sequence 1D No 3: is an amino acid sequence corresponding to the amino acid sequence of the 8A variant of figure 3.

Sequence 1D No 4: is an amino acid sequence corresponding to the amino acid sequence of the 7A variant of figure 2.

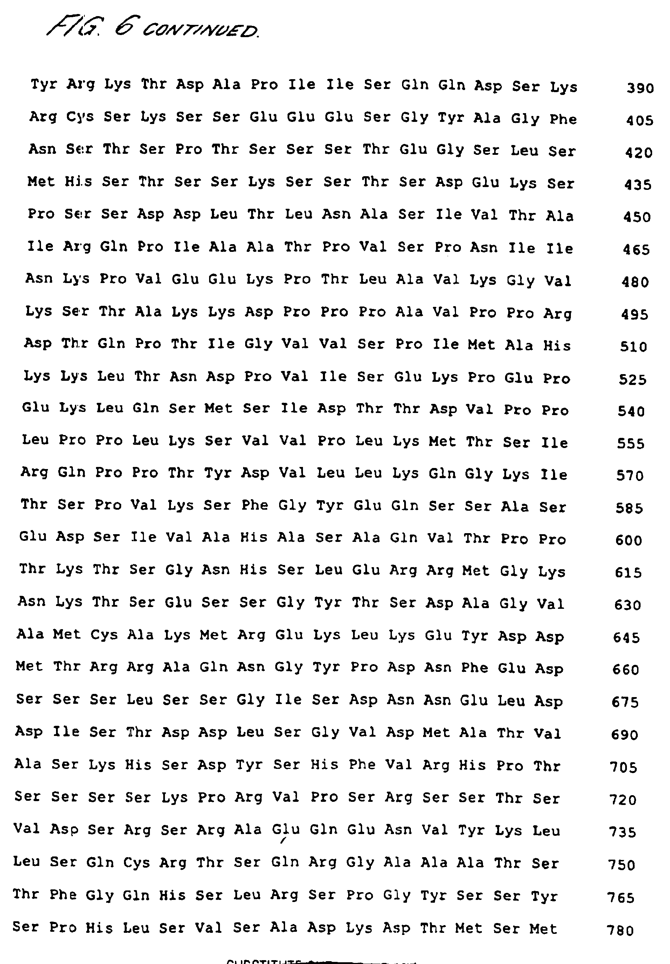

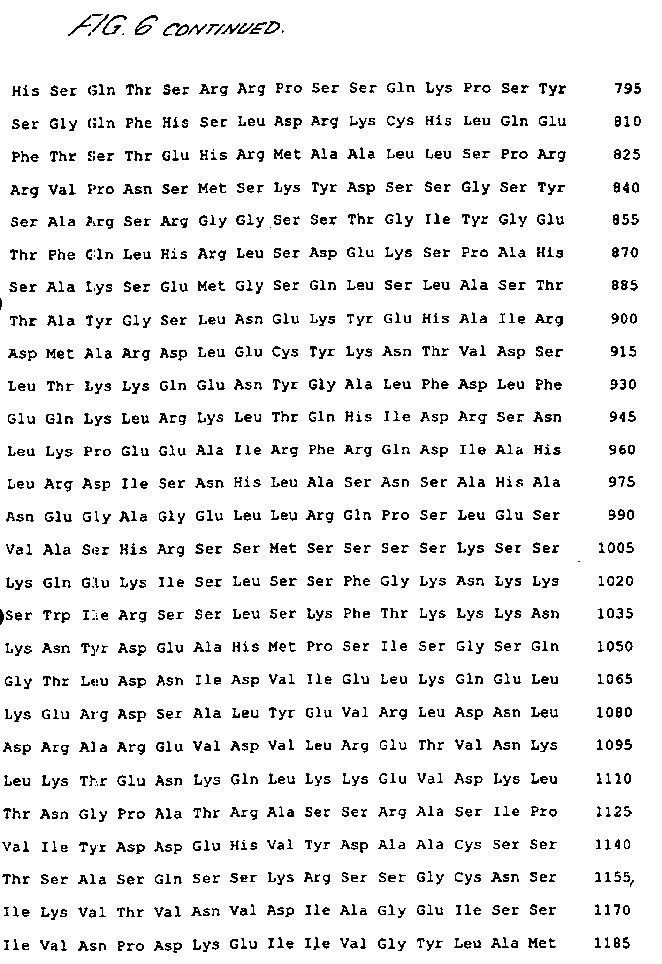

Sequence 1D No 5: is an amino acid corresponding to the amino acid sequence shown in figure 7.

Sequence ID No 6: is a nucleic acid sequence of the oligo BG03 sequence of figure 12.

Sequence 1D No 7: nucleic acid sequence of the oligo BG01 sequence of figure 12.

Sequence ID No 8: is a nucleic acid sequence of the oligo BG02 sequence of figure 12.

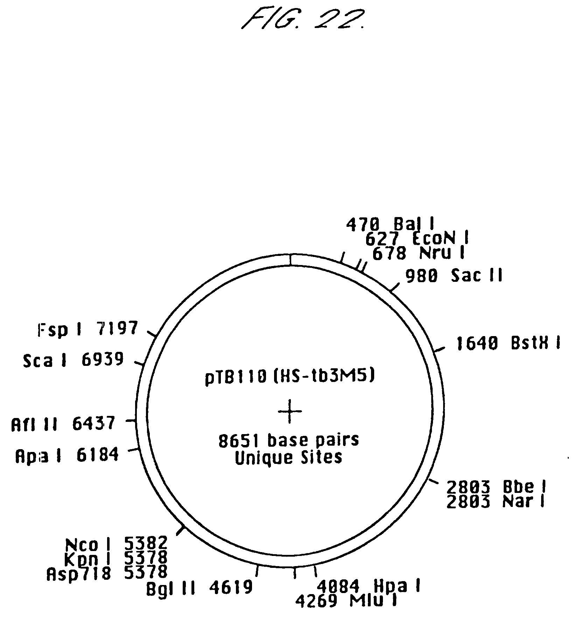

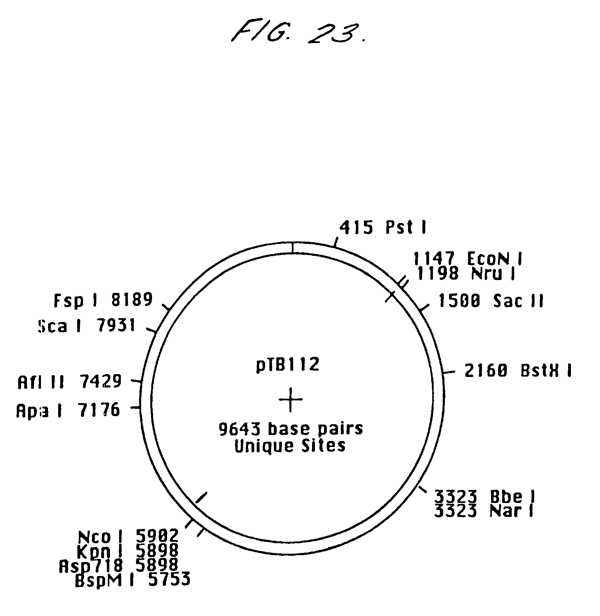

Sequence 1D No 9: is an amino acid sequence corresponding to the amino acid UNC-53(a) sequence shown in figure 17.

Sequence ID No 10: is an amino acid sequence corresponding to amino acid sequence of sequence (b) of UNC-53 shown in figure 17.

Sequence ID No 11: is an amino acid sequence corresponding to the sequence (c) of an SOS shown in figure 17.

Sequence ID No 12: is an amino acid sequence corresponding to the sequence (d) of an SOS shown in figure 17.

Sequence ID No 13: is an amino acid sequence corresponding to the sequence (d) of an SOS shown in figure 17.

Sequence ID No 14: is an amino acid sequence corresponding to the sequence (f) of SOS 1359 shown in figure 17.

Sequence ID No 15: is an amino acid sequence corresponding to the sequence (g) of SOS 1377 shown in figure 17.

Sequence ID No 16: is an amino acid sequence corresponding to the sequence (h) of Dynamin shown in figure 17.

Sequence ID No 17: is an amino acid sequence corresponding to the sequence (i) of dynamin shown in figure 17.

Sequence ID No 18: is an amino acid sequence corresponding to the sequence (j) of PI3K p85 shown in figure 17.

Sequence ID No 19: is an amino acid sequence corresponding to the sequence (k) of P13k p85 shown in figure 17.

Sequence ID NO 20: is an amino acid sequence corresponding to the sequence (l) of AFAP-110 shown in figure 17.

Sequence No 21: is an amino acid sequence corresponding to the sequence (m) of AFAP-110 shown in figure 17.

Sequence No 22: is an amino acid sequence corresponding to the sequence (n) of 3BP-1 shown in figure 17.

Sequence ID No 23: is an amino acid sequence corresponding to the sequence (o) of 3BP-1 shown in figure 17.

Sequence ID No 24: is an amino acid sequence which corresponds to the amino acid sequence from positions 106 to 133 of UNC-53 shown in figure 16.

Sequence ID No 25: is an amino acid sequence which corresponds to the amino acid sequence from positions 1093 to 1120 of UNC-53 shown in figure 16.

Sequence ID No 26: is a nucleotide sequence corresponding to the nucleotide sequence of ptB72 shown in figure 26.

Sequence ID No 27: is a nucleotide sequence corresponding to the nucleotide sequence of ptB73 shown in figure 28.

Sequence ID No 28: is a nucleotide sequence corresponding to the nucleotide sequence of pCB50 shown in figure 30.

Sequence ID No 29: is a nucleotide sequence corresponding to the nucleotide sequence of pCB51 shown in figure 32.

Sequence ID No 30: is a nucleotide sequence corresponding to the sequence of pCB55 shown in figure 34.

Sequence ID No 31: is a nucleotide sequence corresponding to the nucleotide sequence of ptb113 shown in figure 39.

Sequence ID No 32: is an amino acid sequence corresponding to the amino acid sequence as numbered from amino acid 1 to 110 of the sequence figure 3.

Sequence ID No 33: is an amino acid sequence corresponding to the sequence as numbered from amino acid sequence 114 to 133 of the sequence of figure 3.

Sequence ID No 34: is an amino acid sequence corresponding to the sequence as numbered from amino acid sequence 487 to 495 of the sequence of figure 3.

Sequence ID No 35: is an amino acid sequence corresponding to the sequence as numbered from amino acid sequence 537 to 545 of the sequence of figure 3.

Sequence ID No 36: is an amino acid sequence corresponding to the sequence as numbered from amino acid sequence 1032 to 1037 of the sequence of figure 3.

Sequence ID No 37: is an amino acid sequence corresponding to the sequence as numbered from amino acid sequence 1097 to 1116 of the sequence of figure 3.

Sequence ID No 38: is an amino acid sequenc ecorresponding to the sequence as numbered from amino acid sequence 1300 to 1307 of the sequence shown in figure 3.

Sequence ID No 39: is an amino acid sequence corresponding to the amino acid sequence (a) of ∝-actinin (aact) shown in figure 15.

Sequence ID No 40: is an amino acid sequence corresponding to the amino acid sequence (b) of unc-53 shown in figure 15.

Sequence ID No 41: is an amino acid sequence corresponding to the amino acid sequence (c) of β-spectrin (spectrin) shown in figure 15.

Sequence ID No 42: is an amino acid sequence corresponding to the amino acid sequence (d) of ∝actinin (aact) shown in figure 15.

Sequence ID No 43: is an amino acid sequence corresponding to the amino acid sequence (e) of UNC-53 shown in figure 15.

Sequence ID No 44: is a amino acid sequence corresponding to the amino acid sequence (f) of β-spectrin (spectrin) shown in figure 15.

Sequence ID No 45: is an amino acid sequence corresponding to the amino acid sequence (g) of ∝-actinin shown in figure 15.

Sequence ID No 46: is an amino acid sequence corresponding to the amino acid sequence (h) of UNC-53 shown in figure 15.

Sequence ID No 47: is an amino acid sequence corresponding to the amino acid sequence (I) of β-spectrin shown in figure 15.

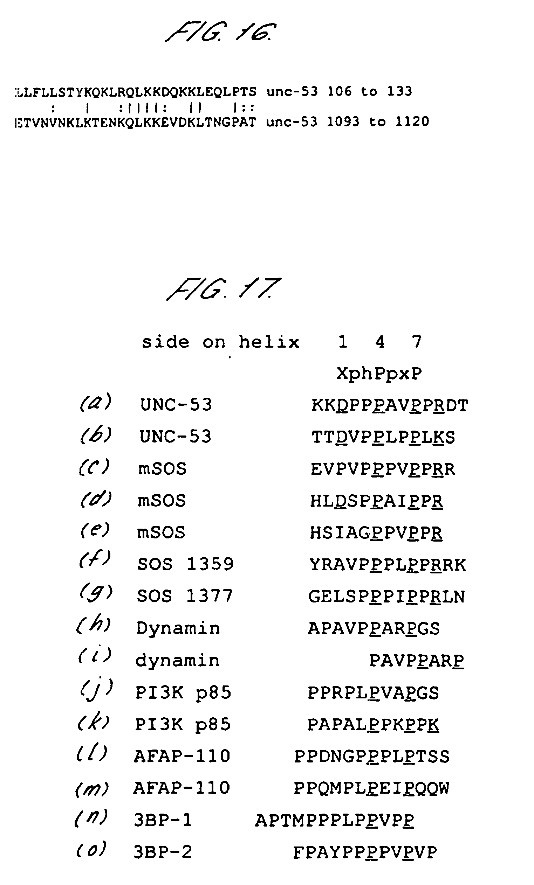

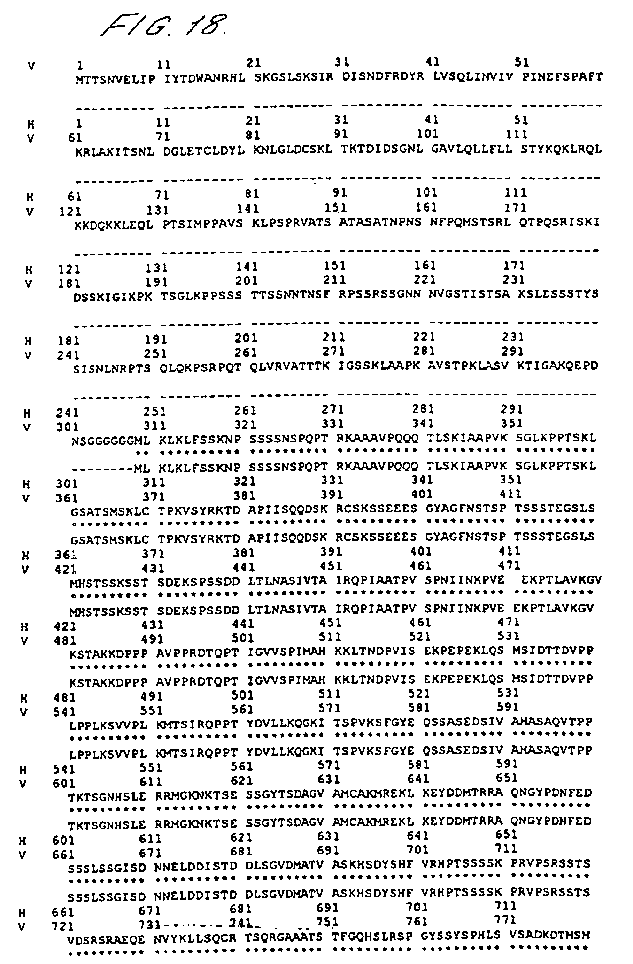

Sequence ID No 48: is a nucleotide sequence corresponding to the nucleotide sequence of S4 lambda clone shown in figure 14(c).

The inventors have established a set of processes particularly in C. elegans to select for inhibitors or enhancers of UNC-53. This screen is based on transgenic or mutant organisms or cells in which we have introduced a nucleic acid sequence encoding UNC-53 under the control of a specific promoter. In these organisms UNC-53 is over-stimulated as judged by increased extension of growth cones of muscle cells which over-express UNC-53 in C. elegans. This leads to a range of phenotypes in both embryonic and postembryonic development(from death to defective morphology and motility). These phenotypes can be scored with simple means at high throughput. Similar results can be obtained with heat shock specific lines. The basis of our test for inhibitors of the UNC-53 signal transduction pathway is reversal of this phenotype to an improved state of health.

We have constructed transgenic strains of C. elegans which over-express UNC-53 in body muscle. This results in increased extension of muscle cells and embryonic lethality (17 to 80% of transgenic organisms depending on the line used). These strains are used to directly screen for drugs which interfere with unc-53 genes, UNC-53 protein activity or any regulatory factor thereof to thereby suppress the background lethality.

Another process which may be used for selecting inhibitors or enhancers of UNC-53 uses a constitutively active unc-53. This is achieved by mutating the nucleotide binding domain such that GTP or ATP is always bound or by covalently attaching SEM-5. In this strategy, transgenics (tissue cultured cell lines, or organisms such as nematodes) are generated which maintain unc-53 in a higher endogenous level of activity. Over-extension and subsequent lethality results in a greater proportion than that observed in the UNC-54/UNC-53 wild type lines. By screening for survivors after drug treatment, this assay specifically identifies inhibitors of downstream components in the signal transduction pathway.

Another process utilises an UNC-53 promoter. In this approach, an UNC-53 promoter is fused to a nucleic acid sequence encoding a reporter molecule, for example green fluorescent protein (GFP). Cells will glow when trans-acting factors bind to the promoter to activate transcription. By screening for cells which do not fluoresce, molecules which inhibit transcription of UNC-53 are identified.

The processes for selecting inhibitors and/or enhancers according to the invention are preferably carried out on whole animals. This can be done using a C. elegans system. The advantages of these tests include:
(1) The screening in a whole animal assay. C. elegans is a complex multicellular organism with a full nervous system, digestive system, etc. Its anatomy and development has been described in extreme detail. It is one of the best-characterised higher organisms at the genetic, molecular, developmental and cell biological level. Any observed changes to phenotype can be checked against this database.
(2) To study effects on rate and directionality of cell migration and the change of cell shape it is important to leave the cells under study in a setting where they are surrounded by the in vivo interacting tissues, cells and substrates for cell migration etc. This can be done using whole C. elegans subjects. A situation has been created where the given pathway is over-stimulated leading to an easily scorable phenotype which can be reverted in any assay or process.
(3) The endpoint of the screen is the substantially increased health of the organism. This permits the exclusion of non-specific and toxic compounds.
(4) A complete and specific inhibition of UNC-53 in the transgenics will lead at the worst to the phenotype of an UNC-53 reduction or loss of function mutant which we have described, can recognise and have shown not to be essential for viability.
(5) The test can be adapted to make full use of the advantages of the C. elegans model system such as the possibility to conduct the test chronically over several generations and the possibility to conduct the test in different genetic backgrounds, e.g. RTK constitutive or defective.
(6) C. elegans exhibits a complex set of wild type, drug- and mutation-induced phenotypes such as changes in body shape, subtle changes in locomotion, mating behaviour, chemotaxis, pharyngeal pumping, egg laying behaviour, which can be used as part of a phenotype analysis or screen.

The results of C. elegans research described herein has provided important breakthroughs in biomedical research fields such as programmed cell death, neuronal guidance, the Receptor Tyrosine Kinase/RAS signal transduction pathway, integrin/cell adhesion receptor signalling, etc.,

The biochemical association of UNC-53 in the RTK signal transduction pathway enables identification of genes or of biochemical pathways which are targets for pharmacologically or pharmaceutically active compounds and the development of high throughput and mode of action specific drug screens using wild type, mutant and transgenic animal strains including, in particular, C. elegans.

Thus pharmacological manipulation of the UNC-53 pathway is now possible on the following rationale:

We have scientific arguments to expect C. elegans UNC-53 to interact in vivo with the other components of RTK signal transduction pathways based on:
(1) The observation that C. elegans SEM-5 and GRB-2 are mutually exchangeable in vivo, combined with our observed in vitro binding of both GRB-2 and SEM-5 to UNC-53. Thus, C. elegans UNC-53 will be able to interact with the activated GRB-2/RTK receptor in mammalian cells.
(2) UNC-53 interacts with the rabbit actin-cytoskeleton

Expression of C. elegans UNC-53 in mammalian cell lines represents a shortcut to develop pharmacological assays and screens to target this pathway. We have shown that over-expression of the C. elegans UNC-53 in C. elegans myoblasts leads to over-extension of these cells in the anterior/posterior axis of the embryo and ultimate disorganisation of the muscle cell and myofilament pattern. (Over)-expression of C. elegans UNC-53 in a human cell line leads to a detectable change in phenotype, in particular increased motility of cells, increased outgrowth of neurons and morphological changes in the elongation and cytoskeletal morphology of differentiating myotubes.

The C. elegans unc-53 Open Reading Frame (ORF) (with and without optimised Kozak consensus sequence) of both 7A and 8A clone variants has been cloned between the CMV major intermediate early promoter/enhancer and bovine growth hormone polyA signal sequence of expression vector pcDNA3 (Invitrogen). This vector is designed for high level stable and transient expression in most mammalian cells.

The following additional considerations require mention:
(1) Genetic analysis of reduction in UNC-53 function and ectopic expression experiments suggest that UNC-53 acts in a highly dosage-dependent manner. As is the case for RAS, increased expression may lead to lowering the threshold of RTK-signal required for a given response or may remove the requirement for an activating signal to obtain a phenotype response (Fig 10). In addition UNC-53 is an unusually low abundance protein in wild type C. elegans. It is therefore likely to be necessary or useful to control the temporal and quantitative expression of UNC-53 in the proposed assay conditions in all organisms or cells to be assayed. The already available or a further optimised expression cassette is then cloned in expression vectors with IPTG- inducible or tetracycline-repressible promoters. It is realised that both the Lac and Tet expression systems are leaky. Additional other repressible/inducible expression systems (e.g. Mx promoter) or weak mammalian promoters might be preferred.
(2) Over-expression of the endocytosis controlling protein dynamin leads to phenotypes which are not associated with dynamin function in the cell but which are thought to be due to sequestration of the GRB-2 pool in the cell (GRB-2 is an adaptor for a variety of signal transduction pathways). Such sequestration is unlikely to lead to "positive effects" on the activity of the cell such as is observed in the presently described assay system (increased cell process extension or motility), see Fig 19. Based on the homology between UNC-53 and GTP-binding, we can also predict specific mutations in the nucleotide-binding pocket or the predicted effector region which should lead to loss of function. Sequence analysis of unc-53 alleles is instructive in determining which amino acids of UNC-53 are essential for function, e.g. as exemplified by the indication that an allele (n152) which has a differential effect on anterior versus posterior guidance has a deletion in a region of differential splicing. The differential splices of the C. elegans unc-53 gene encode different variants of the protein which independently affect posterior or anterior migration and/or cell specificity. One predicted exon in C. elegans unc-53 is indicated in Fig 1. It is conceivable that of two variants of the same protein one is inhibited or enhanced by a particular compound whereas the other is not (or to a lesser degree). Such a compound could then be used to control direction of migration or cell specificity by selective inhibition or enhancement.
(3) To develop pharmacological screens for inhibitors of a biochemical pathway a "gain of function" phenotype has been invented which can be expected to revert to wild type in the presence of specific inhibitors. Overexpression of UNC-53 in C. elegans myoblasts already leads to lethal subviable muscle phenotypes which can be easily scored with high throughput or a scorable heat shock inducible phenotype (Fig 21). They may form the basis for a pharmacological screen for inhibitors. A similar screen is obtained for over-expressing UNC-53 in mammalian cells. An alternative strategy is based on the homology to GTP binding proteins, RAS and dynamin and NTPases. We can introduce amino-acid changes in the nucleotide binding pocket which are predicted/expected to lead to a constitutively activated or inactivated UNC-53. Similar changes are based on homologies with SOS, dynamin or ATP/GTP binding proteins from homology tables.
(4) Correct expression of UNC-53 in each cell line may be assessed by immunofluorescence and western blot analysis with the monoclonal antibody (mab) designated as 16-48-2.

The inventors have thus expressed and stably integrate the expression constructs in the neuronal, myoblast and 3T3 cell lines.

These cell lines are primarily used to:
- Assess the effect of UNC-53 expression on the morphology, motility, metastatic potential and growth cone extension of the cell lines.
- Produce protein and mRNA
- Screen for pharmacological compounds inhibiting observed UNC-53 mediated phenotypes
- Analyse signal transduction pathways associated with UNC-53 activation (for example, phosphorylation,)
- Immunofluorescence studies with mab 16-48-2 to assess changes in subcellular localisation following growth factor treatment.

Thus, the present invention provides for the identification of compounds which inhibit or enhance the UNC-53 signal transduction pathway. Such compounds can be used in the control of cell directional migration, motility and differentiation. These compounds are useful in the treatment of oncogenesis, psoriasis, neuronal degeneration and cell migration (metastasis).

The present invention also provides the ability to identify nucleic acid sequences and proteins which are involved in the UNC-53 pathway in C. elegans. Such nucleic acid sequences and proteins may be UNC-53 equivalents, members of an UNC-53 pathway or may be nucleic acid sequences or proteins which interact in the UNC-53 pathway, for example as demonstrated by the GRB-2/SEM-5 proteins. This knowledge of the UNC-53 pathway in C. elegans can be established as can factors which influence the functioning of the pathway, for example, factors/ proteins which feed into the pathway or are of a parallel pathway which at least, in vitro, compensates for steps in an UNC-53 pathway.

The identification of other components in the UNC-53 signal transduction pathway:
(1) help to determine the interaction of UNC-53 with known signal transduction pathways (RAC-, RHO-, cdc42-RAS-pathway exchange factors, downstream or regulating kinases)
(2) identify the new interacting proteins which may constitute additional potential pharmacological targets.
(3) may assign functions to the more than 1000 amino acids of UNC-53 which have no homology to known proteins.

Accordingly, proteins which cross-react with anti-C. elegans UNC-53 protein antibodies can be isolated. The basic experiment protocol for purifying antigen-antibody complexes is described in Example 11. This system can also be used to identify factors which interact with proteins which bind to anti-UNC-53 C. elegans antibodies.

The following tissue sources may be used for immuno-precipitation:
(1) Mammalian cells which exhibit a phenotype after transfection with unc-53 indicating that it interacts with vertebrate components of its signal transduction pathway.
(2) UNC-53 protein may be too low abundance to make affinity purification from wild type C. elegans feasible. The inventors have affinity-purified UNC-53 from already constructed transgenic C. elegans lines which express UNC-53 under control of the hsp-16 promoter and/or the myosin promoter. These experiments in C. elegans are justified because with the vast amount of sequence information (genomic and cDNA) available, one has a good chance of identifying the corresponding genes in the databases with a minimum of peptide sequence.

Several types of proteins may be expected to co-purify with UNC-53, including GRB-2 and other proteins with SH3 domains of the Grb2 class or phosphorylation sites, RTK-receptors, subunits of an UNC-53 homo-heterodimer complex, downstream regulating kinases or proteins from the microfilament cytoskeleton.

This co-immuno-precipitation approach can also be used to dissect the order of events in this signal transduction pathway. For example: UNC-53 immuno-purified after stimulation of mammalian cell-lines with growth factors and pharmacological agents can also be assayed with respect to its state of phosphorylation, or complex formation with interacting proteins.

Proteins interacting with specific UNC-53 domains are identified using a yeast two-hybrid system, whereby two sets of hybrid proteins are used to assay for functional restoration of the GAL4 transcriptional activator: the first consisting of a GAL4 activation domain/UNC-53 structural domain of unknown function, the second derived from a cDNA library cloned into an expression vector to generate a library of hybrid proteins containing a GAL4 DNA binding domain. The yeast two-hybrid system is well know in the art.

A set of unc-53-fusion constructs can be constructed, including a fusion to
(1) the full length protein,
(2) the carboxyterminal domain (from second actin binding domain to the ATP/GTP binding domain),
(3) The aminoterminus (predicted cortical localisation domain up to the SH3 binding sites),
(4) a variety of overlapping constructs within the central domain of 1000 amino acids to which no function can as yet be assigned.

These are tested in yeast to exclude those which lead to activation of the reporter gene in the absence of the cDNA-activator fusion. cDNA libraries were transformed into these reporter strains and positive clones identified. (In this strategy, screening of multiple libraries requires very little effort (transformation followed by plating on selective and indicator medium)).

A preferred cDNA library is from cell lines in which a phenotypic change is observed following UNC-53 expression such as mouse N4 neuroblastoma cells or MCF-7 breast carcinoma cells. The yeast two hybrid system can identify interacting proteins or "sections" of nucleic acid which may not be translated in vivo but which may inhibit UNC-53.

Candidate positives are tested for the fusion-protein dependence of the reporter gene activation. The cDNA insert in remaining positive clones is sequenced. The obtained sequence is screened through the databases, which provides, especially in the case of C. elegans clones, significant extra sequence.

Another system also exists for the identification of proteins which bind or modify UNC-53. An UNC-53 protein is bound by conventional techniques to a column. A sample to be tested is then passed over the column. This sample may be fractions from cells from C.elegans, mammals or any other organism. These sample fractions may have been incubated with ³²ATP. In this course the "reaction" of the labelled fraction with UNC-53 can be determined. If the UNC-53 on the column becomes ³²P phosphorylated then this indicates that the sample fraction contains an UNC-53 modifying protein. Alternatively a constituent of the sample may bind to the UNC-53 and remain bound therewith on the column. The retention of any fraction of the sample on the column and the identification of the fraction can easily be determined by techniques known in the art.

Example 9 describes the identification of sensitive, dependant or resistant mutations as direct tools for the development of screens for compounds with similar or antagonistic activities. Both resistant and sensitising mutations may have a phenotype in the absence of the compound and no or a different phenotype in the presence of the compound. This permits the introduction of action-specificity in the screens.

High throughput screens are a basic feature of C. elegans genetic methodology. Non-complementation screens for new alleles in a locus require setting up of up to 8000 separate worm populations starting from one hand-picked individual each. This is done in 24 well plates or small Petri-plates. These are subsequently (after 1 or 2 generations) visually screened for a complex behavioural phenotype. For pharmacological screens where populations can be started from multiple individuals pipetted from a pool of synchronised eggs, high throughput screens can also be developed. If the endpoint of the assay can be scored in liquid, populations can be set up in microtitreplates. If the end-point is linked to a reporter gene (e.g. β-galactosidase activity) ELISA type colour-metric assays can be used to score the end-point. C. elegans can also be introduced into soils, exposed to compounds and subsequently recovered and assayed. Such endpoints are used in the heat-shock assay developed by Stressgen (Stringham & Candido (1994), Environ. Toxicology and Chemistry, 13(8), 1211-1220).

Gain of function mutants of C. elegans or transgenic C. elegans in which a pathway of interest has been over- or constitutively activated, causing a dominant phenotype which can be used to develop specific screens for inhibitors.

Transgenic lines expressing UNC-53 ectopically under the C. elegans heat-shock (hsp-16) promoter, and body wall muscle (unc-54) promoter have been constructed. These lines lead to dominant phenotypes in development and are used directly to screen a spectrum of compounds. Where necessary or deemed useful endogenous C. elegans genes can be replaced by or complemented with human signal transduction pathways.

**DEPOSITED CELL LINES AND PLASMIDS**

| STRAIN NAME | DATE OF DEPOSIT | LMBP ACCESSION NUMBER |
|---|---|---|
| pTB54 Plasmid | 22 MAY 1995 | 3296 |
| pTB112 Plasmid | 22 MAY 1995 | 3295 |
| pTB72 | 22 MAY 1996 | 3486 |
| TB4EX25 Cell Line | 22 MAY 1995 | 1384 CB |
| TBAIn76 Cell Line | 22 MAY 1995 | 1385 CB |
| HYBRIDOMA Cell Line | 22 MAY 1995 | 1383 CB |
| MCF-7 TRANSFECTED BREAST CARCINOMA | | |
| CELL LINE | 24 MAY 1996 | 1550 CB |
| TRANSFECTED N4 NEUROBLASTOMA CELL LINE | 24 MAY 1996 | 1549 CB |
| WILD TYPE MCF-7 BREAST CARCINOMA CELL LINE | 24 MAY 1996 | 1551 CB |

The above plasmids and cell-lines were deposited at the Belgian Coordinated Collections of Micro organisms (BCCM) at Laboratorium voor Moleculaire Biologie - Plasmidencollective (LMBP) B-9000, Ghent, Belgium, in accordance with the provisions of the Budapest Treaty of 28 April 1977.
The present invention will now be described with reference to the following Examples.

### Examples

### Example 1 - Molecular Characterisation of unc-53 gene in C. elegans

### Screen for muscle pattern mutants :

C. elegans has two sets of muscles which are suitable to study this problem, the body wall muscles and the sex muscles. The sex muscles are a set of 16 muscle cells (4 muscle types) in the hermaphrodite and 41 cells in the male (10 muscle types) with distinct attachments points on the hypodermis and gonads. The sex muscles develop postembryonically and are not required for viability. The body wall muscles are arranged longitudinally (roughly 2 cells abreast) into four quadrants. At birth there are 81 cells. In postembryonic development, extra muscles interdigitate with these bringing the total number of body wall muscles in the hermaphrodite to 95. Head, neck and body muscles can be distinguished within these rows on the basis of their innervation and patterning within the rows.

We have screened 4800 haploid genomes using Nomarski and polarized microscopy for mutants with specific attachment or pattern defects in a subset of the male sex muscles but with wild type body wall muscle pattern and myofilament organization, wild type movement and wild type male bursa anatomy (a sensitive indicator of wild type morphogenesis). Amongst the 21 identified mutants we selected for further study those with specific phenotypes in both the male and hermaphrodite sex muscles. As these muscles lie in different regions of the animals this was thought to reduce the chance that the male tail phenotype is a pleiotropic consequence of changes in regional identity of the tail or defects in male tail hypodermal lineage or morphogenesis.

### Muscle phenotype of e2432.

Mutant e2432 was isolated on the basis of its phenotype in the male spicule retractor muscles, a pair of bilaterally symmetrical muscles which attach anteriorly to the body wall and posteriorly to the base of the spicules. The spicule retractors of mutant e2432 are shorter than wild type. Their attachment to the spicules is wild type, but their attachment point to the body wall is shifted posteriorly. The spicule protractors sometimes extend processes onto the attachment point of the spicule retractors on the hypodermis, suggesting the defect is not in these attachment points, but rather in the extension of the muscles towards that point. The diagonal muscles are in most specimens wild type but they are occasionally not parallel to one another or are have a dorsal attachment point that is more ventrally positioned than in wild tye. e2432 males have a nicely shaped fan with the normal pattern of rays, suggesting that the sex muscle defect is not pleiotropic due to defects in the hypodermis.

e2432 hermaphrodites have a reduced ability to lay eggs which is variable from animal to animal. This is due to a muscle pattern defect in the vulval sex muscles. The uterine muscles, 8 muscle cells which circle the hermaphrodite uterus, are wild type in e2432. The vulval muscles are a set of 4 pairs of cells arranged symmetrically in a cross-pattern around the vulval slit. Each pair consists of one vm1 and one vm2 muscle cell. The vm2 muscles attach to the junction between uterus and vulva and extend anteriorly to attach to the hypodermis in between two muscle cells of the ventral body wall muscle quadrant. In e2432 these muscles are shorter than in wild type small. In e2432 they can only be visualized by laser confocal microscopy (after FITC-phalloidin staining of the myofilaments). This showed that they attached to the uterus as in wild type, but that their attachment to the body wall is ectopic (in a random position lateral of the vulva, usually on the ventral edge of the muscle row). In e2432 vm2 myofilaments are oriented more dorsoventrally than in wild type (where their orientation is essentially in the longitudinal axis of the animal). This phenotype is not due to a defect in the attachment point on the epidermis to which these cells should attach in wild type, since we frequently observe that the vm1 sex muscles make an apparently wild type attachment to this unoccupied attachment point.

In wild type hermaphrodites, the vm1 muscle cells attach close to the junction between epidermis and vulva and in the adult extend dorsally and anteriorly (under an angle of 45-50 degrees with respect of the vulval slit) to attach to the hypodermis at the dorsal edge of the ventral body wall muscle quadrants. In e2432 the attachment of the vm1 muscles to the vulva is wild type. With their other end they attach, like wild type vm1 cells, along the dorsal of the edge of the ventral body wall muscles. However the angle between the vulval slit and the myofilaments of the vm1 sex muscles is reduced (less than 45 degrees) so that their dorsal attachment point is closer to the vulva than in wild type. The forces acting on the vulva can be separated in an antero-posterior and a dorsal vector. In e2432, the antero-posterior vector of both the vm1 and vm2 muscle is significantly reduced, leading to a reduced ability to open the vulva upon contraction. Studies in which vulval muscles were ablated individually or in groups suggested that 2 vulval muscle cells of wild type orientation are sufficient for wild type function.

Adult C. elegans hermaphrodites have 95 body wall muscle cells arranged longitudinally (roughly 2 cells abreast) into four quadrants. In wild type cells these cells are spindle shaped.

e2432 adults have body wall muscles with a wild type muscle cell and myofilament pattern, except that cells with interdigitating tips occur more frequently than in wild type. Like the unc-53 phenotype in the male and hermaphrodite sex muscles, this body wall muscle defect, which can also be observed in other guidance and attachment mutants like unc-6 and mups, can also be attributed to a reduced ability to extend "growth cones" otherwise referred to as cell processes in the anterior-posterior axis of the animal.

### Position on the genetic map :

e2432 was mapped to the left arm of chromosome II and was found not to complement unc-53(e404). The unc-53 locus was originally identified by Brenner (1974), Genetics, 77, 71-94 as one of the uncoordinated mutants but has received only sporadic attention in general phenotypic surveys of the UNC-collection (Hedgecock et al (1987), Development, 100, 365-382 and Siddiqui (1990), Neurosci. Res. (Suppl) 13, 171-190, in a genome wide screen for egg laying defective mutants (Trent and Horvitz (1983), Genetics, 104, 619-647) and using e2432 as a tool to study the effect of body shape on the pattern of neuronal processes (Hekimi and Kershaw (1993), J. Neuroscience, 13(10) 4254-4271). We initiated a detailed genetic and phenotypic analysis of this locus using the existing available alleles which various colleagues isolated in different screens : The canonical unc-53 allele e404, a strong UNC was isolated by Sydney Brenner. Alleles n152, n166 and n1199 have been obtained in screens for egg laying defective mutants. Alleles NJ234 and NJ222 were isolated by Ed Hedgecock in a screen defective in excretory canal outgrowth. As these screens were aimed at isolating viable fertile alleles, we isolated additional alleles by pre-complementation screens designed to yield loss of function alleles irrespective of their phenotype. e2432/mnDf90 hermaphrodites are egl, weak unc's with a slightly stronger phenotype than e2432. Matings were set up on 3 cm petri dishes between 2 to 3 unc-53(e2432) sqt-1(sc13) /+ males and 2 e2431ts or dpy-6(el4) hermaphrodites mutagenized with EMS in the L4 stage (Brenner, 1974), Genetics, 77 71-94. The F1 egl, unc-53 like hermaphrodites, which may be unc-53(e2432) sqt-1(sc13)/unc-53(new) were cloned on petri dishes and their offspring examined for the segregation of new unc-53 alleles. In two screens, two unc-53 alleles, 5 and 8 were isolated in an estimated 13000 F1 offspring, giving an approx. mutation rate 1/3250 mutagenized chromosomes. Sqt-1(scl3), an allele of sqt-1 that confers a roller phenotype was included because it is closely linked to unc-53 (0.2 m.u.) and marks the original allele e2432. e2431ts, an X-linked ts larval lethal with a mup phenotype was included to eliminate F1 hermaphrodites arising from selfing and F1 males which can mate. In the second screen dpy-6(el4) was included to prevent F1 males from mating with F1 hermaphrodites.

All unc-53 alleles used in this study fail to complement to e2432. Complementation was tested by mating unc-53(e2432) sqt-1(sc13)/+ males to hermaphrodites of the respective alleles. The male sex muscle phenotype described above for e2432 was found to be the only 100% penetrant phenotype in the unc-53 locus (see below) and was the primary phenotype used in complementation tests. Each of these alleles was also complemented to mnDf90 by mating unc-4 mnDf90/mnC1 males to unc-53 homozygotes and temporary unc-53/unc-4 mnDf90 lines were established to evaluate the phenotype. The male and hermaphrodite phenotypes of all alleles over deficiency is identical or slightly, but not substantially stronger than that of the homozygous lines (which is not unusual for a large deficiency).

S. Brenner mapped unc-53 to 2.9 +/- 0.7 map units from dpy-10 (chromosome II). We refined this map position by mapping unc-53 with respect to different deficiencies in the region and doing three factor crosses between unc-4 and sqt-1, a 1.5 map unit interval. Unc-53(e2432)/+ males were mated in unc-4 sqt-1 hermaphrodites. Non-rolling F1 offspring were cloned on petriplates and their broods screened for the segregation of unc-53(e2432). Unc-4 non sqt-1 and sqt-1 non unc-4 hermaphrodites were picked from those plates and cloned on petriplates. 6 out of 42 sqt-1 non unc-4 recombinants segregated unc-53 and 3 out of 18 unc-4 non sqt-1 recombinants did not segregate unc-53. This yields a relative position of unc-4 / 51 /unc-53 / 9 / sqt-1. Or a calculated map position for unc-53 on chromosome II, 0.23 map units left of sqt-1.

Unc-53(e2432) was mapped relative to three deficiencies in the region mnDf90 mnDf87 and mnDf77 by mating e2432/+ males to unc-4 Dfx/mnC1 hermaphrodites and scoring for males and hermaphrodites with the unc-53 phenotype in the F1. The experiment was also performed by mating unc-4 mnDfx/mnC1 males to homozygous unc-53. mnDf87 and mnDf90 do not complement unc-53 while mnDf77 complements unc-53. Ooc-3, the only other gene on the genetic map in the region, was found to complement unc-53 in identical crosses between e2432 and unc-4 ooc-3/mnCl. Further mapping of unc-53 relative to RFLPs between wt strains in the region and the molecular cloning confirmed the map position of unc-53 (see below).

### Molecular characterization :

We started cloning the unc-53 locus because the study and interpretation of the unc-53 phenotype and the different mutants in the locus would be greatly facilitated by having information on and probes for the unc-53 mRNA and gene product.

At the time we initiated cloning of unc-53, a contig extending between unc-4 and sqt-1 (approx. 1500 kb) had been identified by A. Coulson and J. Sulston (C. elegans genome project LMB Cambridge), with no clone markers in between. To correlate the genetic map with the physical map in this region we positioned cosmids of this contig relative to the deficiencies mnDf77, mnD87 and mnDf90 by comparing band intensities of Southern blots of mnDfx/mnC1 strains probed with cosmids throughout the region. Cosmid KO2F7 is deleted in mnDf90 but not deleted in mnDf87 an mnDf77 thus identifying a leftmost location for unc-53. Cosmids W10G4, TO8D11 and F33G3 are in the unc-53 region (not deleted in mnDf77 but deleted in mnDf87 and mnDf90). Cosmid KO4H9 is deleted in mnDf77 and identifies a rightmost location for the gene. The distance between KO2F and KO4H9 is approx. 10 cosmids.

To narrow down the position of unc-53 further we looked for restriction fragment length polymorphisms between wild type strains in this interval and identified N2/RC301 RFLPs in cosmids W10G4, F40F8 and F22G3. We mapped these using three factor crosses with the strains unc-53 sqt-1/RC301 and unc-4 unc-53/RC301. We mapped F22G3 and F40F8 between unc-53 and sqt-1 at the following relative distances :
unc-4 / 9 / W10G4 / 2 / unc-53 / 1 / F40F8 / 1 / F22G3 / sqt-1.

These data localize unc-53 in an interval of approx. 80kb in which more than 15 differently overlapping cosmids are available. Pools of cosmids were injected in unc-53(n152) gonads together with the rol-6 selectable marker. Transient roller lines were established and scored for rescue of the unc-53 phenotype. Cosmid T28D2 was found to rescue the backward movement egg laying phenotypes of allele n152 .

A genomic library of N2 in lambda 2001 was screened with T28D2 and flanking overlapping cosmids. These were assayed in pools and individually for transformation rescue. Lambda clone, S4 carrying a sixteen kb insert was shown to give some rescue activity. Using restriction fragments of S4 as a probe, cDNA clones M5 (3.8 kb) and M18 (1-2 kb) were isolated from a Lamda MGU1 cDNA library. Both M18 and M5 contain an identical 3'-end as judged by restriction fragment analysis. Partial sequence analysis showed that M18 is shorter version of M5. Insert M5 was sequenced on both strands and was found not to be a poly-A tail at its 3'-end but appears not to full length at its 5'-end.

To find the 5' end of the unc-53 transcript we did nested PCR on L2 stage random primed cDNA, between antisense oligos tab2 and tab (43 bp away from the 5' end of cDNA M5) and an oligo to the SL1 trans-spliced leader sequence. This sequence is transspliced to the 5'-end of most C. elegans mRNAs. This yielded at least 6 classes of PCR-fragments which have been subcloned and sequenced. All contain the 43 bp between oligo tab2 and the 5' end of cDNA M5 (bp1281 to 1338). The longest PCR fragment (TB3) extends the sequence of cDNA M5 with 1280 bp. When added to the length of the cDNA M5, this unc-53 transcript which we constructed in vitro and named tb3-M5 would then be 5073 bp long (including some poly-A tail) and have a 1528 AA open reading frame. Recently a 5 kb cDNA, was identified in an embryonic cDNA library which has the TB3-5'-end (including part of the SL1), and the same 3'-end as M5, suggesting that TB3-M5 occurs in vivo. Similar PCR reactions in which the SL1 oligo was replaced by an SL2 transplice oligo gave no reaction products. Preliminary Northern blot analysis identifies a major 5.0 kb transcript and at least 2 smaller transcripts that are expressed in L2, L4 and adult worms. It needs to be examined whether the unc-53 5' ends reported here are made in vivo and encode different proteins or whether they represent PCR noise. The smaller PCR-fragments TB1b, TB16, TB1, TB6b and TB22 are "nested deletions" of clone TB3 with SL1's at their 5' end. The sequence of each is identical in the regions of overlap. The shorter SL1 transspliced transcripts contain ATGs downstream of the SL1 addition sites at positions 466, 988 and 1324. Comparison to the sequence of genomic clones confirmed that the SLls are spliced onto intron exon boundaries. However not all intron-exon boundaries receive SL1, suggesting that there is some specificity to this differential trans-splicing.

Recently the C. elegans sequencing consortium has sequenced cosmids F45E10. We mapped cDNA tb3-M5 onto these cosmids and found that unc-53 is an unusually large locus. It has 23 exons spread over more than 31 kb of genomic DNA.

The lambda clone S4 that rescues does not contain the first 430 bp of the unc-53 transcript. This suggests that the ORF between positions 63 and 430 is not essential for transformation rescue. This rescue may derive from expression of transcripts TB6b or TB22 or from "non-specific" initiation of transcription on the extrachromosomal arrays.

Additional confirmation that M5 was derived from the unc-53 transcription unit is provided by the observation that allele n152 has a 300 bp deletion, disrupting the sequence of cDNA M5 and leading to a large (possibly complete) reduction of UNC-53 protein in n152 embryos stained in immunofluorescence with an anti-unc-53 antibody (16-48-2). In addition, allele e2432 was found to carry a 3-4 kb insertion in this transcription unit.

### Sequence homology:

### Antibody staining :

The NdeI-EcoRI fragment of cDNA M5, the 47 kd fragment of UNC-53 encoded by the NdeI-EcoRI (position 3187 to 4458 (tb-M5 fig 3) protein sequence fig 2) was subcloned in the T7 expression vector prk172 (yielding vector TB66 and expressed in E. coli. Inclusion bodies containing recombinant protein were purified, by processes known in the art solubilized in 8 M Urea and the recombinant protein purified over a DEAE column equilibrated in 8M urea. Purified protein was mixed with complete Freund's adjuvant and injected in a rabbit and 4 Lou rats. This was followed six weeks later by bi-weekly boosts with antigen mixed with incomplete adjuvant. All sera are active in western blotting at titers of 1:30,000 on Western blots of the 47 kd unc-53 fragment expressed in E.coli. With this western blotting assay, a rat-mouse hybridoma cell line was prepared producing a monoclonal antibody to UNC-53. Mab 16-48-2 has the following properties :
- protein G-binding
- binding activity on western blots of
   (1) the 47 kd UNC-53 fragment expressed in E. coli, (pTB66)
   (2) the 57 kd carboxyterminal fragment of UNC-53 expressed in E. coli (construct pTB65.)
   (3) the full length TB3-M5 UNC-53 expressed in E. coli (construct pTB61) and mammalian cells (COS-cells; constructs pTB54 and 56).
- immunoprecipitation of native and SDS denatured full length TB3-M5 UNC-53 construct pTB50 expressed in vitro-transcription translation reactions in reticulocyte lysates.
- immuno-histochemistry in wild-type C. elegans fixed with methanol, acetone or paraformaldehyde and transgenic C. elegans expressing UNC-53 tb3-m5 pTB110, 111 or 112 in epidermis, neurones, gut and muscle.

Mab 16-48-2 fail to detect antigen of the correct size on Western blots of total worm proteins or worm proteins fractioned by progressive extraction with detergents, urea and SDS.

### Excretory canal phenotype

The excretory canal of C. elegans is a large H-shaped cell. It's cell body is positioned ventrally at the level of the pharyngeal bulb and send out two processes dorsally. At the level of the lateral epidermis (seam) each of these bifurcates and extends anteriorly and posteriorly over the seam cells, until they extend over most of the whole body length. It has been reported that in unc-53 the posterior process of the excretory cell does not extend up to the V6/T seam-cell boundary (E. Hedgecock et al., (1987), Development, 100 365-382).

We have done an extensive characterization of this phenotype in all alleles listed, either by direct in vivo Nomarski microscopy or UL6 rol6d marked unc-53 strains which express LacZ in the epidermis and excretory cell (Hope(1991) Development 113(2) 399-408). In wild type the excretory cell processes are straight. In unc-53 the canal is often meandering from left to right over the seam before it arrests prematurely, as if it has lost directional cues in its migration. It never leaves the lateral epidermis seam. Both the anterior and posteriorward processes are affected.

In weak unc-53 alleles the posterior excretory canal processes arrest anywhere between the vulval region and the V6/T boundary. We noticed that in even the strongest alleles or in unc-53/Df heterozygotes the canal arrests unusually frequently at or close to the vulva and never substantially before the vulva. We therefore set out to test whether the gonad dependent attractive signal which attracts the sex myoblasts to the gonad also might attract the excretory canal in an unc-53 independent manner to the vulval region. If this is the case we would expect that in a strong unc-53 mutant n152 in which the 2 somatic gonad cells (the source of the signal) have been ablated, the excretory canal migration would be fully arrested. As a control we ablated one germ cell and one somatic gonad cell (Z1 and Z2 or Z2 and Z4). Embryos were ablated in the comma to 2 fold stage and the position of the excretory canal scored double blind in hatched embryos. At the time of ablation, the canal may already have started growing out. At hatching, the endpoint of our experiment, the growth cone of the posterior canal process has reached just beyond the gonad. Although these are technically difficult laser ablations, the results show a substantial difference in excretory canal outgrowth between embryo with an ablated somatic gonad and control ablated embryos. In the experimental series the canal usually arrested a significant distance from the gonad or any other potentially damaged cells, suggesting the loss of a long range signal as described for the SM myoblast migration (Thomas et al (1990) and Stern (1991)). In the control series the excretory canal usually extended as far as unablated n152 and into region of the partially ablated gonad. This indicates that the premature arrest observed in the experimental series was not due to encountering a damaged region.

A gonad dependent and independent pathway were found to act redundantly in the posteriorard migration of the sex myoblasts. The data suggest that in wild type the migration of excretory cell growth cones is also guided by a gonad dependent and a gonad independent cue. In both cases the gonad dependent cue acts towards the gonad, but from opposite directions. However the gonad independent signal act anteriorward on the SM myoblasts and posteriorward on the posterior excretory cell growth cones. Since single mutants in both the gonad dependent pathway (sem-5) and independent pathway (unc-53) have no excretory cell phenotype these pathways may be redundant in the trajectory up to the gonad. An analogous redundancy has been observed for the sex myoblast migration. In the trajectory between gonad and tail the gonad independent pathway acts in different directions on the SM cells versus the excretory cell. In the excretory cell it acts in both anteriorward and posteriorward migration. A simple explanation which is elaborated in detail below is that unc-53 (like sem-5) may act downstream of a variety of receptors interpreting different cues.

The previously described interaction between the gonad and the sex myoblasts was rationalizable as an interaction between cells due to become part of the same organ. The interaction between the excretory cell and the gonad we report here suggests that the gonad may have a more general role as organizer cell migrations in the embryo. We wish to point out that the described dependent and independent pathways are formal genetic concepts. It is for example possible that in unc-53 embryos or unc-53 embryos in which the gonad dependent pathway has been genetically or laser ablated, as yet to be identified, pathway defining growth cones are misplaced leading indirectly to defective sex myoblast, neuronal (PLM, see below) or excretory canal migration. The observed highly restricted expression of unc-53 is an additional indication of this possibility.

### Sex muscle phenotype :

All unc-53 alleles exhibit the sex muscle phenotype described for e2432. We quantified phenotype in eight alleles :
Young adults grown at 20°C were mounted for polarized light or Nomarski microscopy on 2% agarose pads containing 0.2% phenoxypropanol as described in Sulston and Horvitz (1977) Dev. Biol. 56,110-156 . The vm1 sex muscles were examined under polarized light with a 40x objective and a Brace Kohler compensator and photographed. In addition, adults were fixed, incubated with fitc-coupled phalloidin and mounted for fluorescence microscopy as described in Goh and Bogaert (1991) Dev. Biol. 56, 110-156. The angle between the longitudinal axis of the animal and the central bundle of myofilaments of the anterior and posterior vm1 was measured from the negatives with a protractor. As the vulva is a transverse slit at a right angle to the cylindrical body axis, the angle between the vml and the vulval slit can be measured independently of which side of the animal faces the observer.

### Neuronal phenotype :

Unc-53 animals move poorly backwards when prodded but has good forward movement (Brenner (1974) Genetics 77 71-94). Various aspects of the neuronal phenotype of unc-53 has been reported in general phenotypic surveys of the UNC-collection (Brenner (1974) Genetics 77 71-94). : The posterior branch of the PDE neuron can be abnormal (Hedgecock et al. (1987) Development 100 365-382) and the mechanosensory PLMR & PLML neurons can have commissures into the ventral cord at a position much posterior than in the wild-type. There are also frequently multiple ventralward PLM commissures evenly spaced along the posterior half of the body (Siddiqui (1990) Neurosci. Res. (Suppl) 13 171-190), Hedgecock et al., (1987) Development 100 365-382).

### Examples 2 to 5 - Biochemical Analysis of UNC-53

### Example 2 - Immunoprecipitations of ³⁵S labelled unc-53 gene products.

The rat anti-UNC-53 monoclonal antibody, 16-48-2 (obtained from the hybridoma LMBP Accession no. 1383CB) elicited against a 47 kD fragment of the 3' end of UNC-53 from C. elegans was used to immunoprecipitate UNC-53 proteins. In this experiment, the full length unc-53 construct pTB50 (Fig. 11) was transcribed and translated in vitro in rabbit reticulocyte lysates. The resulting radioactively labelled ³⁵S unc-53 gene products were incubated with the monoclonal antibody under both denaturing (using SDS)and non-denaturing conditions, then incubated with protein G sepharose. The bound products were analysed by SDS-PAGE and fluorography. Monoclonal antibody 16-48-2 recognised both native and SDS denatured radioactive UNC-53 products verifying that the protein translated in vitro was bona fide UNC-53. This result shows that immuno-precipitation is a useful tool in schemes to purify native protein and to identify UNC-53 protein complexes in biochemical experiments.

### Example 3 - Actin sedimentation assays (8A variant).

Besides the N-terminal region of the protein which is similar to actin binding proteins, the predicted protein sequence of UNC-53 identified two putative actin binding sites. The first borders on the 3' end of the region of α-actinin/β-spectrin homology and the second lies in the 3' end of the cDNA sequence. This suggests that UNC-53 could potentially bind two actin molecules and via actin cross-linking, stabilise a particular growth cone spike to promote directional extension. Alternatively, the two actin binding sites may serve to anchor UNC-53 (and its shorter gene products) to the microfilament cytoskeleton to then transduce a signal via the NTPase domain to the downstream pathway.

To test the two site model, full length and truncated versions of UNC-53 (pTB50 and pTB52) were transcribed and translated in rabbit reticulocyte lysates for 90 minutes following standard protocols (Promega). To remove insoluble components, the reactions were airfuged for 1 hour at 100,000 x g and the supernatant containing ³⁵S labelled UNC-53 products introduced in actin co-sedimentation assays according to the method of Vancompernolle et al. (1992), EMBO J. 11, 4739-4746. In this procedure, radioactively labelled UNC-53 was incubated with monomeric G-actin in G buffer (2 mM Tris pH 7.5, 0.2 mM CaCl₂ 0.5 mM β-mercaptoethanol, 0.2 mM ATP) for one hour at room temperature. The salt concentration was then increased with F buffer (1 M KCl, 10 mM MgCl₂) to a final concentration of 100 mM to promote polymerisation of G-actin to F-actin. After an additional one hour incubation, polymerised F-actin/protein complexes were pelleted at 100,000 x g in an airfuge, washed with G buffer, resuspended in Laemmli buffer and separated by denaturing SDS-PAGE. The presence of actin in the pellets was confirmed by Coomasie staining while radioactively labelled UNC-53 products were detected by fluorography. Both the full length UNC-53 protein, pTB50, and the truncated construct, pTB52 translated in vitro in rabbit reticulocyte lysates cosedimented with F-actin at starting G-actin concentrations of 50-100 µg/ml. This suggests that UNC-53 binds to microfilament cytoskeleton. Moreover, deletion of the first putative actin binding site (pTB52) did not eliminate actin binding.

### Example 4 - UNC53 interacts with F-actin cytoskeleton (7A and 8A variant)

Analysis of the predicted protein sequence of UNC-53 identified two putative actin binding sites of the LKK class. The first borders the 3' end of the region of α-actinin/β-spectrin homolgy in the amino terminus of the protein while the second lies in the 3' end of the protein sequence upstream of the putative nucleotide binding domain. A single UNC-53 monomer could thus potentially bind and crosslink two actin molecules.

To test whether UNC-53 associates with the actin cytoskeleton, a 7A (pTB72) and 8A version (pTB73) of unc-53 (Figures 25 and 27 respectively) were transcribed and translated in rabbit reticulocyte lysates and the ³⁵S labelled products introduced into F-actin co-sedimentation assays (Figure 35a). The full length UNC-53 protein (pTB72) translated *in vitro* cosedimented with F-actin at starting G-actin concentrations of 100 mg/ml (Figure 35b) suggesting that UNC-53 interacts with F-actin. By 250 mg/ml, all of the UNC53 protein co-sedimented with the F-actin pellet. In contrast, no UNC53 was present in the pellet of the control reaction without actin. Thus, sedimentation was purely actin dependent. This result also indicated that the *in vitro* UNC-53 protein remained soluble even after the salt concentration was raised.

Deletion of the first putative actin binding site in pTB73 did not eliminate actin binding since the larger pTB73 products, including the largest fragment co-sedimented with F-actin under the identical set of conditions (Figure 35b). However, since the rabbit reticulocyte lysates contain numerous proteins, it is possible that the interaction of UNC-53 with actin may not be direct but rather mediated through another associated protein.

Several smaller radiolabelled protein fragments in the TnT reactions were observed in addition to the predicted protein products. Immunoprecipitation experiments confirmed that these products were UNC53 derived. Most likely they result from additional translational starts at internal methionines, since the identical set of smaller products was observed from reaction to reaction; or from premature termination and proteolytic degradation. Many of these smaller fragments also co-sedimented with F-actin. Since the second predicted actin binding site is within the 3' end of the molecule, truncated proteins that are the result of internal starts would be expected to have this site and to bind actin.

### EXPERIMENTAL PROCEDURES:

### Construction of UNC53 plasmids.

The complete unc53 cDNA was cloned as a 5.1 kb NotI-ApaI cassette in the mammalian expression vector pCDNA3 (Invitrogen) to generate plasmid pTB72, the 7A clone variant. To optimize translational initiation at the first methionine, a mammalian KOZAK consensus sequence was engineered upstream of the start methionine by PCR amplification of DNA coding for the first 139 amino acids of the amino terminus with the oligonucleotides BG03 (5'-ataagaatgcggccgccgccatgacgacgtcaaatgtagaattgata-3') and BG02 (5'-cgcggatcctcaaaccgcgggtggcataatggatg-3'). BG03 contains the mammalian KOZAK consensus sequence in addition to a NotI restriction site. pTB73 is a deletion of the first 408 base pairs of the unc53 cDNA contained in the vector Bluescript II-KS. This construction removes the first two methionines of the unc53 cDNA sequence such that the first possible start methionine in pTB73 is at amino acid position 165 in the cDNA sequence. In all these constructs, (pTB72, pTB73 and pTB50) the unc53 cDNA is inserted into the multiple cloning site such that the T7 promoter is immediately upstream of the 5' end of the cDNA sequence.

The first 139 amino acids of the UNC53 cDNA were amplified by PCR with oligonucleotides BG01 (5'ggaattccaaccatatgacgacgtcaaatgtagaattgaata-3') and BG02 (5'-cgcggatcctcaaaccgcgggtggcataatggatg-3') to generate a convenient NdeI cloning site immediately upstream of the start methionine. This amplification was cloned as an NdeI-BamHI fragment into the prokaryotic expression vector pRK172 (Godedert M. and Jakes R. (1990), EMBO J. Vol. 9, pp 4225-4230 and McLeod M *et al,* 1987 EMBO. J. Vol 6, pp 729-736) to generate construct pTB57. pTB61 contains the PCR derived amino terminus of pTB57 in addition to the 3' end of pTB50. Thus pTB61 contains the identical unc53 8A variant cDNA as in pTB50, but as an NdeI-NcoI fragment in the vector pRK172 for prokaryotic expression.

### In vitro transcription/ translation reactions

The UNC53 cDNA constructs pTB72, pTB73 or pTB50 were transcribed and translated for 90' at 30°C in a cell free T7 polymerase expression system in rabbit reticulocyte lysates following the company's protocols (ProMega). Prior to further manipulations, the reactions were centrifuged for 1 hour at 100,000 x g to remove insoluble components. In all subsequent experiments, the supernatant containing the soluble fraction of ³⁵S labelled UNC-53 products was utilized. Actin co-sedimentation assays

Soluble radioactively labelled "S-Met-UNC53 products were introduced in actin co-sedimentation assays according to the method of Vancompernolle et al. (1992). In this procedure, radioactively labelled UNC-53 was incubated with monomeric G-actin in G buffer (2 mM Tris-pH 7.5, 0.2 mM CaCl2, 0.5 mM b-mercaptoethanol, 0.2 mM ATP) for one hour at room temperature and then the salt concentration increased with F buffer (1 M KCl, 10 mM MgCl2) to a final concentration of 100 mM to promote polymerization of G-actin to F-actin. After an additional one hour incubation, polymerized F-actin/protein complexes were pelleted at 100,000 x g in an airfuge (Beckman), washed with G buffer, resuspended in Laemmli buffer and separated by denaturing SDS-PAGE. The presence of actin in the pellets was confirmed by Coomasie staining while radioactively labelled UNC-53 products were detected by fluorography. Briefly, after destaining, gels were soaked in 45 - methanol, 7.5 % acetic acid (vol/vol) for 30 minutes, followed by 30 min. in dimethyl sulfoxide (DMSO), and 1 hour in 10 % PPO dissolved in DMSO (wt/vol). The scintillant was precipitated by rehydrating the gels with four five minute water washes. After drying, gels were exposed to Xray film (Hyperfilm-Amersham).

### Immunoprecipitations

To confirm that the radioactively labelled proteins translated *in vitro* were of UNC53 origin, an anti-rat monoclonal antibody, 16-48-2, elicited against a 47 kD fragment of the 3' end of UNC-53 was used to immunoprecipitate UNC-53 proteins. In this experiment, the unc-53 construct pTB50 was transcribed and translated *in vitro* in rabbit reticulocyte lysates. The resulting radioactively labelled ³⁵S UNC-53 gene products were incubated with the monoclonal antibody under both denaturing (0.4% SDS, 2.0% Triton X-100) and non-denaturing conditions for 1 hour at room temperature, then incubated with protein G sepharose for 2 hours at room temperature, the beads washed 3 times with PBS and the bound products analyzed by SDS-PAGE and fluorography. Monoclonal antibody 16-48-2 recognized both native and denatured radioactive UNC-53 products. As a control, a reaction without monoclonal antibody 16-48-2 was treated identically.

### Example 5 - Interaction of UNC-53 with SEM-5/GRB-2

The observation that certain alleles of UNC-53 enhance the sex myoblast migration defect of sem-5 mutants is difficult to interpret. While the genetics suggests that UNC-53 and SEM-5 cooperate to regulate sex myoblast migration, it is unclear whether this is the result of a direct molecular interaction. To answer this question, two types of biochemical experiments were used to determine if UNC-53 physically interacts with SEM-5. In the first experiment, radioactively labelled ³⁵S UNC-53, synthesised in reticulocyte lysates, was incubated with SEM-5/GST (glutathione-S-transferase) fusion protein bound to glutathione resin or with GST protein bound to glutathione resin. After incubation, the beads were washed and the bound proteins analysed by SDS-PAGE and fluorography. This demonstrated that UNC-53 made in vitro specifically bound to the SEM-5/GST fusion protein resin. The GST fusion proteins have been previously described. Purification of GST-fusion proteins was facilitated by using a commercially available kit (Pharmacia). All purification methods followed the manufacturer's protocols.

To further characterise the nature of the interaction with SEM-5, a second experiment utilised Western blot overlays. UNC-53 fusion proteins were expressed in E.coli and the denatured protein lysates separated by SDS-PAGE and blotted to Immobilon-P nylon membrane (Milipore). Blots were incubated with biotin labelled SEM-5/GST, GRB-2/GST or GST protein, washed and bound multi-protein biotinylated complexes detected by probing with an avidin-alkaline phosphatase conjugate. The results from this experiment demonstrated that SEM-5 and its mammalian homologue GRB2 can interact with UNC-53 in vitro. Binding was observed in induced cell lysates only and probing with the UNC-53 monoclonal antibody 16-48-2 detected the identical sets of products. In addition, only the full length UNC-53 fusion, pTB61 (Fig. 7), which contained the SH3 binding sites gave a positive result (pTB52 was not tested) No signal was detectable for either of the SH3 binding site minus fusion proteins, pTB57 (Fig. 11) or pTB65 (Fig. 11). This provides supportive evidence that the polyproline repeats of the UNC-53 directly bind to the SH3 domains of SEM-5. Moreover, these results show that a SEM-5 or GRB-2/GST glutathione resin may be used in schemes to affinity purify native UNC-53 from tissue culture cells or nematodes or other organism extracts.

### Detailed Methodology

Radioactively labelled ³⁵S UNC-53 synthesized in reticulocyte lysates was incubated with SEM-5/GST (glutathione-S-transferase) fusion protein bound to glutathione resin or with GST protein alone bound to glutathione resin for one hour at 20°C. After incubation, the beads were washed four times with Phosphate Buffered Saline (PBS)/Triton X-100 (0.2%) and the bound proteins analyzed by SDS-PAGE and fluorography. The SEM5 and GRB2 GST fusions have been previously described (Lowenstein et al., 1992; Stern et al., 1993). Purification of GST-fusion proteins was facilitated using a commercially available kit (Pharmacia). All purification methods followed the company protocols.

### Western blot overlays

Approximately 500-1000 mg each of purified GRB2-GST protein or GST protein were biotin labelled by the following procedure. After overnight dialysis in PBS at 4°C, 1 M Hepes, pH7.4, was added to a final concentration of 100 mM and 50-100 mg of biotinylation reagent, dissolved in dimethyl sulfoxide, and the mixture incubated at 20°C for 90 minutes. The biotinylation reaction was stopped by the addition of 1 M Tris, pH7.4 to a final concentration of 100 mM and the labelled proteins stored on ice.

The UNC-53 construct pTB61 was expressed in *E. coli* strain BL21 (DE3), and the denatured protein lysate separated by SDS-PAGE and electroblotted to Immobilon-P nylon membrane (Millipore). Membranes were blocked with 1 % skim milk powder in TBS-T (20 mM Tris, pH7.6; 0.14 M NaCl; 0.1% Tween-20) for 1 hour at 37°C. Subsequently, membranes were incubated in equimolar amounts of either biotin labelled GRB-2/GST or biotin labelled GST protein for 1 hour at 20°C, washed 4 x with TBS-T and bound multi-protein biotinylated complexes detected by probing for 1 hour at 20°C with an avidin-alkaline phosphatase conjugate (dilution 1:5000). Biotinylated protein conjugate complexes were visualized with a chromogenic solution containing bromochloroindolyl phosphate (BCIP)/nitro blue tetrazolium (NBT) in 100 mM Tris(pH 9.5), 100 mM NaCl, 5 mM MgCl₂. Development was terminated with 10 mM Tris (pH8.0), 1 mM EDTA.

### Example 6 - Transgenic Analysis

To further our understanding of the function of unc-53 we developed an in vivo assay to test gene fusions generated in vitro. Nematode expression vectors containing the full length unc-53 cDNA, TB3M5, downstream of various tissue specific and inducible promoters were constructed.

The mec-7 promoter of pTB112 (Fig. 7) confers tissue specific expression to the mechanosensory neurons, the unc-54 promoter of pTB111 (Fig. 7) confers tissue specific expression to body wall muscle and the hsp16-41 promoter of pTB109 (Fig. 7) confers and pTB110 (Fig. 7) confers heat inducible expression to somatic cells. pTB109 is a transcriptional fusion containing only the hsp16-41 gene promoter and has been shown to confer high levels of inducible expression in embryos. pTB110 contains a larger portion of the hsp16-41/2 intergenic region in addition to a synthetic intron. This plasmid is expected to be highly inducible in embryos and post-embryonic stages in most somatic cell types.

Oocytes of both wild type (N2) and unc-53(n152) hermaphrodites were microinjected according to the method of Fire (1986), EMBO J., 5, 2673-2680. Coinjection of the unc-53 fusion with a selection plasmid, pRF4, a dominant marker of rol-6, allowed identification of transgenic animals by their right rolling phenotype (Mello et al, (1991), EMBO J., 10, 3959-3970. In C. elegans, the injected DNA does not integrate into the genome but rather forms extrachromosomal arrays which are heritable at a frequency ranging from 20-95% (Stinchcomb et al, (1985), Mol. Cell. Biol., 5, 3483-3496; Fire et al, (1990), Gene, 93, 189-198; Mello et al, (1991), EMBO J., 10, 3959-3970. Transgenic extrachromosomal lines were considered stable after the rolling phenotype had passed through four generations. Some transgenic HS-unc-53 strains were mutagenised with 3550 rads of y rays emanating from a ⁶⁰Co source which produces breaks in the chromosomes allowing for insertion of the extrachromosomal array. Stable integrants were identified by screening for homozygous rolling F3 broods. The names and genotypes of all transgenic strains are listed in Table 1 with details of the unc-53 fusions (constructs/vectors) listed in Table 2:

**Table 1 - Extend in other constructs**

| STRAIN NAME | PARENTAL STRAIN | unc53 FUSION | SELECTION | lacZ MARKER |
|---|---|---|---|---|
| TB3In54 | n152 | pTB109 | pRF4 | UL6 |
| TBAInB | N2 | pTB110 | pRF4 | pPCZ1 |
| TBAIn61 | N2 | pTB110 | pRF4 | pPCZ1 |
| TBAIn69 | N2 | pTB110 | pRF4 | pPCZ1 |
| TBAIn76 Accession No 1385CB (See Fig 17A) | N2 | pTB110 | pRF4 | pPCZ1 |
| TBAIn90 | N2 | pTB110 | pRF4 | pPCZ1 |
| TBAIn210 | N2 | pTB110 | pRF4 | pPCZ1 |
| TBAIn222 | N2 | pTB110 | pRF4 | pPCZ1 |
| TBAIn306 | N2 | pTB110 | pRF4 | pPCZ1 |
| TBAIn327 | N2 | pTB110 | pRF4 | pPCZ1 |
| TBBIn3 | N2 | pTB110 | pRF4 | pPCZ1 |
| TBBIn267 | N2 | pTB110 | pRF4 | pPCZ1 |
| TB1Ex10 | n152 | pTB112 | pRF4 | none |
| TB1Ex23 | n152 | pTB112 | pRF4 | none |
| TB1Ex8 | N2 | pTB112 | pRF4 | none |
| TB1Ex16 | N2 | pTB112 | pRF4 | none |
| TB2Ex1 | N2 | pTB112 | pRF4 | none |
| TB2Ex37 | N2 | pTB112 | pRF4 | none |
| TB3Ex10 | N2 | pTB112 | pRF4 | none |
| TB3Ex12 | N2 | pTB112 | pRF4 | none |
| TB3Ex20 | N2 | pTB112 | pRF4 | none |
| TB3Ex37 | N2 | pTB112 | pRF4 | none |
| TB4Ex14 | N2 | pTB112 | pRF4 | none |
| TB4Ex18 | N2 | pTB112 | pRF4 | none |
| TB4Ex22 | N2 | pTB112 | pRF4 | none |
| TB4Ex25 Accession No LMBP 1384CB (See Fig 16) | N2 | pTB112 | pRF4 | none |
| TB1Ex3 | n152 | pTB111 | pRF4 | none |
| TB1Ex6 (See Fig 178, C) | n152 | pTB111 | pRF4 | none |
| TB1Ex11 | n152 | pTB111 | pRF4 | none |

| | | | | |
|---|---|---|---|---|
| Notes for Table 1: Ex-extrachromosomal | | | | |
| In-integrated | | | | |
| pTB109, pTB110-Heat shock unc-53 fusions | | | | |
| pTB111-mec-7 fusion | | | | |
| pTB112-unc-54 fusion | | | | |
| pRF4-rol-6 (su1006) (Mello et al, (1991), EMBO J., 5, 3959-3970) | | | | |
| UL6-excretory canal promoter lacZ fusion | | | | |
| pPCZ1-Hsp16-48/1 lacZ fusion (Stringham et al, (1992) Molec.Biol.Cell 3, 221-233) | | | | |

**Table 2**

| Full length cDNA tb3M5 (still has SL1 and 5' UTR) | |
|---|---|
| pTB50 | (NotI-ApaI fragment in Bluescript II-KS, for in vitro transcription) |
| pTB51 | (NotI-ApaI fragment in Bluescript II-SK, for in vitro transcription) |
| pTB54 | (NotI-ApaI fragment in pCDNA3, for mammalian expression) (Deposited as accession no. LMBP3296) |
| pTB109 | (NotI-ApaI fragment in hsp16-pucBM21, for in vivo expression) |
| pTB67 | (Notl-Apa fragment in pGEM5 +) |

| PCR1 of amino terminus of cDNA | |
|---|---|
| (*PCR using oligos BG01 and BG02) | |
| pTB57 | (NdeI-BamHI fragment in pRK172, for E. coli expression) |
| pTB58 | (NdeI-NcoI fragment in pGEM5) |
| pTB63 | (SacI-NcoI fragment in pRSETA, for E. coli expression) |
| pTB64 | (BamHI fragment in pBluescriptII-KS) |

| Full length cDNA utilizing PCR1 amino terminus | |
|---|---|
| pTB61 | (NdeI-NcoI fragment in pRK172, for E. coli expression) |
| pTB110 | (XbaI-KpnI fragment in pPD49.83, for in vivo expression) |
| pTB111 | (XbaI-KpnI fragment in pPD52.102, for in vivo expression) |
| pTB112 | (XbaI-KpnI fragment in pPD30.38, for in vivo expression) (Deposited as accession no. LMBP3295) |

| PCR2 of amino terminus of cDNA | |
|---|---|
| (*PCR using oligos BG03 and BG01) | |
| pTB59 | (NotI-BamHI fragment in pBluescript II-KS) |
| pTB60 | (NotI-XhoI fragment in pCDNA3, for mammalian expression) |

| Full length cDNA utilizing PCR2 amino terminus | |
|---|---|
| pTB55 | (NotI-EaeI fragment in pBluescriptII-KS) |
| pTB56 | (NotI-ApaI fragment in pCDNA3, for mammalian expression) |

| Other constructs | |
|---|---|
| pTB52 | (SacII deletion of amino terminus of pTB50) |
| pTB53 | (SacII deletion of amino terminus of pTB51) |
| pTB62 | (SmaI fragment of pTB52 in pGEX2T, for prokaryotic expression) |
| pTB65 | (NdeI-NcoI fragment of 3' terminus in pRK172, for prokaryotic expression) |
| pTB66 | (NdeI-EcoRI fragment of 3' terminus in pRK172, for prokaryotic expression, MAB 16-48-2) |

Initially, the phenotype of each transgenic line was characterised by inspection with a dissecting microscope and/or Nomarski optics. Transgenic strains were directly analysed for expression of unc-53 by immunohistochemistry. Briefly, embryos were adhered to polylysine coated slides and permeabilised by a combination of freeze fracturing and immersion in cold methanol and acetone (3-4 minutes each). Embryos were rehydrated through an acetone/distilled water series and then incubated for 30 minutes at room temperature in TBS-Tween (0.1%). The anti-UNC-53 monoclonal 16-48-2 anti-sera was applied undiluted and the slides incubated at 4°C overnight. The embryos were washed three times with TBS-T and then incubated in a secondary rhodamine like (Cy3-M)conjugated antibody for 1 hour at 37°C. After 3-4 washed in TBS-T the slides were mounted for fluorescence microscopy in 2% propylgallate, 80% glycerol-pH 8.0.

### Characterisation of transgenic strains carrying pTB112

UNC-53 was over-expressed in the muscle of wild type animals (pTB112 in N2). Each extrachromosomal pTB112/N2 line consisted of wild type and rolling animals as expected, but in addition, several mutant phenotypes were observed at low frequency. These animals varied considerably in phenotype and included embryos which arrested at the two fold stage, larvae which hatched but died soon afterward, animals with extra protrusions on the epidermis and animals with a truncated posterior end. This phenotype is consistent with that of the mup or mua classes of muscle mutants in which the positioning and/or integrity of muscle attachments to the hypodermis has been disrupted. Most of these animals were either inviable or sterile. The progeny of the viable mutants contained the same frequency of rollers, wild type and mutants as did the progeny of rolling individuals. Since the extrachromosomal array may be lost at each cell division, every animal is a mosaic. The healthy rollers may have lost the transgene from most muscle cells and may represent weak phenotypes whereas the 2 fold arrests represent the situation where the array has been lost from few muscle cells. Nomarski and polarised light microscopy of the severe larval lethals showed that the muscle cells were disorganised and over-extended.

Detailed analysis of the underlying defect in embryonic development that leads to this terminal phenotype were performed with immunofluorescence microscopy (Fig 21).

Since the unc-54 gene encodes the myosin heavy chain, we expected that this promoter would be active in body muscle descendants from the comma stage onwards. In the unc-54 - unc-53 strains, signal was indeed localised to the body muscle cells in comma and later stages as predicted. The immunofluorescence was localised to the cytoplasm of the cell bodies and was particularly intense at the tips of the extending processes. Increased process length was observed very early in muscle development (comma to 1.5 fold stage) and increased up to the three fold stage. No other abnormalities in shape or muscle myofilament pattern were observed in the anterior-posterior axis of the animal. Two and three fold embryos which were stained with the monoclonal antibody NE8(4c6.3) (Goh and Bogaert,(1991), Dev. Biol. 56, 110-156) appeared to have a relatively wild type myofilament structure. As these animals are mosaic, it may be possible that severe cases die in late morphogenesis and those which survive through embryogenesis to adulthood can tolerate a few distorted muscle cells.

### pTB111 transgenic lines

Immunostains indicates that the transgene is expressed efficiently in the mechanosensory neurons of a transgenic extrachromosomal line carrying the pTB111 transgene in an unc-53 (n152) genetic background (Fig 20).

### pTB109 and pTB110 lines

Twelve integrated lines derived from three separate mutageneses of extrachromosomal lines have been isolated. TB3In54 carries the pTB109 fusion in addition to pRF4. Nine TBA strains were isolated after mutagenesis of an extrachromosomal strain, HSA. There are two strains (TBB) derived from mutagenesis of the extrachromosomal strain HS B. Both TBA and TBB strains contain the transgenes pT8110, pPCZ1 and pRF4. Inclusion of the HS-lacZ plasmid, pPCZ1 (Stringham et al, (1992), Molec.Bio.Cell 3, 221-233) allows one to monitor the strength of the heat shock induction by assaying for β-galactosidase activity.

Immunostains of embryos freeze fractured after a two hour heat shock showed that the signal was most prominent in the pharynx, gut and neurons. Surprisingly, the signal had a speckled appearance. This may be a feature of heat shock. Heat shock proteins may sequester UNC-53 to "chaperone" it during stress. Alternatively, UNC-53 may be targeted for degradation. In one experiment, embryos were heat shocked for two hours, allowed to recover overnight and then freeze fractured the next morning. While levels were reduced, there was a little residual UNC-53 signal in the gut cells. Thus, about 16 hours later most the protein has gone.

Level of heat shock and recovery times are therefore important factors in the mutant rescue experiments and the preferred assay system described in example 10. In addition, experiments suggest that heat shock induction in liquid culture versus agar plates or dry incubators versus water baths need careful calibration.

After a strong three hour heat shock, a high percentage of animals were not able to recover from the stress. Embryos which were not subjected to a double shock (2-two hour heat shocks at 33°C separated by a two-hour recovery) hatch out as malformed worms reminiscent of the muscle overexpression lines (Fig 21). The heat shock promoter used is especially active in the pharynx. Consistent with this, a strong pharyngeal morphogenetic phenotype was observed (Fig 21). Pharyngeal phenotypes are easy to score and quantify (feeding rate, dye uptake, LacZ lines staining the pharynx) by anyone skilled in the C. elegans field and may form a preferred embodiment of the assay.

### Example 7

Over-expression of UNC-53 results in directional over-extension : Assay with 7A variant.

In wild type *C. elegans,* body muscle cells are normally spindle shaped while in UNC53 mutants, a number of these cells have a reduced process which results in a fork shaped tip. This phenotype is consistent with the general reduction of extension observed in many growth cone types along the longitudinal axis of the animal in unc-53 mutants. Recalling the extremely limited pattern of UNC53 expression in embryogenesis detected by immunostaining with monoclonal antibody 16-48-2;no UNC53 activity was discernable in wild type body muscle cells during outgrowth suggesting that the levels of UNC53 activity required for this extension may be extremely low.

We overexpressed unc-53 in the muscle of wild type animals by expressing the full length cDNA under the control of the unc-54 myosin heavy chain promoter in the fusion pTB113. Plasmid pTB113 is a translational fusion containing the 7A variant unc-53 cDNA sequence as an XbaI-KpnI fragment starting from the first methionine and including the unc-53 cDNA poly adenylation tail under control of the myosin heavy chain unc-54 promoter of the nematode expression vector pPD30.38 available on Internet web site ftp archive: ciwl, ciwemb.edu. Plasmid pTB114 contains the identical cDNA fragment under control of the hsp16-41 -2 promoter (Jones et al., 1995, Dev. Biol. VOL. 171, PAGES 60-72) which confers heat inducible expression to somatic cells, in the expression vector pPD 49.83 (Fire, pers. comm.) The amino terminus of the UNC53 cDNA is identical to the PCR amplification with BG01 and BG02 of pTB57. Thus, both pTB113 and pTB114 are in frame translational fusions devoid of the SL1 leader sequence and upstream untranslated region of the cDNA.

Each transgenic mosaic line (3 were examined) consisted of wild type and rolling animals as expected, but in addition, several mutant phenotypes were observed at a low frequency. These animals varied considerably in phenotype and included, embryos which arrested at the two fold stage, larvae which hatched but died soon afterwards, animals with extra protrusions on the epidermis and animals with a truncated posterior end. Most of these latter animals were either inviable or sterile. The progeny of the viable mutants contained the same frequency of rollers, wild type and mutants as did the progeny of rolling individuals. Since the extrachromosomal array may be lost at each cell division, every animal is a mosaic. The healthy rollers may have lost the transgene from most muscle cells and may represent weak phenotypes whereas the 2 fold arrests represent the situation where the array has been retained in most muscle cells. The truncated posterior end may be the result of lethality in the D lineage due to mosaicism. Nomarski and polarized light microscopy of the severe larval lethals showed that the muscle cells were disorganized and over-extended in the longitudinal axis. In some cases the muscle cells appeared detached from the hypodermis. As these animals are mosaic, it may be possible that severe cases die early in morphogenesis whereas those which survive through embryogenesis to adulthood can tolerate a few distorted muscle cells.

In transgenic pTB113 strains, UNC53 expression, as detected by immunostaining with monoclonal antibody 16-48-2, was localized to the body muscle cells in comma and later stages as predicted for the UNC-53 promoter (myosin heavy chain). The pattern of immunofluoresence with the anti UNC-53 antibody was localized to the cytoplasm of the cell bodies and was particularly intense at the tips of the extending processes and in the cytoskeleton, when compared to phalloidin staining which specifically stains the actin cytoskeleton. The identical pattern of subcellular localization, in the cytoplasm and cytoskeleton, was also observed in the intestinal cells of pTB114 transgenic embryos expressing UNC-53 ectopically after heat shock.

In addition, the growth cone processes appeared to be overextended specifically in the anterior-posterior axis of the animal. To verify this, the length of body muscle cells over-expressing the UNC53 cDNA in the pTB113 strains were measured and compared to the length of wild-type muscle growth cones expressing an unc-54 promoter-GFP (green fluorescent protein) fusion, pPD49.83 (available on Internet Web Ste Ftp archive: ciwl. ciwemb.edu. The GFP reporter allowed visualization of the entire cell body and boundaries of the muscle cells in wild-type animals. We estimated that the processes of the pTB113 expressing cells were roughly 1½ times the length of pPD49.83 expressing wild type cells.

The lethality in the transgenic progeny of the three pTB113 strains examined ranged from 32% to 78%. Thus a significant proportion of the transformed mosaic progeny did not survive morphogenesis. In contrast, no lethality was observed in the pPD93.48 (unc-54-GFP) control strains. The lethality observed in the pTB113 lines is likely the consequence of overextension of muscle cells during embryogenesis because (a) both pTB113 and pPD93.48 utilize the identical promoter and should be expressed in the same cells at the same point in development, and (b) rol-6 selection was used to identify transformants for both constructs.

### Example 8

### Transient and stable transfection of UNC-53 in N4 neuroblastoma cells.

pTB72 and a plasmid expressing LacZ under the CMV promoter were transfected transiently with the Ca-phosphate method in N4 neuroblastoma cells.

N4 cells and their stably transfected counterparts were grown in Minimum Essential Medium (MEM)-REGA 3 (GIBCO BRL) supplemented with 10% Foetal Calf Serum, 1% L-Glutamine, 2% Sodium Bicarbonate, 200 units/ml penicilline and 200 µg/ml Streptomycine, in a humidified atmosphere of 90% air and 10% CO₂ at 37ºC. Transfections were performed by the Lipofectamine method (GIBCO BRL). 18 to 24 hrs before transfection cells were seeded in complete growth medium at a density of 7x10⁵ per well in a six well tissue culture plate, and incubated at 37º C in a CO₂ incubator. For each transfection the following solutions were prepared.:
SolA = 4 *µ*g of DNA diluted in 200 ul of Optimem (GIBCO BRL)
SolB = 12 ul of Lipofectamine reagent diluted in 200 ul of Optimem (GIBCO BRL)
Solutions A and B were combined, gently mixed and incubated at room temperature for 30 minutes. For each transfection 0.6 ml of Optimem was added to the lipid-DNA complex to reach the final volume of 1 ml. This mixture was then added onto the cells (which had been previously rinsed once with 2 ml of Optimem). The cells were incubated in the transfection mixture for 5 hrs at 37C in a CO2 incubator. At the beginning of the sixth hour from transfection, 1 ml of complete growth medium supplemented with 20% of Foetal calf serum was added to the transfected cells. The cells were incubated for 18 hrs at 37C in a CO2 incubator. 24 hrs following the beginning of transfection the supernatans was replaced with fresh growth medium. 72hrs post transfection cell cultures from each well were harvested, diluted 1:24 and distributed over 24 well plates with the growth medium containing 500, 750 ug/ml or 1mg/ml of geneticin (G418, GIBCO BRL). After -12 days from the start of selection, single clones were picked and allowed to grow in the absence of selection. Of 27 initial clones, 7 were lost while expanding the clones because of their slow growth rate and the apparent general toxicity of caused by the transfected construct. Clone 9 was selected for further analysis.

### Functional assay for neurite extension in N4 neuroblastoma

Step (1): Quantitative determination of neuronal morphology, i.e. length of neurites and fraction of positive cells is performed fully automatically. As an example we studied the degree of morphological differentiation in the wild-type N4 cells to a stably transfected C9 clone.

### Step (2): Quantitative neuronal morphology

Morphological changes of neurones were quantitated as described in GEERTS et al (1992 Restorative Neurology and Neuroscience 4: 21-32 and Katsuhito *et al* Neurodegeration, 2: 173-181). Briefly, at appropriate times, glutaraldehyde was applied to cell cultures. No washing steps were performed. This ensured that the morphology of the cells at that time point was frozen. The cells were observed in transmitted light mode on an Axiovert microscope, equipped with a Marzhauser scanning stage driven by an Indy workstation (Silicon graphics). Images were captured using a MCS video camera (HCS). About 3000 cells were detected in 64 neatly aligned images, forming a 8x8 square matrix of images. The exact alignment of the images ensured that neurites could be followed from one image field to the next. The analysis software automatically detected cell bodies and neurites and saved cell body size and length of each individual neurite on a file. Different parameters were subsequently calculated. The neurite length per cell was calculated on freely lying cells (not within a cluster). The fraction positive cells is the fraction of cells having at least one neurite with a length exceeding twice the cell body diameter. Figure 40 clearly shows that clone C9 increases both neurite length (free length) and fraction of positive cells, compared to wild-type N4 cells clone.

### Example 9

### Transient and stable transfection of UNC-53 in MCF-7 breast carcinoma cells.

pTB72 and a plasmid expressing Lac Z under the CMV promoter where transfected transiently with the Ca-phosphate method in MCF-7 breast carcinoma cells.

MCF7 cells and their stably transfected counterparts were grown in Dulbecco's Modified Eagle's Medium (DMEM, GIBCO BRL) supplemented with 10% foetal Calf Serum, 1% L-Glutamine, 1% of a 5mg/ml stock of Gentamicine and 1% of a 100mM stock of Sodium Pyruvate in an humidified atmosphere of 90% air and 10% CO2 at 37 C. Construct pTB72 was transfected by the Calcium-phosphate method (ref): 18-24hrs before transfection. cells were seeded at a density of 3x10⁵ in a six well tissue culture plate with complete growth medium. Two hours before transfection the culture medium was removed and replaced with 1.8 ml of fresh medium. The cells were put back in the incubator until the moment of transfection. DNA-Ca₃(PO4)₂ precipitates were prepared one hour before transfection : For each transfection (1 well): 4 ug of DNA (=3-4 ul) was combined with 76 ul of TE (Tris HCl-EDTA pH 8) 0.1M to a final volume of 80 ul. To these DNA's diluted in TE, 20 ul of CaCl₂, Hepes solution was added to a final volume of 100 ul of DNA/CaCl₂ mixture. The 100 ul of DNA/CaCl₂ mixture was added very slowly, drop-by-drop to 100ul of 2x BS/Hepes while shaking, to a final volume of 200 ul. The resulting 200 ul DNA/Calcium Phosphate mixture was added to the cells and the mixture incubated for 8 hrs at 37 C in a CO₂ incubator. At the beginning of the ninth hour from the start of transfection, the supernatans with the DNA/Calcium phosphate mixture was replaced with 3 ml of complete culture medium. 72hrs post transfection, cells from each well were harvested, split1:24 in complete growth medium supplemented with 1mg/ml of Geneticin (G418, GIBCO-BRL) and plated out in 24 well plates. 15 days from the start of selection, single clones where picked and allowed to grow without selection. Three clones MCF7-pTB72-clone9, MCF7-pTB72-14 and MCF7-pTB72-15 were retained all of which have a similar phenotype.

### 1) Phenotyping UNC-53 transfected MCF-7 breast carcinoma cells:

The general morphology and motile behaviour of the three transfected MCF-7 clones are different from non-transfected cells.

The assay consists of a tyramide amplification of a classical immunofluorescent reaction. The cells were grown in defined medium with 10% charcoal treated serum and supplemented by 10 µg/ml insulin (final concentration) and 5 ng/ml basic fibroblast growth factor (final concentration). The substrate consisted of 50 *µ*g/ml poly-L-lysine in chamber slides; cultures were maintained in a humidified atmosphere of 95/5% air/CO₂.

Inductin of expression of vimentin and of increased levels of fosfotyrosine was found in the transfected subclones. Vimentin formed dense clusters around the cell nucleus with some filamentous structures in the pseudo-podes. Fosfotyrosine, on the other hand, was predominantly found at the border of the cell ruffles, at the same subcellular area where UNC53 expression was found. This provides evidence of a controlling molecule functioning in a signal transduction pathway and that vimentin is an indicator of metastasis in cancerous cell lines.

### 2) Functional assay to establish the signal transduction role of UNC-53.

Cells locomote in tissues and on substrates. The type and amount of cell locomotion depends on different factors: (1) the physiological conditions perceived through receptors, which can be - for example - stimulation with or deprivation of serum, growth factor(s), cytokine(s), chemokine(s) or (pro-) inflammatory mediators; (2) the type and functionality of cell adhesion molecules expressed by cells and extracellular matrix molecules present in tissue or in culture model, (3) the actin, tubulin and/or intermediate filament cytoskeleton and (4) proper functioning of integrator proteins such as UNC-53, homologues or other molecules that translate physiological stimuli (or lack of stimuli) into increased or decreased cell motility, directional or random motility or different types of motility. Cell locomotion can be measured in different types of assays, such as disperse cells or in monolayer cultures, as cellular outgrowth from tissues in culture or in organotype cultures. Motility of live cells can be quantified microscopically as in example 8 or by time-lapse video or cinematography or by phagokinetic assays (Albrecht-Buehler, 1977, Cell, 11:395) amongst other methods.

Cell motility assays are interesting tools to study the functioning and pharmacology of UNC-53 and the unc-53 pathway.

All previous observations were performed on MCF-7 cells grown in defined medium supplemented by 10 µg/ml insulin (final concentration) and 5ng/ml basic fibroblast growth factor (final concentration). This approach offers the possibility of investigating the role of FGF in the UNC53 role of signal transmission. Indeed, by comparing wild-type versus UNC53 transfected cells cultured in medium with or without FGF/insulin and/or by microinjection of UNC53 protein, it can be investigated if UNC53 is responsible directly for regulating a signal transduction pathway linking extracellular growth factors to the assembly of, amongst others, focal adhesions.

### Example 10: Enhanced phagokinesis in Ce-unc-53 transfected MCF-7 cells.

In this example evidence is presented that transfection of a plasmid containing the Ce-unc-53 sequence under a suitable promoter enhances cell motility in the phagokinesis assay.

When culture plastics are coated with colloidal gold particles, a variety of cells types were shown to migrate over the plate and displace or phagocytose the gold lawn on their way while locomoting. The track left bare is a qualitative and quantitative measure of cell motility and/or locomotion. The basic methods have been described in detail elsewhere (Albrecht-Buehler, 1977, Cell, 11:395; Zetter, 1980, Nature, 285:41; O'Keefe et al., 1983, J. Invest. Dermatol., 85:130).

### Methods

12 well plates were coated for 15 minutes with 5 *µ*g/ml gelatin in water and gold coated as described by Albrecht-Bueller (1977). Ce-unc-53 transfected MCF-7 cells and the parent MCF-7 were cultured in parallel, trypsinised dispersed in culture medium and seeded in 12-well plates at a density of 2550 cells per well. The cells were allowed to adhere to the plate and to locomote for 16 hours. After incubation the cells were chemically fixed to the plate using paraformaldehyde, washed with distilled water and finally air-dried.

Subsequently, images of the gold lawns were captured using automated videomicroscopy, composite images of the wells were generated and single-cell phagokinetic tracks were measured using a home-made routine in SCIL^{™} software.

### Results

The parent MCF-7 line displayed two cell populations with different motile behaviour in phagokinesis assays. In table 3 the fraction of parent and Ce-unc-53 transfected MCF-7 cells that produced linear tracks in the phagokinesis assay are shown. In the parent MCF-7 cells, 88% of the cells produce a round track (long and short axis less than 2-fold different) and 12% cells produce 'linear' tracks (long and short axis more than 2-fold different). Ce-unc-53 transfection of MCF-7 cells produced an increase of the fraction of cells displaying 'linear' tracks to 28% at the cost of the cells producing round tracks.

These observation suggest that Ce-unc-53 transfection into MCF-7 is capable of increasing in situ locomotion of MCF-7 e.g. by increased spreading, ruffling or other forms of non-directional motility in the 'round' population as well as by driving a fraction of transfected MCF-7 cells from non-directional motility (round tracks) into directional migration (linear tracks).

In tissue culture, cells are provided with non-directional signals. It is likely that providing directionality to these signals will enhance observed effects. Significant enhancement was observed for the fraction of linear tracks.

In addition, a significant increase of 35% in the area of tracks was observed in the Ce-unc-53 transfected MCF-7 cells versus the parent MCF-7 cells (Table 3). This increase occurred in the round track population; the area of linear tracks was found not to be changed by transfection.

These obsevations in phagokinesis suggest that Ce-unc-53 transfection into MCF-7 cells is capable of increasing insitu locomotion in Ce-unc-53 MCF-7, e.g. by increasing spreading, ruffling, or other forms of non-directional motility in the "round" population. In addition the Ce-unc-53 transgene in MCF-7 cells drives a fraction of the MCF-7 cells from non-directional motility (round tracks) into directional migration (linear tracks).

| Table 3. Analysis of phagokinesis assays with parent and *Ce*-*unc-53* transfected MCF-7 cells. | | | | | |
|---|---|---|---|---|---|
| | *parent MCF-7* | | *Ce*-*unc*-*53 MCF-7* | | *increase* |
| *Fraction linear* | % +- *SD(n)* | | *%*+-*SD(n)* | | |
| *tracks (*)* | 12+-3(8) | | 28+-6(8) | | 2.33 |
| *Track area (**)* | *pixels*+-*SD(n)* | | *picels*+-*SD (n)* | | |
| *all tracks* | 1261+-128(8) | | 1698+-179(8) | | 1.35 |
| *round tracks* | 1229+-162(8) | | 1464+-204(8) | | 1.19 |
| *linear tracks* | 2367+-424(8) | | 2300+-319(8) | | 0.97 |
| (*) the fraction of linear tracks in 8 wells was pooled | | | | | |

MCF-7 cells expressing low levels of UNC-53 exhibit increased motility.

Individual transfected cells are much more flattened in appearance than wild type and have a broad lamellipodium extending from the edge of the cell. Ruffling edges are more frequent than in wild type. Transfected cells in clusters have a broad lamellipodium edge around the cluster while cluster of the non-transfected. Within the cluster the nuclei are more widely spaced from one-another than in wild type cells (also due to a lamellipodium edge).

### Example 11

Method for Protein micro-sequencing of co-affinity purifying proteins

UNC-53 protein was immuno-affinity purified from extracts of cells expressing C. elegans UNC-53 using monoclonal antibody 16-48-2. One to five mg of Mab 16-48-2 was prepared, purified on protein-G sepharose and subsequently covalently linked to sepharose beads. A column of such beads was loaded with both crude cytosolic and Triton-X100 extracts (containing solubilised RTKs) and eluted with 4M MgCl₂ or other chaotropic agents. A co-immuno-purifying band was identified on SDS-denaturing PAGE gels, eluted from these gels and micro-sequenced. This protein sequence or mass information of peptides generated by proteolysis was used to identify the co-immunoprecipitation directly from the sequence databases.

Alternatively the sequence was reverse translated and oligonucleotides based on the sequence prepared. This is used to clone the corresponding gene as well as other techniques well known in the art.

### Example 12 C. elegans as a model assay system.

We have constructed transgenic strains which overexpress UNC-53 in body muscle. This results in increased extension of muscle cells and embryonic lethality at low frequency. These strains were used to screen for drugs which interfere with UNC-53 activity and thereby suppress the background lethality.

Another related assay was used to screen specifically to identify inhibitors of downstream components in the signal transduction pathway. This assay utilised constitutively active mutant cDNA (or corresponding nucleic acid sequence). Such a mutant may be formed by mutating the nucleotide binding domain such that GTP or ATP is always bound or by covalently attaching SEM-5. In this strategy, transgenics/mutants (nematodes or tissue cultured cell lines) were generated which maintain the pathway in a permanently switched on state. Over-extension and subsequent lethality results in a greater frequency than that observed in the unc-54 - unc-53 wild-type lines. By screening for survivors after drug treatment, this assay specifically identifies inhibitors of downstream components in the signal transduction pathway.

A range of other embodiments of the assay are obvious to a person skilled in the art of C. elegans genetics, including the use of alternative selectable markers, genetic backgrounds, histochemical detection and visual detection systems to identify phenotypic changes following contacting a single worm or a population of worms with a compound.

Another assay previously described herein utilizes the unc-53 promoter. The unc-53 promoter is fused to a nucleic acid sequence encoding a reporter molecule. By screening for cells which do not express the wild type pattern, molecules which increase or reduce transcription of unc-53 may be identified.

### Example 13 - Heterologous expression of C. elegans UNC-53 in insect cells.

C. elegans UNC53 cDNAs have been expressed in a Baculovirus system to obtain sufficient amounts of protein for biochemical and structural studies.

Two UNC53 cDNA clones (UNC53(7A) and UNC53(8A) have been documented differing in the number of adenosine (A) residues (7 or 8) in a polyA stretch of the of the 3' coding region; the two clones therefore have different reading frames in the carboxyterminal coding region.

The 5' (N-terminal) part of the UNC53 coding region was excised from pTB564 with *SacII* after linearizing the plasmid with *NdeI* . The *Ndei* site was blunted with Klenow. The remaining C-terminal part of the coding region was excised from pTB68(7A) and pTB50(8A) with *SacII* plus *KpnI.* The *NdeI*/*SacII* fragment from pTB64 and the *SacII*/*KpnI* fragment from either pTB68 or pTB50 were ligated simultaneously into pBacPAK9 (Clontech) which had been linearized with *Ec1136II* (blunt end) and *KpnI*. In this way, a minimum amount of 5' untranslated region is left in the final construct.

The desired recombinant viruses were obtained by co-transfection of Sf21 cells (*Spodoptera frugiperda*) with one of the aforementioned pBacPAK9 constructs and BacPAK6 *Bsu*361-digested DNA (Clontech). Several candidate recombinant viruses plaques were picked and screened by PCR for the presence of the target gene and the absence of wild-type virus.

*Sf*9 cells were infected at a high multiplicity with UNC53(7A) or UNC53 (8A) recombinant Baculoviruses for protein expression. Proteins from whole cell lysates were separated by denaturing (SDS) polyacrylamide gel electrophoresis and transferred to nitrocellulose membranes. The expression of UNC53 in those cell lysates was confirmed by immunoreaction with a monoclonal antibody (16-49-2) to UNC53 and subsequent chemiluminescent detection (ECL^{™} Amersham). A Coomassie-stained band of the expected size was observed in lysates of *Sf*9 cells infected with UNC-53(7A) or UNC53(8A) recombinant baculoviruses, but not with control constructs. Within the accuracy of the methods, this Coomassie-stained band coincided with the largest immunoreactive band. Their estimated mass was approximately 180 kDa, which is compatible with the theoretically calculated mass (167 kDa). We therefore conclude that this band most likely corresponds to intact UNC53.

For both UNC53(7A) and UNC53(8A) baculoviral expression constructs, mostly intact recombinant UNC53-protein was detected by immunoblotting in lysates from infected cells harvested 24 hours post infection. Larger amounts of recombinant protein could be detected in lysates from cells prepared during later stages of infection (48 and 72 hours post infection) but in those preparations a considerable amount of smaller fragments (presumptive degradation products) is observed.

### Example 14

The UNC-53 protein expressed in Sf9 cells using a Baculovirus expression system is a valid tool to study its biochemical functions and a valid tool to identify interacting proteins.

3x10+6 SF9 cells infected with recombinant virus UNC53 7A(L2.3)/pBacPAK9 were resuspended in 100 microliter Phosphate Buffered Saline supplemented with 0.14 micromolar of pepstatin, 10 mM of benzamidine and 0.015 micromolar aprotinin. The cells were briefly sonicated and the obtained material was centrifuged at 30,000 g for 30 minutes at 4 degrees centrigade. The clear supernatant (soluble fraction) was frozen in 50% glycerol. An aliquot of this fraction was incubated in the cold room for 48 hrs. The protein samples were analyzed by SDS-PAGE, blotted to nitrocellulose and probed with mab 16-48-2. This showed that UNC-53 protein made in SF9 cells is soluble and stable under the conditions tested.

20 microlitres of the UNC-53 SF9 lysate were incubated with 5 microlitre GST-Sepharose beads loaded with equal amouts (approx. 10 microgram) of GST-GRB-2 or GST alone. The beads were rinsed 3 times in 500 microlitres of solution PBS-0.29. Tween 20 and eluted with 50 microliter SDS sample buffer. The eluted material was analyzed by SDS-PAGE and Western blot analysis with mab 16-48-2. UNC-53 was retained on the GST-GRB2 column and not on the GST demonstrating that UNC-53 interacts *in vitro* with GRB-2.

### Example 15

### Identification of proteins interacting with UNC-53 :

Vectors pCB50 and pCB51 were constructed as bait vectors for the yeast two hybrid system expressing resp. the full length and the carboxyterminal part of UNC-53.

pCB50 was constructed by cloning the full length UNC-53 cDNA (7A variant; *Nde*I-*Nco*I fragment from pTB74) into pAS1-CYH2 vector from Clontech. (Figure 30).

pCB51 (Figure 32) was constructed by cloning the 1880 bp *Nde*I-*Nco*I fragment from pTB74 into vector pAS1-CYH2 from Clontech. This protein encodes among others, the GTP/ATP binding domains, a leucine zipper domain, and an additional coiled-coil domain.

pCB50 and pCB51 were transformed in yeast strain Hf7C (YRG2). Expression was confirmed by western blotting using antibodies to the GAL4 protein fused to UNC-53 in these constructs. Bands of expected size (190 kd for pCB50 and 90 kd for pcB51) were observed both in yeast strains with pCB50 and pCB51 indicating that both fusion proteins are expressed in the yeast. The expression of the pCB50 and pCB51 fusion proteins in yeast strain Hf7C does not lead to expression of the LacZ or HIS reporter genes. These experiments demonstrate that the constructed fusions are useful baits in yeast two hybrid screens.

Vector pCB55 was made by cloning the 984 bp *BamH*I-*Bgl*II of pTB74 construct into the yeast two hybrid activation vector (pGAD-424 vector from Clontech) (Figure 34). In order to check the possible interactions of UNC-53 either with itself (homodimerization) or other proteins.

This vector expresses a Gal-4 activation domain fused to amongst others the predicted coiled coil or leucine zipper domain of UNC-53.

The following combinations of plasmids were co-transformed in yeast strain HF7C : (1) pCB51 and pCB55 (2) pCB55 with control plasmid- pTD1 and (3) positive control plasmids pTD1 and PVA3 (two proteins known to interact (Bartel,P.L et al., Biotechniques Vol. 14 nr.6 (1993)). Yeast cotransformed with combination (1) and (3) grew well on -LEU;-TRYP plates and -LEU;-TRYP;-HIS plates indicating that an interacting protein is present in both co-transformations. Only yeast co-transformed with (3) was positive in a lacZ assay indicating that the observed interaction in (1) (between pCB50 and pCB 55) is weak.For co-transformation (2), colonies grew on -LEU;-TRYP plates and as expected not on -LEU;-TRYP;-HIS plates. The positive control were thus positive whereas the negative controls were negative. We conclude that there is a weak but significant interaction between pCB51 and pCB55, which is strong enough to activate the HIS but not the lacZ reporter gene in this Hf7c strain.

### Example 16

### Protocol to screen for components which inhibit or enhance UNC-53 using C. elegans cell line pTBIn76

Embryos from large liquid C. elegans cultures of line pTBIn76 (table 1) are collected by sucrose flotation of a bleached population (Goh and Bogaert (1991), Dev. Biol. 56, 110-156). Embryos are dispensed in 96 well microtiter plates with M9 medium and various concentrations of the compound to be tested. The embryos are allowed to hatch and are synchronised in the L1 stage by starvation. After a suitable exposure to the compound (by standard calibration) a standard quantity of E. coli (food) is dispersed in the 96 well plates, which starts C. elegans post-embryonic development. The microtiter plates are then placed in an incubator to induce heat shock and subsequently placed at 25°C to permit continued development. After 0 to 1 generations of C. elegans development wells are inspected to assess the degree of population growth inhibition. This inspection can consist of an optical density measurement to assess the amount of food consumed by the developing nematodes. Very little food is consumed when no test compound is present: most food is consumed if an UNC-53 inhibitor has blocked the lethal or subviable phenotype induced by the transgene. The inspection can also be a visual inspection of the number of healthy or subviable worms or a histochemical measurement of C. elegans viability or of the remainder of E. coli (food).

### Example 17 - Protocol to screen for compounds which inhibit or enhance cell regulation or motility.

Transfected cells used in this example were the same as those obtained from example 8. Compounds to be tested were added to each of the cells and their effects on the cells monitored. Functional assays to determine neurite extension were also the same as used in example 8 as described by Geests *et al*. One compound (of the Formula I below) was used for further testing.

### Example 18 - Compounds targetted at the unc-53 pathway.

### Snythesis of (1-(1H-pyrrol-2-ylmethyl)-2-piperidone.

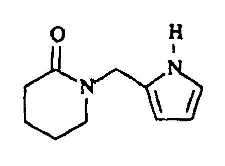

### Step 1

To a stirred solution of 150g of 1H-pyrrol-2-carboxaldehyde in 1500g parts of trichloromethane were added 690, of 5Å molecular Sieves. A kit solution of 264, of methyl 5-aminopentanoate hydrochloride in 1500g of tricholoromethane was added. After stirring for 5 minutes, 465g of thiethylamine were added over 10 minutes. Upon complete addition, the reaction mixture was stirred for 20 hours at ambient temperature. The mixture was filtered over diatomaceous earth and the filtrate was concentrated by evaporation of the solvent. The concentrate was triturated in 1,1'-oxybisethane. The precipitate was filtered off and the filtrate was concentrated, yielding 300g (91.1%) of 5-[[(1H*-*pyrrol-2-

### Step 2

A mixture of 150g of 5-[[(1H-pyrrol-2-yl)methylen]amino]pentanoate hydrogenated at 3.10⁵Pa and at ambient temperature with 3.3 parts of platinum oxide. After the calculated amount of hydrogen was consumed, the catalyst was filtered off and the filtrate was evaporated. The residue was dissolved in dichloromethane and the organic phase was washed three times with a sodium hydroxide 3 N solution. The product was distilled at 13.30 Pa (bp 100-130ºC). The residue was crystallized from cyclohexane and hexane. The product was filtered off and dried, yielding 193 parts (100%) of 1-(1H-pyrrol-2-ylmethyl)-2-piperidone. ; mp. 105.8ºC.

The compound (1-(1H-pyrrol-2-ylmethyl)-2-piperidinone) when applied for 24 hours to cultures of both wild-type and transfected N4 (mouse neuroblastoma) cells displays a differential behaviour. There is no effect (or at most a small stimulatory) effect on the wild-type N4 cells, up to concentrations of 1 *µ*M, the compound clearly becomes toxic for both types of cells. The results indicate that this compound conteracts the effects of overexpression of UNC-53 and may have beneficial effects therefore in for example metastasis.

### SEQUENCE LISTING

(1) GENERAL INFORMATION:
   (i) APPLICANT:
      (A) NAME: BOGAERT; THIERRY
      (B) STREET: Voorstraat 36 bus 11
      (C) CITY: Kortrijk
      (E) COUNTRY: Belgium
      (F) POSTAL CODE (ZIP): B-8500

      (A) NAME: STRINGHAM; EVE
      (B) STREET: 9326-133 A Street
      (C) CITY: Surrey
      (D) STATE: British Columbia
      (E) COUNTRY: Canada
      (F) POSTAL CODE (ZIP): V3V 5R5

      (A) NAME: VANDEKERCKHOVE; JOEL
      (B) STREET: Rode Benkendreef 27
      (C) CITY: Loppem
      (D) STATE: -
      (E) COUNTRY: Belgium
      (F) POSTAL CODE (ZIP): none
   (ii) TITLE OF INVENTION: Processes for the identification of compounds which control cell behaviour, the compounds identified and pharmaceutical compositions containing them and their use in the control of cell behaviour
   (iii) NUMBER OF SEQUENCES: 48
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA: APPLICATION NUMBER: EP PCT/EP96/02311
   (vi) PRIOR APPLICATION DATA:
      (A) APPLICATION NUMBER: GB 9510944.3
      (B) FILING DATE: 31-MAY-1995
(2) INFORMATION FOR SEQ ID NO: 1:
   (i) SEQUENCE CHARACTERISTICS:
      (A) LENGTH: 5073 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (vi) ORIGINAL SOURCE:
      (A) ORGANISM: Caenorhabditis elegans
   (xi) SEQUENCE DESCRIPTION: SEQ ID NO: 1:
   GGTTTAATTA CCCAAGTTTG AGACATCAAT TCCATCGAAC GAAATGTTGG TGCTCCGAAT 60
   AAAATGACGA CGTCAAATGT AGAATTGATA CCAATCTACA CGGATTGGGC CAATCGGCAC 120
   CTTTCGAAGG GCAGCTTATC AAAGTCGATT AGGGATATTT CCAATGATTT TCGCGACTAT 180
   CGACTGGTTT CTCAGCTTAT TAATGTGATC GTTCCGATCA ACGAATTCTC GCCTGCATTC 240
   ACGAAACGTT TGGCAAAAAT CACATCGAAC CTGGATGGCC TCGAAACGTG TCTCGACTAC 300
   CTGAAAAATC TGGGTCTCGA CTGCTCGAAA CTCACCAAAA CCGATATCGA CAGCGGAAAC 360
   TTGGGTGCAG TTCTCCAGCT GCTCTTCCTG CTCTCCACCT ACAAGCAGAA GCTTCGGCAA 420
   CTGAAAAAAG ATCAGAAGAA ATTGGAGCAA CTACCCACAT CCATTATGCC ACCCGCGGTT 480
   TCTAAATTAC CCTCGCCACG TGTCGCCACG TCAGCAACCG CTTCAGCAAC TAACCCAAAT 540
   TCCAACTTTC CACAAATGTC AACATCCAGG CTTCAGACTC CACAGTCAAG AATATCGAAA 600
   ATTGATTCAT CAAAGATTGG TATCAAGCCA AAGACGTCTG GACTTAAACC ACCCTCATCA 660
   TCAACCACTT CATCAAATAA TACAAATTCA TTCCGTCCGT CGAGCCGTTC GAGTGGCAAT 720
   AATAATGTTG GCTCGACGAT ATCCACATCT GCGAAGAGCT TAGAATCATC ATCAACGTAC 780
   AGCTCTATTT CGAATCTAAA CCGACCTACC TCCCAACTCC AAAAACCTTC TAGACCACAA 840
   ACCCAGCTAG TTCGTGTTGC TACAACTACA AAAATCGGAA GCTCAAAGCT AGCCGCTCCG 900
   AAAGCCGTGA GCACCCCAAA ACTTGCTTCT GTGAAGACTA TTGGAGCAAA ACAAGAGCCC 960
   GATAACAGCG GTGGTGGTGG TGGTGGAATG CTGAAATTAA AGTTATTCAG TAGCAAAAAC 1020
   CCATCTTCCT CATCGAATAG CCCACAACCT ACGAGAAAGG CGGCGGCGGT GCCTCAACAA 1080
   CAAACTTTGT CGAAAATCGC TGCCCCAGTG AAAAGTGGCC TGAAGCCGCC GACCAGTAAG 1140
   CTGGGAAGTG CCACGTCTAT GTCGAAGCTT TGTACGCCAA AAGTTTCCTA CCGTAAAACG 1200
   GACGCCCCAA TCATATCTCA ACAAGACTCG AAACGATGCT CAAAGAGCAG TGAAGAAGAG 1260
   TCCGGATACG CTGGATTCAA CAGCACGTCG CCAACGTCAT CATCGACGGA AGGTTCCCTA 1320
   AGCATGCATT CCACATCTTC CAAGAGTTCA ACGTCAGACG AAAAGTCTCC GTCATCAGAC 1380
   GATCTTACTC TTAACGCCTC CATCGTGACA GCTATCAGAC AGCCGATAGC CGCAACACCG 1440
   GTTTCTCCAA ATATTATCAA CAAGCCTGTT GAGGAAAAAC CAACACTGGC AGTGAAAGGA 1500
   GTGAAAAGCA CAGCGAAAAA AGATCCACCT CCAGCTGTTC CGCCACGTGA CACCCAGCCA 1560
   ACAATCGGAG TTGTTAGTCC AATTATGGCA CATAAGAAGT TGACAAATGA CCCCGTGATA 1620
   TCTGAAAAAC CAGAACCTGA AAAGCTCCAA TCAATGAGCA TCGACACGAC GGACGTTCCA 1680
   CCGCTTCCAC CTCTAAAATC AGTTGTTCCA CTTAAAATGA CTTCAATCCG ACAACCACCA 1740
   ACGTACGATG TTCTTCTAAA ACAAGGAAAA ATCACATCGC CTGTCAAGTC GTTTGGATAT 1800
   GAGCAGTCGT CCGCGTCTGA AGACTCCATT GTGGCTCATG CGTCGGCTCA GGTGACTCCG 1860
   CCGACAAAAA CTTCTGGTAA TCATTCGCTG GAGAGAAGGA TGGGAAAGAA TAAGACATCA 1920
   GAATCCAGCG GCTACACCTC TGACGCCGGT GTTGCGATGT GCGCCAAAAT GAGGGAGAAG 1980
   CTGAAAGAAT ACGATGACAT GACTCGTCGA GCACAGAACG GCTATCCTGA CAACTTCGAA 2040
   GACAGTTCCT CCTTGTCGTC TGGAATATCC GATAACAACG AGCTCGACGA CATATCCACG 2100
   GACGATTTGT CCGGAGTAGA CATGGCAACA GTCGCCTCCA AACATAGCGA CTATTCCCAC 2160
   TTTGTTCGCC ATCCCACGTC TTCTTCCTCA AAGCCCCGAG TCCCCAGTCG GTCCTCCACA 2220
   TCAGTCGATT CTCGATCTCG AGCAGAACAG GAGAATGTGT ACAAACTTCT GTCCCAGTGC 2280
   CGAACGAGCC AACGTGGCGC CGCTGCCACC TCAACCTTCG GACAACATTC GCTAAGATCC 2340
   CCGGGATACT CATCCTATTC TCCACACTTA TCAGTGTCAG CTGATAAGGA CACAATGTCT 2400
   ATGCACTCAC AGACTAGTCG ACGACCTTCT TCACAAAAAC CAAGCTATTC AGGCCAATTT 2460
   CATTCACTTG ATCGTAAATG CCACCTTCAA GAGTTCACAT CCACCGAGCA CAGAATGGCG 2520
   GCTCTCTTGA GCCCGAGACG GGTGCCGAAC TCGATGTCGA AATATGATTC TTCAGGATCC 2580
   TACTCGGCGC GTTCCCGAGG TGGAAGCTCT ACTGGTATCT ATGGAGAGAC GTTCCAACTG 2640
   CACAGACTAT CCGATGAAAA ATCCCCCGCA CATTCTGCCA AAAGTGAGAT GGGATCCCAA 2700
   CTATCACTGG CTAGCACGAC AGCATATGGA TCTCTCAATG AGAAGTACGA ACATGCTATT 2760
   CGGGACATGG CACGTGACTT GGAGTGTTAC AAGAACACTG TCGACTCACT AACCAAGAAA 2820
   CAGGAGAACT ATGGAGCATT GTTTGATCTT TTTGAGCAAA AGCTTAGAAA ACTCACTCAA 2880
   CACATTGATC GATCCAACTT GAAGCCTGAA GAGGCAATAC GATTCAGGCA GGACATTGCT 2940
   CATTTGAGGG ATATTAGCAA TCATCTTGCA TCCAACTCAG CTCATGCTAA CGAAGGCGCT 3000
   GGTGAGCTTC TTCGTCAACC ATCTCTGGAA TCAGTTGCAT CCCATCGATC ATCGATGTCA 3060
   TCGTCGTCGA AAAGCAGCAA GCAGGAGAAG ATCAGCTTGA GCTCGTTTGG CAAGAACAAG 3120
   AAGAGCTGGA TCCGCTCCTC ACTCTCCAAG TTCACCAAGA AGAAGAACAA GAACTACGAC 3180
   GAAGCACATA TGCCATCAAT TTCCGGATCT CAAGGAACTC TTGACAACAT TGATGTGATT 3240
   GAGTTGAAGC AAGAGCTCAA AGAACGCGAT AGTGCACTTT ACGAAGTCCG CCTTGACAAT 3300
   CTGGATCGTG CCCGCGAAGT TGATGTTCTG AGGGAGACAG TGAACAAGTT GAAAACCGAG 3360
   AACAAGCAAT TAAAGAAAGA AGTGGACAAA CTCACCAACG GTCCAGCCAC TCGTGCTTCT 3420
   TCCCGCGCCT CAATTCCAGT TATCTACGAC GATGAGCATG TCTATGATGC AGCGTGTAGC 3480
   AGTACATCAG CTAGTCAATC TTCGAAACGA TCCTCTGGCT GCAACTCAAT CAAGGTTACT 3540
   GTAAACGTGG ACATCGCTGG AGAAATCAGT TCGATCGTTA ACCCGGACAA AGAGATAATC 3600
   GTAGGATATC TTGCCATGTC AACCAGTCAG TCATGCTGGA AAGACATTGA TGTTTCTATT 3660
   CTAGGACTAT TTGAAGTCTA CCTATCCAGA ATTGATGTGG AGCATCAACT TGGAATCGAT 3720
   GCTCGTGATT CTATCCTTGG CTATCAAATT GGTGAACTTC GACGCGTCAT TGGAGACTCC 3780
   ACAACCATGA TAACCAGCCA TCCAACTGAC ATTCTTACTT CCTCAACTAC AATCCGAATG 3840
   TTCATGCACG GTGCCGCACA GAGTCGCGTA GACAGTCTGG TCCTTGATAT GCTTCTTCCA   3900
   AAGCAAATGA TTCTCCAACT CGTCAAGTCA ATTTTGACAG AGAGACGTCT GGTGTTAGCT   3960
   GGAGCAACTG GAATTGGAAA GAGCAAACTG GCGAAGACCC TGGCTGCTTA TGTATCTATT   4020
   CGAACAAATC AATCCGAAGA TAGTATTGTT AATATCAGCA TTCCTGAAAA CAATAAAGAA   4080
   GAATTGCTTC AAGTGGAACG ACGCCTGGAA AAGATCTTGA GAAGCAAAGA ATCATGCATC   4140
   GTAATTCTAG ATAATATCCC AAAGAATCGA ATTGCATTTG TTGTATCCGT TTTTGCAAAT   4200
   GTCCCACTTC AAAACAACGA AGGTCCATTT GTAGTATGCA CAGTCAACCG ATATCAAATC   4260
   CCTGAGCTTC AAATTCACCA CAATTTCAAA ATGTCAGTAA TGTCGAATCG TCTCGAAGGA   4320
   TTCATCCTAC GTTACCTCCG ACGACGGGCG GTAGAGGATG AGTATCGTCT AACTGTACAG   4380
   ATGCCATCAG AGCTCTTCAA AATCATTGAC TTCTTCCCAA TAGCTCTTCA GGCCGTCAAT   4440
   AATTTTATTG AGAAAACGAA TTCTGTTGAT GTGACAGTTG GTCCAAGAGC ATGCTTGAAC   4500
   TGTCCTCTAA CTGTCGATGG ATCCCGTGAA TGGTTCATTC GATTGTGGAA TGAGAACTTC   4560
   ATTCCATATT TGGAACGTGT TGCTAGAGAT GGCAAAAAAA ACCTTCGGTC GCTGCACTTC   4620
   CTTCGAGGAT CCCACCGACA TCGTCTCTAA AAAATGGCCG TGGTTCGATG GTGAAAACCC   4680
   GGAGAATGTG CTCAAACGTC TTCAACTCCA AGACCTCGTC CCGTCACCTG CCAACTCATC   4740
   CCGACAACAC TTCAATCCCC TCGAGTCGTT GATCCAATTG CATGCTACCA AGCATCAGAC   4800
   CATCGACAAC ATTTGAACAG AAGACTCTAA TCTTCTCTCG CCTCTCCCCC GCTTTCCTTA   4860
   TCTTCGTACC GGTACCTGAT GATTCCCCAT TTTCCCCCTT TTCCCCCCAA TTTCCCAGAA   4920
   CCTCCTGTTC CCTTTGTTCC TAGTCCTCCC GGGTGCCGAC GCCGAAGCGA TTTAAAAACC   4980
   TTTTTCTTTC CGAAACATTT CCCATTGCTC ATTAATAGTC AAATTGAATA AACAGTGTAT   5040
   GTACTTAAAA AAAAAAAAAA AAAAAAAAAA AAA                                 5073
(2) INFORMATION FOR SEQ ID NO: 2:
   (i) SEQUENCE CHARACTERISTICS:
      (A) LENGTH: 5072 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO: 2:
   GGTTTAATTA CCCAAGTTTG AGACATCAAT TCCATCGAAC GAAATGTTGG TGCTCCGAAT     60
   AAAATGACGA CGTCAAATGT AGAATTGATA CCAATCTACA CGGATTGGGC CAATCGGCAC    120
   CTTTCGAAGG GCAGCTTATC AAAGTCGATT AGGGATATTT CCAATGATTT TCGCGACTAT    180
   CGACTGGTTT CTCAGCTTAT TAATGTGATC GTTCCGATCA ACGAATTCTC GCCTGCATTC 240
   ACGAAACGTT TGGCAAAAAT CACATCGAAC CTGGATGGCC TCGAAACGTG TCTCGACTAC 300
   CTGAAAAATC TGGGTCTCGA CTGCTCGAAA CTCACCAAAA CCGATATCGA CAGCGGAAAC 360
   TTGGGTGCAG TTCTCCAGCT GCTCTTCCTG CTCTCCACCT ACAAGCAGAA GCTTCGGCAA 420
   CTGAAAAAAG ATCAGAAGAA ATTGGAGCAA CTACCCACAT CCATTATGCC ACCCGCGGTT 480
   TCTAAATTAC CCTCGCCACG TGTCGCCACG TCAGCAACCG CTTCAGCAAC TAACCCAAAT 540
   TCCAACTTTC CACAAATGTC AACATCCAGG CTTCAGACTC CACAGTCAAG AATATCGAAA 600
   ATTGATTCAT CAAAGATTGG TATCAAGCCA AAGACGTCTG GACTTAAACC ACCCTCATCA 660
   TCAACCACTT CATCAAATAA TACAAATTCA TTCCGTCCGT CGAGCCGTTC GAGTGGCAAT 720
   AATAATGTTG GCTCGACGAT ATCCACATCT GCGAAGAGCT TAGAATCATC ATCAACGTAC 780
   AGCTCTATTT CGAATCTAAA CCGACCTACC TCCCAACTCC AAAAACCTTC TAGACCACAA 840
   ACCCAGCTAG TTCGTGTTGC TACAACTACA AAAATCGGAA GCTCAAAGCT AGCCGCTCCG 900
   AAAGCCGTGA GCACCCCAAA ACTTGCTTCT GTGAAGACTA TTGGAGCAAA ACAAGAGCCC 960
   GATAACAGCG GTGGTGGTGG TGGTGGAATG CTGAAATTAA AGTTATTCAG TAGCAAAAAC 1020
   CCATCTTCCT CATCGAATAG CCCACAACCT ACGAGAAAGG CGGCGGCGGT GCCTCAACAA 1080
   CAAACTTTGT CGAAAATCGC TGCCCCAGTG AAAAGTGGCC TGAAGCCGCC GACCAGTAAG 1140
   CTGGGAAGTG CCACGTCTAT GTCGAAGCTT TGTACGCCAA AAGTTTCCTA CCGTAAAACG 1200
   GACGCCCCAA TCATATCTCA ACAAGACTCG AAACGATGCT CAAAGAGCAG TGAAGAAGAG 1260
   TCCGGATACG CTGGATTCAA CAGCACGTCG CCAACGTCAT CATCGACGGA AGGTTCCCTA 1320
   AGCATGCATT CCACATCTTC CAAGAGTTCA ACGTCAGACG AAAAGTCTCC GTCATCAGAC 1380
   GATCTTACTC TTAACGCCTC CATCGTGACA GCTATCAGAC AGCCGATAGC CGCAACACCG 1440
   GTTTCTCCAA ATATTATCAA CAAGCCTGTT GAGGAAAAAC CAACACTGGC AGTGAAAGGA 1500
   GTGAAAAGCA CAGCGAAAAA AGATCCACCT CCAGCTGTTC CGCCACGTGA CACCCAGCCA 1560
   ACAATCGGAG TTGTTAGTCC AATTATGGCA CATAAGAAGT TGACAAATGA CCCCGTGATA 1620
   TCTGAAAAAC CAGAACCTGA AAAGCTCCAA TCAATGAGCA TCGACACGAC GGACGTTCCA 1680
   CCGCTTCCAC CTCTAAAATC AGTTGTTCCA CTTAAAATGA CTTCAATCCG ACAACCACCA 1740
   ACGTACGATG TTCTTCTAAA ACAAGGAAAA ATCACATCGC CTGTCAAGTC GTTTGGATAT 1800
   GAGCAGTCGT CCGCGTCTGA AGACTCCATT GTGGCTCATG CGTCGGCTCA GGTGACTCCG 1860
   CCGACAAAAA CTTCTGGTAA TCATTCGCTG GAGAGAAGGA TGGGAAAGAA TAAGACATCA 1920
   GAATCCAGCG GCTACACCTC TGACGCCGGT GTTGCGATGT GCGCCAAAAT GAGGGAGAAG 1980
   CTGAAAGAAT ACGATGACAT GACTCGTCGA GCACAGAACG GCTATCCTGA CAACTTCGAA 2040
   GACAGTTCCT CCTTGTCGTC TGGAATATCC GATAACAACG AGCTCGACGA CATATCCACG 2100
   GACGATTTGT CCGGAGTAGA CATGGCAACA GTCGCCTCCA AACATAGCGA CTATTCCCAC 2160
   TTTGTTCGCC ATCCCACGTC TTCTTCCTCA AAGCCCCGAG TCCCCAGTCG GTCCTCCACA 2220
   TCAGTCGATT CTCGATCTCG AGCAGAACAG GAGAATGTGT ACAAACTTCT GTCCCAGTGC 2280
   CGAACGAGCC AACGTGGCGC CGCTGCCACC TCAACCTTCG GACAACATTC GCTAAGATCC 2340
   CCGGGATACT CATCCTATTC TCCACACTTA TCAGTGTCAG CTGATAAGGA CACAATGTCT 2400
   ATGCACTCAC AGACTAGTCG ACGACCTTCT TCACAAAAAC CAAGCTATTC AGGCCAATTT 2460
   CATTCACTTG ATCGTAAATG CCACCTTCAA GAGTTCACAT CCACCGAGCA CAGAATGGCG 2520
   GCTCTCTTGA GCCCGAGACG GGTGCCGAAC TCGATGTCGA AATATGATTC TTCAGGATCC 2580
   TACTCGGCGC GTTCCCGAGG TGGAAGCTCT ACTGGTATCT ATGGAGAGAC GTTCCAACTG 2640
   CACAGACTAT CCGATGAAAA ATCCCCCGCA CATTCTGCCA AAAGTGAGAT GGGATCCCAA 2700
   CTATCACTGG CTAGCACGAC AGCATATGGA TCTCTCAATG AGAAGTACGA ACATGCTATT 2760
   CGGGACATGG CACGTGACTT GGAGTGTTAC AAGAACACTG TCGACTCACT AACCAAGAAA 2820
   CAGGAGAACT ATGGAGCATT GTTTGATCTT TTTGAGCAAA AGCTTAGAAA ACTCACTCAA 2880
   CACATTGATC GATCCAACTT GAAGCCTGAA GAGGCAATAC GATTCAGGCA GGACATTGCT 2940
   CATTTGAGGG ATATTAGCAA TCATCTTGCA TCCAACTCAG CTCATGCTAA CGAAGGCGCT 3000
   GGTGAGCTTC TTCGTCAACC ATCTCTGGAA TCAGTTGCAT CCCATCGATC ATCGATGTCA 3060
   TCGTCGTCGA AAAGCAGCAA GCAGGAGAAG ATCAGCTTGA GCTCGTTTGG CAAGAACAAG 3120
   AAGAGCTGGA TCCGCTCCTC ACTCTCCAAG TTCACCAAGA AGAAGAACAA GAACTACGAC 3180
   GAAGCACATA TGCCATCAAT TTCCGGATCT CAAGGAACTC TTGACAACAT TGATGTGATT 3240
   GAGTTGAAGC AAGAGCTCAA AGAACGCGAT AGTGCACTTT ACGAAGTCCG CCTTGACAAT 3300
   CTGGATCGTG CCCGCGAAGT TGATGTTCTG AGGGAGACAG TGAACAAGTT GAAAACCGAG 3360
   AACAAGCAAT TAAAGAAAGA AGTGGACAAA CTCACCAACG GTCCAGCCAC TCGTGCTTCT 3420
   TCCCGCGCCT CAATTCCAGT TATCTACGAC GATGAGCATG TCTATGATGC AGCGTGTAGC 3480
   AGTACATCAG CTAGTCAATC TTCGAAACGA TCCTCTGGCT GCAACTCAAT CAAGGTTACT 3540
   GTAAACGTGG ACATCGCTGG AGAAATCAGT TCGATCGTTA ACCCGGACAA AGAGATAATC 3600
   GTAGGATATC TTGCCATGTC AACCAGTCAG TCATGCTGGA AAGACATTGA TGTTTCTATT 3660
   CTAGGACTAT TTGAAGTCTA CCTATCCAGA ATTGATGTGG AGCATCAACT TGGAATCGAT 3720
   GCTCGTGATT CTATCCTTGG CTATCAAATT GGTGAACTTC GACGCGTCAT TGGAGACTCC 3780
   ACAACCATGA TAACCAGCCA TCCAACTGAC ATTCTTACTT CCTCAACTAC AATCCGAATG 3840
   TTCATGCACG GTGCCGCACA GAGTCGCGTA GACAGTCTGG TCCTTGATAT GCTTCTTCCA 3900
   AAGCAAATGA TTCTCCAACT CGTCAAGTCA ATTTTGACAG AGAGACGTCT GGTGTTAGCT 3960
   GGAGCAACTG GAATTGGAAA GAGCAAACTG GCGAAGACCC TGGCTGCTTA TGTATCTATT 4020
   CGAACAAATC AATCCGAAGA TAGTATTGTT AATATCAGCA TTCCTGAAAA CAATAAAGAA    4080
   GAATTGCTTC AAGTGGAACG ACGCCTGGAA AAGATCTTGA GAAGCAAAGA ATCATGCATC    4140
   GTAATTCTAG ATAATATCCC AAAGAATCGA ATTGCATTTG TTGTATCCGT TTTTGCAAAT    4200
   GTCCCACTTC AAAACAACGA AGGTCCATTT GTAGTATGCA CAGTCAACCG ATATCAAATC    4260
   CCTGAGCTTC AAATTCACCA CAATTTCAAA ATGTCAGTAA TGTCGAATCG TCTCGAAGGA    4320
   TTCATCCTAC GTTACCTCCG ACGACGGGCG GTAGAGGATG AGTATCGTCT AACTGTACAG    4380
   ATGCCATCAG AGCTCTTCAA AATCATTGAC TTCTTCCCAA TAGCTCTTCA GGCCGTCAAT    4440
   AATTTTATTG AGAAAACGAA TTCTGTTGAT GTGACAGTTG GTCCAAGAGC ATGCTTGAAC    4500
   TGTCCTCTAA CTGTCGATGG ATCCCGTGAA TGGTTCATTC GATTGTGGAA TGAGAACTTC    4560
   ATTCCATATT TGGAACGTGT TGCTAGAGAT GGCAAAAAAA CCTTCGGTCG CTGCACTTCC    4620
   TTCGAGGATC CCACCGACAT CGTCTCTAAA AAATGGCCGT GGTTCGATGG TGAAAACCCG    4680
   GAGAATGTGC TCAAACGTCT TCAACTCCAA GACCTCGTCC CGTCACCTGC CAACTCATCC    4740
   CGACAACACT TCAATCCCCT CGAGTCGTTG ATCCAATTGC ATGCTACCAA GCATCAGACC    4800
   ATCGACAACA TTTGAACAGA AGACTCTAAT CTTCTCTCGC CTCTCCCCCG CTTTCCTTAT    4860
   CTTCGTACCG GTACCTGATG ATTCCCCATT TTCCCCCTTT TCCCCCCAAT TTCCCAGAAC    4920
   CTCCTGTTCC CTTTGTTCCT AGTCCTCCCG GGTGCCGACG CCGAAGCGAT TTAAAAACCT    4980
   TTTTCTTTCC GAAACATTTC CCATTGCTCA TTAATAGTCA AATTGAATAA ACAGTGTATG    5040
   TACTTAAAAA AAAAAAAAAA AAAAAAAAAA AA                                  5072
(2) INFORMATION FOR SEQ ID NO: 3:
   (i) SEQUENCE CHARACTERISTICS:
      (A) LENGTH: 1528 amino acids
      (B) TYPE: amino acid
      (C) STRANDEDNESS:
      (D) TOPOLOGY: unknown
   (ii) MOLECULE TYPE: protein
   (xi) SEQUENCE DESCRIPTION: SEQ ID NO: 3:
   Met Thr Thr Ser Asn Val Glu Leu Ile Pro Ile Tyr Thr Asp Trp Ala
   1               5                   10                  15
   Asn Arg His Leu Ser Lys Gly Ser Leu Ser Lys Ser Ile Arg Asp Ile
               20                  25                  30
   Ser Asn Asp Phe Arg Asp Tyr Arg Leu Val Ser Gln Leu Ile Asn Val
           35                  40                  45
   Ile Val Pro Ile Asn Glu Phe Ser Pro Ala Phe Thr Lys Arg Leu Ala
       50                  55                  60
   Lys Ile Thr Ser Asn Leu Asp Gly Leu Glu Thr Cys Leu Asp Tyr Leu
   65 70 75 80
   Lys Asn Leu Gly Leu Asp Cys Ser Lys Leu Thr Lys Thr Asp Ile Asp
   85 90 95
   Ser Gly Asn Leu Gly Ala Val Leu Gln Leu Leu Phe Leu Leu Ser Thr
   100 105 110
   Tyr Lys Gln Lys Leu Arg Gln Leu Lys Lys Asp Gln Lys Lys Leu Glu
   115 120 125
   Gln Leu Pro Thr Ser Ile Met Pro Pro Ala Val Ser Lys Leu Pro Ser
   130 135 140
   Pro Arg Val Ala Thr Ser Ala Thr Ala Ser Ala Thr Asn Pro Asn Ser
   145 150 155 160
   Asn Phe Pro Gln Met Ser Thr Ser Arg Leu Gln Thr Pro Gln Ser Arg
   165 170 175
   Ile Ser Lys Ile Asp Ser Ser Lys Ile Gly Ile Lys Pro Lys Thr Ser
   180 185 190
   Gly Leu Lys Pro Pro Ser Ser Ser Thr Thr Ser Ser Asn Asn Thr Asn
   195 200 205
   Ser Phe Arg Pro Ser Ser Arg Ser Ser Gly Asn Asn Asn Val Gly Ser
   210 215 220
   Thr Ile Ser Thr Ser Ala Lys Ser Leu Glu Ser Ser Ser Thr Tyr Ser
   225 230 235 240
   Ser Ile Ser Asn Leu Asn Arg Pro Thr Ser Gln Leu Gln Lys Pro Ser
   245 250 255
   Arg Pro Gln Thr Gln Leu Val Arg Val Ala Thr Thr Thr Lys Ile Gly
   260 265 270
   Ser Ser Lys Leu Ala Ala Pro Lys Ala Val Ser Thr Pro Lys Leu Ala
   275 280 285
   Ser Val Lys Thr Ile Gly Ala Lys Gln Glu Pro Asp Asn Ser Gly Gly
   290 295 300
   Gly Gly Gly Gly Met Leu Lys Leu Lys Leu Phe Ser Ser Lys Asn Pro
   305 310 315 320
   Ser Ser Ser Ser Asn Ser Pro Gln Pro Thr Arg Lys Ala Ala Ala Val
   325 330 335
   Pro Gln Gln Gln Thr Leu Ser Lys Ile Ala Ala Pro Val Lys Ser Gly
   340 345 350
   Leu Lys Pro Pro Thr Ser Lys Leu Gly Ser Ala Thr Ser Met Ser Lys
   355 360 365
   Leu Cys Thr Pro Lys Val Ser Tyr Arg Lys Thr Asp Ala Pro Ile Ile
   370 375 380
   Ser Gln Gln Asp Ser Lys Arg Cys Ser Lys Ser Ser Glu Glu Glu Ser
   385 390 395 400
   Gly Tyr Ala Gly Phe Asn Ser Thr Ser Pro Thr Ser Ser Ser Thr Glu
                   405                 410                 415
   Gly Ser Leu Ser Met His Ser Thr Ser Ser Lys Ser Ser Thr Ser Asp
               420                 425                 430
   Glu Lys Ser Pro Ser Ser Asp Asp Leu Thr Leu Asn Ala Ser Ile Val
           435                 440                 445
   Thr Ala Ile Arg Gln Pro Ile Ala Ala Thr Pro Val Ser Pro Asn Ile
       450                 455                 460
   Ile Asn Lys Pro Val Glu Glu Lys Pro Thr Leu Ala Val Lys Gly Val
   465                 470                 475                 480
   Lys Ser Thr Ala Lys Lys Asp Pro Pro Pro Ala Val Pro Pro Arg Asp
                   485                 490                 495
   Thr Gln Pro Thr Ile Gly Val Val Ser Pro Ile Met Ala His Lys Lys
               500                 505                 510
   Leu Thr Asn Asp Pro Val Ile Ser Glu Lys Pro Glu Pro Glu Lys Leu
           515                 520                 525
   Gln Ser Met Ser Ile Asp Thr Thr Asp Val Pro Pro Leu Pro Pro Leu
       530                 535                 540
   Lys Ser Val Val Pro Leu Lys Met Thr Ser Ile Arg Gln Pro Pro Thr
   545                 550                 555                 560
   Tyr Asp Val Leu Leu Lys Gln Gly Lys Ile Thr Ser Pro Val Lys Ser
                   565                 570                 575
   Phe Gly Tyr Glu Gln Ser Ser Ala Ser Glu Asp Ser Ile Val Ala His
               580                 585                 590
   Ala Ser Ala Gln Val Thr Pro Pro Thr Lys Thr Ser Gly Asn His Ser
           595                 600                 605
   Leu Glu Arg Arg Met Gly Lys Asn Lys Thr Ser Glu Ser Ser Gly Tyr
       610                 615                 620
   Thr Ser Asp Ala Gly Val Ala Met Cys Ala Lys Met Arg Glu Lys Leu
   625                 630                 635                 640
   Lys Glu Tyr Asp Asp Met Thr Arg Arg Ala Gln Asn Gly Tyr Pro Asp
                   645                 650                 655
   Asn Phe Glu Asp Ser Ser Ser Leu Ser Ser Gly Ile Ser Asp Asn Asn
               660                 665                 670
   Glu Leu Asp Asp Ile Ser Thr Asp Asp Leu Ser Gly Val Asp Met Ala
           675                 680                 685
   Thr Val Ala Ser Lys His Ser Asp Tyr Ser His Phe Val Arg His Pro
       690                 695                 700
   Thr Ser Ser Ser Ser Lys Pro Arg Val Pro Ser Arg Ser Ser Thr Ser
   705                 710                 715                 720
   Val Asp Ser Arg Ser Arg Ala Glu Gln Glu Asn Val Tyr Lys Leu Leu
                   725                 730                 735
   Ser Gln Cys Arg Thr Ser Gln Arg Gly Ala Ala Ala Thr Ser Thr Phe
               740                 745                 750
   Gly Gln His Ser Leu Arg Ser Pro Gly Tyr Ser Ser Tyr Ser Pro His
           755                 760                 765
   Leu Ser Val Ser Ala Asp Lys Asp Thr Met Ser Met His Ser Gln Thr
       770                 775                 780
   Ser Arg Arg Pro Ser Ser Gln Lys Pro Ser Tyr Ser Gly Gln Phe His
   785                 790                 795                 800
   Ser Leu Asp Arg Lys Cys His Leu Gln Glu Phe Thr Ser Thr Glu His
                   805                 810                 815
   Arg Met Ala Ala Leu Leu Ser Pro Arg Arg Val Pro Asn Ser Met Ser
               820                 825                 830
   Lys Tyr Asp Ser Ser Gly Ser Tyr Ser Ala Arg Ser Arg Gly Gly Ser
           835                 840                 845
   Ser Thr Gly Ile Tyr Gly Glu Thr Phe Gln Leu His Arg Leu Ser Asp
       850                 855                 860
   Glu Lys Ser Pro Ala His Ser Ala Lys Ser Glu Met Gly Ser Gln Leu
   865                 870                 875                 880
   Ser Leu Ala Ser Thr Thr Ala Tyr Gly Ser Leu Asn Glu Lys Tyr Glu
                   885                 890                 895
   His Ala Ile Arg Asp Met Ala Arg Asp Leu Glu Cys Tyr Lys Asn Thr
               900                 905                 910
   Val Asp Ser Leu Thr Lys Lys Gln Glu Asn Tyr Gly Ala Leu Phe Asp
           915                 920                 925
   Leu Phe Glu Gln Lys Leu Arg Lys Leu Thr Gln His Ile Asp Arg Ser
       930                 935                 940
   Asn Leu Lys Pro Glu Glu Ala Ile Arg Phe Arg Gln Asp Ile Ala His
   945                 950                 955                 960
   Leu Arg Asp Ile Ser Asn His Leu Ala Ser Asn Ser Ala His Ala Asn
                   965                 970                 975
   Glu Gly Ala Gly Glu Leu Leu Arg Gln Pro Ser Leu Glu Ser Val Ala
               980                 985                 990
   Ser His Arg Ser Ser Met Ser Ser Ser Ser Lys Ser Ser Lys Gln Glu
           995                 1000                1005
   Lys Ile Ser Leu Ser Ser Phe Gly Lys Asn Lys Lys Ser Trp Ile Arg
       1010                1015                1020
   Ser Ser Leu Ser Lys Phe Thr Lys Lys Lys Asn Lys Asn Tyr Asp Glu
   1025                1030                1035                1040
   Ala His Met Pro Ser Ile Ser Gly Ser Gln Gly Thr Leu Asp Asn Ile
                   1045                1050                1055
   Asp Val Ile Glu Leu Lys Gln Glu Leu Lys Glu Arg Asp Ser Ala Leu
               1060                1065                1070
   Tyr Glu Val Arg Leu Asp Asn Leu Asp Arg Ala Arg Glu Val Asp Val
         1075                1080                1085
   Leu Arg Glu Thr Val Asn Lys Leu Lys Thr Glu Asn Lys Gln Leu Lys
      1090                1095                1100
   Lys Glu Val Asp Lys Leu Thr Asn Gly Pro Ala Thr Arg Ala Ser Ser
   1105                1110                1115                1120
   Arg Ala Ser Ile Pro Val Ile Tyr Asp Asp Glu His Val Tyr Asp Ala
               1125                1130                1135
   Ala Cys Ser Ser Thr Ser Ala Ser Gln Ser Ser Lys Arg Ser Ser Gly
            1140                1145                1150
   Cys Asn Ser Ile Lys Val Thr Val Asn Val Asp Ile Ala Gly Glu Ile
         1155                1160                1165
   Ser Ser Ile Val Asn Pro Asp Lys Glu Ile Ile Val Gly Tyr Leu Ala
      1170                1175                1180
   Met Ser Thr Ser Gln Ser Cys Trp Lys Asp Ile Asp Val Ser Ile Leu
   1185                1190                1195                1200
   Gly Leu Phe Glu Val Tyr Leu Ser Arg Ile Asp Val Glu His Gln Leu
               1205                1210                1215
   Gly Ile Asp Ala Arg Asp Ser Ile Leu Gly Tyr Gln Ile Gly Glu Leu
            1220                1225                1230
   Arg Arg Val Ile Gly Asp Ser Thr Thr Met Ile Thr Ser His Pro Thr
         1235                1240                1245
   Asp Ile Leu Thr Ser Ser Thr Thr Ile Arg Met Phe Met His Gly Ala
      1250                1255                1260
   Ala Gln Ser Arg Val Asp Ser Leu Val Leu Asp Met Leu Leu Pro Lys
   1265                1270                1275                1280
   Gln Met Ile Leu Gln Leu Val Lys Ser Ile Leu Thr Glu Arg Arg Leu
               1285                1290                1295
   Val Leu Ala Gly Ala Thr Gly Ile Gly Lys Ser Lys Leu Ala Lys Thr
            1300                1305                1310
   Leu Ala Ala Tyr Val Ser Ile Arg Thr Asn Gln Ser Glu Asp Ser Ile
         1315                1320                1325
   Val Asn Ile Ser Ile Pro Glu Asn Asn Lys Glu Glu Leu Leu Gln Val
      1330                1335                1340
   Glu Arg Arg Leu Glu Lys Ile Leu Arg Ser Lys Glu Ser Cys Ile Val
   1345                1350                1355                1360
   Ile Leu Asp Asn Ile Pro Lys Asn Arg Ile Ala Phe Val Val Ser Val
               1365                1370                1375
   Phe Ala Asn Val Pro Leu Gln Asn Asn Glu Gly Pro Phe Val Val Cys
            1380                1385                1390
   Thr Val Asn Arg Tyr Gln Ile Pro Glu Leu Gln Ile His His Asn Phe
         1395                1400                1405
   Lys Met Ser Val Met Ser Asn Arg Leu Glu Gly Phe Ile Leu Arg Tyr
       1410                1415                1420
   Leu Arg Arg Arg Ala Val Glu Asp Glu Tyr Arg Leu Thr Val Gln Met
   1425                1430                1435                1440
   Pro Ser Glu Leu Phe Lys Ile Ile Asp Phe Phe Pro Ile Ala Leu Gln
                   1445                1450                1455
   Ala Val Asn Asn Phe Ile Glu Lys Thr Asn Ser Val Asp Val Thr Val
               1460                1465                1470
   Gly Pro Arg Ala Cys Leu Asn Cys Pro Leu Thr Val Asp Gly Ser Arg
           1475                1480                1485
   Glu Trp Phe Ile Arg Leu Trp Asn Glu Asn Phe Ile Pro Tyr Leu Glu
       1490                1495                1500
   Arg Val Ala Arg Asp Gly Lys Lys Asn Leu Arg Ser Leu His Phe Leu
   1505                1510                1515                1520
   Arg Gly Ser His Arg His Arg Leu
                   1525
(2) INFORMATION FOR SEQ ID NO: 4:
   (i) SEQUENCE CHARACTERISTICS:
      (A) LENGTH: 1583 amino acids
      (B) TYPE: amino acid
      (C) STRANDEDNESS:
      (D) TOPOLOGY: unknown
   (ii) MOLECULE TYPE: protein
   (xi) SEQUENCE DESCRIPTION: SEQ ID NO: 4:
   Met Thr Thr Ser Asn Val Glu Leu Ile Pro Ile Tyr Thr Asp Trp Ala
   1               5                   10                  15
   Asn Arg His Leu Ser Lys Gly Ser Leu Ser Lys Ser Ile Arg Asp Ile
               20                  25                  30
   Ser Asn Asp Phe Arg Asp Tyr Arg Leu Val Ser Gln Leu Ile Asn Val
           35                  40                  45
   Ile Val Pro Ile Asn Glu Phe Ser Pro Ala Phe Thr Lys Arg Leu Ala
       50                  55                  60
   Lys Ile Thr Ser Asn Leu Asp Gly Leu Glu Thr Cys Leu Asp Tyr Leu
   65                  70                  75                  80
   Lys Asn Leu Gly Leu Asp Cys Ser Lys Leu Thr Lys Thr Asp Ile Asp
                   85                  90                  95
   Ser Gly Asn Leu Gly Ala Val Leu Gln Leu Leu Phe Leu Leu Ser Thr
               100                 105                 110
   Tyr Lys Gln Lys Leu Arg Gln Leu Lys Lys Asp Gln Lys Lys Leu Glu
           115                 120                 125
   Gln Leu Pro Thr Ser Ile Met Pro Pro Ala Val Ser Lys Leu Pro Ser
       130                 135                 140
   Pro Arg Val Ala Thr Ser Ala Thr Ala Ser Ala Thr Asn Pro Asn Ser
   145                 150                 155                 160
   Asn Phe Pro Gln Met Ser Thr Ser Arg Leu Gln Thr Pro Gln Ser Arg
                   165                 170                 175
   Ile Ser Lys Ile Asp Ser Ser Lys Ile Gly Ile Lys Pro Lys Thr Ser
               180                 185                 190
   Gly Leu Lys Pro Pro Ser Ser Ser Thr Thr Ser Ser Asn Asn Thr Asn
           195                 200                 205
   Ser Phe Arg Pro Ser Ser Arg Ser Ser Gly Asn Asn Asn Val Gly Ser
       210                 215                 220
   Thr Ile Ser Thr Ser Ala Lys Ser Leu Glu Ser Ser Ser Thr Tyr Ser
   225                 230                 235                 240
   Ser Ile Ser Asn Leu Asn Arg Pro Thr Ser Gln Leu Gln Lys Pro Ser
                   245                 250                 255
   Arg Pro Gln Thr Gln Leu Val Arg Val Ala Thr Thr Thr Lys Ile Gly
               260                 265                 270
   Ser Ser Lys Leu Ala Ala Pro Lys Ala Val Ser Thr Pro Lys Leu Ala
           275                 280                 285
   Ser Val Lys Thr Ile Gly Ala Lys Gln Glu Pro Asp Asn Ser Gly Gly
       290                 295                 300
   Gly Gly Gly Gly Met Leu Lys Leu Lys Leu Phe Ser Ser Lys Asn Pro
   305                 310                 315                 320
   Ser Ser Ser Ser Asn Ser Pro Gln Pro Thr Arg Lys Ala Ala Ala Val
                   325                 330                 335
   Pro Gln Gln Gln Thr Leu Ser Lys Ile Ala Ala Pro Val Lys Ser Gly
               340                 345                 350
   Leu Lys Pro Pro Thr Ser Lys Leu Gly Ser Ala Thr Ser Met Ser Lys
           355                 360                 365
   Leu Cys Thr Pro Lys Val Ser Tyr Arg Lys Thr Asp Ala Pro Ile Ile
       370                 375                 380
   Ser Gln Gln Asp Ser Lys Arg Cys Ser Lys Ser Ser Glu Glu Glu Ser
   385                 390                 395                 400
   Gly Tyr Ala Gly Phe Asn Ser Thr Ser Pro Thr Ser Ser Ser Thr Glu
                   405                 410                 415
   Gly Ser Leu Ser Met His Ser Thr Ser Ser Lys Ser Ser Thr Ser Asp
               420                 425                 430
   Glu Lys Ser Pro Ser Ser Asp Asp Leu Thr Leu Asn Ala Ser Ile Val
           435                 440                 445
   Thr Ala Ile Arg Gln Pro Ile Ala Ala Thr Pro Val Ser Pro Asn Ile
       450                 455                 460
   Ile Asn Lys Pro Val Glu Glu Lys Pro Thr Leu Ala Val Lys Gly Val
   465                 470                 475                 480
   Lys Ser Thr Ala Lys Lys Asp Pro Pro Pro Ala Val Pro Pro Arg Asp
                   485                 490                 495
   Thr Gln Pro Thr Ile Gly Val Val Ser Pro Ile Met Ala His Lys Lys
               500                 505                 510
   Leu Thr Asn Asp Pro Val Ile Ser Glu Lys Pro Glu Pro Glu Lys Leu
           515                 520                 525
   Gln Ser Met Ser Ile Asp Thr Thr Asp Val Pro Pro Leu Pro Pro Leu
       530                 535                 540
   Lys Ser Val Val Pro Leu Lys Met Thr Ser Ile Arg Gln Pro Pro Thr
   545                 550                 555                 560
   Tyr Asp Val Leu Leu Lys Gln Gly Lys Ile Thr Ser Pro Val Lys Ser
                   565                 570                 575
   Phe Gly Tyr Glu Gln Ser Ser Ala Ser Glu Asp Ser Ile Val Ala His
               580                 585                 590
   Ala Ser Ala Gln Val Thr Pro Pro Thr Lys Thr Ser Gly Asn His Ser
           595                 600                 605
   Leu Glu Arg Arg Met Gly Lys Asn Lys Thr Ser Glu Ser Ser Gly Tyr
       610                 615                 620
   Thr Ser Asp Ala Gly Val Ala Met Cys Ala Lys Met Arg Glu Lys Leu
   625                 630                 635                 640
   Lys Glu Tyr Asp Asp Met Thr Arg Arg Ala Gln Asn Gly Tyr Pro Asp
                   645                 650                 655
   Asn Phe Glu Asp Ser Ser Ser Leu Ser Ser Gly Ile Ser Asp Asn Asn
               660                 665                 670
   Glu Leu Asp Asp Ile Ser Thr Asp Asp Leu Ser Gly Val Asp Met Ala
           675                 680                 685
   Thr Val Ala Ser Lys His Ser Asp Tyr Ser His Phe Val Arg His Pro
       690                 695                 700
   Thr Ser Ser Ser Ser Lys Pro Arg Val Pro Ser Arg Ser Ser Thr Ser
   705                 710                 715                 720
   Val Asp Ser Arg Ser Arg Ala Glu Gln Glu Asn Val Tyr Lys Leu Leu
                   725                 730                 735
   Ser Gln Cys Arg Thr Ser Gln Arg Gly Ala Ala Ala Thr Ser Thr Phe
               740                 745                 750
   Gly Gln His Ser Leu Arg Ser Pro Gly Tyr Ser Ser Tyr Ser Pro His
           755                 760                 765
   Leu Ser Val Ser Ala Asp Lys Asp Thr Met Ser Met His Ser Gln Thr
       770                 775                 780
   Ser Arg Arg Pro Ser Ser Gln Lys Pro Ser Tyr Ser Gly Gln Phe His
   785                 790                 795                 800
   Ser Leu Asp Arg Lys Cys His Leu Gln Glu Phe Thr Ser Thr Glu His
   805 810 815
   Arg Met Ala Ala Leu Leu Ser Pro Arg Arg Val Pro Asn Ser Met Ser
   820 825 830
   Lys Tyr Asp Ser Ser Gly Ser Tyr Ser Ala Arg Ser Arg Gly Gly Ser
   835 840 845
   Ser Thr Gly Ile Tyr Gly Glu Thr Phe Gln Leu His Arg Leu Ser Asp
   850 855 860
   Glu Lys Ser Pro Ala His Ser Ala Lys Ser Glu Met Gly Ser Gln Leu
   865 870 875 880
   Ser Leu Ala Ser Thr Thr Ala Tyr Gly Ser Leu Asn Glu Lys Tyr Glu
   885 890 895
   His Ala Ile Arg Asp Met Ala Arg Asp Leu Glu Cys Tyr Lys Asn Thr
   900 905 910
   Val Asp Ser Leu Thr Lys Lys Gln Glu Asn Tyr Gly Ala Leu Phe Asp
   915 920 925
   Leu Phe Glu Gln Lys Leu Arg Lys Leu Thr Gln His Ile Asp Arg Ser
   930 935 940
   Asn Leu Lys Pro Glu Glu Ala Ile Arg Phe Arg Gln Asp Ile Ala His
   945 950 955 960
   Leu Arg Asp Ile Ser Asn His Leu Ala Ser Asn Ser Ala His Ala Asn
   965 970 975
   Glu Gly Ala Gly Glu Leu Leu Arg Gln Pro Ser Leu Glu Ser Val Ala
   980 985 990
   Ser His Arg Ser Ser Met Ser Ser Ser Ser Lys Ser Ser Lys Gln Glu
   995 1000 1005
   Lys Ile Ser Leu Ser Ser Phe Gly Lys Asn Lys Lys Ser Trp Ile Arg
   1010 1015 1020
   Ser Ser Leu Ser Lys Phe Thr Lys Lys Lys Asn Lys Asn Tyr Asp Glu
   1025 1030 1035 1040
   Ala His Met Pro Ser Ile Ser Gly Ser Gln Gly Thr Leu Asp Asn Ile
   1045 1050 1055
   Asp Val Ile Glu Leu Lys Gln Glu Leu Lys Glu Arg Asp Ser Ala Leu
   1060 1065 1070
   Tyr Glu Val Arg Leu Asp Asn Leu Asp Arg Ala Arg Glu Val Asp Val
   1075 1080 1085
   Leu Arg Glu Thr Val Asn Lys Leu Lys Thr Glu Asn Lys Gln Leu Lys
   1090 1095 1100
   Lys Glu Val Asp Lys Leu Thr Asn Gly Pro Ala Thr Arg Ala Ser Ser
   1105 1110 1115 1120
   Arg Ala Ser Ile Pro Val Ile Tyr Asp Asp Glu His Val Tyr Asp Ala
   1125 1130 1135
   Ala Cys Ser Ser Thr Ser Ala Ser Gln Ser Ser Lys Arg Ser Ser Gly
   1140 1145 1150
   Cys Asn Ser Ile Lys Val Thr Val Asn Val Asp Ile Ala Gly Glu Ile
   1155 1160 1165
   Ser Ser Ile Val Asn Pro Asp Lys Glu Ile Ile Val Gly Tyr Leu Ala
   1170 1175 1180
   Met Ser Thr Ser Gln Ser Cys Trp Lys Asp Ile Asp Val Ser Ile Leu
   1185 1190 1195 1200
   Gly Leu Phe Glu Val Tyr Leu Ser Arg Ile Asp Val Glu His Gln Leu
   1205 1210 1215
   Gly Ile Asp Ala Arg Asp Ser Ile Leu Gly Tyr Gln Ile Gly Glu Leu
   1220 1225 1230
   Arg Arg Val Ile Gly Asp Ser Thr Thr Met Ile Thr Ser His Pro Thr
   1235 1240 1245
   Asp Ile Leu Thr Ser Ser Thr Thr Ile Arg Met Phe Met His Gly Ala
   1250 1255 1260
   Ala Gln Ser Arg Val Asp Ser Leu Val Leu Asp Met Leu Leu Pro Lys
   1265 1270 1275 1280
   Gln Met Ile Leu Gln Leu Val Lys Ser Ile Leu Thr Glu Arg Arg Leu
   1285 1290 1295
   Val Leu Ala Gly Ala Thr Gly Ile Gly Lys Ser Lys Leu Ala Lys Thr
   1300 1305 1310
   Leu Ala Ala Tyr Val Ser Ile Arg Thr Asn Gln Ser Glu Asp Ser Ile
   1315 1320 1325
   Val Asn Ile Ser Ile Pro Glu Asn Asn Lys Glu Glu Leu Leu Gln Val
   1330 1335 1340
   Glu Arg Arg Leu Glu Lys Ile Leu Arg Ser Lys Glu Ser Cys Ile Val
   1345 1350 1355 1360
   Ile Leu Asp Asn Ile Pro Lys Asn Arg Ile Ala Phe Val Val Ser Val
   1365 1370 1375
   Phe Ala Asn Val Pro Leu Gln Asn Asn Glu Gly Pro Phe Val Val Cys
   1380 1385 1390
   Thr Val Asn Arg Tyr Gln Ile Pro Glu Leu Gln Ile His His Asn Phe
   1395 1400 1405
   Lys Met Ser Val Met Ser Asn Arg Leu Glu Gly Phe Ile Leu Arg Tyr
   1410 1415 1420
   Leu Arg Arg Arg Ala Val Glu Asp Glu Tyr Arg Leu Thr Val Gln Met
   1425 1430 1435 1440
   Pro Ser Glu Leu Phe Lys Ile Ile Asp Phe Phe Pro Ile Ala Leu Gln
   1445 1450 1455
   Ala Val Asn Asn Phe Ile Glu Lys Thr Asn Ser Val Asp Val Thr Val
   1460 1465 1470
   Gly Pro Arg Ala Cys Leu Asn Cys Pro Leu Thr Val Asp Gly Ser Arg
           1475                1480                1485
   Glu Trp Phe Ile Arg Leu Trp Asn Glu Asn Phe Ile Pro Tyr Leu Glu
       1490                1495                1500
   Arg Val Ala Arg Asp Gly Lys Lys Thr Phe Gly Arg Cys Thr Ser Phe
   1505                1510                1515                1520
   Glu Asp Pro Thr Asp Ile Val Ser Lys Lys Trp Pro Trp Phe Asp Gly
                   1525                1530                1535
   Glu Asn Pro Glu Asn Val Leu Lys Arg Leu Gln Leu Gln Asp Leu Val
               1540                1545                1550
   Pro Ser Pro Ala Asn Ser Ser Arg Gln His Phe Asn Pro Leu Glu Ser
           1555                1560                1565
   Leu Ile Gln Leu His Ala Thr Lys His Gln Thr Ile Asp Asn Ile
       1570                1575                1580
(2) INFORMATION FOR SEQ ID NO: 5:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO: 5:
      ATAAGAATGC GGCCGCCGCC ATGACGACGT CAAATGTAGA ATTGATA 47
(2) INFORMATION FOR SEQ ID NO: 6:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO: 6:
      GGAATTCCAA CCATATGACG ACGTCAAATG TAGAATTGAT A 41
(2) INFORMATION FOR SEQ ID NO: 7:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO: 7:
      CGCGGATCCT CAAACCGCGG GTGGCATAAT GGATG 35
(2) INFORMATION FOR SEQ ID NO: 8:
   (i) SEQUENCE CHARACTERISTICS:
      (A) LENGTH: 13 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 8:
   Lys Lys Asp Pro Pro Pro Ala Val Pro Pro Arg Asp Thr
   1               5                   10
(2) INFORMATION FOR SEQ ID NO: 9:
   (i) SEQUENCE CHARACTERISTICS:
      (A) LENGTH: 12 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 9:
   Thr Thr Asp Val Pro Pro Leu Pro Pro Leu Lys Ser
   1               5                   10
(2) INFORMATION FOR SEQ ID NO: 10:
   (i) SEQUENCE CHARACTERISTICS:
      (A) LENGTH: 12 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 10:
   Glu Val Pro Val Pro Pro Pro Val Pro Pro Arg Arg
   1               5                   10
(2) INFORMATION FOR SEQ ID NO: 11:
   (i) SEQUENCE CHARACTERISTICS:
      (A) LENGTH: 11 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 11:
   His Leu Asp Ser Pro Pro Ala Ile Pro Pro Arg
   1               5                   10
(2) INFORMATION FOR SEQ ID NO: 12:
   (i) SEQUENCE CHARACTERISTICS:
      (A) LENGTH: 11 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 12:
   His Ser Ile Ala Gly Pro Pro Val Pro Pro Arg
   1               5                   10
(2) INFORMATION FOR SEQ ID NO: 13:
   (i) SEQUENCE CHARACTERISTICS:
      (A) LENGTH: 13 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 13:
   Tyr Arg Ala Val Pro Pro Pro Leu Pro Pro Arg Arg Lys
   1               5                   10
(2) INFORMATION FOR SEQ ID NO: 14:
   (i) SEQUENCE CHARACTERISTICS:
      (A) LENGTH: 13 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 14:
   Gly Glu Leu Ser Pro Pro Pro Ile Pro Pro Arg Leu Asn
   1 5 10
(2) INFORMATION FOR SEQ ID NO: 15:
   (i) SEQUENCE CHARACTERISTICS:
      (A) LENGTH: 11 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 15:
   Ala Pro Ala Val Pro Pro Ala Arg Pro Gly Ser
   1 5 10
(2) INFORMATION FOR SEQ ID NO: 16:
   (i) SEQUENCE CHARACTERISTICS:
      (A) LENGTH: 8 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 16:
   Pro Ala Val Pro Pro Ala Arg Pro
   1 5
(2) INFORMATION FOR SEQ ID NO: 17:
   (i) SEQUENCE CHARACTERISTICS:
      (A) LENGTH: 11 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 17:
   Pro Pro Arg Pro Leu Pro Val Ala Pro Gly Ser
   1 5 10
(2) INFORMATION FOR SEQ ID NO: 18:
   (i) SEQUENCE CHARACTERISTICS:
      (A) LENGTH: 10 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 18:
   Pro Ala Pro Ala Pro Pro Lys Pro Pro Lys
   1               5                   10
(2) INFORMATION FOR SEQ ID NO: 19:
   (i) SEQUENCE CHARACTERISTICS:
      (A) LENGTH: 13 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 19:
   Pro Pro Asp Asn Gly Pro Pro Pro Leu Pro Thr Ser Ser
   1               5                   10
(2) INFORMATION FOR SEQ ID NO: 20:
   (i) SEQUENCE CHARACTERISTICS:
      (A) LENGTH: 13 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 20:
   Pro Pro Gln Met Pro Leu Pro Glu Ile Pro Gln Gln Trp
   1               5                   10
(2) INFORMATION FOR SEQ ID NO: 21:
   (i) SEQUENCE CHARACTERISTICS:
      (A) LENGTH: 13 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 21:
   Ala Pro Thr Met Pro Pro Pro Leu Pro Pro Val Pro Pro
   1 5 10
(2) INFORMATION FOR SEQ ID NO: 22:
   (i) SEQUENCE CHARACTERISTICS:
      (A) LENGTH: 12 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 22:
   Phe Pro Ala Tyr Pro Pro Pro Pro Val Pro Val Pro
   1 5 10
(2) INFORMATION FOR SEQ ID NO: 23:
   (i) SEQUENCE CHARACTERISTICS:
      (A) LENGTH: 28 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 23:
   Leu Leu Phe Leu Leu Ser Thr Tyr Lys Gln Lys Leu Arg Gln Leu Lys
   1 5 10 15
   Lys Asp Gln Lys Lys Leu Glu Gln Leu Pro Thr Ser
   20 25
(2) INFORMATION FOR SEQ ID NO: 24:
   (i) SEQUENCE CHARACTERISTICS:
      (A) LENGTH: 28 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 24:
   Glu Thr Val Asn Val Asn Lys Leu Lys Thr Glu Asn Lys Gln Leu Lys
   1 5 10 15
   Lys Glu Val Asp Lys Leu Thr Asn Gly Pro Ala Thr
   20 25
(2) INFORMATION FOR SEQ ID NO: 25:
   (i) SEQUENCE CHARACTERISTICS:
      (A) LENGTH: 10443 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "plasmid"
   (iii) HYPOTHETICAL: NO
   (xi) SEQUENCE DESCRIPTION: SEQ ID NO: 25:
   GGCCGCCGCC ATGACGACGT CAAATGTAGA ATTGATACCA ATCTACACGG ATTGGGCCAA 60
   TCGGCACCTT TCGAAGGGCA GCTTATCAAA GTCGATTAGG GATATTTCCA ATGATTTTCG 120
   CGACTATCGA CTGGTTTCTC AGCTTATTAA TGTGATCGTT CCGATCAACG AATTCTCGCC 180
   TGCATTCACG AAACGTTTGG CAAAAATCAC ATCGAACCTG GATGGCCTCG AAACGTGTCT 240
   CGACTACCTG AAAAATCTGG GTCTCGACTG CTCGAAACTC ACCAAAACCG ATATCGACAG 300
   CGGAAACTTG GGTGCAGTTC TCCAGCTGCT CTTCCTGCTC TCCACCTACA AGCAGAAGCT 360
   TCGGCAACTG AAAAAAGATC AGAAGAAATT GGAGCAACTA CCCACATCCA TTATGCCACC 420
   CGCGGTTTCT AAATTACCCT CGCCACGTGT CGCCACGTCA GCAACCGCTT CAGCAACTAA 480
   CCCAAATTCC AACTTTCCAC AAATGTCAAC ATCCAGGCTT CAGACTCCAC AGTCAAGAAT 540
   ATCGAAAATT GATTCATCAA AGATTGGTAT CAAGCCAAAG ACGTCTGGAC TTAAACCACC 600
   CTCATCATCA ACCACTTCAT CAAATAATAC AAATTCATTC CGTCCGTCGA GCCGTTCGAG 660
   TGGCAATAAT AATGTTGGCT CGACGATATC CACATCTGCG AAGAGCTTAG AATCATCATC 720
   AACGTACAGC TCTATTTCGA ATCTAAACCG ACCTACCTCC CAACTCCAAA AACCTTCTAG 780
   ACCACAAACC CAGCTAGTTC GTGTTGCTAC AACTACAAAA ATCGGAAGCT CAAAGCTAGC 840
   CGCTCCGAAA GCCGTGAGCA CCCCAAAACT TGCTTCTGTG AAGACTATTG GAGCAAAACA 900
   AGAGCCCGAT AACAGCGGTG GTGGTGGTGG TGGAATGCTG AAATTAAAGT TATTCAGTAG 960
   CAAAAACCCA TCTTCCTCAT CGAATAGCCC ACAACCTACG AGAAAGGCGG CGGCGGTGCC 1020
   TCAACAACAA ACTTTGTCGA AAATCGCTGC CCCAGTGAAA AGTGGCCTGA AGCCGCCGAC 1080
   CAGTAAGCTG GGAAGTGCCA CGTCTATGTC GAAGCTTTGT ACGCCAAAAG TTTCCTACCG 1140
   TAAAACGGAC GCCCCAATCA TATCTCAACA AGACTCGAAA CGATGCTCAA AGAGCAGTGA 1200
   AGAAGAGTCC GGATACGCTG GATTCAACAG CACGTCGCCA ACGTCATCAT CGACGGAAGG 1260
   TTCCCTAAGC ATGCATTCCA CATCTTCCAA GAGTTCAACG TCAGACGAAA AGTCTCCGTC 1320
   ATCAGACGAT CTTACTCTTA ACGCCTCCAT CGTGACAGCT ATCAGACAGC CGATAGCCGC 1380
   AACACCGGTT TCTCCAAATA TTATCAACAA GCCTGTTGAG GAAAAACCAA CACTGGCAGT 1440
   GAAAGGAGTG AAAAGCACAG CGAAAAAAGA TCCACCTCCA GCTGTTCCGC CACGTGACAC 1500
   CCAGCCAACA ATCGGAGTTG TTAGTCCAAT TATGGCACAT AAGAAGTTGA CAAATGACCC 1560
   CGTGATATCT GAAAAACCAG AACCTGAAAA GCTCCAATCA ATGAGCATCG ACACGACGGA 1620
   CGTTCCACCG CTTCCACCTC TAAAATCAGT TGTTCCACTT AAAATGACTT CAATCCGACA 1680
   ACCACCAACG TACGATGTTC TTCTAAAACA AGGAAAAATC ACATCGCCTG TCAAGTCGTT 1740
   TGGATATGAG CAGTCGTCCG CGTCTGAAGA CTCCATTGTG GCTCATGCGT CGGCTCAGGT 1800
   GACTCCGCCG ACAAAAACTT CTGGTAATCA TTCGCTGGAG AGAAGGATGG GAAAGAATAA 1860
   GACATCAGAA TCCAGCGGCT ACACCTCTGA CGCCGGTGTT GCGATGTGCG CCAAAATGAG 1920
   GGAGAAGCTG AAAGAATACG ATGACATGAC TCGTCGAGCA CAGAACGGCT ATCCTGACAA 1980
   CTTCGAAGAC AGTTCCTCCT TGTCGTCTGG AATATCCGAT AACAACGAGC TCGACGACAT 2040
   ATCCACGGAC GATTTGTCCG GAGTAGACAT GGCAACAGTC GCCTCCAAAC ATAGCGACTA 2100
   TTCCCACTTT GTTCGCCATC CCACGTCTTC TTCCTCAAAG CCCCGAGTCC CCAGTCGGTC 2160
   CTCCACATCA GTCGATTCTC GATCTCGAGC AGAACAGGAG AATGTGTACA AACTTCTGTC 2220
   CCAGTGCCGA ACGAGCCAAC GTGGCGCCGC TGCCACCTCA ACCTTCGGAC AACATTCGCT 2280
   AAGATCCCCG GGATACTCAT CCTATTCTCC ACACTTATCA GTGTCAGCTG ATAAGGACAC 2340
   AATGTCTATG CACTCACAGA CTAGTCGACG ACCTTCTTCA CAAAAACCAA GCTATTCAGG 2400
   CCAATTTCAT TCACTTGATC GTAAATGCCA CCTTCAAGAG TTCACATCCA CCGAGCACAG 2460
   AATGGCGGCT CTCTTGAGCC CGAGACGGGT GCCGAACTCG ATGTCGAAAT ATGATTCTTC 2520
   AGGATCCTAC TCGGCGCGTT CCCGAGGTGG AAGCTCTACT GGTATCTATG GAGAGACGTT 2580
   CCAACTGCAC AGACTATCCG ATGAAAAATC CCCCGCACAT TCTGCCAAAA GTGAGATGGG 2640
   ATCCCAACTA TCACTGGCTA GCACGACAGC ATATGGATCT CTCAATGAGA AGTACGAACA 2700
   TGCTATTCGG GACATGGCAC GTGACTTGGA GTGTTACAAG AACACTGTCG ACTCACTAAC 2760
   CAAGAAACAG GAGAACTATG GAGCATTGTT TGATCTTTTT GAGCAAAAGC TTAGAAAACT 2820
   CACTCAACAC ATTGATCGAT CCAACTTGAA GCCTGAAGAG GCAATACGAT TCAGGCAGGA 2880
   CATTGCTCAT TTGAGGGATA TTAGCAATCA TCTTGCATCC AACTCAGCTC ATGCTAACGA 2940
   AGGCGCTGGT GAGCTTCTTC GTCAACCATC TCTGGAATCA GTTGCATCCC ATCGATCATC 3000
   GATGTCACCG TCGTCGAAAA GCAGCAAGCA GGAGAAGATC AGCTTGAGCT CGTTTGGCAA 3060
   GAACAAGAAG AGCTGGATCC GCTCCTCACT CTCCAAGTTC ACCAAGAAGA AGAACAAGAA 3120
   CTACGACGAA GCACATATGC CATCAATTTC CGGATCTCAA GGAACTCTTG ACAACATTGA 3180
   TGTGATTGAG TTGAAGCAAG AGCTCAAAGA ACGCGATAGT GCACTTTACG AAGTCCGCCT 3240
   TGACAATCTG GATCGTGCCC GCGAAGTTGA TGTTCTGAGG GAGACAGTGA ACAAGTTGAA 3300
   AACCGAGAAC AAGCAATTAA AGAAAGAAGT GGACAAACTC ACCAACGGTC CAGCCACTCG 3360
   TGCTTCTTCC CGCGCCTCAA TTCCAGTTAT CTACGACGAT GAGCATGTCT ATGATGCAGC 3420
   GTGTAGCAGT ACATCAGCTA GTCAATCTTC GAAACGATCC TCTGGCTGCA ACTCAATCAA 3480
   GGTTACTGTA AACGTGGACA TCGCTGGAGA AATCAGTTCG ATCGTTAACC CGGACAAAGA 3540
   GATAATCGTA GGATATCTTG CCATGTCAAC CAGTCAGTCA TGCTGGAAAG ACATTGATGT 3600
   TTCTATTCTA GGACTATTTG AAGTCTACCT ATCCAGAATT GATGTGGAGC ATCAACTTGG 3660
   AATCGATGCT CGTGATTCTA TCCTTGGCTA TCAAATTGGT GAACTTCGAC GCGTCATTGG 3720
   AGACTCCACA ACCATGATAA CCAGCCATCC AACTGACATT CTTACTTCCT CAACTACAAT 3780
   CCGAATGTTC ATGCACGGTG CCGCACAGAG TCGCGTAGAC AGTCTGGTCC TTGATATGCT 3840
   TCTTCCAAAG CAAATGATTC TCCAACTCGT CAAGTCAATT TTGACAGAGA GACGTCTGGT 3900
   GTTAGCTGGA GCAACTGGAA TTGGAAAGAG CAAACTGGCG AAGACCCTGG CTGCTTATGT 3960
   ATCTATTCGA ACAAATCAAT CCGAAGATAG TATTGTTAAT ATCAGCATTC CTGAAAACAA 4020
   TAAAGAAGAA TTGCTTCAAG TGGAACGACG CCTGGAAAAG ATCTTGAGAA GCAAAGAATC 4080
   ATGCATCGTA ATTCTAGATA ATATCCCAAA GAATCGAATT GCATTTGTTG TATCCGTTTT 4140
   TGCAAATGTC CCACTTCAAA ACAACGAAGG TCCATTTGTA GTATGCACAG TCAACCGATA 4200
   TCAAATCCCT GAGCTTCAAA TTCACCACAA TTTCAAAATG TCAGTAATGT CGAATCGTCT 4260
   CGAAGGATTC ATCCTACGTT ACCTCCGACG ACGGGCGGTA GAGGATGAGT ATCGTCTAAC 4320
   TGTACAGATG CCATCAGAGC TCTTCAAAAT CATTGACTTC TTCCCAATAG CTCTTCAGGC 4380
   CGTCAATAAT TTTATTGAGA AAACGAATTC TGTTGATGTG ACAGTTGGTC CAAGAGCATG 4440
   CTTGAACTGT CCTCTAACTG TCGATGGATC CCGTGAATGG TTCATTCGAT TGTGGAATGA 4500
   GAACTTCATT CCATATTTGG AACGTGTTGC TAGAGATGGC AAAAAAACCT TCGGTCGCTG 4560
   CACTTCCTTC GAGGATCCCA CCGACATCGT CTCTAAAAAA TGGCCGTGGT TCGATGGTGA 4620
   AAACCCGGAG AATGTGCTCA AACGTCTTCA ACTCCAAGAC CTCGTCCCGT CACCTGCCAA 4680
   CTCATCCCGA CAACACTTCA ATCCCCTCGA GTCGTTGATC CAATTGCATG CTACCAAGCA 4740
   TCAGACCATC GACAACATTT GAACAGAAGA CTCTAATCTT CTCTCGCCTC TCCCCCGCTT 4800
   TCCTTATCTT CGTACCGGTA CCTGATGATT CCCCATTTTC CCCCTTTTCC CCCCAATTTC 4860
   CCAGAACCTC CTGTTCCCTT TGTTCCTAGT CCTCCCGGGT GCCGACGCCG AAGCGATTTA 4920
   AAAACCTTTT TCTTTCCGAA ACATTTCCCA TTGCTCATTA ATAGTCAAAT TGAATAAACA 4980
   GTGTATGTAC TTAAAAAAAA AAAAAAAAAA ACTCGAGGGG GGGCCCTATT CTATAGTGTC 5040
   ACCTAAATGC TAGAGCTCGC TGATCAGCCT CGACTGTGCC TTCTAGTTGC CAGCCATCTG 5100
   TTGTTTGCCC CTCCCCCGTG CCTTCCTTGA CCCTGGAAGG TGCCACTCCC ACTGTCCTTT 5160
   CCTAATAAAA TGAGGAAATT GCATCGCATT GTCTGAGTAG GTGTCATTCT ATTCTGGGGG 5220
   GTGGGGTGGG GCAGGACAGC AAGGGGGAGG ATTGGGAAGA CAATAGCAGG CATGCTGGGG 5280
   ATGCGGTGGG CTCTATGGCT TCTGAGGCGG AAAGAACCAG CTGGGGCTCT AGGGGGTATC 5340
   CCCACGCGCC CTGTAGCGGC GCATTAAGCG CGGCGGGTGT GGTGGTTACG CGCAGCGTGA 5400
   CCGCTACACT TGCCAGCGCC CTAGCGCCCG CTCCTTTCGC TTTCTTCCCT TCCTTTCTCG 5460
   CCACGTTCGC CGGCTTTCCC CGTCAAGCTC TAAATCGGGG CATCCCTTTA GGGTTCCGAT 5520
   TTAGTGCTTT ACGGCACCTC GACCCCAAAA AACTTGATTA GGGTGATGGT TCACGTAGTG 5580
   GGCCATCGCC CTGATAGACG GTTTTTCGCC CTTTGACGTT GGAGTCCACG TTCTTTAATA 5640
   GTGGACTCTT GTTCCAAACT GGAACAACAC TCAACCCTAT CTCGGTCTAT TCTTTTGATT 5700
   TATAAGGGAT TTTGGGGATT TCGGCCTATT GGTTAAAAAA TGAGCTGATT TAACAAAAAT 5760
   TTAACGCGAA TTAATTCTGT GGAATGTGTG TCAGTTAGGG TGTGGAAAGT CCCCAGGCTC 5820
   CCCAGGCAGG CAGAAGTATG CAAAGCATGC ATCTCAATTA GTCAGCAACC AGGTGTGGAA 5880
   AGTCCCCAGG CTCCCCAGCA GGCAGAAGTA TGCAAAGCAT GCATCTCAAT TAGTCAGCAA 5940
   CCATAGTCCC GCCCCTAACT CCGCCCATCC CGCCCCTAAC TCCGCCCAGT TCCGCCCATT 6000
   CTCCGCCCCA TGGCTGACTA ATTTTTTTTA TTTATGCAGA GGCCGAGGCC GCCTCTGCCT 6060
   CTGAGCTATT CCAGAAGTAG TGAGGAGGCT TTTTTGGAGG CCTAGGCTTT TGCAAAAAGC 6120
   TCCCGGGAGC TTGTATATCC ATTTTCGGAT CTGATCAAGA GACAGGATGA GGATCGTTTC 6180
   GCATGATTGA ACAAGATGGA TTGCACGCAG GTTCTCCGGC CGCTTGGGTG GAGAGGCTAT 6240
   TCGGCTATGA CTGGGCACAA CAGACAATCG GCTGCTCTGA TGCCGCCGTG TTCCGGCTGT 6300
   CAGCGCAGGG GCGCCCGGTT CTTTTTGTCA AGACCGACCT GTCCGGTGCC CTGAATGAAC 6360
   TGCAGGACGA GGCAGCGCGG CTATCGTGGC TGGCCACGAC GGGCGTTCCT TGCGCAGCTG 6420
   TGCTCGACGT TGTCACTGAA GCGGGAAGGG ACTGGCTGCT ATTGGGCGAA GTGCCGGGGC 6480
   AGGATCTCCT GTCATCTCAC CTTGCTCCTG CCGAGAAAGT ATCCATCATG GCTGATGCAA 6540
   TGCGGCGGCT GCATACGCTT GATCCGGCTA CCTGCCCATT CGACCACCAA GCGAAACATC 6600
   GCATCGAGCG AGCACGTACT CGGATGGAAG CCGGTCTTGT CGATCAGGAT GATCTGGACG 6660
   AAGAGCATCA GGGGCTCGCG CCAGCCGAAC TGTTCGCCAG GCTCAAGGCG CGCATGCCCG 6720
   ACGGCGAGGA TCTCGTCGTG ACCCATGGCG ATGCCTGCTT GCCGAATATC ATGGTGGAAA 6780
   ATGGCCGCTT TTCTGGATTC ATCGACTGTG GCCGGCTGGG TGTGGCGGAC CGCTATCAGG 6840
   ACATAGCGTT GGCTACCCGT GATATTGCTG AAGAGCTTGG CGGCGAATGG GCTGACCGCT 6900
   TCCTCGTGCT TTACGGTATC GCCGCTCCCG ATTCGCAGCG CATCGCCTTC TATCGCCTTC 6960
   TTGACGAGTT CTTCTGAGCG GGACTCTGGG GTTCGAAATG ACCGACCAAG CGACGCCCAA 7020
   CCTGCCATCA CGAGATTTCG ATTCCACCGC CGCCTTCTAT GAAAGGTTGG GCTTCGGAAT 7080
   CGTTTTCCGG GACGCCGGCT GGATGATCCT CCAGCGCGGG GATCTCATGC TGGAGTTCTT 7140
   CGCCCACCCC AACTTGTTTA TTGCAGCTTA TAATGGTTAC AAATAAAGCA ATAGCATCAC 7200
   AAATTTCACA AATAAAGCAT TTTTTTCACT GCATTCTAGT TGTGGTTTGT CCAAACTCAT 7260
   CAATGTATCT TATCATGTCT GTATACCGTC GACCTCTAGC TAGAGCTTGG CGTAATCATG 7320
   GTCATAGCTG TTTCCTGTGT GAAATTGTTA TCCGCTCACA ATTCCACACA ACATACGAGC 7380
   CGGAAGCATA AAGTGTAAAG CCTGGGGTGC CTAATGAGTG AGCTAACTCA CATTAATTGC 7440
   GTTGCGCTCA CTGCCCGCTT TCCAGTCGGG AAACCTGTCG TGCCAGCTGC ATTAATGAAT 7500
   CGGCCAACGC GCGGGGAGAG GCGGTTTGCG TATTGGGCGC TCTTCCGCTT CCTCGCTCAC 7560
   TGACTCGCTG CGCTCGGTCG TTCGGCTGCG GCGAGCGGTA TCAGCTCACT CAAAGGCGGT 7620
   AATACGGTTA TCCACAGAAT CAGGGGATAA CGCAGGAAAG AACATGTGAG CAAAAGGCCA 7680
   GCAAAAGGCC AGGAACCGTA AAAAGGCCGC GTTGCTGGCG TTTTTCCATA GGCTCCGCCC 7740
   CCCTGACGAG CATCACAAAA ATCGACGCTC AAGTCAGAGG TGGCGAAACC CGACAGGACT 7800
   ATAAAGATAC CAGGCGTTTC CCCCTGGAAG CTCCCTCGTG CGCTCTCCTG TTCCGACCCT 7860
   GCCGCTTACC GGATACCTGT CCGCCTTTCT CCCTTCGGGA AGCGTGGCGC TTTCTCAATG 7920
   CTCACGCTGT AGGTATCTCA GTTCGGTGTA GGTCGTTCGC TCCAAGCTGG GCTGTGTGCA 7980
   CGAACCCCCC GTTCAGCCCG ACCGCTGCGC CTTATCCGGT AACTATCGTC TTGAGTCCAA 8040
   CCCGGTAAGA CACGACTTAT CGCCACTGGC AGCAGCCACT GGTAACAGGA TTAGCAGAGC 8100
   GAGGTATGTA GGCGGTGCTA CAGAGTTCTT GAAGTGGTGG CCTAACTACG GCTACACTAG 8160
   AAGGACAGTA TTTGGTATCT GCGCTCTGCT GAAGCCAGTT ACCTTCGGAA AAAGAGTTGG 8220
   TAGCTCTTGA TCCGGCAAAC AAACCACCGC TGGTAGCGGT GGTTTTTTTG TTTGCAAGCA 8280
   GCAGATTACG CGCAGAAAAA AAGGATCTCA AGAAGATCCT TTGATCTTTT CTACGGGGTC 8340
   TGACGCTCAG TGGAACGAAA ACTCACGTTA AGGGATTTTG GTCATGAGAT TATCAAAAAG 8400
   GATCTTCACC TAGATCCTTT TAAATTAAAA ATGAAGTTTT AAATCAATCT AAAGTATATA 8460
   TGAGTAAACT TGGTCTGACA GTTACCAATG CTTAATCAGT GAGGCACCTA TCTCAGCGAT 8520
   CTGTCTATTT CGTTCATCCA TAGTTGCCTG ACTCCCCGTC GTGTAGATAA CTACGATACG 8580
   GGAGGGCTTA CCATCTGGCC CCAGTGCTGC AATGATACCG CGAGACCCAC GCTCACCGGC 8640
   TCCAGATTTA TCAGCAATAA ACCAGCCAGC CGGAAGGGCC GAGCGCAGAA GTGGTCCTGC 8700
   AACTTTATCC GCCTCCATCC AGTCTATTAA TTGTTGCCGG GAAGCTAGAG TAAGTAGTTC 8760
   GCCAGTTAAT AGTTTGCGCA ACGTTGTTGC CATTGCTACA GGCATCGTGG TGTCACGCTC 8820
   GTCGTTTGGT ATGGCTTCAT TCAGCTCCGG TTCCCAACGA TCAAGGCGAG TTACATGATC 8880
   CCCCATGTTG TGCAAAAAAG CGGTTAGCTC CTTCGGTCCT CCGATCGTTG TCAGAAGTAA 8940
   GTTGGCCGCA GTGTTATCAC TCATGGTTAT GGCAGCACTG CATAATTCTC TTACTGTCAT 9000
   GCCATCCGTA AGATGCTTTT CTGTGACTGG TGAGTACTCA ACCAAGTCAT TCTGAGAATA 9060
   GTGTATGCGG CGACCGAGTT GCTCTTGCCC GGCGTCAATA CGGGATAATA CCGCGCCACA 9120
   TAGCAGAACT TTAAAAGTGC TCATCATTGG AAAACGTTCT TCGGGGCGAA AACTCTCAAG    9180
   GATCTTACCG CTGTTGAGAT CCAGTTCGAT GTAACCCACT CGTGCACCCA ACTGATCTTC    9240
   AGCATCTTTT ACTTTCACCA GCGTTTCTGG GTGAGCAAAA ACAGGAAGGC AAAATGCCGC    9300
   AAAAAAGGGA ATAAGGGCGA CACGGAAATG TTGAATACTC ATACTCTTCC TTTTTCAATA    9360
   TTATTGAAGC ATTTATCAGG GTTATTGTCT CATGAGCGGA TACATATTTG AATGTATTTA    9420
   GAAAAATAAA CAAATAGGGG TTCCGCGCAC ATTTCCCCGA AAAGTGCCAC CTGACGTCGA    9480
   CGGATCGGGA GATCTCCCGA TCCCCTATGG TCGACTCTCA GTACAATCTG CTCTGATGCC    9540
   GCATAGTTAA GCCAGTATCT GCTCCCTGCT TGTGTGTTGG AGGTCGCTGA GTAGTGCGCG    9600
   AGCAAAATTT AAGCTACAAC AAGGCAAGGC TTGACCGACA ATTGCATGAA GAATCTGCTT    9660
   AGGGTTAGGC GTTTTGCGCT GCTTCGCGAT GTACGGGCCA GATATACGCG TTGACATTGA    9720
   TTATTGACTA GTTATTAATA GTAATCAATT ACGGGGTCAT TAGTTCATAG CCCATATATG    9780
   GAGTTCCGCG TTACATAACT TACGGTAAAT GGCCCGCCTG GCTGACCGCC CAACGACCCC    9840
   CGCCCATTGA CGTCAATAAT GACGTATGTT CCCATAGTAA CGCCAATAGG GACTTTCCAT    9900
   TGACGTCAAT GGGTGGACTA TTTACGGTAA ACTGCCCACT TGGCAGTACA TCAAGTGTAT    9960
   CATATGCCAA GTACGCCCCC TATTGACGTC AATGACGGTA AATGGCCCGC CTGGCATTAT   10020
   GCCCAGTACA TGACCTTATG GGACTTTCCT ACTTGGCAGT ACATCTACGT ATTAGTCATC   10080
   GCTATTACCA TGGTGATGCG GTTTTGGCAG TACATCAATG GGCGTGGATA GCGGTTTGAC   10140
   TCACGGGGAT TTCCAAGTCT CCACCCCATT GACGTCAATG GGAGTTTGTT TTGGCACCAA   10200
   AATCAACGGG ACTTTCCAAA ATGTCGTAAC AACTCCGCCC CATTGACGCA AATGGGCGGT   10260
   AGGCGTGTAC GGTGGGAGGT CTATATAAGC AGAGCTCTCT GGCTAACTAG AGAACCCACT   10320
   GCTTACTGGC TTATCGAAAT TAATACGACT CACTATAGGG AGACCCAAGC TTGGTACCGA   10380
   GCTCGGATCC ACTAGTAACG GCCGCCAGTG TGCTGGAATT CTGCAGATAT CCATCACACT   10440
   GGC                                                                  10443
(2) INFORMATION FOR SEQ ID NO: 26:
   (i) SEQUENCE CHARACTERISTICS:
      (A) LENGTH: 7474 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "plasmid"
   (iii) HYPOTHETICAL: NO
   (xi) SEQUENCE DESCRIPTION: SEQ ID NO: 26:
   CTAAATTGTA AGCGTTAATA TTTTGTTAAA ATTCGCGTTA AATTTTTGTT AAATCAGCTC     60
   ATTTTTTAAC CAATAGGCCG AAATCGGCAA AATCCCTTAT AAATCAAAAG AATAGACCGA    120
   GATAGGGTTG AGTGTTGTTC CAGTTTGGAA CAAGAGTCCA CTATTAAAGA ACGTGGACTC    180
   CAACGTCAAA GGGCGAAAAA CCGTCTATCA GGGCGATGGC CCACTACGTG AACCATCACC    240
   CTAATCAAGT TTTTTGGGGT CGAGGTGCCG TAAAGCACTA AATCGGAACC CTAAAGGGAG    300
   CCCCCGATTT AGAGCTTGAC GGGGAAAGCC GGCGAACGTG GCGAGAAAGG AAGGGAAGAA    360
   AGCGAAAGGA GCGGGCGCTA GGGCGCTGGC AAGTGTAGCG GTCACGCTGC GCGTAACCAC    420
   CACACCCGCC GCGCTTAATG CGCCGCTACA GGGCGCGTCC CATTCGCCAT TCAGGCTGCG    480
   CAACTGTTGG GAAGGGCGAT CGGTGCGGGC CTCTTCGCTA TTACGCCAGC TGGCGAAAGG    540
   GGGATGTGCT GCAAGGCGAT TAAGTTGGGT AACGCCAGGG TTTTCCCAGT CACGACGTTG    600
   TAAAACGACG GCCAGTGAGC GCGCGTAATA CGACTCACTA TAGGGCGAAT TGGAGCTCCA    660
   CCGCGGTTTC TAAATTACCC TCGCCACGTG TCGCCACGTC AGCAACCGCT TCAGCAACTA    720
   ACCCAAATTC CAACTTTCCA CAAATGTCAA CATCCAGGCT TCAGACTCCA CAGTCAAGAA    780
   TATCGAAAAT TGATTCATCA AAGATTGGTA TCAAGCCAAA GACGTCTGGA CTTAAACCAC    840
   CCTCATCATC AACCACTTCA TCAAATAATA CAAATTCATT CCGTCCGTCG AGCCGTTCGA    900
   GTGGCAATAA TAATGTTGGC TCGACGATAT CCACATCTGC GAAGAGCTTA GAATCATCAT    960
   CAACGTACAG CTCTATTTCG AATCTAAACC GACCTACCTC CCAACTCCAA AAACCTTCTA   1020
   GACCACAAAC CCAGCTAGTT CGTGTTGCTA CAACTACAAA AATCGGAAGC TCAAAGCTAG   1080
   CCGCTCCGAA AGCCGTGAGC ACCCCAAAAC TTGCTTCTGT GAAGACTATT GGAGCAAAAC   1140
   AAGAGCCCGA TAACAGCGGT GGTGGTGGTG GTGGAATGCT GAAATTAAAG TTATTCAGTA   1200
   GCAAAAACCC ATCTTCCTCA TCGAATAGCC CACAACCTAC GAGAAAGGCG GCGGCGGTGC   1260
   CTCAACAACA AACTTTGTCG AAAATCGCTG CCCCAGTGAA AAGTGGCCTG AAGCCGCCGA   1320
   CCAGTAAGCT GGGAAGTGCC ACGTCTATGT CGAAGCTTTG TACGCCAAAA GTTTCCTACC   1380
   GTAAAACGGA CGCCCCAATC ATATCTCAAC AAGACTCGAA ACGATGCTCA AAGAGCAGTG   1440
   AAGAAGAGTC CGGATACGCT GGATTCAACA GCACGTCGCC AACGTCATCA TCGACGGAAG   1500
   GTTCCCTAAG CATGCATTCC ACATCTTCCA AGAGTTCAAC GTCAGACGAA AAGTCTCCGT   1560
   CATCAGACGA TCTTACTCTT AACGCCTCCA TCGTGACAGC TATCAGACAG CCGATAGCCG   1620
   CAACACCGGT TTCTCCAAAT ATTATCAACA AGCCTGTTGA GGAAAAACCA ACACTGGCAG   1680
   TGAAAGGAGT GAAAAGCACA GCGAAAAAAG ATCCACCTCC AGCTGTTCCG CCACGTGACA   1740
   CCCAGCCAAC AATCGGAGTT GTTAGTCCAA TTATGGCACA TAAGAAGTTG ACAAATGACC   1800
   CCGTGATATC TGAAAAACCA GAACCTGAAA AGCTCCAATC AATGAGCATC GACACGACGG    1860
   ACGTTCCACC GCTTCCACCT CTAAAATCAG TTGTTCCACT TAAAATGACT TCAATCCGAC   1920
   AACCACCAAC GTACGATGTT CTTCTAAAAC AAGGAAAAAT CACATCGCCT GTCAAGTCGT   1980
   TTGGATATGA GCAGTCGTCC GCGTCTGAAG ACTCCATTGT GGCTCATGCG TCGGCTCAGG   2040
   TGACTCCGCC GACAAAAACT TCTGGTAATC ATTCGCTGGA GAGAAGGATG GGAAAGAATA   2100
   AGACATCAGA ATCCAGCGGC TACACCTCTG ACGCCGGTGT TGCGATGTGC GCCAAAATGA   2160
   GGGAGAAGCT GAAAGAATAC GATGACATGA CTCGTCGAGC ACAGAACGGC TATCCTGACA   2220
   ACTTCGAAGA CAGTTCCTCC TTGTCGTCTG GAATATCCGA TAACAACGAG CTCGACGACA   2280
   TATCCACGGA CGATTTGTCC GGAGTAGACA TGGCAACAGT CGCCTCCAAA CATAGCGACT   2340
   ATTCCCACTT TGTTCGCCAT CCCACGTCTT CTTCCTCAAA GCCCCGAGTC CCCAGTCGGT   2400
   CCTCCACATC AGTCGATTCT CGATCTCGAG CAGAACAGGA GAATGTGTAC AAACTTCTGT   2460
   CCCAGTGCCG AACGAGCCAA CGTGGCGCCG CTGCCACCTC AACCTTCGGA CAACATTCGC   2520
   TAAGATCCCC GGGATACTCA TCCTATTCTC CACACTTATC AGTGTCAGCT GATAAGGACA   2580
   CAATGTCTAT GCACTCACAG ACTAGTCGAC GACCTTCTTC ACAAAAACCA AGCTATTCAG   2640
   GCCAATTTCA TTCACTTGAT CGTAAATGCC ACCTTCAAGA GTTCACATCC ACCGAGCACA   2700
   GAATGGCGGC TCTCTTGAGC CCGAGACGGG TGCCGAACTC GATGTCGAAA TATGATTCTT   2760
   CAGGATCCTA CTCGGCGCGT TCCCGAGGTG GAAGCTCTAC TGGTATCTAT GGAGAGACGT   2820
   TCCAACTGCA CAGACTATCC GATGAAAAAT CCCCCGCACA TTCTGCCAAA AGTGAGATGG   2880
   GATCCCAACT ATCACTGGCT AGCACGACAG CATATGGATC TCTCAATGAG AAGTACGAAC   2940
   ATGCTATTCG GGACATGGCA CGTGACTTGG AGTGTTACAA GAACACTGTC GACTCACTAA   3000
   CCAAGAAACA GGAGAACTAT GGAGCATTGT TTGATCTTTT TGAGCAAAAG CTTAGAAAAC   3060
   TCACTCAACA CATTGATCGA TCCAACTTGA AGCCTGAAGA GGCAATACGA TTCAGGCAGG   3120
   ACATTGCTCA TTTGAGGGAT ATTAGCAATC ATCTTGCATC CAACTCAGCT CATGCTAACG   3180
   AAGGCGCTGG TGAGCTTCTT CGTCAACCAT CTCTGGAATC AGTTGCATCC CATCGATCAT   3240
   CGATGTCATC GTCGTCGAAA AGCAGCAAGC AGGAGAAGAT CAGCTTGAGC TCGTTTGGCA   3300
   AGAACAAGAA GAGCTGGATC CGCTCCTCAC TCTCCAAGTT CACCAAGAAG AAGAACAAGA   3360
   ACTACGACGA AGCACATATG CCATCAATTT CCGGATCTCA AGGAACTCTT GACAACATTG   3420
   ATGTGATTGA GTTGAAGCAA GAGCTCAAAG AACGCGATAG TGCACTTTAC GAAGTCCGCC   3480
   TTGACAATCT GGATCGTGCC CGCGAAGTTG ATGTTCTGAG GGAGACAGTG AACAAGTTGA   3540
   AAACCGAGAA CAAGCAATTA AAGAAAGAAG TGGACAAACT CACCAACGGT CCAGCCACTC   3600
   GTGCTTCTTC CCGCGCCTCA ATTCCAGTTA TCTACGACGA TGAGCATGTC TATGATGCAG   3660
   CGTGTAGCAG TACATCAGCT AGTCAATCTT CGAAACGATC CTCTGGCTGC AACTCAATCA   3720
   AGGTTACTGT AAACGTGGAC ATCGCTGGAG AAATCAGTTC GATCGTTAAC CCGGACAAAG   3780
   AGATAATCGT AGGATATCTT GCCATGTCAA CCAGTCAGTC ATGCTGGAAA GACATTGATG 3840
   TTTCTATTCT AGGACTATTT GAAGTCTACC TATCCAGAAT TGATGTGGAG CATCAACTTG 3900
   GAATCGATGC TCGTGATTCT ATCCTTGGCT ATCAAATTGG TGAACTTCGA CGCGTCATTG 3960
   GAGACTCCAC AACCATGATA ACCAGCCATC CAACTGACAT TCTTACTTCC TCAACTACAA 4020
   TCCGAATGTT CATGCACGGT GCCGCACAGA GTCGCGTAGA CAGTCTGGTC CTTGATATGC 4080
   TTCTTCCAAA GCAAATGATT CTCCAACTCG TCAAGTCAAT TTTGACAGAG AGACGTCTGG 4140
   TGTTAGCTGG AGCAACTGGA ATTGGAAAGA GCAAACTGGC GAAGACCCTG GCTGCTTATG 4200
   TATCTATTCG AACAAATCAA TCCGAAGATA GTATTGTTAA TATCAGCATT CCTGAAAACA 4260
   ATAAAGAAGA ATTGCTTCAA GTGGAACGAC GCCTGGAAAA GATCTTGAGA AGCAAAGAAT 4320
   CATGCATCGT AATTCTAGAT AATATCCCAA AGAATCGAAT TGCATTTGTT GTATCCGTTT 4380
   TTGCAAATGT CCCACTTCAA AACAACGAAG GTCCATTTGT AGTATGCACA GTCAACCGAT 4440
   ATCAAATCCC TGAGCTTCAA ATTCACCACA ATTTCAAAAT GTCAGTAATG TCGAATCGTC 4500
   TCGAAGGATT CATCCTACGT TACCTCCGAC GACGGGCGGT AGAGGATGAG TATCGTCTAA 4560
   CTGTACAGAT GCCATCAGAG CTCTTCAAAA TCATTGACTT CTTCCCAATA GCTCTTCAGG 4620
   CCGTCAATAA TTTTATTGAG AAAACGAATT CTGTTGATGT GACAGTTGGT CCAAGAGCAT 4680
   GCTTGAACTG TCCTCTAACT GTCGATGGAT CCCGTGAATG GTTCATTCGA TTGTGGAATG 4740
   AGAACTTCAT TCCATATTTG GAACGTGTTG CTAGAGATGG CAAAAAAACC TTCGGTCGCT 4800
   GCACTTCCTT CGAGGATCCC ACCGACATCG TCTCTAAAAA ATGGCCGTGG TTCGATGGTG 4860
   AAAACCCGGA GAATGTGCTC AAACGTCTTC AACTCCAAGA CCTCGTCCCG TCACCTGCCA 4920
   ACTCATCCCG ACAACACTTC AATCCCCTCG AGTCGTTGAT CCAATTGCAT GCTACCAAGC 4980
   ATCAGACCAT CGACAACATT TGAACAGAAG ACTCTAATCT TCTCTCGCCT CTCCCCCGCT 5040
   TTCCTTATCT TCGTACCGGT ACCTGATGAT TCCCCATTTT CCCCCTTTTC CCCCCAATTT 5100
   CCCAGAACCT CCTGTTCCCT TTGTTCCTAG TCCTCCCGGG TGCCGACGCC GAAGCGATTT    5160
   AAAAACCTTT TTCTTTCCGA AACATTTCCC ATTGCTCATT AATAGTCAAA TTGAATAAAC    5220
   AGTGTATGTA CTTAAAAAAA AAAAAAAAAA AACTCGAGGG GGGGCCCGGT ACCCAGCTTT 5280
   TGTTCCCTTT AGTGAGGGTT AATTGCGCGC TTGGCGTAAT CATGGTCATA GCTGTTTCCT 5340
   GTGTGAAATT GTTATCCGCT CACAATTCCA CACAACATAC GAGCCGGAAG CATAAAGTGT 5400
   AAAGCCTGGG GTGCCTAATG AGTGAGCTAA CTCACATTAA TTGCGTTGCG CTCACTGCCC 5460
   GCTTTCCAGT CGGGAAACCT GTCGTGCCAG CTGCATTAAT GAATCGGCCA ACGCGCGGGG 5520
   AGAGGCGGTT TGCGTATTGG GCGCTCTTCC GCTTCCTCGC TCACTGACTC GCTGCGCTCG 5580
   GTCGTTCGGC TGCGGCGAGC GGTATCAGCT CACTCAAAGG CGGTAATACG GTTATCCACA 5640
   GAATCAGGGG ATAACGCAGG AAAGAACATG TGAGCAAAAG GCCAGCAAAA GGCCAGGAAC 5700
   CGTAAAAAGG CCGCGTTGCT GGCGTTTTTC CATAGGCTCC GCCCCCCTGA CGAGCATCAC 5760
   AAAAATCGAC GCTCAAGTCA GAGGTGGCGA AACCCGACAG GACTATAAAG ATACCAGGCG 5820
   TTTCCCCCTG GAAGCTCCCT CGTGCGCTCT CCTGTTCCGA CCCTGCCGCT TACCGGATAC 5880
   CTGTCCGCCT TTCTCCCTTC GGGAAGCGTG GCGCTTTCTC ATAGCTCACG CTGTAGGTAT 5940
   CTCAGTTCGG TGTAGGTCGT TCGCTCCAAG CTGGGCTGTG TGCACGAACC CCCCGTTCAG 6000
   CCCGACCGCT GCGCCTTATC CGGTAACTAT CGTCTTGAGT CCAACCCGGT AAGACACGAC 6060
   TTATCGCCAC TGGCAGCAGC CACTGGTAAC AGGATTAGCA GAGCGAGGTA TGTAGGCGGT 6120
   GCTACAGAGT TCTTGAAGTG GTGGCCTAAC TACGGCTACA CTAGAAGGAC AGTATTTGGT 6180
   ATCTGCGCTC TGCTGAAGCC AGTTACCTTC GGAAAAAGAG TTGGTAGCTC TTGATCCGGC 6240
   AAACAAACCA CCGCTGGTAG CGGTGGTTTT TTTGTTTGCA AGCAGCAGAT TACGCGCAGA 6300
   AAAAAAGGAT CTCAAGAAGA TCCTTTGATC TTTTCTACGG GGTCTGACGC TCAGTGGAAC 6360
   GAAAACTCAC GTTAAGGGAT TTTGGTCATG AGATTATCAA AAAGGATCTT CACCTAGATC 6420
   CTTTTAAATT AAAAATGAAG TTTTAAATCA ATCTAAAGTA TATATGAGTA AACTTGGTCT 6480
   GACAGTTACC AATGCTTAAT CAGTGAGGCA CCTATCTCAG CGATCTGTCT ATTTCGTTCA 6540
   TCCATAGTTG CCTGACTCCC CGTCGTGTAG ATAACTACGA TACGGGAGGG CTTACCATCT 6600
   GGCCCCAGTG CTGCAATGAT ACCGCGAGAC CCACGCTCAC CGGCTCCAGA TTTATCAGCA 6660
   ATAAACCAGC CAGCCGGAAG GGCCGAGCGC AGAAGTGGTC CTGCAACTTT ATCCGCCTCC 6720
   ATCCAGTCTA TTAATTGTTG CCGGGAAGCT AGAGTAAGTA GTTCGCCAGT TAATAGTTTG 6780
   CGCAACGTTG TTGCCATTGC TACAGGCATC GTGGTGTCAC GCTCGTCGTT TGGTATGGCT 6840
   TCATTCAGCT CCGGTTCCCA ACGATCAAGG CGAGTTACAT GATCCCCCAT GTTGTGCAAA 6900
   AAAGCGGTTA GCTCCTTCGG TCCTCCGATC GTTGTCAGAA GTAAGTTGGC CGCAGTGTTA 6960
   TCACTCATGG TTATGGCAGC ACTGCATAAT TCTCTTACTG TCATGCCATC CGTAAGATGC 7020
   TTTTCTGTGA CTGGTGAGTA CTCAACCAAG TCATTCTGAG AATAGTGTAT GCGGCGACCG 7080
   AGTTGCTCTT GCCCGGCGTC AATACGGGAT AATACCGCGC CACATAGCAG AACTTTAAAA 7140
   GTGCTCATCA TTGGAAAACG TTCTTCGGGG CGAAAACTCT CAAGGATCTT ACCGCTGTTG 7200
   AGATCCAGTT CGATGTAACC CACTCGTGCA CCCAACTGAT CTTCAGCATC TTTTACTTTC 7260
   ACCAGCGTTT CTGGGTGAGC AAAAACAGGA AGGCAAAATG CCGCAAAAAA GGGAATAAGG 7320
   GCGACACGGA AATGTTGAAT ACTCATACTC TTCCTTTTTC AATATTATTG AAGCATTTAT 7380
   CAGGGTTATT GTCTCATGAG CGGATACATA TTTGAATGTA TTTAGAAAAA TAAACAAATA 7440
   GGGGTTCCGC GCACATTTCC CCGAAAAGTG CCAC 7474
(2) INFORMATION FOR SEQ ID NO: 27:
   (i) SEQUENCE CHARACTERISTICS:
      (A) LENGTH: 13414 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "plasmid"
   (iii) HYPOTHETICAL: NO
   (ix) FEATURE:
      (A) NAME/KEY: misc_feature
      (B) LOCATION:11582
      (D) OTHER INFORMATION:/note= "N is A,G,C or T"
   (xi) SEQUENCE DESCRIPTION: SEQ ID NO: 27:
   TATGACGACG TCAAATGTAG AATTGATACC ATTCTACACG GATTGGGCCA ATCGGCACCT      60
   TTCGAAGGGC AGCTTATCAA AGTCGATTAG GGATATTTCC AATGATTTTC GCGACTATCG     120
   ACTGGTTTCT CAGCTTATTA ATGTGATCGT TCCGATCAAC GAATTCTCGC CTGCATTCAC     180
   GAAACGTTTG GCAAAAATCA CATCGAACCT GGATGGCCTC GAAACGTGTC TCGACTACCT     240
   GAAAAATCTG GGTCTCGACT GCTCGAAACT CACCAAAACC GATATCGACA GCGGAAACTT     300
   GGGTGCAGTT CTCCAGCTGC TCTTCCTGCT CTCCACCTAC AAGCAGAAGC TTCGGCAACT     360
   GAAAAAAGAT CAGAAGAAAT TGGAGCAACT ACCCACATCC ATTATGCCAC CCGCGGTTTC     420
   TAAATTACCC TCGCCACGTG TCGCCACGTC AGCAACCGCT TCAGCAACTA ACCCAAATTC     480
   CAACTTTCCA CAAATGTCAA CATCCAGGCT TCAGACTCCA CAGTCAAGAA TATCGAAAAT     540
   TGATTCATCA AAGATTGGTA TCAAGCCAAA GACGTCTGGA CTTAAACCAC CCTCATCATC     600
   AACCACTTCA TCAAATAATA CAAATTCATT CCGTCCGTCG AGCCGTTCGA GTGGCAATAA     660
   TAATGTTGGC TCGACGATAT CCACATCTGC GAAGAGCTTA GAATCATCAT CAACGTACAG     720
   CTCTATTTCG AATCTAAACC GACCTACCTC CCAACTCCAA AAACCTTCTA GACCACAAAC     780
   CCAGCTAGTT CGTGTTGCTA CAACTACAAA AATCGGAAGC TCAAAGCTAG CCGCTCCGAA     840
   AGCCGTGAGC ACCCCAAAAC TTGCTTCTGT GAAGACTATT GGAGCAAAAC AAGAGCCCGA     900
   TAACAGCGGT GGTGGTGGTG GTGGAATGCT GAAATTAAAG TTATTCAGTA GCAAAAACCC     960
   ATCTTCCTCA TCGAATAGCC CACAACCTAC GAGAAAGGCG GCGGCGGTGC CTCAACAACA    1020
   AACTTTGTCG AAAATCGCTG CCCCAGTGAA AAGTGGCCTG AAGCCGCCGA CCAGTAAGCT    1080
   GGGAAGTGCC ACGTCTATGT CGAAGCTTTG TACGCCAAAA GTTTCCTACC GTAAAACGGA    1140
   CGCCCCAATC ATATCTCAAC AAGACTCGAA ACGATGCTCA AAGAGCAGTG AAGAAGAGTC    1200
   CGGATACGCT GGATTCAACA GCACGTCGCC AACGTCATCA TCGACGGAAG GTTCCCTAAG    1260
   CATGCATTCC ACATCTTCCA AGAGTTCAAC GTCAGACGAA AAGTCTCCGT CATCAGACGA 1320
   TCTTACTCTT AACGCCTCCA TCGTGACAGC TATCAGACAG CCGATAGCCG CAACACCGGT 1380
   TTCTCCAAAT ATTATCAACA AGCCTGTTGA GGAAAAACCA ACACTGGCAG TGAAAGGAGT 1440
   GAAAAGCACA GCGAAAAAAG ATCCACCTCC AGCTGTTCCG CCACGTGACA CCCAGCCAAC 1500
   AATCGGAGTT GTTAGTCCAA TTATGGCACA TAAGAAGTTG ACAAATGACC CCGTGATATC 1560
   TGAAAAACCA GAACCTGAAA AGCTCCAATC AATGAGCATC GACACGACGG ACGTTCCACC 1620
   GCTTCCACCT CTAAAATCAG TTGTTCCACT TAAAATGACT TCAATCCGAC AACCACCAAC 1680
   GTACGATGTT CTTCTAAAAC AAGGAAAAAT CACATCGCCT GTCAAGTCGT TTGGATATGA 1740
   GCAGTCGTCC GCGTCTGAAG ACTCCATTGT GGCTCATGCG TCGGCTCAGG TGACTCCGCC 1800
   GACAAAAACT TCTGGTAATC ATTCGCTGGA GAGAAGGATG GGAAAGAATA AGACATCAGA 1860
   ATCCAGCGGC TACACCTCTG ACGCCGGTGT TGCGATGTGC GCCAAAATGA GGGAGAAGCT 1920
   GAAAGAATAC GATGACATGA CTCGTCGAGC ACAGAACGGC TATCCTGACA ACTTCGAAGA 1980
   CAGTTCCTCC TTGTCGTCTG GAATATCCGA TAACAACGAG CTCGACGACA TATCCACGGA 2040
   CGATTTGTCC GGAGTAGACA TGGCAACAGT CGCCTCCAAA CATAGCGACT ATTCCCACTT 2100
   TGTTCGCCAT CCCACGTCTT CTTCCTCAAA GCCCCGAGTC CCCAGTCGGT CCTCCACATC 2160
   AGTCGATTCT CGATCTCGAG CAGAACAGGA GAATGTGTAC AAACTTCTGT CCCAGTGCCG 2220
   AACGAGCCAA CGTGGCGCCG CTGCCACCTC AACCTTCGGA CAACATTCGC TAAGATCCCC 2280
   GGGATACTCA TCCTATTCTC CACACTTATC AGTGTCAGCT GATAAGGACA CAATGTCTAT 2340
   GCACTCACAG ACTAGTCGAC GACCTTCTTC ACAAAAACCA AGCTATTCAG GCCAATTTCA 2400
   TTCACTTGAT CGTAAATGCC ACCTTCAAGA GTTCACATCC ACCGAGCACA GAATGGCGGC 2460
   TCTCTTGAGC CCGAGACGGG TGCCGAACTC GATGTCGAAA TATGATTCTT CAGGATCCTA 2520
   CTCGGCGCGT TCCCGAGGTG GAAGCTCTAC TGGTATCTAT GGAGAGACGT TCCAACTGCA 2580
   CAGACTATCC GATGAAAAAT CCCCCGCACA TTCTGCCAAA AGTGAGATGG GATCCCAACT 2640
   ATCACTGGCT AGCACGACAG CATATGGATC TCTCAATGAG AAGTACGAAC ATGCTATTCG 2700
   GGACATGGCA CGTGACTTGG AGTGTTACAA GAACACTGTC GACTCACTAA CCAAGAAACA 2760
   GGAGAACTAT GGAGCATTGT TTGATCTTTT TGAGCAAAAG CTTAGAAAAC TCACTCAACA 2820
   CATTGATCGA TCCAACTTGA AGCCTGAAGA GGCAATACGA TTCAGGCAGG ACATTGCTCA 2880
   TTTGAGGGAT ATTAGCAATC ATCTTGCATC CAACTCAGCT CATGCTAACG AAGGCGCTGG 2940
   TGAGCTTCTT CGTCAACCAT CTCTGGAATC AGTTGCATCC CATCGATCAT CGATGTCATC 3000
   GTCGTCGAAA AGCAGCAAGC AGGAGAAGAT CAGCTTGAGC TCGTTTGGCA AGAACAAGAA 3060
   GAGCTGGATC CGCTCCTCAC TCTCCAAGTT CACCAAGAAG AAGAACAAGA ACTACGACGA 3120
   AGCACATATG CCATCAATTT CCGGATCTCA AGGAACTCTT GACAACATTG ATGTGATTGA 3180
   GTTGAAGCAA GAGCTCAAAG AACGCGATAG TGCACTTTAC GAAGTCCGCC TTGACAATCT 3240
   GGATCGTGCC CGCGAAGTTG ATGTTCTGAG GGAGACAGTG AACAAGTTGA AAACCGAGAA 3300
   CAAGCAATTA AAGAAAGAAG TGGACAAACT CACCAACGGT CCAGCCACTC GTGCTTCTTC 3360
   CCGCGCCTCA ATTCCAGTTA TCTACGACGA TGAGCATGTC TATGATGCAG CGTGTAGCAG 3420
   TACATCAGCT AGTCAATCTT CGAAACGATC CTCTGGCTGC AACTCAATCA AGGTTACTGT 3480
   AAACGTGGAC ATCGCTGGAG AAATCAGTTC GATCGTTAAC CCGGACAAAG AGATAATCGT 3540
   AGGATATCTT GCCATGTCAA CCAGTCAGTC ATGCTGGAAA GACATTGATG TTTCTATTCT 3600
   AGGACTATTT GAAGTCTACC TATCCAGAAT TGATGTGGAG CATCAACTTG GAATCGATGC 3660
   TCGTGATTCT ATCCTTGGCT ATCAAATTGG TGAACTTCGA CGCGTCATTG GAGACTCCAC 3720
   AACCATGATA ACCAGCCATC CAACTGACAT TCTTACTTCC TCAACTACAA TCCGAATGTT 3780
   CATGCACGGT GCCGCACAGA GTCGCGTAGA CAGTCTGGTC CTTGATATGC TTCTTCCAAA 3840
   GCAAATGATT CTCCAACTCG TCAAGTCAAT TTTGACAGAG AGACGTCTGG TGTTAGCTGG 3900
   AGCAACTGGA ATTGGAAAGA GCAAACTGGC GAAGACCCTG GCTGCTTATG TATCTATTCG 3960
   AACAAATCAA TCCGAAGATA GTATTGTTAA TATCAGCATT CCTGAAAACA ATAAAGAAGA 4020
   ATTGCTTCAA GTGGAACGAC GCCTGGAAAA GATCTTGAGA AGCAAAGAAT CATGCATCGT 4080
   AATTCTAGAT AATATCCCAA AGAATCGAAT TGCATTTGTT GTATCCGTTT TTGCAAATGT 4140
   CCCACTTCAA AACAACGAAG GTCCATTTGT AGTATGCACA GTCAACCGAT ATCAAATCCC 4200
   TGAGCTTCAA ATTCACCACA ATTTCAAAAT GTCAGTAATG TCGAATCGTC TCGAAGGATT 4260
   CATCCTACGT TACCTCCGAC GACGGGCGGT AGAGGATGAG TATCGTCTAA CTGTACAGAT 4320
   GCCATCAGAG CTCTTCAAAA TCATTGACTT CTTCCCAATA GCTCTTCAGG CCGTCAATAA 4380
   TTTTATTGAG AAAACGAATT CTGTTGATGT GACAGTTGGT CCAAGAGCAT GCTTGAACTG 4440
   TCCTCTAACT GTCGATGGAT CCCGTGAATG GTTCATTCGA TTGTGGAATG AGAACTTCAT 4500
   TCCATATTTG GAACGTGTTG CTAGAGATGG CAAAAAAACC TTCGGTCGCT GCACTTCCTT 4560
   CGAGGATCCC ACCGACATCG TCTCTAAAAA ATGGCCGTGG TTCGATGGTG AAAACCCGGA 4620
   GAATGTGCTC AAACGTCTTC AACTCCAAGA CCTCGTCCCG TCACCTGCCA ACTCATCCCG 4680
   ACAACACTTC AATCCCCTCG AGTCGTTGAT CCAATTGCAT GCTACCAAGC ATCAGACCAT 4740
   CGACAACATT TGAACAGAAG ACTCTAATCT TCTCTCGCCT CTCCCCCGCT TTCCTTATCT 4800
   TCGTACCGGT ACCTGATGAT TCCCCATTTT CCCCCTTTTC CCCCCAATTT CCCAGAACCT 4860
   CCTGTTCCCT TTGTTCCTAG TCCTCCCGGG TGCCGACGCC GAAGCGATTT AAAAACCTTT 4920
   TTCTTTCCGA AACATTTCCC ATTGCTCATT AATAGTCAAA TTGAATAAAC AGTGTATGTA 4980
   CTTAAAAAAA AAAAAAAAAA AAAAAAAAAA GGCCTATGCG GCCGGGCCAT GGAGGCCGAA 5040
   TTCCCGGGGA TCCGTCGACC TGCAGCCAAG CTAATTCCGG GCGAATTTCT TATGATTTAT 5100
   GATTTTTATT ATTAAATAAG TTATAAAAAA AATAAGTGTA TACAAATTTT AAAGTGACTC 5160
   TTAGGTTTTA AAACGAAAAT TCTTGTTCTT GAGTAACTCT TTCCTGTAGG TCAGGTTGCT 5220
   TTCTCAGGTA TAGCATGAGG TCGCTCTTAT TGACCACACC TCTACCGGCA TGCAAGCTTG 5280
   GCGTAATCAT GGTCATAGCT GTTTCCTGTG TGAAATTGTT ATCCGCTCAC AATTCCACAC 5340
   AACATACGAG CCGGAAGCAT AAAGTGTAAA GCCTGGGGTG CCTAATGAGT GAGGTAACTC 5400
   ACATTAATTG CGTTGCGCTC ACTGCCCGCT TTCCAGTCGG GAAACCTGTC GTGCCAGCTG 5460
   GATTAATGAA TCGGCCAACG CGCGGGGAGA GGCGGTTTGC GTATTGGGCG CTCTTCCGCT 5520
   TCCTCGCTCA CTGACTCGCT GCGCTCGGTC GTTCGGCTGC GGCGAGCGGT ATCAGCTCAC 5580
   TCAAAGGCGG TAATACGGTT ATCCACAGAA TCAGGGGATA ACGCAGGAAA GAACATGTGA 5640
   GCAAAAGGCC AGCAAAAGGC CAGGAACCGT AAAAAGGCCG CGTTGCTGGC GTTTTTCCAT 5700
   AGGCTCCGCC CCCCTGACGA GCATCACAAA AATCGACGCT CAAGTCAGAG GTGGCGAAAC 5760
   CCGACAGGAC TATAAAGATA CCAGGCGTTT CCCCCTGGAA GCTCCCTCGT GCGCTCTCCT 5820
   GTTCCGACCC TGCCGCTTAC CGGATACCTG TCCGCCTTTC TCCCTTCGGG AAGCGTGGCG 5880
   CTTTCTCATA GCTCACGCTG TAGGTATCTC AGTTCGGTGT AGGTCGTTCG CTCCAAGCTG 5940
   GGCTGTGTGC ACGAACCCCC CGTTCAGCCC GACCGCTGCG CCTTATCCGG TAACTATCGT 6000
   CTTGAGTCCA ACCCGGTAAG ACACGACTTA TCGCCACTGG CAGCAGCCAC TGGTAACAGG 6060
   ATTAGCAGAG CGAGGTATGT AGGCGGTGCT ACAGAGTTCT TGAAGTGGTG GCCTAACTAC 6120
   GGCTACACTA GAAGGACAGT ATTTGGTATC TGCGCTCTGC TGAAGCCAGT TACCTTCGGA 6180
   AAAAGAGTTG GTAGCTCTTG ATCCGGCAAA CAAACCACCG CTGGTAGCGG TGGTTTTTTT 6240
   GTTTGCAAGC AGCAGATTAC GCGCAGAAAA AAAGGATCTC AAGAAGATCC TTTGATCTTT 6300
   TCTACGGGGT CTGACGCTCA GTGGAACGAA AACTCACGTT AAGGGATTTT GGTCATGAGA 6360
   TTATCAAAAA GGATCTTCAC CTAGATCCTT TTAAATTAAA AATGAAGTTT TAAATCAATC 6420
   TAAAGTATAT ATGAGTAAAC TTGGTCTGAC AGTTACCAAT GCTTAATCAG TGAGGCACCT 6480
   ATCTCAGCGA TCTGTCTATT TCGTTCATCC ATAGTTGCCT GACTCCCCGT CGTGTAGATA 6540
   ACTACGATAC GGGAGGGCTT ACCATCTGGC CCCAGTGCTG CAATGATACC GCGAGACCCA 6600
   CGCTCACCGG CTCCAGATTT ATCAGCAATA AACCAGCCAG CCGGAAGGGC CGAGCGCAGA 6660
   AGTGGTCCTG CAACTTTATC CGCCTCCATC CAGTCTATTA ATTGTTGCCG GGAAGCTAGA 6720
   GTAAGTAGTT CGCCAGTTAA TAGTTTGCGC AACGTTGTTG CCATTGCTAC AGGCATCGTG 6780
   GTGTCACGCT CGTCGTTTGG TATGGCTTCA TTCAGCTCCG GTTCCCAACG ATCAAGGCGA 6840
   GTTACATGAT CCCCCATGTT GTGCAAAAAA GCGGTTAGCT CCTTCGGTCC TCCGATCGTT 6900
   GTCAGAAGTA AGTTGGCCGC AGTGTTATCA CTCATGGTTA TGGCAGCACT GCATAATTCT 6960
   CTTACTGTCA TGCCATCCGT AAGATGCTTT TCTGTGACTG GTGAGTACTC AACCAAGTCA 7020
   TTCTGAGAAT AGTGTATGCG GCGACCGAGT TGCTCTTGCC CGGCGTCAAT ACGGGATAAT 7080
   ACCGCGCCAC ATAGCAGAAC TTTAAAAGTG CTCATCATTG GAAAACGTTC TTCGGGGCGA 7140
   AAACTCTCAA GGATCTTACC GCTGTTGAGA TCCAGTTCGA TGTAACCCAC TCGTGCACCC 7200
   AACTGATCTT CAGCATCTTT TACTTTCACC AGCGTTTCTG GGTGAGCAAA AACAGGAAGG 7260
   CAAAATGCCG CAAAAAAGGG AATAAGGGCG ACACGGAAAT GTTGAATACT CATACTCTTC 7320
   CTTTTTCAAT ATTATTGAAG CATTTATCAG GGTTATTGTC TCATGAGCGG ATACATATTT 7380
   GAATGTATTT AGAAAAATAA ACAAATAGGG GTTCCGCGCA CATTTCCCCG AAAAGTGCCA 7440
   CCTGAACGAA GCATCTGTGC TTCATTTTGT AGAACAAAAA TGCAACGCGA GAGCGCTAAT 7500
   TTTTCAAACA AAGAATCTGA GCTGCATTTT TACAGAACAG AAATGCAACG CGAAAGCGCT 7560
   ATTTTACCAA CGAAGAATCT GTGCTTCATT TTTGTAAAAC AAAAATGCAA CGCGAGAGCG 7620
   CTAATTTTTC AAACAAAGAA TCTGAGCTGC ATTTTTACAG AACAGAAATG CAACGCGAGA 7680
   GCGCTATTTT ACCAACAAAG AATCTATACT TCTTTTTTGT TCTACAAAAA TGCATCCCGA 7740
   GAGCGCTATT TTTCTAACAA AGCATCTTAG ATTACTTTTT TTCTCCTTTG TGCGCTCTAT 7800
   AATGCAGTCT CTTGATAACT TTTTGCACTG TAGGTCCGTT AAGGTTAGAA GAAGGCTACT 7860
   TTGGTGTCTA TTTTCTCTTC CATAAAAAAA GCCTGACTCC ACTTCCCGCG TTTACTGATT 7920
   ACTAGCGAAG CTGCGGGTGC ATTTTTTCAA GATAAAGGCA TCCCCGATTA TATTCTATAC 7980
   CGATGTGGAT TGCGCATACT TTGTGAACAG AAAGTGATAG CGTTGATGAT TCTTCATTGG 8040
   TCAGAAAATT ATGAACGGTT TCTTCTATTT TGTCTCTATA TACTACGTAT AGGAAATGTT 8100
   TACATTTTCG TATTGTTTTC GATTCACTCT ATGAATAGTT CTTACTACAA TTTTTTTGTC 8160
   TAAAGAGTAA TACTAGAGAT AAACATAAAA AATGTAGAGG TCGAGTTTAG ATGCAAGTTC 8220
   AAGGAGCGAA AGGTGGATGG GTAGGTTATA TAGGGATATA GCACAGAGAT ATATAGCAAA 8280
   GAGATACTTT TGAGCAATGT TTGTGGAAGC GGTATTCGCA ATATTTTAGT AGCTCGTTAC 8340
   AGTCCGGTGC GTTTTTGGTT TTTTGAAAGT GCGTCTTCAG AGCGCTTTTG GTTTTCAAAA 8400
   GCGCTCTGAA GTTCCTATAC TTTCTAGAGA ATAGGAACTT CGGAATAGGA ACTTCAAAGC 8460
   GTTTCCGAAA ACGAGCGCTT CCGAAAATGC AACGCGAGCT GCGCACATAC AGCTCACTGT 8520
   TCACGTCGCA CCTATATCTG CGTGTTGCCT GTATATATAT ATACATGAGA AGAACGGCAT 8580
   AGTGCGTGTT TATGCTTAAA TGCGTACTTA TATGCGTCTA TTTATGTAGG ATGAAAGGTA 8640
   GTCTAGTACC TCCTGTGATA TTATCCCATT CCATGCGGGG TATCGTATGC TTCCTTCAGC 8700
   ACTACCCTTT AGCTGTTCTA TATGCTGCCA CTCCTCAATT GGATTAGTCT CATCCTTCAA 8760
   TGCTATCATT TCCTTTGATA TTGGATCATA TTAAGAAACC ATTATTATCA TGACATTAAC 8820
   CTATAAAAAT AGGCGTATCA CGAGGCCCTT TCGTCTCGCG CGTTTCGGTG ATGACGGTGA 8880
   AAACCTCTGA CACATGCAGC TCCCGGAGAC GGTCACAGCT TGTCTGTAAG CGGATGCCGG 8940
   GAGCAGACAA GCCCGTCAGG GCGCGTCAGC GGGTGTTGGC GGGTGTCGGG GCTGGCTTAA 9000
   CTATGCGGCA TCAGAGCAGA TTGTACTGAG AGTGCACCAT AGATCAACGA CATTACTATA 9060
   TATATAATAT AGGAAGCATT TAATAGACAG CATCGTAATA TATGTGTACT TTGCAGTTAT 9120
   GACGCCAGAT GGCAGTAGTG GAAGATATTC TTTATTGAAA AATAGCTTGT CACCTTACGT 9180
   ACAATCTTGA TCCGGAGCTT TTCTTTTTTT GCCGATTAAG AATTAATTCG GTCGAAAAAA 9240
   GAAAAGGAGA GGGCCAAGAG GGAGGGCATT GGTGACTATT GAGCACGTGA GTATACGTGA 9300
   TTAAGCACAC AAAGGCAGCT TGGAGTATGT CTGTTATTAA TTTCACAGGT AGTTCTGGTC 9360
   CATTGGTGAA AGTTTGCGGC TTGCAGAGCA CAGAGGCCGC AGAATGTGCT CTAGATTCCG 9420
   ATGCTGACTT GCTGGGTATT ATATGTGTGC CCAATAGAAA GAGAACAATT GACCCGGTTA 9480
   TTGCAAGGAA AATTTCAAGT CTTGTAAAAG CATATAAAAA TAGTTCAGGC ACTCCGAAAT 9540
   ACTTGGTTGG CGTGTTTCGT AATCAACCTA AGGAGGATGT TTTGGCTCTG GTCAATGATT 9600
   ACGGCATTGA TATCGTCCAA CTGCATGGAG ATGAGTCGTG GCAAGAATAC CAAGAGTTCC 9660
   TCGGTTTGCC AGTTATTAAA AGACTCGTAT TTCCAAAAGA CTGCAACATA CTACTCAGTG 9720
   CAGCTTCACA GAAACCTCAT TCGTTTATTC CCTTGTTTGA TTCAGAAGCA GGTGGGACAG 9780
   GTGAACTTTT GGATTGGAAC TCGATTTCTG ACTGGGTTGG AAGGCAAGAG AGCCCCGAAA 9840
   GCTTACATTT TATGTTAGCT GGTGGACTGA CGCCAGAAAA TGTTGGTGAT GCGCTTAGAT 9900
   TAAATGGCGT TATTGGTGTT GATGTAAGCG GAGGTGTGGA GACAAATGGT GTAAAAGACT 9960
   CTAACAAAAT AGCAAATTTC GTCAAAAATG CTAAGAAATA GGTTATTACT GAGTAGTATT 10020
   TATTTAAGTA TTGTTTGTGC ACTTGCCGAT CTATGCGGTG TGAAATACCG CACAGATGCG 10080
   TAAGGAGAAA ATACCGCATC AGGAAATTGT AAACGTTAAT ATTTTGTTAA AATTCGCGTT 10140
   AAATTTTTGT TAAATCAGCT CATTTTTTAA CCAATAGGCC GAAATCGGCA AAATCCCTTA 10200
   TAAATCAAAA GAATAGACCG AGATAGGGTT GAGTGTTGTT CCAGTTTGGA ACAAGAGTCC 10260
   ACTATTAAAG AACGTGGACT CCAACGTCAA AGGGCGAAAA ACCGTCTATC AGGGCGATGG 10320
   CCCACTACGT GAACCATCAC CCTAATCAAG TTTTTTGGGG TCGAGGTGCC GTAAAGCACT 10380
   AAATCGGAAC CCTAAAGGGA GCCCCCGATT TAGAGCTTGA CGGGGAAAGC CGGCGAACGT 10440
   GGCGAGAAAG GAAGGGAAGA AAGCGAAAGG AGCGGGCGCT AGGGCGCTGG CAAGTGTAGC 10500
   GGTCACGCTG CGCGTAACCA CCACACCCGC CGCGCTTAAT GCGCCGCTAC AGGGCGCGTC 10560
   GCGCCATTCG CCATTCAGGC TGCGCAACTG TTGGGAAGGG CGATCGGTGC GGGCCTCTTC 10620
   GCTATTACGC CAGCTGGCGA AAGGGGGATG TGCTGCAAGG CGATTAAGTT GGGTAACGCC 10680
   AGGGTTTTCC CAGTCACGAC GTTGTAAAAC GACGGCCAGT CGTCCAAGCT TTCGCGAGCT 10740
   CGAGATCCCG AGCTTTGCAA ATTAAAGCCT TCGAGCGTCC CAAAACCTTC TCAAGCAAGG 10800
   TTTTCAGTAT AATGTTACAT GCGTACACGC GTCTGTACAG AAAAAAAAGA AAAATTTGAA 10860
   ATATAAATAA CGTTCTTAAT ACTAACATAA CTATAAAAAA ATAAATAGGG ACCTAGACTT 10920
   CAGGTTGTCT AACTCCTTCC TTTTCGGTTA GAGCGGATGT GGGGGGAGGG CGTGAATGTA 10980
   AGCGTGACAT AACTAATTAC ATGATATCGA CAAAGGAAAA GGGGCCTGTT TACTCACAGG 11040
   CTTTTTTCAA GTAGGTAATT AAGTCGTTTC TGTCTTTTTC CTTCTTCAAC CCACCAAAGG 11100
   CCATCTTGGT ACTTTTTTTT TTTTTTTTTT TTTTTTTTTT TTTTTTTTTT TTTTTTTTTT 11160
   TTTTTTTTTT TTTTTTTTTT TTTTTTTTTT TTTTTTTTTT TTTTTTCATA GAAATAATAC 11220
   AGAAGTAGAT GTTGAATTAG ATTAAACTGA AGATATATAA TTTATTGGAA AATACATAGA 11280
   GCTTTTTGTT GATGCGCTTA AGCGATCAAT TCAACAACAC CACCAGCAGC TCTGATTTTT 11340
   TCTTCAGCCA ACTTGGAGAC GAATCTAGCT TTGACGATAA CTGGAACATT TGGGATTCTA 11400
   CCCTTACCCA AGATCTTACC GTAACCGGCT GCCAAAGTGT CAATAACTGG AGCAGTTTCC 11460
   TTAGAAGCAG ATTTCAAGTA TTGGTCTCTC TTGTCTTCTG GGATCAATGT CCACAATTTG 11520
   TCCAAGTTCA AGACTGGCTT CCAGAAATGA GCTTGTTGCT TGTGGAAGTA TCTCATACCA 11580
   ANCCTTACCG AAATAACCTG GATGGTATTT ATCCATGTTA ATTCTGTGGT GATGTTGACC 11640
   ACCGGCCATA CCTCTACCAC CGGGGTGCTT TCTGTGCTTA CCGATACGAC CTTTACCGGC 11700
   TGAGACGTGA CCTCTGTGCT TTCTAGTCTT AGTGAATCTG GAAGGCATTC TTGATTAGTT 11760
   GGATGATTGT TCTGGGATTT AATGCAAAAA AATCACTAAG AAGGAAAAAA ATCAACGGAG 11820
   AAAGCAAACG CCATCTTAAA TATACGGGAT ACAGATGAAA GGTTTGAACC TATCTGGGAA 11880
   AATACGCATT AAACAAGCGA AAAACTGCGA GGAAAATTGT TTGCGTCTCT GCGGGCTATT 11940
   CACGCGCCAG AGGAAAATAG GAAAAATAAC AGGGCATTAG AAAAATAATT TTGATTTTGG 12000
   TAATGTGTGG GTCCCTGGTG TACAGATGTT ACATTGGTTA CAGTACTCTT GTTTTTGCTG 12060
   TGTTTTTCGA TGAATCTCCA AAATGGTTGT TAGCACATGG AAGAGTCACC GATGCTAAGT 12120
   TATCTCTATG TAAGCTACGT GGCGTGACTT TTGATGAAGC CGCACAAGAG ATACAGGATT 12180
   GGCAACTGCA AATAGAATCT GGGGATCTAG ATATCCTTTT GTTGTTTCCG GGTGTACAAT 12240
   ATGGACTTCC TCTTTTCTGG CAACCAAACC CATACATCGG GATTCCTATA ATACCTTCGT 12300
   TGGTCTCCCT AACATGTAGG TGGCGGAGGG GAGATATACA ATAGAACAGA TACCAGACAA 12360
   GACATAATGG GCTAAACAAG ACTACACCAA TTACACTGCC TCATTGATGG TGGTACATAA 12420
   CGAACTAATA CTGTAGCCCT AGACTTGATA GCCATCATCA TATCGAAGTT TCACTACCCT 12480
   TTTTCCATTT GCCATCTATT GAAGTAATAA TAGGCGCATG CAACTTCTTT TCTTTTTTTT 12540
   TCTTTTCTCT CTCCCCCGTT GTTGTCTCAC CATATCCGCA ATGACAAAAA AAATGATGGA 12600
   AGACACTAAA GGAAAAAATT AACGACAAAG ACAGCACCAA CAGATGTCGT TGTTCCAGAG 12660
   CTGATGAGGG GTATCTTCGA ACACACGAAA CTTTTTCCTT CCTTCATTCA CGCACACTAC 12720
   TCTCTAATGA GCAACGGTAT ACGGCCTTCC TTCCAGTTAC TTGAATTTGA AATAAAAAAA 12780
   GTTTGCCGCT TTGCTATCAA GTATAAATAG ACCTGCAATT ATTAATCTTT TGTTTCCTCG 12840
   TCATTGTTCT CGTTCCCTTT CTTCCTTGTT TCTTTTTCTG CACAATATTT CAAGCTATAC 12900
   CAAGCATACA ATCAACTCCA AGCTTGAAGC AAGCCTCCTG AAAGATGAAG CTACTGTCTT 12960
   CTATCGAACA AGCATGCGAT ATTTGCCGAC TTAAAAAGCT CAAGTGCTCC AAAGAAAAAC 13020
   CGAAGTGCGC CAAGTGTCTG AAGAACAACT GGGAGTGTCG CTACTCTCCC AAAACCAAAA 13080
   GGTCTCCGCT GACTAGGGCA CATCTGACAG AAGTGGAATC AAGGCTAGAA AGACTGGAAC 13140
   AGCTATTTCT ACTGATTTTT CCTCGAGAAG ACCTTGACAT GATTTTGAAA ATGGATTCTT 13200
   TACAGGATAT AAAAGCATTG TTAACAGGAT TATTTGTACA AGATAATGTG AATAAAGATG 13260
   CCGTCACAGA TAGATTGGCT TCAGTGGAGA CTGATATGCC TCTAACATTG AGACAGCATA 13320
   GAATAAGTGC GACATCATCA TCGGAAGAGA GTAGTAACAA AGGTCAAAGA CAGTTGACTG 13380
   TATCGCCGGA ATTGCAATAC CCAGCTTTGA CTCA 13414
(2) INFORMATION FOR SEQ ID NO: 28:
   (i) SEQUENCE CHARACTERISTICS:
      (A) LENGTH: 10288 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "plasmid"
   (iii) HYPOTHETICAL: NO
   (ix) FEATURE:
      (A) NAME/KEY: misc_feature
      (B) LOCATION:8456
      (D) OTHER INFORMATION:/note= "N is A,C,G, or T"
   (xi) SEQUENCE DESCRIPTION: SEQ ID NO: 28:
   TATGCCATCA ATTTCCGGAT CTCAAGGAAC TCTTGACAAC ATTGATGTGA TTGAGTTGAA 60
   GCAAGAGCTC AAAGAACGCG ATAGTGCACT TTACGAAGTC CGCCTTGACA ATCTGGATCG 120
   TGCCCGCGAA GTTGATGTTC TGAGGGAGAC AGTGAACAAG TTGAAAACCG AGAACAAGCA 180
   ATTAAAGAAA GAAGTGGACA AACTCACCAA CGGTCCAGCC ACTCGTGCTT CTTCCCGCGC 240
   CTCAATTCCA GTTATCTACG ACGATGAGCA TGTCTATGAT GCAGCGTGTA GCAGTACATC 300
   AGCTAGTCAA TCTTCGAAAC GATCCTCTGG CTGCAACTCA ATCAAGGTTA CTGTAAACGT 360
   GGACATCGCT GGAGAAATCA GTTCGATCGT TAACCCGGAC AAAGAGATAA TCGTAGGATA 420
   TCTTGCCATG TCAACCAGTC AGTCATGCTG GAAAGACATT GATGTTTCTA TTCTAGGACT 480
   ATTTGAAGTC TACCTATCCA GAATTGATGT GGAGCATCAA CTTGGAATCG ATGCTCGTGA 540
   TTCTATCCTT GGCTATCAAA TTGGTGAACT TCGACGCGTC ATTGGAGACT CCACAACCAT 600
   GATAACCAGC CATCCAACTG ACATTCTTAC TTCCTCAACT ACAATCCGAA TGTTCATGCA 660
   CGGTGCCGCA CAGAGTCGCG TAGACAGTCT GGTCCTTGAT ATGCTTCTTC CAAAGCAAAT 720
   GATTCTCCAA CTCGTCAAGT CAATTTTGAC AGAGAGACGT CTGGTGTTAG CTGGAGCAAC 780
   TGGAATTGGA AAGAGCAAAC TGGCGAAGAC CCTGGCTGCT TATGTATCTA TTCGAACAAA 840
   TCAATCCGAA GATAGTATTG TTAATATCAG CATTCCTGAA AACAATAAAG AAGAATTGCT 900
   TCAAGTGGAA CGACGCCTGG AAAAGATCTT GAGAAGCAAA GAATCATGCA TCGTAATTCT 960
   AGATAATATC CCAAAGAATC GAATTGCATT TGTTGTATCC GTTTTTGCAA ATGTCCCACT 1020
   TCAAAACAAC GAAGGTCCAT TTGTAGTATG CACAGTCAAC CGATATCAAA TCCCTGAGCT 1080
   TCAAATTCAC CACAATTTCA AAATGTCAGT AATGTCGAAT CGTCTCGAAG GATTCATCCT 1140
   ACGTTACCTC CGACGACGGG CGGTAGAGGA TGAGTATCGT CTAACTGTAC AGATGCCATC 1200
   AGAGCTCTTC AAAATCATTG ACTTCTTCCC AATAGCTCTT CAGGCCGTCA ATAATTTTAT 1260
   TGAGAAAACG AATTCTGTTG ATGTGACAGT TGGTCCAAGA GCATGCTTGA ACTGTCCTCT 1320
   AACTGTCGAT GGATCCCGTG AATGGTTCAT TCGATTGTGG AATGAGAACT TCATTCCATA 1380
   TTTGGAACGT GTTGCTAGAG ATGGCAAAAA AACCTTCGGT CGCTGCACTT CCTTCGAGGA 1440
   TCCCACCGAC ATCGTCTCTA AAAAATGGCC GTGGTTCGAT GGTGAAAACC CGGAGAATGT 1500
   GCTCAAACGT CTTCAACTCC AAGACCTCGT CCCGTCACCT GCCAACTCAT CCCGACAACA 1560
   CTTCAATCCC CTCGAGTCGT TGATCCAATT GCATGCTACC AAGCATCAGA CCATCGACAA 1620
   CATTTGAACA GAAGACTCTA ATCTTCTCTC GCCTCTCCCC CGCTTTCCTT ATCTTCGTAC 1680
   CGGTACCTGA TGATTCCCCA TTTTCCCCCT TTTCCCCCCA ATTTCCCAGA ACCTCCTGTT 1740
   CCCTTTGTTC CTAGTCCTCC CGGGTGCCGA CGCCGAAGCG ATTTAAAAAC CTTTTTCTTT 1800
   CCGAAACATT TCCCATTGCT CATTAATAGT CAAATTGAAT AAACAGTGTA TGTACTTAAA 1860
   AAAAAAAAAA AAAAAAAAAA AAAAGGCCTA TGCGGCCGGG CCATGGAGGC CGAATTCCCG 1920
   GGGATCCGTC GACCTGCAGC CAAGCTAATT CCGGGCGAAT TTCTTATGAT TTATGATTTT 1980
   TATTATTAAA TAAGTTATAA AAAAAATAAG TGTATACAAA TTTTAAAGTG ACTCTTAGGT 2040
   TTTAAAACGA AAATTCTTGT TCTTGAGTAA CTCTTTCCTG TAGGTCAGGT TGCTTTCTCA 2100
   GGTATAGCAT GAGGTCGCTC TTATTGACCA CACCTCTACC GGCATGCAAG CTTGGCGTAA 2160
   TCATGGTCAT AGCTGTTTCC TGTGTGAAAT TGTTATCCGC TCACAATTCC ACACAACATA 2220
   CGAGCCGGAA GCATAAAGTG TAAAGCCTGG GGTGCCTAAT GAGTGAGGTA ACTCACATTA 2280
   ATTGCGTTGC GCTCACTGCC CGCTTTCCAG TCGGGAAACC TGTCGTGCCA GCTGGATTAA 2340
   TGAATCGGCC AACGCGCGGG GAGAGGCGGT TTGCGTATTG GGCGCTCTTC CGCTTCCTCG 2400
   CTCACTGACT CGCTGCGCTC GGTCGTTCGG CTGCGGCGAG CGGTATCAGC TCACTCAAAG 2460
   GCGGTAATAC GGTTATCCAC AGAATCAGGG GATAACGCAG GAAAGAACAT GTGAGCAAAA 2520
   GGCCAGCAAA AGGCCAGGAA CCGTAAAAAG GCCGCGTTGC TGGCGTTTTT CCATAGGCTC 2580
   CGCCCCCCTG ACGAGCATCA CAAAAATCGA CGCTCAAGTC AGAGGTGGCG AAACCCGACA 2640
   GGACTATAAA GATACCAGGC GTTTCCCCCT GGAAGCTCCC TCGTGCGCTC TCCTGTTCCG 2700
   ACCCTGCCGC TTACCGGATA CCTGTCCGCC TTTCTCCCTT CGGGAAGCGT GGCGCTTTCT 2760
   CATAGCTCAC GCTGTAGGTA TCTCAGTTCG GTGTAGGTCG TTCGCTCCAA GCTGGGCTGT 2820
   GTGCACGAAC CCCCCGTTCA GCCCGACCGC TGCGCCTTAT CCGGTAACTA TCGTCTTGAG 2880
   TCCAACCCGG TAAGACACGA CTTATCGCCA CTGGCAGCAG CCACTGGTAA CAGGATTAGC 2940
   AGAGCGAGGT ATGTAGGCGG TGCTACAGAG TTCTTGAAGT GGTGGCCTAA CTACGGCTAC 3000
   ACTAGAAGGA CAGTATTTGG TATCTGCGCT CTGCTGAAGC CAGTTACCTT CGGAAAAAGA 3060
   GTTGGTAGCT CTTGATCCGG CAAACAAACC ACCGCTGGTA GCGGTGGTTT TTTTGTTTGC 3120
   AAGCAGCAGA TTACGCGCAG AAAAAAAGGA TCTCAAGAAG ATCCTTTGAT CTTTTCTACG 3180
   GGGTCTGACG CTCAGTGGAA CGAAAACTCA CGTTAAGGGA TTTTGGTCAT GAGATTATCA 3240
   AAAAGGATCT TCACCTAGAT CCTTTTAAAT TAAAAATGAA GTTTTAAATC AATCTAAAGT 3300
   ATATATGAGT AAACTTGGTC TGACAGTTAC CAATGCTTAA TCAGTGAGGC ACCTATCTCA 3360
   GCGATCTGTC TATTTCGTTC ATCCATAGTT GCCTGACTCC CCGTCGTGTA GATAACTACG 3420
   ATACGGGAGG GCTTACCATC TGGCCCCAGT GCTGCAATGA TACCGCGAGA CCCACGCTCA 3480
   CCGGCTCCAG ATTTATCAGC AATAAACCAG CCAGCCGGAA GGGCCGAGCG CAGAAGTGGT 3540
   CCTGCAACTT TATCCGCCTC CATCCAGTCT ATTAATTGTT GCCGGGAAGC TAGAGTAAGT 3600
   AGTTCGCCAG TTAATAGTTT GCGCAACGTT GTTGCCATTG CTACAGGCAT CGTGGTGTCA 3660
   CGCTCGTCGT TTGGTATGGC TTCATTCAGC TCCGGTTCCC AACGATCAAG GCGAGTTACA 3720
   TGATCCCCCA TGTTGTGCAA AAAAGCGGTT AGCTCCTTCG GTCCTCCGAT CGTTGTCAGA 3780
   AGTAAGTTGG CCGCAGTGTT ATCACTCATG GTTATGGCAG CACTGCATAA TTCTCTTACT 3840
   GTCATGCCAT CCGTAAGATG CTTTTCTGTG ACTGGTGAGT ACTCAACCAA GTCATTCTGA 3900
   GAATAGTGTA TGCGGCGACC GAGTTGCTCT TGCCCGGCGT CAATACGGGA TAATACCGCG 3960
   CCACATAGCA GAACTTTAAA AGTGCTCATC ATTGGAAAAC GTTCTTCGGG GCGAAAACTC 4020
   TCAAGGATCT TACCGCTGTT GAGATCCAGT TCGATGTAAC CCACTCGTGC ACCCAACTGA 4080
   TCTTCAGCAT CTTTTACTTT CACCAGCGTT TCTGGGTGAG CAAAAACAGG AAGGCAAAAT 4140
   GCCGCAAAAA AGGGAATAAG GGCGACACGG AAATGTTGAA TACTCATACT CTTCCTTTTT 4200
   CAATATTATT GAAGCATTTA TCAGGGTTAT TGTCTCATGA GCGGATACAT ATTTGAATGT 4260
   ATTTAGAAAA ATAAACAAAT AGGGGTTCCG CGCACATTTC CCCGAAAAGT GCCACCTGAA 4320
   CGAAGCATCT GTGCTTCATT TTGTAGAACA AAAATGCAAC GCGAGAGCGC TAATTTTTCA 4380
   AACAAAGAAT CTGAGCTGCA TTTTTACAGA ACAGAAATGC AACGCGAAAG CGCTATTTTA 4440
   CCAACGAAGA ATCTGTGCTT CATTTTTGTA AAACAAAAAT GCAACGCGAG AGCGCTAATT 4500
   TTTCAAACAA AGAATCTGAG CTGCATTTTT ACAGAACAGA AATGCAACGC GAGAGCGCTA 4560
   TTTTACCAAC AAAGAATCTA TACTTCTTTT TTGTTCTACA AAAATGCATC CCGAGAGCGC 4620
   TATTTTTCTA ACAAAGCATC TTAGATTACT TTTTTTCTCC TTTGTGCGCT CTATAATGCA 4680
   GTCTCTTGAT AACTTTTTGC ACTGTAGGTC CGTTAAGGTT AGAAGAAGGC TACTTTGGTG 4740
   TCTATTTTCT CTTCCATAAA AAAAGCCTGA CTCCACTTCC CGCGTTTACT GATTACTAGC 4800
   GAAGCTGCGG GTGCATTTTT TCAAGATAAA GGCATCCCCG ATTATATTCT ATACCGATGT 4860
   GGATTGCGCA TACTTTGTGA ACAGAAAGTG ATAGCGTTGA TGATTCTTCA TTGGTCAGAA 4920
   AATTATGAAC GGTTTCTTCT ATTTTGTCTC TATATACTAC GTATAGGAAA TGTTTACATT 4980
   TTCGTATTGT TTTCGATTCA CTCTATGAAT AGTTCTTACT ACAATTTTTT TGTCTAAAGA 5040
   GTAATACTAG AGATAAACAT AAAAAATGTA GAGGTCGAGT TTAGATGCAA GTTCAAGGAG 5100
   CGAAAGGTGG ATGGGTAGGT TATATAGGGA TATAGCACAG AGATATATAG CAAAGAGATA 5160
   CTTTTGAGCA ATGTTTGTGG AAGCGGTATT CGCAATATTT TAGTAGCTCG TTACAGTCCG 5220
   GTGCGTTTTT GGTTTTTTGA AAGTGCGTCT TCAGAGCGCT TTTGGTTTTC AAAAGCGCTC 5280
   TGAAGTTCCT ATACTTTCTA GAGAATAGGA ACTTCGGAAT AGGAACTTCA AAGCGTTTCC 5340
   GAAAACGAGC GCTTCCGAAA ATGCAACGCG AGCTGCGCAC ATACAGCTCA CTGTTCACGT 5400
   CGCACCTATA TCTGCGTGTT GCCTGTATAT ATATATACAT GAGAAGAACG GCATAGTGCG 5460
   TGTTTATGCT TAAATGCGTA CTTATATGCG TCTATTTATG TAGGATGAAA GGTAGTCTAG 5520
   TACCTCCTGT GATATTATCC CATTCCATGC GGGGTATCGT ATGCTTCCTT CAGCACTACC 5580
   CTTTAGCTGT TCTATATGCT GCCACTCCTC AATTGGATTA GTCTCATCCT TCAATGCTAT 5640
   CATTTCCTTT GATATTGGAT CATATTAAGA AACCATTATT ATCATGACAT TAACCTATAA 5700
   AAATAGGCGT ATCACGAGGC CCTTTCGTCT CGCGCGTTTC GGTGATGACG GTGAAAACCT 5760
   CTGACACATG CAGCTCCCGG AGACGGTCAC AGCTTGTCTG TAAGCGGATG CCGGGAGCAG 5820
   ACAAGCCCGT CAGGGCGCGT CAGCGGGTGT TGGCGGGTGT CGGGGCTGGC TTAACTATGC 5880
   GGCATCAGAG CAGATTGTAC TGAGAGTGCA CCATAGATCA ACGACATTAC TATATATATA 5940
   ATATAGGAAG CATTTAATAG ACAGCATCGT AATATATGTG TACTTTGCAG TTATGACGCC 6000
   AGATGGCAGT AGTGGAAGAT ATTCTTTATT GAAAAATAGC TTGTCACCTT ACGTACAATC 6060
   TTGATCCGGA GCTTTTCTTT TTTTGCCGAT TAAGAATTAA TTCGGTCGAA AAAAGAAAAG 6120
   GAGAGGGCCA AGAGGGAGGG CATTGGTGAC TATTGAGCAC GTGAGTATAC GTGATTAAGC 6180
   ACACAAAGGC AGCTTGGAGT ATGTCTGTTA TTAATTTCAC AGGTAGTTCT GGTCCATTGG 6240
   TGAAAGTTTG CGGCTTGCAG AGCACAGAGG CCGCAGAATG TGCTCTAGAT TCCGATGCTG 6300
   ACTTGCTGGG TATTATATGT GTGCCCAATA GAAAGAGAAC AATTGACCCG GTTATTGCAA 6360
   GGAAAATTTC AAGTCTTGTA AAAGCATATA AAAATAGTTC AGGCACTCCG AAATACTTGG 6420
   TTGGCGTGTT TCGTAATCAA CCTAAGGAGG ATGTTTTGGC TCTGGTCAAT GATTACGGCA 6480
   TTGATATCGT CCAACTGCAT GGAGATGAGT CGTGGCAAGA ATACCAAGAG TTCCTCGGTT 6540
   TGCCAGTTAT TAAAAGACTC GTATTTCCAA AAGACTGCAA CATACTACTC AGTGCAGCTT 6600
   CACAGAAACC TCATTCGTTT ATTCCCTTGT TTGATTCAGA AGCAGGTGGG ACAGGTGAAC 6660
   TTTTGGATTG GAACTCGATT TCTGACTGGG TTGGAAGGCA AGAGAGCCCC GAAAGCTTAC 6720
   ATTTTATGTT AGCTGGTGGA CTGACGCCAG AAAATGTTGG TGATGCGCTT AGATTAAATG 6780
   GCGTTATTGG TGTTGATGTA AGCGGAGGTG TGGAGACAAA TGGTGTAAAA GACTCTAACA 6840
   AAATAGCAAA TTTCGTCAAA AATGCTAAGA AATAGGTTAT TACTGAGTAG TATTTATTTA 6900
   AGTATTGTTT GTGCACTTGC CGATCTATGC GGTGTGAAAT ACCGCACAGA TGCGTAAGGA 6960
   GAAAATACCG CATCAGGAAA TTGTAAACGT TAATATTTTG TTAAAATTCG CGTTAAATTT 7020
   TTGTTAAATC AGCTCATTTT TTAACCAATA GGCCGAAATC GGCAAAATCC CTTATAAATC 7080
   AAAAGAATAG ACCGAGATAG GGTTGAGTGT TGTTCCAGTT TGGAACAAGA GTCCACTATT 7140
   AAAGAACGTG GACTCCAACG TCAAAGGGCG AAAAACCGTC TATCAGGGCG ATGGCCCACT 7200
   ACGTGAACCA TCACCCTAAT CAAGTTTTTT GGGGTCGAGG TGCCGTAAAG CACTAAATCG 7260
   GAACCCTAAA GGGAGCCCCC GATTTAGAGC TTGACGGGGA AAGCCGGCGA ACGTGGCGAG 7320
   AAAGGAAGGG AAGAAAGCGA AAGGAGCGGG CGCTAGGGCG CTGGCAAGTG TAGCGGTCAC 7380
   GCTGCGCGTA ACCACCACAC CCGCCGCGCT TAATGCGCCG CTACAGGGCG CGTCGCGCCA 7440
   TTCGCCATTC AGGCTGCGCA ACTGTTGGGA AGGGCGATCG GTGCGGGCCT CTTCGCTATT 7500
   ACGCCAGCTG GCGAAAGGGG GATGTGCTGC AAGGCGATTA AGTTGGGTAA CGCCAGGGTT 7560
   TTCCCAGTCA CGACGTTGTA AAACGACGGC CAGTCGTCCA AGCTTTCGCG AGCTCGAGAT 7620
   CCCGAGCTTT GCAAATTAAA GCCTTCGAGC GTCCCAAAAC CTTCTCAAGC AAGGTTTTCA 7680
   GTATAATGTT ACATGCGTAC ACGCGTCTGT ACAGAAAAAA AAGAAAAATT TGAAATATAA 7740
   ATAACGTTCT TAATACTAAC ATAACTATAA AAAAATAAAT AGGGACCTAG ACTTCAGGTT 7800
   GTCTAACTCC TTCCTTTTCG GTTAGAGCGG ATGTGGGGGG AGGGCGTGAA TGTAAGCGTG 7860
   ACATAACTAA TTACATGATA TCGACAAAGG AAAAGGGGCC TGTTTACTCA CAGGCTTTTT 7920
   TCAAGTAGGT AATTAAGTCG TTTCTGTCTT TTTCCTTCTT CAACCCACCA AAGGCCATCT 7980
   TGGTACTTTT TTTTTTTTTT TTTTTTTTTT TTTTTTTTTT TTTTTTTTTT TTTTTTTTTT 8040
   TTTTTTTTTT TTTTTTTTTT TTTTTTTTTT TTTTTTTTTT CATAGAAATA ATACAGAAGT 8100
   AGATGTTGAA TTAGATTAAA CTGAAGATAT ATAATTTATT GGAAAATACA TAGAGCTTTT 8160
   TGTTGATGCG CTTAAGCGAT CAATTCAACA ACACCACCAG CAGCTCTGAT TTTTTCTTCA 8220
   GCCAACTTGG AGACGAATCT AGCTTTGACG ATAACTGGAA CATTTGGGAT TCTACCCTTA 8280
   CCCAAGATCT TACCGTAACC GGCTGCCAAA GTGTCAATAA CTGGAGCAGT TTCCTTAGAA 8340
   GCAGATTTCA AGTATTGGTC TCTCTTGTCT TCTGGGATCA ATGTCCACAA TTTGTCCAAG 8400
   TTCAAGACTG GCTTCCAGAA ATGAGCTTGT TGCTTGTGGA AGTATCTCAT ACCAANCCTT 8460
   ACCGAAATAA CCTGGATGGT ATTTATCCAT GTTAATTCTG TGGTGATGTT GACCACCGGC 8520
   CATACCTCTA CCACCGGGGT GCTTTCTGTG CTTACCGATA CGACCTTTAC CGGCTGAGAC 8580
   GTGACCTCTG TGCTTTCTAG TCTTAGTGAA TCTGGAAGGC ATTCTTGATT AGTTGGATGA 8640
   TTGTTCTGGG ATTTAATGCA AAAAAATCAC TAAGAAGGAA AAAAATCAAC GGAGAAAGCA 8700
   AACGCCATCT TAAATATACG GGATACAGAT GAAAGGTTTG AACCTATCTG GGAAAATACG 8760
   CATTAAACAA GCGAAAAACT GCGAGGAAAA TTGTTTGCGT CTCTGCGGGC TATTCACGCG 8820
   CCAGAGGAAA ATAGGAAAAA TAACAGGGCA TTAGAAAAAT AATTTTGATT TTGGTAATGT 8880
   GTGGGTCCCT GGTGTACAGA TGTTACATTG GTTACAGTAC TCTTGTTTTT GCTGTGTTTT 8940
   TCGATGAATC TCCAAAATGG TTGTTAGCAC ATGGAAGAGT CACCGATGCT AAGTTATCTC 9000
   TATGTAAGCT ACGTGGCGTG ACTTTTGATG AAGCCGCACA AGAGATACAG GATTGGCAAC 9060
   TGCAAATAGA ATCTGGGGAT CTAGATATCC TTTTGTTGTT TCCGGGTGTA CAATATGGAC 9120
   TTCCTCTTTT CTGGCAACCA AACCCATACA TCGGGATTCC TATAATACCT TCGTTGGTCT 9180
   CCCTAACATG TAGGTGGCGG AGGGGAGATA TACAATAGAA CAGATACCAG ACAAGACATA 9240
   ATGGGCTAAA CAAGACTACA CCAATTACAC TGCCTCATTG ATGGTGGTAC ATAACGAACT 9300
   AATACTGTAG CCCTAGACTT GATAGCCATC ATCATATCGA AGTTTCACTA CCCTTTTTCC 9360
   ATTTGCCATC TATTGAAGTA ATAATAGGCG CATGCAACTT CTTTTCTTTT TTTTTCTTTT 9420
   CTCTCTCCCC CGTTGTTGTC TCACCATATC CGCAATGACA AAAAAAATGA TGGAAGACAC 9480
   TAAAGGAAAA AATTAACGAC AAAGACAGCA CCAACAGATG TCGTTGTTCC AGAGCTGATG 9540
   AGGGGTATCT TCGAACACAC GAAACTTTTT CCTTCCTTCA TTCACGCACA CTACTCTCTA 9600
   ATGAGCAACG GTATACGGCC TTCCTTCCAG TTACTTGAAT TTGAAATAAA AAAAGTTTGC 9660
   CGCTTTGCTA TCAAGTATAA ATAGACCTGC AATTATTAAT CTTTTGTTTC CTCGTCATTG 9720
   TTCTCGTTCC CTTTCTTCCT TGTTTCTTTT TCTGCACAAT ATTTCAAGCT ATACCAAGCA 9780
   TACAATCAAC TCCAAGCTTG AAGCAAGCCT CCTGAAAGAT GAAGCTACTG TCTTCTATCG 9840
   AACAAGCATG CGATATTTGC CGACTTAAAA AGCTCAAGTG CTCCAAAGAA AAACCGAAGT 9900
   GCGCCAAGTG TCTGAAGAAC AACTGGGAGT GTCGCTACTC TCCCAAAACC AAAAGGTCTC 9960
   CGCTGACTAG GGCACATCTG ACAGAAGTGG AATCAAGGCT AGAAAGACTG GAACAGCTAT 10020
   TTCTACTGAT TTTTCCTCGA GAAGACCTTG ACATGATTTT GAAAATGGAT TCTTTACAGG 10080
   ATATAAAAGC ATTGTTAACA GGATTATTTG TACAAGATAA TGTGAATAAA GATGCCGTCA 10140
   CAGATAGATT GGCTTCAGTG GAGACTGATA TGCCTCTAAC ATTGAGACAG CATAGAATAA    10200
   GTGCGACATC ATCATCGGAA GAGAGTAGTA ACAAAGGTCA AAGACAGTTG ACTGTATCGC    10260
   CGGAATTGCA ATACCCAGCT TTGACTCA                                       10288
(2) INFORMATION FOR SEQ ID NO: 29:
   (i) SEQUENCE CHARACTERISTICS:
      (A) LENGTH: 7625 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "plasmid"
   (iii) HYPOTHETICAL: NO
   (xi) SEQUENCE DESCRIPTION: SEQ ID NO: 29:
   GCTTGCATGC AACTTCTTTT CTTTTTTTTT CTTTTCTCTC TCCCCCGTTG TTGTCTCACC 60
   ATATCCGCAA TGACAAAAAA AATGATGGAA GACACTAAAG GAAAAAATTA ACGACAAAGA 120
   CAGCACCAAC AGATGTCGTT GTTCCAGAGC TGATGAGGGG TATCTTCGAA CACACGAAAC 180
   TTTTTCCTTC CTTCATTCAC GCACACTACT CTCTAATGAG CAACGGTATA CGGCCTTCCT 240
   TCCAGTTACT TGAATTTGAA ATAAAAAAAG TTTGCCGCTT TGCTATCAAG TATAAATAGA 300
   CCTGCAATTA TTAATCTTTT GTTTCCTCGT CATTGTTCTC GTTCCCTTTC TTCCTTGTTT 360
   CTTTTTCTGC ACAATATTTC AAGCTATACC AAGCATACAA TCAACTCCAA GCTTTGCAAA 420
   GATGGATAAA GCGGAATTAA TTCCCGAGCC TCCAAAAAAG AAGAGAAAGG TCGAATTGGG 480
   TACCGCCGCC AATTTTAATC AAAGTGGGAA TATTGCTGAT AGCTCATTGT CCTTCACTTT 540
   CACTAACAGT AGCAACGGTC CGAACCTCAT AACAACTCAA ACAAATTCTC AAGCGCTTTC 600
   ACAACCAATT GCCTCCTCTA ACGTTCATGA TAACTTCATG AATAATGAAA TCACGGCTAG 660
   TAAAATTGAT GATGGTAATA ATTCAAAACC ACTGTCACCT GGTTGGACGG ACCAAACTGC 720
   GTATAACGCG TTTGGAATCA CTACAGGGAT GTTTAATACC ACTACAATGG ATGATGTATA 780
   TAACTATCTA TTCGATGATG AAGATACCCC ACCAAACCCA AAAAAAGAGA TCGAATTCCC 840
   GGGGATCCGC TCCTCACTCT CCAAGTTCAC CAAGAAGAAG AACAAGAACT ACGACGAAGC 900
   ACATATGCCA TCAATTTCCG GATCTCAAGG AACTCTTGAC AACATTGATG TGATTGAGTT 960
   GAAGCAAGAG CTCAAAGAAC GCGATAGTGC ACTTTACGAA GTCCGCCTTG ACAATCTGGA 1020
   TCGTGCCCGC GAAGTTGATG TTCTGAGGGA GACAGTGAAC AAGTTGAAAA CCGAGAACAA 1080
   GCAATTAAAG AAAGAAGTGG ACAAACTCAC CAACGGTCCA GCCACTCGTG CTTCTTCCCG 1140
   CGCCTCAATT CCAGTTATCT ACGACGATGA GCATGTCTAT GATGCAGCGT GTAGCAGTAC 1200
   ATCAGCTAGT CAATCTTCGA AACGATCCTC TGGCTGCAAC TCAATCAAGG TTACTGTAAA 1260
   CGTGGACATC GCTGGAGAAA TCAGTTCGAT CGTTAACCCG GACAAAGAGA TAATCGTAGG 1320
   ATATCTTGCC ATGTCAACCA GTCAGTCATG CTGGAAAGAC ATTGATGTTT CTATTCTAGG 1380
   ACTATTTGAA GTCTACCTAT CCAGAATTGA TGTGGAGCAT CAACTTGGAA TCGATGCTCG 1440
   TGATTCTATC CTTGGCTATC AAATTGGTGA ACTTCGACGC GTCATTGGAG ACTCCACAAC 1500
   CATGATAACC AGCCATCCAA CTGACATTCT TACTTCCTCA ACTACAATCC GAATGTTCAT 1560
   GCACGGTGCC GCACAGAGTC GCGTAGACAG TCTGGTCCTT GATATGCTTC TTCCAAAGCA 1620
   AATGATTCTC CAACTCGTCA AGTCAATTTT GACAGAGAGA CGTCTGGTGT TAGCTGGAGC 1680
   AACTGGAATT GGAAAGAGCA AACTGGCGAA GACCCTGGCT GCTTATGTAT CTATTCGAAC 1740
   AAATCAATCC GAAGATAGTA TTGTTAATAT CAGCATTCCT GAAAACAATA AAGAAGAATT 1800
   GCTTCAAGTG GAACGACGCC TGGAAAAGAT CTATGAATCG TAGATACTGA AAAACCCCGC 1860
   AAGTTCACTT CAACTGTGCA TCGTGCACCA TCTCAATTTC TTTCATTTAT ACATCGTTTT 1920
   GCCTTCTTTT ATGTAACTAT ACTCCTCTAA GTTTCAATCT TGGCCATGTA ACCTCTGATC 1980
   TATAGAATTT TTTAAATGAC TAGAATTAAT GCCCATCTTT TTTTTGGACC TAAATTCTTC 2040
   ATGAAAATAT ATTACGAGGG CTTATTCAGA AGCTTTGGAC TTCTTCGCCA GAGGTTTGGT 2100
   CAAGTCTCCA ATCAAGGTTG TCGGCTTGTC TACCTTGCCA GAAATTTACG AAAAGATGGA 2160
   AAAGGGTCAA ATCGTTGGTA GATACGTTGT TGACACTTCT AAATAAGCGA ATTTCTTATG 2220
   ATTTATGATT TTTATTATTA AATAAGTTAT AAAAAAAATA AGTGTATACA AATTTTAAAG 2280
   TGACTCTTAG GTTTTAAAAC GAAAATTCTT GTTCTTGAGT AACTCTTTCC TGTAGGTCAG 2340
   GTTGCTTTCT CAGGTATAGC ATGAGGTCGC TCTTATTGAC CACACCTCTA CCGGCATGCC 2400
   CGAAATTCCC CTACCCTATG AACATATTCC ATTTTGTAAT TTCGTGTCGT TTCTATTATG 2460
   AATTTCATTT ATAAAGTTTA TGTACAAATA TCATAAAAAA AGAGAATCTT TTTAAGCAAG 2520
   GATTTTCTTA ACTTCTTCGG CGACAGCATC ACCGACTTCG GTGGTACTGT TGGAACCACC 2580
   TAAATCACCA GTTCTGATAC CTGCATCCAA AACCTTTTTA ACTGCATCTT CAATGGCCTT 2640
   ACCTTCTTCA GGCAAGTTCA ATGACAATTT CAACATCATT GCAGCAGACA AGATAGTGGC 2700
   GATAGGGTCA ACCTTATTCT TTGGCAAATC TGGAGCAGAA CCGTGGCATG GTTCGTACAA 2760
   ACCAAATGCG GTGTTCTTGT CTGGCAAAGA GGCCAAGGAC GCAGATGGCA ACAAACCCAA 2820
   GGAACCTGGG ATAACGGAGG CTTCATCGGA GATGATATCA CCAAACATGT TGCTGGTGAT 2880
   TATAATACCA TTTAGGTGGG TTGGGTTCTT AACTAGGATC ATGGCGGCAG AATCAATCAA 2940
   TTGATGTTGA ACCTTCAATG TAGGAAATTC GTTCTTGATG GTTTCCTCCA CAGTTTTTCT 3000
   CCATAATCTT GAAGAGGCCA AAACATTAGC TTTATCCAAG GACCAAATAG GCAATGGTGG 3060
   CTCATGTTGT AGGGCCATGA AAGCGGCCAT TCTTGTGATT CTTTGCACTT CTGGAACGGT 3120
   GTATTGTTCA CTATCCCAAG CGACACCATC ACCATCGTCT TCCTTTCTCT TACCAAAGTA 3180
   AATACCTCCC ACTAATTCTC TGACAACAAC GAAGTCAGTA CCTTTAGCAA ATTGTGGCTT 3240
   GATTGGAGAT AAGTCTAAAA GAGAGTCGGA TGCAAAGTTA CATGGTCTTA AGTTGGCGTA 3300
   CAATTGAAGT TCTTTACGGA TTTTTAGTAA ACCTTGTTCA GGTCTAACAC TACCTGTACC 3360
   CCATTTAGGA CCACCCACAG CACCTAACAA AACGGCATCA ACCTTCTTGG AGGCTTCCAG 3420
   CGCCTCATCT GGAAGTGGGA CACCTGTAGC ATCGATAGCA GCACCACCAA TTAAATGATT 3480
   TTCGAAATCG AACTTGACAT TGGAACGAAC ATCAGAAATA GCTTTAAGAA CCTTAATGGC 3540
   TTCGGCTGTG ATTTCTTGAC CAACGTGGTC ACCTGGCAAA ACGACGATCT TCTTAGGGGC 3600
   AGACATTAGA ATGGTATATC CTTGAAATAT ATATATATAT TGCTGAAATG TAAAAGGTAA 3660
   GAAAAGTTAG AAAGTAAGAC GATTGCTAAC CACCTATTGG AAAAAACAAT AGGTCCTTAA 3720
   ATAATATTGT CAACTTCAAG TATTGTGATG CAAGCATTTA GTCATGAACG CTTCTCTATT 3780
   CTATATGAAA AGCCGGTTCC GGCCTCTCAC CTTTCCTTTT TCTCCCAATT TTTCAGTTGA 3840
   AAAAGGTATA TGCGTCAGGC GACCTCTGAA ATTAACAAAA AATTTCCAGT CATCGAATTT 3900
   GATTCTGTGC GATAGCGCCC CTGTGTGTTC TCGTTATGTT GAGGAAAAAA ATAATGGTTG 3960
   CTAAGAGATT CGAACTCTTG CATCTTACGA TACCTGAGTA TTCCCACAGT TGGGGATCTC 4020
   GACTCTAGCT AGAGGATCAA TTCGTAATCA TGGTCATAGC TGTTTCCTGT GTGAAATTGT 4080
   TATCCGCTCA CAATTCCACA CAACATACGA GCCGGAAGCA TAAAGTGTAA AGCCTGGGGT 4140
   GCCTAATGAG TGAGGTAACT CACATTAATT GCGTTGCGCT CACTGCCCGC TTTCCAGTCG 4200
   GGAAACCTGT CGTGCCAGCT GGATTAATGA ATCGGCCAAC GCGCGGGGAG AGGCGGTTTG 4260
   CGTATTGGGC GCTCTTCCGC TTCCTCGCTC ACTGACTCGC TGCGCTCGGT CGTTCGGCTG 4320
   CGGCGAGCGG TATCAGCTCA CTCAAAGGCG GTAATACGGT TATCCACAGA ATCAGGGGAT 4380
   AACGCAGGAA AGAACATGTG AGCAAAAGGC CAGCAAAAGG CCAGGAACCG TAAAAAGGCC 4440
   GCGTTGCTGG CGTTTTTCCA TAGGCTCCGC CCCCCTGACG AGCATCACAA AAATCGACGC 4500
   TCAAGTCAGA GGTGGCGAAA CCCGACAGGA CTATAAAGAT ACCAGGCGTT TCCCCCTGGA 4560
   AGCTCCCTCG TGCGCTCTCC TGTTCCGACC CTGCCGCTTA CCGGATACCT GTCCGCCTTT 4620
   CTCCCTTCGG GAAGCGTGGC GCTTTCTCAT AGCTCACGCT GTAGGTATCT CAGTTCGGTG 4680
   TAGGTCGTTC GCTCCAAGCT GGGCTGTGTG CACGAACCCC CCGTTCAGCC CGACCGCTGC 4740
   GCCTTATCCG GTAACTATCG TCTTGAGTCC AACCCGGTAA GACACGACTT ATCGCCACTG 4800
   GCAGCAGCCA CTGGTAACAG GATTAGCAGA GCGAGGTATG TAGGCGGTGC TACAGAGTTC 4860
   TTGAAGTGGT GGCCTAACTA CGGCTACACT AGAAGGACAG TATTTGGTAT CTGCGCTCTG 4920
   CTGAAGCCAG TTACCTTCGG AAAAAGAGTT GGTAGCTCTT GATCCGGCAA ACAAACCACC 4980
   GCTGGTAGCG GTGGTTTTTT TGTTTGCAAG CAGCAGATTA CGCGCAGAAA AAAAGGATCT 5040
   CAAGAAGATC CTTTGATCTT TTCTACGGGG TCTGACGCTC AGTGGAACGA AAACTCACGT 5100
   TAAGGGATTT TGGTCATGAG ATTATCAAAA AGGATCTTCA CCTAGATCCT TTTAAATTAA 5160
   AAATGAAGTT TTAAATCAAT CTAAAGTATA TATGAGTAAA CTTGGTCTGA CAGTTACCAA 5220
   TGCTTAATCA GTGAGGCACC TATCTCAGCG ATCTGTCTAT TTCGTTCATC CATAGTTGCC 5280
   TGACTCCCCG TCGTGTAGAT AACTACGATA CGGGAGGGCT TACCATCTGG CCCCAGTGCT 5340
   GCAATGATAC CGCGAGACCC ACGCTCACCG GCTCCAGATT TATCAGCAAT AAACCAGCCA 5400
   GCCGGAAGGG CCGAGCGCAG AAGTGGTCCT GCAACTTTAT CCGCCTCCAT CCAGTCTATT 5460
   AATTGTTGCC GGGAAGCTAG AGTAAGTAGT TCGCCAGTTA ATAGTTTGCG CAACGTTGTT 5520
   GCCATTGCTA CAGGCATCGT GGTGTCACGC TCGTCGTTTG GTATGGCTTC ATTCAGCTCC 5580
   GGTTCCCAAC GATCAAGGCG AGTTACATGA TCCCCCATGT TGTGCAAAAA AGCGGTTAGC 5640
   TCCTTCGGTC CTCCGATCGT TGTCAGAAGT AAGTTGGCCG CAGTGTTATC ACTCATGGTT 5700
   ATGGCAGCAC TGCATAATTC TCTTACTGTC ATGCCATCCG TAAGATGCTT TTCTGTGACT 5760
   GGTGAGTACT CAACCAAGTC ATTCTGAGAA TAGTGTATGC GGCGACCGAG TTGCTCTTGC 5820
   CCGGCGTCAA TACGGGATAA TACCGCGCCA CATAGCAGAA CTTTAAAAGT GCTCATCATT 5880
   GGAAAACGTT CTTCGGGGCG AAAACTCTCA AGGATCTTAC CGCTGTTGAG ATCCAGTTCG 5940
   ATGTAACCCA CTCGTGCACC CAACTGATCT TCAGCATCTT TTACTTTCAC CAGCGTTTCT 6000
   GGGTGAGCAA AAACAGGAAG GCAAAATGCC GCAAAAAAGG GAATAAGGGC GACACGGAAA 6060
   TGTTGAATAC TCATACTCTT CCTTTTTCAA TATTATTGAA GCATTTATCA GGGTTATTGT 6120
   CTCATGAGCG GATACATATT TGAATGTATT TAGAAAAATA AACAAATAGG GGTTCCGCGC 6180
   ACATTTCCCC GAAAAGTGCC ACCTGACGTC TAAGAAACCA TTATTATCAT GACATTAACC 6240
   TATAAAAATA GGCGTATCAC GAGGCCCTTT CGTCTCGCGC GTTTCGGTGA TGACGGTGAA 6300
   AACCTCTGAC ACATGCAGCT CCCGGAGACG GTCACAGCTT GTCTGTAAGC GGATGCCGGG 6360
   AGCAGACAAG CCCGTCAGGG CGCGTCAGCG GGTGTTGGCG GGTGTCGGGG CTGGCTTAAC 6420
   TATGCGGCAT CAGAGCAGAT TGTACTGAGA GTGCACCATA ACGCATTTAA GCATAAACAC 6480
   GCACTATGCC GTTCTTCTCA TGTATATATA TATACAGGCA ACACGCAGAT ATAGGTGCGA 6540
   CGTGAACAGT GAGCTGTATG TGCGCAGCTC GCGTTGCATT TTCGGAAGCG CTCGTTTTCG 6600
   GAAACGCTTT GAAGTTCCTA TTCCGAAGTT CCTATTCTCT AGCTAGAAAG TATAGGAACT 6660
   TCAGAGCGCT TTTGAAAACC AAAAGCGCTC TGAAGACGCA CTTTCAAAAA ACCAAAAACG 6720
   CACCGGACTG TAACGAGCTA CTAAAATATT GCGAATACCG CTTCCACAAA CATTGCTCAA 6780
   AAGTATCTCT TTGCTATATA TCTCTGTGCT ATATCCCTAT ATAACCTACC CATCCACCTT 6840
   TCGCTCCTTG AACTTGCATC TAAACTCGAC CTCTACATTT TTTATGTTTA TCTCTAGTAT 6900
   TACTCTTTAG ACAAAAAAAT TGTAGTAAGA ACTATTCATA GAGTGAATCG AAAACAATAC 6960
   GAAAATGTAA ACATTTCCTA TACGTAGTAT ATAGAGACAA AATAGAAGAA ACCGTTCATA 7020
   ATTTTCTGAC CAATGAAGAA TCATCAACGC TATCACTTTC TGTTCACAAA GTATGCGCAA 7080
   TCCACATCGG TATAGAATAT AATCGGGGAT GCCTTTATCT TGAAAAAATG CACCCGCAGC 7140
   TTCGCTAGTA ATCAGTAAAC GCGGGAAGTG GAGTCAGGCT TTTTTTATGG AAGAGAAAAT 7200
   AGACACCAAA GTAGCCTTCT TCTAACCTTA ACGGACCTAC AGTGCAAAAA GTTATCAAGA 7260
   GACTGCATTA TAGAGCGCAC AAAGGAGAAA AAAAGTAATC TAAGATGCTT TGTTAGAAAA 7320
   ATAGCGCTCT CGGGATGCAT TTTTGTAGAA CAAAAAAGAA GTATAGATTC TTTGTTGGTA 7380
   AAATAGCGCT CTCGCGTTGC ATTTCTGTTC TGTAAAAATG CAGCTCAGAT TCTTTGTTTG 7440
   AAAAATTAGC GCTCTCGCGT TGCATTTTTG TTTTACAAAA ATGAAGCACA GATTCTTCGT 7500
   TGGTAAAATA GCGCTTTCGC GTTGCATTTC TGTTCTGTAA AAATGCAGCT CAGATTCTTT 7560
   GTTTGAAAAA TTAGCGCTCT CGCGTTGCAT TTTTGTTCTA CAAAATGAAG CACAGATGCT 7620
   TCGTT 7625
(2) INFORMATION FOR SEQ ID NO: 30:
   (i) SEQUENCE CHARACTERISTICS:
      (A) LENGTH: 9642 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "plasmid"
   (iii) HYPOTHETICAL: NO
   (xi) SEQUENCE DESCRIPTION: SEQ ID NO: 30:
   ATGACCATGA TTACGCCAAG CTTGTCTTCT TCTAAATTCC CATAAAATCC CGAAACTCCT    60
   TCCCTCTATC TTCTTTTTCT TCTCGTTTTC AAATGTTTCT CTCTATCCCA TTCTCTCATC   120
   AATTGAGTGG GATGAGGCTA TCTCTGCCTC TCTTCTGAAT CTCTGAACCA TCTTACATTA   180
   CACTGTGGAT GACGAGCCCC ACAGGCTCCC TTGCATCAGA TACTGCCATT GGGGATGGCA   240
   AAGAAGAGAG AAGGTATTGT GAGGATATAT TTTTCTAAGA AAAAACGTTT GAAGAAAAGA   300
   AGATGAAGAA GATCTGCTTG ATTCATTGCA CAAGTTAGAA GTAACAGGGG TCTATATTTC   360
   GAAGAACTTA AAGGGAATGC AACTGAACAT AAAATTAAAC AAAGGGATTG AATCCTGCAG   420
   TGAGTATTTT CGGTTTTTCA CTGGTTCTCT GTAAAAAGAG TAATGCAAAG GGCAAGTTAA   480
   CTTAGGTCGT AAATGTATTG AATTTGCTTA AAATCTGAAG ATCTAGTGGT GAACCGTGGA   540
   AGATTATCAA GAGGAGGCTG AAGATCTGTT TAAGAACCAT TAATCAAACT GGTATTCTAT   600
   TTTCACTGGT TGTATGTAAA CATTCTATCT TATTCCTTTT ATCACTGTTC TGCACTTTCC   660
   TATAAAAAAA GTTGACCGAC CGTACTCTCT GAATTCATTT TTCCCGATCT TACCAACTCC 720
   CGATCTATCT CTATCCCTGG TTTTTTCTTC GTGCTCCAAT GGAATTCTTG AGACTTCCAC 780
   TATCTTCTCT GGCACCCTCC ACTACGCGTA GGCGTCTCTC GCTTCGTGTA TTCCCGGGAA 840
   GCCGGTTCCC GTCTCTCCCG CCGCTGCCGC TGCCGCACAC AGCTTTACAC CTCGTAGAAT 900
   CCCCAAAGAG GGGCGTGGCT TGCGGGTGCC AACATCCTCC TGCCGAGGAA GAAGCAGGCA 960
   CTCATCACTC GCATCATCAA CCTCGGGATT GGCCAAAGGA CCCAAAGGTA TGTTTCGAAT 1020
   GATACTAACA TAACATAGAA CATTTTCAGG AGGACCCTTG GCTAGAACTA GTGGATCCGA 1080
   GCTCTCCCAT ATGACGACGT CAAATGTAGA ATTGATACCA ATCTACACGG ATTGGGCCAA 1140
   TCGGCACCTT TCGAAGGGCA GCTTATCAAA GTCGATTAGG GATATTTCCA ATGATTTTCG 1200
   CGACTATCGA CTGGTTTCTC AGCTTATTAA TGTGATCGTT CCGATCAACG AATTCTCGCC 1260
   TGCATTCACG AAACGTTTGG CAAAAATCAC ATCGAACCTG GATGGCCTCG AAACGTGTCT 1320
   CGACTACCTG AAAAATCTGG GTCTCGACTG CTCGAAACTC ACCAAAACCG ATATCGACAG 1380
   CGGAAACTTG GGTGCAGTTC TCCAGCTGCT CTTCCTGCTC TCCACCTACA AGCAGAAGCT 1440
   TCGGCAACTG AAAAAAGATC AGAAGAAATT GGAGCAACTA CCCACATCCA TTATGCCACC 1500
   CGCGGTTTCT AAATTACCCT CGCCACGTGT CGCCACGTCA GCAACCGCTT CAGCAACTAA 1560
   CCCAAATTCC AACTTTCCAC AAATGTCAAC ATCCAGGCTT CAGACTCCAC AGTCAAGAAT 1620
   ATCGAAAATT GATTCATCAA AGATTGGTAT CAAGCCAAAG ACGTCTGGAC TTAAACCACC 1680
   CTCATCATCA ACCACTTCAT CAAATAATAC AAATTCATTC CGTCCGTCGA GCCGTTCGAG 1740
   TGGCAATAAT AATGTTGGCT CGACGATATC CACATCTGCG AAGAGCTTAG AATCATCATC 1800
   AACGTACAGC TCTATTTCGA ATCTAAACCG ACCTACCTCC CAACTCCAAA AACCTTCTAG 1860
   ACCACAAACC CAGCTAGTTC GTGTTGCTAC AACTACAAAA ATCGGAAGCT CAAAGCTAGC 1920
   CGCTCCGAAA GCCGTGAGCA CCCCAAAACT TGCTTCTGTG AAGACTATTG GAGCAAAACA 1980
   AGAGCCCGAT AACAGCGGTG GTGGTGGTGG TGGAATGCTG AAATTAAAGT TATTCAGTAG 2040
   CAAAAACCCA TCTTCCTCAT CGAATAGCCC ACAACCTACG AGAAAGGCGG CGGCGGTGCC 2100
   TCAACAACAA ACTTTGTCGA AAATCGCTGC CCCAGTGAAA AGTGGCCTGA AGCCGCCGAC 2160
   CAGTAAGCTG GGAAGTGCCA CGTCTATGTC GAAGCTTTGT ACGCCAAAAG TTTCCTACCG 2220
   TAAAACGGAC GCCCCAATCA TATCTCAACA AGACTCGAAA CGATGCTCAA AGAGCAGTGA 2280
   AGAAGAGTCC GGATACGCTG GATTCAACAG CACGTCGCCA ACGTCATCAT CGACGGAAGG 2340
   TTCCCTAAGC ATGCATTCCA CATCTTCCAA GAGTTCAACG TCAGACGAAA AGTCTCCGTC 2400
   ATCAGACGAT CTTACTCTTA ACGCCTCCAT CGTGACAGCT ATCAGACAGC CGATAGCCGC 2460
   AACACCGGTT TCTCCAAATA TTATCAACAA GCCTGTTGAG GAAAAACCAA CACTGGCAGT 2520
   GAAAGGAGTG AAAAGCACAG CGAAAAAAGA TCCACCTCCA GCTGTTCCGC CACGTGACAC 2580
   CCAGCCAACA ATCGGAGTTG TTAGTCCAAT TATGGCACAT AAGAAGTTGA CAAATGACCC 2640
   CGTGATATCT GAAAAACCAG AACCTGAAAA GCTCCAATCA ATGAGCATCG ACACGACGGA 2700
   CGTTCCACCG CTTCCACCTC TAAAATCAGT TGTTCCACTT AAAATGACTT CAATCCGACA 2760
   ACCACCAACG TACGATGTTC TTCTAAAACA AGGAAAAATC ACATCGCCTG TCAAGTCGTT 2820
   TGGATATGAG CAGTCGTCCG CGTCTGAAGA CTCCATTGTG GCTCATGCGT CGGCTCAGGT 2880
   GACTCCGCCG ACAAAAACTT CTGGTAATCA TTCGCTGGAG AGAAGGATGG GAAAGAATAA 2940
   GACATCAGAA TCCAGCGGCT ACACCTCTGA CGCCGGTGTT GCGATGTGCG CCAAAATGAG 3000
   GGAGAAGCTG AAAGAATACG ATGACATGAC TCGTCGAGCA CAGAACGGCT ATCCTGACAA 3060
   CTTCGAAGAC AGTTCCTCCT TGTCGTCTGG AATATCCGAT AACAACGAGC TCGACGACAT 3120
   ATCCACGGAC GATTTGTCCG GAGTAGACAT GGCAACAGTC GCCTCCAAAC ATAGCGACTA 3180
   TTCCCACTTT GTTCGCCATC CCACGTCTTC TTCCTCAAAG CCCCGAGTCC CCAGTCGGTC 3240
   CTCCACATCA GTCGATTCTC GATCTCGAGC AGAACAGGAG AATGTGTACA AACTTCTGTC 3300
   CCAGTGCCGA ACGAGCCAAC GTGGCGCCGC TGCCACCTCA ACCTTCGGAC AACATTCGCT 3360
   AAGATCCCCG GGATACTCAT CCTATTCTCC ACACTTATCA GTGTCAGCTG ATAAGGACAC 3420
   AATGTCTATG CACTCACAGA CTAGTCGACG ACCTTCTTCA CAAAAACCAA GCTATTCAGG 3480
   CCAATTTCAT TCACTTGATC GTAAATGCCA CCTTCAAGAG TTCACATCCA CCGAGCACAG 3540
   AATGGCGGCT CTCTTGAGCC CGAGACGGGT GCCGAACTCG ATGTCGAAAT ATGATTCTTC 3600
   AGGATCCTAC TCGGCGCGTT CCCGAGGTGG AAGCTCTACT GGTATCTATG GAGAGACGTT 3660
   CCAACTGCAC AGACTATCCG ATGAAAAATC CCCCGCACAT TCTGCCAAAA GTGAGATGGG 3720
   ATCCCAACTA TCACTGGCTA GCACGACAGC ATATGGATCT CTCAATGAGA AGTACGAACA 3780
   TGCTATTCGG GACATGGCAC GTGACTTGGA GTGTTACAAG AACACTGTCG ACTCACTAAC 3840
   CAAGAAACAG GAGAACTATG GAGCATTGTT TGATCTTTTT GAGCAAAAGC TTAGAAAACT 3900
   CACTCAACAC ATTGATCGAT CCAACTTGAA GCCTGAAGAG GCAATACGAT TCAGGCAGGA 3960
   CATTGCTCAT TTGAGGGATA TTAGCAATCA TCTTGCATCC AACTCAGCTC ATGCTAACGA 4020
   AGGCGCTGGT GAGCTTCTTC GTCAACCATC TCTGGAATCA GTTGCATCCC ATCGATCATC 4080
   GATGTCATCG TCGTCGAAAA GCAGCAAGCA GGAGAAGATC AGCTTGAGCT CGTTTGGCAA 4140
   GAACAAGAAG AGCTGGATCC GCTCCTCACT CTCCAAGTTC ACCAAGAAGA AGAACAAGAA 4200
   CTACGACGAA GCACATATGC CATCAATTTC CGGATCTCAA GGAACTCTTG ACAACATTGA 4260
   TGTGATTGAG TTGAAGCAAG AGCTCAAAGA ACGCGATAGT GCACTTTACG AAGTCCGCCT 4320
   TGACAATCTG GATCGTGCCC GCGAAGTTGA TGTTCTGAGG GAGACAGTGA ACAAGTTGAA 4380
   AACCGAGAAC AAGCAATTAA AGAAAGAAGT GGACAAACTC ACCAACGGTC CAGCCACTCG 4440
   TGCTTCTTCC CGCGCCTCAA TTCCAGTTAT CTACGACGAT GAGCATGTCT ATGATGCAGC 4500
   GTGTAGCAGT ACATCAGCTA GTCAATCTTC GAAACGATCC TCTGGCTGCA ACTCAATCAA 4560
   GGTTACTGTA AACGTGGACA TCGCTGGAGA AATCAGTTCG ATCGTTAACC CGGACAAAGA 4620
   GATAATCGTA GGATATCTTG CCATGTCAAC CAGTCAGTCA TGCTGGAAAG ACATTGATGT 4680
   TTCTATTCTA GGACTATTTG AAGTCTACCT ATCCAGAATT GATGTGGAGC ATCAACTTGG 4740
   AATCGATGCT CGTGATTCTA TCCTTGGCTA TCAAATTGGT GAACTTCGAC GCGTCATTGG 4800
   AGACTCCACA ACCATGATAA CCAGCCATCC AACTGACATT CTTACTTCCT CAACTACAAT 4860
   CCGAATGTTC ATGCACGGTG CCGCACAGAG TCGCGTAGAC AGTCTGGTCC TTGATATGCT 4920
   TCTTCCAAAG CAAATGATTC TCCAACTCGT CAAGTCAATT TTGACAGAGA GACGTCTGGT 4980
   GTTAGCTGGA GCAACTGGAA TTGGAAAGAG CAAACTGGCG AAGACCCTGG CTGCTTATGT 5040
   ATCTATTCGA ACAAATCAAT CCGAAGATAG TATTGTTAAT ATCAGCATTC CTGAAAACAA 5100
   TAAAGAAGAA TTGCTTCAAG TGGAACGACG CCTGGAAAAG ATCTTGAGAA GCAAAGAATC 5160
   ATGCATCGTA ATTCTAGATA ATATCCCAAA GAATCGAATT GCATTTGTTG TATCCGTTTT 5220
   TGCAAATGTC CCACTTCAAA ACAACGAAGG TCCATTTGTA GTATGCACAG TCAACCGATA 5280
   TCAAATCCCT GAGCTTCAAA TTCACCACAA TTTCAAAATG TCAGTAATGT CGAATCGTCT 5340
   CGAAGGATTC ATCCTACGTT ACCTCCGACG ACGGGCGGTA GAGGATGAGT ATCGTCTAAC 5400
   TGTACAGATG CCATCAGAGC TCTTCAAAAT CATTGACTTC TTCCCAATAG CTCTTCAGGC 5460
   CGTCAATAAT TTTATTGAGA AAACGAATTC TGTTGATGTG ACAGTTGGTC CAAGAGCATG 5520
   CTTGAACTGT CCTCTAACTG TCGATGGATC CCGTGAATGG TTCATTCGAT TGTGGAATGA 5580
   GAACTTCATT CCATATTTGG AACGTGTTGC TAGAGATGGC AAAAAAACCT TCGGTCGCTG 5640
   CACTTCCTTC GAGGATCCCA CCGACATCGT CTCTAAAAAA TGGCCGTGGT TCGATGGTGA 5700
   AAACCCGGAG AATGTGCTCA AACGTCTTCA ACTCCAAGAC CTCGTCCCGT CACCTGCCAA 5760
   CTCATCCCGA CAACACTTCA ATCCCCTCGA GTCGTTGATC CAATTGCATG CTACCAAGCA 5820
   TCAGACCATC GACAACATTT GAACAGAAGA CTCTAATCTT CTCTCGCCTC TCCCCCGCTT 5880
   TCCTTATCTT CGTACCGGTA CCATGGTATT GATATCTGAG CTCCGCATCG GCCGCTGTCA 5940
   TCAGATCGCC ATCTCGCGCC CGTGCCTCTG ACTTCTAAGT CCAATTACTC TTCAACATCC 6000
   CTACATGCTC TTTCTCCCTG TGCTCCCACC CCCTATTTTT GTTATTATCA AAAAAACTTC 6060
   TTCTTAATTT CTTTGTTTTT TAGCTTCTTT TAAGTCACCT CTAACAATGA AATTGTGTAG 6120
   ATTCAAAAAT AGAATTAATT CGTAATAAAA AGTCGAAAAA AATTGTGCTC CCTCCCCCCA 6180
   TTAATAATAA TTCTATCCCA AAATCTACAC AATGTTCTGT GTACACTTCT TATGTTTTTT 6240
   TTACTTCTGA TAAATTTTTT TTGAAACATC ATAGAAAAAA CCGCACACAA AATACCTTAT 6300
   CATATGTTAC GTTTCAGTTT ATGACCGCAA TTTTTATTTC TTCGCACGTC TGGGCCTCTC 6360
   ATGACGTCAA ATCATGCTCA TCGTGAAAAA GTTTTGGAGT ATTTTTGGAA TTTTTCAATC 6420
   AAGTGAAAGT TTATGAAATT AATTTTCCTG CTTTTGCTTT TTGGGGGTTT CCCCTATTGT 6480
   TTGTCAAGAG TTTCGAGGAC GGCGTTTTTC TTGCTAAAAT CACAAGTATT GATGAGCACG 6540
   ATGCAAGAAA GATCGGAAGA AGGTTTGGGT TTGAGGCTCA GTGGAAGGTG AGTAGAAGTT 6600
   GATAATTTGA AAGTGGAGTA GTGTCTATGG GGTTTTTGCC TTAAATGACA GAATACATTC 6660
   CCAATATACC AAACATAACT GTTTAAAATT AAACATTTTT CTAAATTTTA TATGATTTCT 6720
   TTTAAATTTG CAAAAATTAC TTAAATTTGA ATTCCCGCGC AAATGAGTGA CTTCATTTTC 6780
   TGCATTATTG TGTTTTCCGG CTATATTAAT AGGTATTTGT TTGTGTTTTT CTTTATTTTA 6840
   TGATTCGAAC TCCAATTTGT AAATTTTCGA ACATATTTCC CTAAAGAAAA AATATGATTA 6900
   ATCTGGAAAA ATTGGAAAAT TATTTTTCAA ATAAAAAACA AAGAAAAAAA TGAAGAAAAA 6960
   CCTATTAGTT TGGCCATAAA ACGCAAAAAT GTCGAAAATG ACGTCACTCA TCTGCGCGGG 7020
   AAATCAAGAA TAATTCGGCC TTTTTTATTT TTTTGGAAAA TCGTAAAACA TTTAGAAAAA 7080
   TTTTTTAATA GTTATAGTGG GACTGTATTC TGTCATTTAG GGCAAAAGCC AGAGACGCTA 7140
   CTCCACCGTT GGGGGATCCA CTAGTCGGCC GTACGGGCCC TTTCGTCTCG CGCGTTTCGG 7200
   TGATGACGGT GAAAACCTCT GACACATGCA GCTCCCGGAG ACGGTCACAG CTTGTCTGTA 7260
   AGCGGATGCC GGGAGCAGAC AAGCCCGTCA GGGCGCGTCA GCGGGTGTTG GCGGGTGTCG 7320
   GGGCTGGCTT AACTATGCGG CATCAGAGCA GATTGTACTG AGAGTGCACC ATATGCGGTG 7380
   TGAAATACCG CACAGATGCG TAAGGAGAAA ATACCGCATC AGGCGGCCTT AAGGGCCTCG 7440
   TGATACGCCT ATTTTTATAG GTTAATGTCA TGATAATAAT GGTTTCTTAG ACGTCAGGTG 7500
   GCACTTTTCG GGGAAATGTG CGCGGAACCC CTATTTGTTT ATTTTTCTAA ATACATTCAA 7560
   ATATGTATCC GCTCATGAGA CAATAACCCT GATAAATGCT TCAATAATAT TGAAAAAGGA 7620
   AGAGTATGAG TATTCAACAT TTCCGTGTCG CCCTTATTCC CTTTTTTGCG GCATTTTGCC 7680
   TTCCTGTTTT TGCTCACCCA GAAACGCTGG TGAAAGTAAA AGATGCTGAA GATCAGTTGG 7740
   GTGCACGAGT GGGTTACATC GAACTGGATC TCAACAGCGG TAAGATCCTT GAGAGTTTTC 7800
   GCCCCGAAGA ACGTTTTCCA ATGATGAGCA CTTTTAAAGT TCTGCTATGT GGCGCGGTAT 7860
   TATCCCGTAT TGACGCCGGG CAAGAGCAAC TCGGTCGCCG CATACACTAT TCTCAGAATG 7920
   ACTTGGTTGA GTACTCACCA GTCACAGAAA AGCATCTTAC GGATGGCATG ACAGTAAGAG 7980
   AATTATGCAG TGCTGCCATA ACCATGAGTG ATAACACTGC GGCCAACTTA CTTCTGACAA 8040
   CGATCGGAGG ACCGAAGGAG CTAACCGCTT TTTTGCACAA CATGGGGGAT CATGTAACTC 8100
   GCCTTGATCG TTGGGAACCG GAGCTGAATG AAGCCATACC AAACGACGAG CGTGACACCA 8160
   CGATGCCTGT AGCAATGGCA ACAACGTTGC GCAAACTATT AACTGGCGAA CTACTTACTC 8220
   TAGCTTCCCG GCAACAATTA ATAGACTGGA TGGAGGCGGA TAAAGTTGCA GGACCACTTC 8280
   TGCGCTCGGC CCTTCCGGCT GGCTGGTTTA TTGCTGATAA ATCTGGAGCC GGTGAGCGTG 8340
   GGTCTCGCGG TATCATTGCA GCACTGGGGC CAGATGGTAA GCCCTCCCGT ATCGTAGTTA    8400
   TCTACACGAC GGGGAGTCAG GCAACTATGG ATGAACGAAA TAGACAGATC GCTGAGATAG    8460
   GTGCCTCACT GATTAAGCAT TGGTAACTGT CAGACCAAGT TTACTCATAT ATACTTTAGA    8520
   TTGATTTAAA ACTTCATTTT TAATTTAAAA GGATCTAGGT GAAGATCCTT TTTGATAATC    8580
   TCATGACCAA AATCCCTTAA CGTGAGTTTT CGTTCCACTG AGCGTCAGAC CCCGTAGAAA    8640
   AGATCAAAGG ATCTTCTTGA GATCCTTTTT TTCTGCGCGT AATCTGCTGC TTGCAAACAA    8700
   AAAAACCACC GCTACCAGCG GTGGTTTGTT TGCCGGATCA AGAGCTACCA ACTCTTTTTC    8760
   CGAAGGTAAC TGGCTTCAGC AGAGCGCAGA TACCAAATAC TGTCCTTCTA GTGTAGCCGT    8820
   AGTTAGGCCA CCACTTCAAG AACTCTGTAG CACCGCCTAC ATACCTCGCT CTGCTAATCC    8880
   TGTTACCAGT GGCTGCTGCC AGTGGCGATA AGTCGTGTCT TACCGGGTTG GACTCAAGAC    8940
   GATAGTTACC GGATAAGGCG CAGCGGTCGG GCTGAACGGG GGGTTCGTGC ACACAGCCCA    9000
   GCTTGGAGCG AACGACCTAC ACCGAACTGA GATACCTACA GCGTGAGCAT TGAGAAAGCG    9060
   CCACGCTTCC CGAAGGGAGA AAGGCGGACA GGTATCCGGT AAGCGGCAGG GTCGGAACAG    9120
   GAGAGCGCAC GAGGGAGCTT CCAGGGGGAA ACGCCTGGTA TCTTTATAGT CCTGTCGGGT    9180
   TTCGCCACCT CTGACTTGAG CGTCGATTTT TGTGATGCTC GTCAGGGGGG CGGAGCCTAT    9240
   GGAAAAACGC CAGCAACGCG GCCTTTTTAC GGTTCCTGGC CTTTTGCTGG CCTTTTGCTC    9300
   ACATGTTCTT TCCTGCGTTA TCCCCTGATT CTGTGGATAA CCGTATTACC GCCTTTGAGT    9360
   GAGCTGATAC CGCTCGCCGC AGCCGAACGA CCGAGCGCAG CGAGTCAGTG AGCGAGGAAG    9420
   CGGAAGAGCG CCCAATACGC AAACCGCCTC TCCCCGCGCG TTGGCCGATT CATTAATGCA    9480
   GCTGGCACGA CAGGTTTCCC GACTGGAAAG CGGGCAGTGA GCGCAACGCA ATTAATGTGA    9540
   GTTAGCTCAC TCATTAGGCA CCCCAGGCTT TACACTTTAT GCTTCCGGCT CGTATGTTGT    9600
   GTGGAATTGT GAGCGGATAA CAATTTCACA CAGGAAACAG CT                       9642
(2) INFORMATION FOR SEQ ID NO: 31:
   (i) SEQUENCE CHARACTERISTICS:
      (A) LENGTH: 110 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 31:
   Met Thr Thr Ser Asn Val Glu Leu Ile Pro Ile Tyr Thr Asp Trp Ala
   1               5                   10                  15
   Asn Arg His Leu Ser Lys Gly Ser Leu Ser Lys Ser Ile Arg Asp Ile
               20                  25                  30
   Ser Asn Asp Phe Arg Asp Tyr Arg Leu Val Ser Gln Leu Ile Asn Val
           35                  40                  45
   Ile Val Pro Ile Asn Glu Phe Ser Pro Ala Phe Thr Lys Arg Leu Ala
       50                  55                  60
   Lys Ile Thr Ser Asn Leu Asp Gly Leu Glu Thr Cys Leu Asp Tyr Leu
   65                  70                  75                  80
   Lys Asn Leu Gly Leu Asp Cys Ser Lys Leu Thr Lys Thr Asp Ile Asp
                   85                  90                  95
   Ser Gly Asn Leu Gly Ala Val Leu Gln Leu Leu Phe Leu Leu
               100                 105                 110
(2) INFORMATION FOR SEQ ID NO: 32:
   (i) SEQUENCE CHARACTERISTICS:
      (A) LENGTH: 20 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 32:
   Lys Gln Lys Leu Arg Gln Leu Lys Lys Asp Gln Lys Lys Leu Glu Gln
   1               5                   10                  15
   Leu Pro Thr Ser
               20
(2) INFORMATION FOR SEQ ID NO: 33:
   (i) SEQUENCE CHARACTERISTICS:
      (A) LENGTH: 9 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 33:
   Asp Pro Pro Pro Ala Val Pro Pro Arg
   1               5
(2) INFORMATION FOR SEQ ID NO: 34:
   (i) SEQUENCE CHARACTERISTICS:
      (A) LENGTH: 9 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 34:
   Asp Val Pro Pro Leu Pro Pro Leu Lys
   1               5
(2) INFORMATION FOR SEQ ID NO: 35:
   (i) SEQUENCE CHARACTERISTICS:
      (A) LENGTH: 5 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 35:
   Lys Lys Lys Asn Lys
   1               5
(2) INFORMATION FOR SEQ ID NO: 36:
   (i) SEQUENCE CHARACTERISTICS:
      (A) LENGTH: 20 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 36:
   Lys Thr Glu Asn Lys Gln Leu Lys Lys Glu Val Asp Lys Leu Thr Asn
   1               5                   10                  15
   Gly Pro Ala Thr
               20
(2) INFORMATION FOR SEQ ID NO: 37:
   (i) SEQUENCE CHARACTERISTICS:
      (A) LENGTH: 8 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION:'SEQ ID NO: 37:
   Gly Ala Thr Gly Ile Gly Lys Ser
   1               5
(2) INFORMATION FOR SEQ ID NO: 38:
   (i) SEQUENCE CHARACTERISTICS:
      (A) LENGTH: 58 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 38:
   Met Ser Glu Glu Pro Thr Pro Val Ser Gly Asn Asp Lys Gln Leu Leu
   1               5                   10                  15
   Asn Lys Ala Trp Glu Ile Thr Gln Lys Lys Thr Phe Thr Ala Trp Cys
               20                  25                  30
   Asn Ser His Leu Arg Lys Leu Gly Ser Ser Ile Glu Gln Ile Asp Thr
           35                  40                  45
   Asp Phe Thr Asp Gly Ile Lys Leu Ala Gln
       50                  55
(2) INFORMATION FOR SEQ ID NO: 39:
   (i) SEQUENCE CHARACTERISTICS:
      (A) LENGTH: 44 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 39:
   Met Thr Thr Ser Asn Val Glu Leu Ile Pro Ile Tyr Thr Asp Trp Ala
   1               5                   10                  15
   Asn Arg His Leu Ser Lys Gly Ser Leu Ser Lys Ser Ile Arg Asp Ile
               20                  25                  30
   Ser Asn Asp Phe Arg Asp Tyr Arg Leu Val Ser Gln
           35                  40
(2) INFORMATION FOR SEQ ID NO: 40:
   (i) SEQUENCE CHARACTERISTICS:
      (A) LENGTH: 51 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 40:
   Phe Glu Arg Ser Arg Ile Lys Ala Leu Ala Asp Glu Arg Glu Val Val
   1               5                   10                  15
   Gln Lys Lys Thr Phe Thr Lys Trp Val Asn Ser His Leu Ala Arg Val
               20                  25                  30
   Ser Cys Arg Ile Thr Asp Leu Tyr Lys Asp Leu Arg Asp Gly Arg Met
           35                  40                  45
   Leu Ile Lys
       50
(2) INFORMATION FOR SEQ ID NO: 41:
   (i) SEQUENCE CHARACTERISTICS:
      (A) LENGTH: 59 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 41:
   Leu Leu Glu Val Ile Ser Asn Asp Pro Val Phe Lys Val Asn Lys Thr
   1               5                   10                  15
   Pro Lys Leu Arg Arg Ile His Asn Ile Gln Asn Val Gly Leu Cys Leu
               20                  25                  30
   Lys His Ile Glu Ser His Gly Val Lys Leu Val Gly Ile Gly Ala Glu
           35                  40                  45
   Glu Leu Val Asp Lys Asn Leu Lys Met Thr Leu
       50                  55
(2) INFORMATION FOR SEQ ID NO: 42:
   (i) SEQUENCE CHARACTERISTICS:
      (A) LENGTH: 60 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 42:
   Leu Ile Asn Val Ile Val Pro Ile Asn Glu Phe Ser Pro Ala Phe Thr
   1               5                   10                  15
   Lys Arg Leu Ala Lys Ile Thr Ser Asn Leu Asp Gly Leu Glu Thr Cys
               20                  25                  30
   Leu Asp Tyr Leu Lys Asn Leu Gly Leu Asp Cys Ser Lys Leu Thr Lys
           35                  40                  45
   Thr Asp Ile Asp Ser Gly Asn Leu Gly Ala Val Leu
       50                  55                  60
(2) INFORMATION FOR SEQ ID NO: 43:
   (i) SEQUENCE CHARACTERISTICS:
      (A) LENGTH: 57 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 43:
   Leu Leu Glu Val Leu Ser Gly Glu Met Leu Pro Lys Pro Thr Lys Gly
   1               5                   10                  15
   Lys Met Arg Ile His Cys Leu Glu Asn Val Asp Lys Ala Leu Gln Phe
               20                  25                  30
   Leu Lys Glu Gln Arg Val His Leu Glu Asn Met Gly Ser His Asp Ile
           35                  40                  45
   Val Asp Gly Asn His Arg Leu Val Leu
       50                  55
(2) INFORMATION FOR SEQ ID NO: 44:
   (i) SEQUENCE CHARACTERISTICS:
      (A) LENGTH: 42 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 44:
   Gly Met Ile Trp Thr Ile Ile Leu Arg Phe Ala Ile Gln Asp Ile Ser
   1               5                   10                  15
   Ile Glu Glu Leu Ser Ala Lys Glu Ala Leu Leu Leu Trp Cys Gln Arg
               20                  25                  30
   Lys Thr Glu Gly Tyr Asp Arg Val Lys Val
           35                  40
(2) INFORMATION FOR SEQ ID NO: 45:
   (i) SEQUENCE CHARACTERISTICS:
      (A) LENGTH: 46 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 45:
   Gln Leu Leu Phe Leu Leu Ser Thr Tyr Lys Gln Lys Leu Arg Gln Leu
   1               5                   10                  15
   Lys Lys Asp Gln Lys Lys Leu Glu Gln Leu Pro Thr Ser Ile Met Pro
               20                  25                  30
   Pro Ala Val Ser Lys Leu Pro Ser Pro Arg Val Ala Thr Ser
           35                  40                  45
(2) INFORMATION FOR SEQ ID NO: 46:
   (i) SEQUENCE CHARACTERISTICS:
      (A) LENGTH: 48 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO: 46:
   Gly Leu Ile Trp Thr Ile Ile Leu Arg Phe Gln Ile Gln Asp Ile Val
   1               5                   10                  15
   Val Gln Thr Gln Glu Gly Arg Glu Thr Arg Ser Ala Lys Asp Ala Leu
               20                  25                  30
   Leu Gln Phe Leu Lys Glu Gln Arg Val His Leu Glu Asn Met Gly Ser
           35                  40                  45
(2) INFORMATION FOR SEQ ID NO: 47:
   (i) SEQUENCE CHARACTERISTICS:
      (A) LENGTH: 100 base pairs
      (B) TYPE: nucleic acid
      (C) STRANDEDNESS: single
      (D) TOPOLOGY: linear
   (ii) MOLECULE TYPE: cosmid DNA
   (xi) SEQUENCE DESCRIPTION: SEQ ID NO: 47:
   GATCAGAAGA AATTGGAGCA ACTACCCACA TCCATTATGC CACCCGCGGT TTCTAAGTGA 60
   GTTTAATTTT GAGTTTACGA CTACAAAAAT GTGTTCTTTA 100
(2) INFORMATION FOR SEQ ID NO: 48:
   (i) SEQUENCE CHARACTERISTICS:
      (A) LENGTH: 91 base pairs
      (B) TYPE: nucleic acid
      (C) STRANDEDNESS: single
      (D) TOPOLOGY: linear
   (ii) MOLECULE TYPE: cosmid DNA
   (xi) SEQUENCE DESCRIPTION: SEQ ID NO: 48:
   CCGCCTTCTG ACTTCGTGAC GACAGTCTCG ACACGTGGGG TTGCAGGTAG GAGTGGATGA 60
   GTCGAAACTG ATAAGATAGT CATTTGAGAT C 91

## Claims

1. A cDNA encoding an UNC-53 protein of C.elegans, said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3.

2. A cDNA as claimed in claim 1 comprising at least from nucleotide position 431 to the 3' end of the sequence shown in SEQ ID No 1.

3. A cDNA as claimed in Claim 1 comprising at least from nucleotide position 64 to nucleotide position 4647 of the sequence as shown in SEQ ID No 1.

4. A cDNA as claimed in claim 3 comprising at least from nucleotide position 64 to the 3' end of the sequence shown in SEQ ID No 1.

5. A cDNA as claimed in Claims 1 to 4 comprising the nucleotide sequence shown in SEQ ID No 1.

6. A cDNA encoding an UNC-53 protein of C.elegans, said UNC-53 protein being selected from the group consisting of a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4.

7. A cDNA as claimed in claim 6 comprising at least from nucleotide position 431 to the 3' end of the sequence shown in SEQ ID No 2.

8. A cDNA as claimed in Claim 6 comprising at least from nucleotide position 64 to nucleotide position 4812 of the sequence shown in SEQ ID No 2.

9. A cDNA as claimed in claim 8 comprising at least from nucleotide position 64 to the 3' end of sequence shown in SEQ ID No 2.

10. A cDNA as claimed in any of claims 6 to 9 comprising the nucleotide sequence shown in SEQ ID No 2.

11. A DNA expression vector which comprises a cDNA as claimed in any one of Claims 1 to 10.

12. A host cell transformed or transfected with the vector of Claim 11.

13. A host cell as claimed in Claim 12 which is a bacterial, an animal, a plant or an insect cell.

14. A transgenic cell comprising a transgene capable of expressing an UNC-53 protein of C. elegans; said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3; or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4.

15. A transgenic cell as claimed in Claim 14 which cell is a C. elegans cell, an N4 neuroblastoma cell or an MCF-7 breast carcinoma cell.

16. A transgenic non-human organism comprising a transgene capable of expressing UNC-53 protein of C. elegans said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3 or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4.

17. A transgenic organism as claimed in Claim 16 wherein said organism is C. elegans.

18. A transgenic organism as claimed in Claim 16 wherein said organism is an insect, a non-human mammal or a plant.

19. An UNC-53 protein encoded by the cDNA of Claim 1 and which protein has the amino acid sequence shown in SEQ ID No 3 from amino acid position 135 to amino acid position 1528.

20. An UNC-53 protein encoded by the cDNA sequence of any of Claims 2 to 5 and which protein has the amino acid sequence shown in SEQ ID No 3.

21. An UNC-53 protein encoded by the cDNA sequence of Claim 6 and which protein has the amino acid sequence shown in SEQ ID NO 4 from amino acid position 135 to amino acid position 1583.

22. An UNC-53 protein encoded by the cDNA sequence according to any of Claims 7 to 10 and which protein has the amino acid sequence shown in SEQ ID No 4.

23. An UNC-53 protein of C. elegans, said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3 or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4, for use as a medicament to promote neuronal regeneration, revascularisation or wound healing, or for treatment of chronic neuro-degenerative diseases or acute traumatic injuries.

24. An UNC-53 protein as claimed in any one of Claims 19 to 22 for use as a medicament to promote neuronal regeneration, revascularisation or wound healing, or for treatment of chronic neuro-degenerative diseases or acute traumatic injuries.

25. Use of an UNC-53 protein of C. elegans, said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3 or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO 4 in the manufacture of a medicament for promoting neuronal regeneration, revascularisation or wound healing, or for treatment of chronic neuro-degenerative diseases or acute traumatic injuries.

26. Use of an UNC-53 protein as claimed in any one of Claims 19 to 22 in the manufacture of a medicament for promoting neuronal regeneration, revascularisation or wound healing, or for treatment of chronic neuro-degenerative or acute traumatic injuries.

27. A pharmaceutical composition comprising an UNC-53 protein of C. elegans, said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3 or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4 and an acceptable carrier, diluent or excipient therefor.

28. A pharmaceutical composition as claimed in Claim 27 which comprises an UNC-53 protein as claimed in any one of Claims 19 to 22.

29. A nucleic acid sequence encoding an UNC-53 protein of C. elegans said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising, the sequence from position 366 to 1528 of SEQ. ID. NO. 3, and a protein comprising the sequence from position 421 to 1528 of SEQ. ID. NO. 3 or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4, for use as a medicament to promote neuronal regeneration, vascularisation or wound healing, or for treatment of chronic neuro-degenerative diseases or acute traumatic injuries.

30. A nucleic acid sequence for use as claimed in Claim 29 wherein said sequence is a cDNA sequence as claimed in any one of Claims 1 to 10.

31. Use of a nucleic acid sequence encoding and UNC-53 protein of C. elegans said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3 or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4 in the manufacture of a medicament to promote neuronal regeneration, vascularization or wound healing, or for treatment of chronic neuro-degenerative diseases or acute traumatic injuries.

32. Use of a nucleic acid sequence as claimed in Claim 31 wherein said sequence is a cDNA sequence as claimed in any one of Claims 1 to 10.

33. A pharmaceutical composition comprising a nucleic acid encoding an UNC-53 protein of C. elegans said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3 or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4 and an acceptable carrier, diluent, or excipient therefor.

34. A pharmaceutical composition as claimed in Claim 33 wherein said nucleic acid sequence is a cDNA sequence as claimed in any one of Claims 1 to 10.

35. A method of determining whether a compound is an inhibitor or an enhancer of the regulation of cell shape or motility or the direction of cell migration, which method comprises contacting said compound with a transgenic cell as claimed in Claims 14 or 15 and screening for a phenotypic change in said cell.

36. A method as claimed in Claim 35 wherein said phenotypic change to be screened is a change in cell shape or a change in cell motility.

37. A method as claimed in claim 35 wherein said phenotypic change to be screened is a change in filipodia outgrowth, ruffling behaviour, cell adhesion or the length of neurite growth.

38. A method as claimed in any of Claims 35 to 37 wherein said transgenic cell is an N4 neuroblastoma cell and the phenotypic change to be screened is the length of neurite growth.

39. A method as claimed in any of Claims 35 to 37 wherein said transgenic cell is an MCF-7 breast carcinoma cell and the phenotypic change to be screened is the extent of phagokinesis.

40. A method of determining whether a compound is an inhibitor or an enhancer of the regulation of cell shape or motility or of the direction of cell migration which method comprises administering said compound to a transgenic organism as claimed in any one of Claims 16 to 19, , and screening for a phenotypic change in said organism.

41. A method as claimed in Claim 40 wherein said compound is an inhibitor or enhancer of a protein of the signal transduction pathway of said transgenic organisms, said protein being an UNC-53 protein said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3. or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4 or said compound is an inhibitor or an enhancer of a parallel or redundant signal transduction pathway in said cell.

42. A kit for determining whether a compound is an enhancer or an inhibitor of the activity of UNC-53 protein, which kit comprises at least, a transgenic organism as claimed in any of claims 16 to 18 and a wild type organism of C.elegans.

43. A process for producing an UNC-53 protein of C.elegans, said UNC-53 protein being selected from the group consisting of a protein comprising the sequence shown in SEQ. ID. No. 3, a protein comprising the sequence from position 135 to 1528 of SEQ. ID. NO. 3, a protein comprising the sequence from position 165 to 1528 of SEQ. ID. NO. 3, a protein comprising the sequence from position 309 to 1528 of SEQ. ID. NO. 3, a protein comprising the sequence from position 366 to 1528 of SEQ. ID. NO. 3, and a protein comprising the sequence from position 421 to 1528 of SEQ. ID. NO. 3 or a protein comprising the sequence shown in SEQ. ID. NO. 4, a protein comprising the sequence from position 135 to 1583 of SEQ. ID. NO. 4, a protein comprising the sequence from position 165 to 1583 of SEQ. ID. NO. 4, a protein comprising the sequence the sequence from position 309 to 1583 of SEQ. ID. NO. 4, a protein comprising the sequence from position 366 to 1583 of SEQ. ID. NO. 4, and a protein comprising the sequence from protein 421 to 1583 of SEQ. ID. NO. 4, which process comprises culturing the transfected or transformed cells of Claim 12 or Claim 13 and recovering the expressed UNC-53 protein.

44. A hybridoma cell line deposited under the LMBP Accession No. 1383CB.

45. Monoclonal antibody 16-48-2 obtainable from the hybridoma deposited under the LMBP Accession No. 1383CB.

46. Plasmid pTB54 deposited under the LMBP Accession No. 3296.

47. Plasmid pBT112 deposited under the Accession No. 3295.

48. Plasmid pTB72 deposited under the LMBP Accession No. 3486.

49. Transgenic cell-line of C.elegans designated TB4EX25 and deposited under the LMBP Accession No. 1384CB.

50. Transgenic cell-line of C.elegans designated TBAIn76 and deposited under the Accession NO. 1385CB.

51. A transgenic cell-line of MCF-7 breast carcinoma cells deposited under the LMBP Accession No. 1550CB.

52. A transgenic cell-line of N4 neuroblastoma cells deposited under LMBP Accession No. 1549CB.

## Patentansprüche

1. cDNA kodierend ein UNC-53 Protein aus C. elegans, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3.

2. cDNA gemäß Anspruch 1 umfassend wenigstens von Nukleotidposition 431 bis zum 3'-Ende der in SEQ. ID. NO. 1 gezeigten Sequenz.

3. cDNA gemäß Anspruch 1 umfassend wenigstens von Nukleotidposition 64 bis Nukleotidposition 4647 der Sequenz wie in SEQ. ID. NO. 1 gezeigt.

4. cDNA gemäß Anspruch 3 umfassend wenigstens von Nukleotidposition 64 bis zum 3'-Ende der in SEQ. ID. NO. 1 gezeigten Sequenz.

5. cDNA gemäß einem der Ansprüche 1 bis 4 umfassend die in SEQ. ID. NO. 1 gezeigte Nukleotidsequenz.

6. cDNA kodierend ein UNC-53 Protein aus C. elegans, wobei das UNC-53 Protein ausgewählt wird aus der Gruppe bestehend aus einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4.

7. cDNA gemäß Anspruch 6 umfassend wenigstens von Nukleotidposition 431 bis zum 3'-Ende der in SEQ. ID. NO. 2 gezeigten Sequenz.

8. cDNA gemäß Anspruch 6 umfassend wenigstens von Nukleotidposition 64 bis Nukleotidposition 4812 der in SEQ. ID. NO. 2 gezeigten Sequenz.

9. cDNA gemäß Anspruch 8 umfassend wenigstens von Nukleotidposition 64 bis zum 3'-Ende der in SEQ. ID. NO. 2 gezeigten Sequenz.

10. cDNA gemäß einem der Ansprüche 6 bis 9 umfassend die in SEQ. ID. NO. 2 gezeigte Nukleotidsequenz.

11. DNA-Expressionsvektor, welcher eine cDNA gemäß einem der Ansprüche 1 bis 10 umfaßt.

12. Wirtszelle transformiert oder transfiziert mit dem Vektor gemäß Anspruch 11.

13. Wirtszelle gemäß Anspruch 12, welche eine bakterielle, tierische, pflanzliche oder Insektenzelle ist.

14. Transgene Zelle umfassend ein Transgen, welches ein UNC-53 Protein aus C. elegans exprimieren kann, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4.

15. Transgene Zelle gemäß Anspruch 14, wobei die Zelle eine C. elegans-Zelle, eine N4 Neuroblastomzelle oder eine MCF-7 Brustkrebszelle ist.

16. Transgener nichtmenschlicher Organismus, umfassend ein Transgen, welches ein UNC-53 Protein aus C. elegans exprimieren kann, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4.

17. Transgener Organismus gemäß Anspruch 16, wobei der Organismus C. elegans ist.

18. Transgener Organismus gemäß Anspruch 16, wobei der Organismus ein Insekt, ein nichtmenschliches Säugetier oder eine Pflanze ist.

19. UNC-53 Protein kodiert durch die cDNA gemäß Anspruch 1 und wobei das Protein die in SEQ. ID. NO. 3 gezeigte Aminosäuresequenz von Aminosäureposition 135 bis Aminosäureposition 1528 hat.

20. UNC-53 Protein kodiert durch die cDNA-Sequenz gemäß einem der Ansprüche 2 bis 5 und wobei das Protein die in SEQ. ID. NO. 3 gezeigte Aminosäuresequenz hat.

21. UNC-53 Protein kodiert durch die cDNA-Sequenz gemäß Anspruch 6 und wobei das Protein die in SEQ. ID. NO. 4 gezeigte Aminosäuresequenz von Aminosäureposition 135 bis Aminosäureposition 1583 hat.

22. UNC-53 Protein kodiert durch die cDNA-Sequenz gemäß einem der Ansprüche 7 bis 10 und wobei das Protein die in SEQ. ID. NO. 4 gezeigte Aminosäuresequenz hat.

23. UNC-53 Protein aus C. elegans, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4, zur Verwendung als ein Medikament zur Unterstützung neuronaler Regeneration, Revaskularisation oder Wundheilung oder zur Behandlung von chronischen neurodegenerativen Krankheiten oder akuten traumatischen Verletzungen.

24. UNC-53 Protein gemäß einem der Ansprüche 19 bis 22 zur Verwendung als ein Medikament zur Unterstützung neuronaler Regeneration, Revaskularisation oder Wundheilung oder zur Behandlung von chronischen neurodegenerativen Krankheiten oder akuten traumatischen Verletzungen.

25. Verwendung eines UNC-53 Proteins aus C. elegans, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4, bei der Herstellung eines Medikamentes zur Unterstützung neuronaler Regeneration, Revaskularisation oder Wundheilung oder zur Behandlung von chronischen neurodegenerativen Krankheiten oder akuten traumatischen Verletzungen.

26. Verwendung eines UNC-53 Proteins gemäß einem der Ansprüche 19 bis 22 bei der Herstellung eines Medikamentes zur Unterstützung neuronaler Regeneration, Revaskularisation oder Wundheilung oder zur Behandlung von chronischen neurodegenerativen oder akuten traumatischen Verletzungen.

27. Pharmazeutische Zusammensetzung umfassend ein UNC-53 Protein aus C. elegans, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4, und einen akzeptablen Träger, Verdünner oder Hilfsstoff dafür.

28. Pharmazeutische Zusammensetzung gemäß Anspruch 27, welche ein UNC-53 Protein gemäß einem der Ansprüche 19 bis 22 umfaßt.

29. Nukleinsäuresequenz kodierend ein UNC-53 Protein aus C. elegans, wobei das UNC-53 Protein ausgewählt wird aus der Gruppe bestehend aus einem Protein umfassend die Sequenz gezeigt in SEQ. ID NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4, zur Verwendung als ein Medikament zur Unterstützung neuronaler Regeneration, Vaskularisation oder Wundheilung oder zur Behandlung von chronischen neurodegenerativen Krankheiten oder akuten traumatischen Verletzungen.

30. Nukleinsäuresequenz zur Verwendung gemäß Anspruch 29, wobei die Sequenz eine cDNA-Sequenz gemäß einem der Ansprüche 1 bis 10 ist.

31. Verwendung einer Nukleinsäuresequenz kodierend ein UNC-53 Protein aus C. elegans, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4, bei der Herstellung eines Medikamentes zur Unterstützung neuronaler Regeneration, Vaskularisation oder Wundheilung oder zur Behandlung von chronischen neurodegenerativen Krankheiten oder akuten traumatischen Verletzungen.

32. Verwendung einer Nukleinsäuresequenz gemäß Anspruch 31 wobei die Sequenz eine cDNA-Sequenz gemäß einem der Ansprüche 1 bis 10 ist.

33. Pharmazeutische Zusammensetzung umfassend eine Nukleinsäure kodierend ein UNC-53 Protein aus C. elegans, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4, und einen akzeptablen Träger, Verdünner oder Hilfsstoff dafür.

34. Pharmazeutische Zusammensetzung gemäß Anspruch 33, wobei die Nukleinsäuresequenz eine cDNA-Sequenz gemäß einem der Ansprüche 1 bis 10 ist.

35. Verfahren zur Bestimmung, ob eine Verbindung ein Inhibitor oder ein Aktivator der Regulation der Zellform oder -beweglichkeit oder der Richtung der Zellmigration ist, wobei das Verfahren Inkontaktbringen der Verbindung mit einer transgenen Zelle gemäß Anspruch 14 oder 15 und Screenen nach einer phänotypischen Veränderung in der Zelle umfasst.

36. Verfahren gemäß Anspruch 35, wobei die phänotypische Veränderung, nach der gescreent werden soll, eine Veränderung der Zellform oder eine Veränderung der Zellbeweglichkeit ist.

37. Verfahren gemäß Anspruch 35, wobei die phänotypische Veränderung, nach der gescreent werden soll, eine Veränderung des Auswachsens der Filipodien, des Kräuselungsverhaltens, der Zelladhäsion oder der Länge des Neuritenwachstums ist.

38. Verfahren gemäß einem der Ansprüche 35 bis 37, wobei die transgene Zelle eine N4 Neuroblastomzelle ist und die phänotypische Veränderung, nach der gescreent werden soll, die Länge des Neuritenwachstums ist.

39. Verfahren gemäß einem der Ansprüche 35 bis 37, wobei die transgene Zelle eine MCF-7 Brustkrebszelle und die phänotypische Veränderung, nach der gescreent werden soll, das Ausmaß der Phagokinese ist.

40. Verfahren zur Bestimmung, ob eine Verbindung ein Inhibitor oder ein Aktivator der Regulation der Zellform oder -beweglichkeit oder der Richtung der Zellmigration ist, wobei das Verfahren Verabreichen der Verbindung an einen transgenen Organismus gemäß einem der Ansprüche 16 bis 19 und Screenen nach einer phänotypischen Veränderung in dem Organismus umfasst.

41. Verfahren gemäß Anspruch 40, wobei die Verbindung ein Inhibitor oder ein Aktivator eines Proteins des Signaltransduktionsweges des transgenen Organismus' ist, wobei das Protein ein UNC-53 Protein ist, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4, oder die Verbindung ein Inhibitor oder ein Aktivator eines parallelen oder redundanten Signaltransduktionsweges in der Zelle ist.

42. Kit zur Bestimmung, ob eine Verbindung ein Aktivator oder ein Inhibitor der Aktivität eines UNC-53 Proteins ist, wobei der Kit wenigstens einen transgenen Organismus gemäß einem der Ansprüche 16 bis 18 und einen Wildtyporganismus von C. elegans umfaßt.

43. Verfahren zur Herstellung eines UNC-53 Proteins aus C. elegans, wobei das UNC-53 Protein ausgewählt wird aus der Gruppe bestehend aus einem Protein umfassend die Sequenz gezeigt in SEQ. ID. NO. 3, einem Protein umfassend die Sequenz von Position 135 bis 1528 aus SEQ. ID. NO. 3, einem Protein umfassend die Sequenz von Position 165 bis 1528 aus SEQ. ID. NO. 3, einem Protein umfassend die Sequenz von Position 309 bis 1528 aus SEQ. ID. NO. 3, einem Protein umfassend die Sequenz von Position 366 bis 1528 aus SEQ. ID. NO. 3 und einem Protein umfassend die Sequenz von Position 421 bis 1528 aus SEQ. ID. NO. 3 oder einem Protein umfassend die Sequenz gezeigt in SEQ. ID. NO. 4, einem Protein umfassend die Sequenz von Position 135 bis 1583 aus SEQ. ID. NO. 4, einem Protein umfassend die Sequenz von Position 165 bis 1583 aus SEQ. ID. NO. 4, einem Protein umfassend die Sequenz von Position 309 bis 1583 aus SEQ. ID. NO. 4, einem Protein umfassend die Sequenz von Position 366 bis 1583 aus SEQ. ID. NO. 4 und einem Protein umfassend die Sequenz von Position 421 bis 1583 aus SEQ. ID. NO. 4, wobei das Verfahren Kultivieren der transfizierten oder transformierten Zellen gemäß Anspruch 12 oder Anspruch 13 und Gewinnen des exprimierten UNC-53 Proteins umfaßt.

44. Hybridomzelllinie hinterlegt unter der LMBP-Zugriffsnr. 1383CB.

45. Monoklonaler Antikörper 16-48-2 erhältlich aus dem Hybridom hinterlegt unter der LMBP-Zugriffsnr. 1383CB.

46. Plasmid pTB54 hinterlegt unter der LMBP-Zugriffsnr. 3296.

47. Plasmid pTB112 hinterlegt unter der LMBP-Zugriffsnr. 3295.

48. Plasmid pTB72 hinterlegt unter der LMBP-Zugriffsnr. 3486.

49. Transgene Zelllinie aus C. elegans bezeichnet als TB4EX25 und hinterlegt unter der LMBP-Zugriffsnr. 1384CB.

50. Transgene Zelllinie aus C. elegans bezeichnet als TBAIn76 und hinterlegt unter der LMBP-Zugriffsnr. 1385CB.

51. Transgene Zelllinie aus MCF-7 Brustkrebszellen hinterlegt unter der LMBP-Zugriffsnr. 1550CB.

52. Transgene Zelllinie aus N4 Neuroblastomzellen hinterlegt unter der LMBP-Zugriffsnr. 1549CB.

## Revendications

1. ADNc codant pour une protéine UNC-53 de C. elegans, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3.

2. ADNc suivant la revendication 1, comprenant au moins la position de nucléotide 431 jusqu'à l'extrémité 3' de la séquence représentée dans la SEQ ID N° 1.

3. ADNc suivant la revendication 1, comprenant au moins la position de nucléotide 64 jusqu'à la position de nucléotide 4647 de la séquence représentée dans la SEQ ID N° 1.

4. ADNc suivant la revendication 3, comprenant au moins la position de nucléotide 64 jusqu'à l'extrémité 3' de la séquence représentée dans la SEQ ID N° 1.

5. ADNc suivant les revendications 1 à 4, comprenant la séquence de nucléotides représentée dans la SEQ ID N° 1.

6. ADNc codant pour une protéine UNC-53 de C. elegans, ladite protéine UNC-53 étant choisie dans le groupe consistant en une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4.

7. ADNc suivant la revendication 6, comprenant au moins la position de nucléotide 431 jusqu'à l'extrémité 3' de la séquence représentée dans la SEQ ID N° 2.

8. ADNc suivant la revendication 6, comprenant au moins la position de nucléotide 64 jusqu'à la position de nucléotide 4812 de la séquence représentée dans la SEQ ID N° 2.

9. ADNc suivant la revendication 8, comprenant au moins la position de nucléotide 64 jusqu'à l'extrémité 3' de la séquence représentée dans la SEQ ID N° 2.

10. ADNc suivant les revendications 6 à 9, comprenant la séquence de nucléotides représentée dans la SEQ ID N° 2.

11. Vecteur d'expression d'ADN, qui comprend un ADNc suivant l'une quelconque des revendications 1 à 10.

12. Cellule hôte transformée ou transfectée avec le vecteur de la revendication 11.

13. Cellule hôte suivant la revendication 12, qui est une cellule bactérienne, une cellule animale, une cellule végétale ou une cellule d'insecte.

14. Cellule transgénique comprenant un transgène capable d'exprimer une protéine UNC-53 de C. elegans ; ladite protéine UCN-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3 ; ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4.

15. Cellule transgénique suivant la revendication 14, cellule qui est une cellule de C. elegans, une cellule de neuroblastome N4 ou une cellule de carcinome du sein MCF-7.

16. Organisme non humain transgénique comprenant un transgène capable d'exprimer la protéine UNC-53 de C. elegans ; ladite protéine UCN-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4, et une protéine comprenant la séquence de la position 421 à la position 1583 de la SEQ ID n° 4.

17. Organisme transgénique suivant la revendication 16, ledit organisme étant C. elegans.

18. Organisme transgénique suivant la revendication 16, ledit organisme étant un insecte, un mammifère non humain ou une plante.

19. Protéine UCN-53 codée par l'ADNc de la revendication 1, protéine qui a la séquence d'aminoacides représentée dans la SEQ ID N°3 de la position d'aminoacide 135 à la position d'aminoacide 1528.

20. Protéine UCN-53 codée par la séquence d'ADNc de l'une quelconque des revendications 2 à 5, protéine qui a la séquence d'aminoacides représentée dans la SEQ ID N° 3.

21. Protéine UCN-53 codée par la séquence d'ADNc de la revendication 6, protéine qui a la séquence d'aminoacides représentée dans la SEQ ID N° 4 de la position d'aminoacide 135 à la position d'aminoacide 1583.

22. Protéine UCN-53 codée par la séquence d'ADNc de l'une quelconque des revendications 7 à 10, protéine qui a la séquence d'aminoacides représentée dans la SEQ ID N° 4.

23. Protéine UNC-53 de C. elegans, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4, destinée à être utilisée comme médicament pour favoriser la régénérescence neuronale, la revascularisation ou la cicatrisation d'une plaie, ou destinée au traitement de maladies neurodégénératives chroniques ou de lésions traumatiques aiguës.

24. Protéine UNC-53 suivant l'une quelconque des revendications 19 à 22, destinée à être utilisée comme médicament pour favoriser la régénérescence neuronale, la revascularisation ou la cicatrisation d'une plaie, ou destinée au traitement de maladies neurodégénératives chroniques ou de lésions traumatiques aiguës.

25. Utilisation d'une protéine UNC-53 de C. elegans, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4, dans la production d'un médicament destiné à favoriser la régénérescence neuronale, la revascularisation ou la cicatrisation d'une plaie, ou destiné au traitement de maladies neurodégénératives chroniques ou de lésions traumatiques aiguës.

26. Utilisation d'une protéine UNC-53 suivant l'une quelconque des revendications 19 à 22 dans la production d'un médicament destiné à favoriser la régénérescence neuronale, la revascularisation ou la cicatrisation d'une plaie, ou destiné au traitement de lésions neurodégénératives chroniques ou de lésions traumatiques aiguës.

27. Composition pharmaceutique comprenant une protéine UNC-53 de C. elegans, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4, et un support, diluant ou excipient acceptable à cette fin.

28. Composition pharmaceutique suivant la revendication 27, qui comprend une protéine UNC-53 suivant l'une quelconque des revendications 19 à 22.

29. Séquence d'acide nucléique codant pour une protéine UNC-53 de C. elegans, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4, destinée à être utilisée comme médicament pour favoriser la régénérescence neuronale, la vascularisation ou la cicatrisation d'une plaie, ou destinée au traitement de maladies neurodégénératives chroniques ou de lésions traumatiques aiguës.

30. Séquence d'acide nucléique destinée à être utilisée suivant la revendication 29, ladite séquence étant une séquence d'ADNc suivant l'une quelconque des revendications 1 à 10.

31. Utilisation d'une séquence d'acide nucléique codant pour une protéine UNC-53 de C. elegans, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4, dans la production d'un médicament destiné à favoriser la régénérescence neuronale, la vascularisation ou la cicatrisation d'une plaie, ou destiné au traitement de maladies neurodégénératives chroniques ou de lésions traumatiques aiguës.

32. Utilisation d'une séquence d'acide nucléique suivant la revendication 31, dans laquelle ladite séquence est une séquence d'ADNc suivant l'une quelconque des revendications 1 à 10.

33. Composition pharmaceutique comprenant un acide nucléique codant pour une protéine UNC-53 de C. elegans, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4, et un support, diluant ou excipient acceptable à cette fin.

34. Composition pharmaceutique suivant la revendication 33, dans laquelle ladite séquence d'acide nucléique est une séquence d'ADNc suivant l'une quelconque des revendications 1 à 10.

35. Méthode pour déterminer si un composé est un inhibiteur ou un activateur de la régulation de la forme ou de la motilité cellulaire ou de la direction de migration cellulaire, méthode qui comprend la mise en contact dudit composé avec une cellule transgénique suivant les revendications 14 ou 15 et la sélection d'une variation phénotypique dans ladite cellule.

36. Procédé suivant la revendication 35, dans lequel ladite variation phénotypique à sélectionner est une variation de la forme des cellules ou une variation de la motilité cellulaire.

37. Procédé suivant la revendication 35, dans lequel ladite variation phénotypique à sélectionner est une variation de l'extension des filipodes, du comportement de plissement, de l'adhésion cellulaire ou de la longueur de croissance des neurites.

38. Procédé suivant l'une quelconque des revendications 35 à 37, dans lequel ladite cellule transgénique est une cellule de neuroblastome N4 et la variation phénotypique à sélectionner est la longueur de croissance des neurites.

39. Procédé suivant l'une quelconque des revendications 35 à 37, dans lequel ladite cellule transgénique est une cellule du carcinome du sein MCF-7 et la variation phénotypique à sélectionner est l'étendue de la phagokinèse.

40. Méthode pour déterminer si un composé est un inhibiteur ou un activateur de la régulation de la forme ou de la motilité de cellules ou de la direction de migration cellulaire, méthode qui comprend l'administration du composé à un organisme transgénique suivant l'une quelconque des revendications 16 à 19, et la sélection d'une variation phénotypique chez ledit organisme.

41. Procédé suivant la revendication 40, dans lequel ledit composé est un inhibiteur ou activateur d'une protéine de la voie de transduction de signal desdits organismes transgéniques, ladite protéine étant une protéine UNC-53, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4, ou bien ledit composé est un inhibiteur ou un activateur d'une voie de transduction de signal parallèle ou redondante dans ladite cellule.

42. Kit pour déterminer si un composé est un activateur ou un inhibiteur de l'activité de la protéine UNC-53, kit qui comprend au moins un organisme transgénique suivant l'une quelconque des revendications 16 à 18 et un organisme de C. elegans de type sauvage.

43. Procédé pour la production d'une protéine UNC-53 de C. elegans, ladite protéine UNC-53 étant choisie dans le groupe consistant en une protéine comprenant la séquence représentée dans la SEQ ID N° 3, une protéine comprenant la séquence de la position 135 à la position 1528 de la SEQ ID n° 3, une protéine comprenant la séquence de la position 165 à la position 1528 de la SEQ ID n° 3, une protéine comprenant la séquence de la position 309 à la position 1528 de la SEQ ID n° 3, une protéine comprenant la séquence de la position 366 à la position 1528 de la SEQ ID n° 3 et une protéine comprenant la séquence de la position 421 à la position 1528 de la SEQ ID n° 3, ou une protéine comprenant la séquence représentée dans la SEQ ID N° 4, une protéine comprenant la séquence de la position 135 à la position 1583 de la SEQ ID n° 4, une protéine comprenant la séquence de la position 165 à la position 1583 de la SEQ ID n° 4, une protéine comprenant la séquence de la position 309 à la position 1583 de la SEQ ID n° 4, une protéine comprenant la séquence de la position 366 à la position 1583 de la SEQ ID n° 4 et une protéine comprenant la séquence de la position 421 à la position 1583 de la SEQ ID n° 4, procédé qui comprend la culture des cellules transfectées ou transformées de la revendication 12 ou la revendication 13 et la récupération de la protéine UNC-53 exprimée.

44. Lignée de cellules d'hybridome déposé sous le numéro de dépôt LMBP 1383CB.

45. Anticorps monoclonal 16-48-2 pouvant être obtenu à partir de l'hybridome déposé sous le numéro de dépôt LMBP 1383CB.

46. Plasmide pTB54 déposé sous le numéro de dépôt 3296.

47. Plasmide pTB112 déposé sous le numéro de dépôt LMBP 3295.

48. Plasmide pTB72 déposé sous le numéro de dépôt LMBP 3486.

49. Lignée de cellules transgéniques de C. elegans, appelée TB4EX25 et déposée sous le numéro de dépôt LMBP 1384CB.

50. Lignée de cellules transgéniques de C. elegans, appelée TBAIn76 et déposée sous le numéro de dépôt LMBP 1385CB.

51. Lignée de cellules transgéniques de cellules du carcinome du sein MCF-7 déposée sous le numéro de dépôt LMBP 1550CB.

52. Lignée de cellules transgéniques de cellules de neuroblastome N4 déposée sous le numéro de dépôt LMBP 1549CB.
